# EUROPEAN PATENT SPECIFICATION

(11) **EP 2 487 240 B1**
(45) Date of publication and mention of the grant of the patent: **16.11.2016**
(21) Application number: 12159733.0
(22) Date of filing: 19.09.2007
(51) Int. Cl.: C12N 15/11

(54) **Micrornas differentially expressed in pancreatic diseases and uses thereof**
Mikro-RNA, die differentiell in Pankreaserkrankungen exprimiert sind, und ihre Verwendung
Micro ARN différemment exprimés dans des maladies pancréatiques et leurs utilisations

(30) Priority: 19.09.2006 US 826173 P
(43) Date of publication of application: 15.08.2012
(62) Divisional of application: 07842816.6
(73) Proprietor: Interpace Diagnostics, LLC, Parsippany, NJ 07054 (US)
(72) Inventor: Labourier, Emmanuel, Austin, TX Texas 78744 (US); Szafranska, Anna, E., Austin, TX Texas 78744 (US); Davison, Tim, Austin, TX Texas 78744 (US); John, Jeremy, Austin, TX Texas 78744 (US)
(74) Representative: Sonn & Partner Patentanwälte

(56) References cited:
- US-A1- 2006 185 027
- HOUBAVIY HRISTO B ET AL: "Embryonic stem cell-specific microRNAs", DEVELOPMENTAL CELL, CELL PRESS, US, vol. 5, no. 2, 1 August 2003 (2003-08-01), pages 351-358, XP002540101, ISSN: 1534-5807, DOI: 10.1016/S1534-5807(03)00227-2
- VOLINIA STEFANO ET AL: "A microRNA expression signature of human solid tumors defines cancer gene targets", PROCEEDINGS OF THE NATIONAL ACADEMY OF SCIENCES, NATIONAL ACADEMY OF SCIENCES, vol. 103, no. 7, 14 February 2006 (2006-02-14), pages 2257-2261, XP002608380, ISSN: 0027-8424, DOI: 10.1073/PNAS.0510565103 [retrieved on 2006-02-03]
- SZAFRANSKA A E ET AL: "MicroRNA expression alterations are linked to tumorigenesis and non-neoplastic processes in pancreatic ductal adenocarcinoma.", ONCOGENE 28 JUN 2007 LNKD- PUBMED:17237814, vol. 26, no. 30, 28 June 2007 (2007-06-28) , pages 4442-4452, XP002675917, ISSN: 0950-9232
- KUTAY HUBAN ET AL: "Downregulation of miR-122 in the rodent and human hepatocellular carcinomas", JOURNAL OF CELLULAR BIOCHEMISTRY, WILEY-LISS INC, US, vol. 99, no. 3, 15 October 2006 (2006-10-15), pages 671-678, XP002554082, ISSN: 0730-2312, DOI: 10.1002/JCB.20982 [retrieved on 2006-06-30]
- BURMISTROVA O A ET AL: "MicroRNA in schizophrenia: Genetic and expression analysis of miR-130b (22q 11)", BIOCHEMISTRY (MOSCOW), vol. 72, no. 5, May 2007 (2007-05), pages 578-582, XP002675918, ISSN: 0006-2979, DOI: DOI:10.1134/S0006297907050161

## Description

### BACKGROUND OF THE INVENTION

### .I FIELD OF THE INVENTION

The present invention relates generally to the field of molecular biology. More particularly, it concerns methods and compositions involving microRNA (miRNAs) molecules. Certain aspects of the invention include applications for miRNAs in diagnostics, therapeutics, and prognostics of pancreatic cancer.

### .II BACKGROUND

In 2001, several groups used a cloning method to isolate and identify a large group of "microRNAs" (miRNAs) from C. *elegans, Drosophila,* and human s (Lagos-Quintana *et al.,* 2001; Lau *et al.,* 2001; Lee and Ambros, 2001). Several hundreds of miRNAs have been identified in plants and animals - including humans - which do not appear to have endogenous siRNAs. Thus, while similar to siRNAs, miRNAs are nonetheless distinct.

miRNAs thus far observed have been approximately 21-22 nucleotides in length and they arise from longer precursors, which are transcribed from non-protein-encoding genes. See review of Carrington *et al.* (2003). The precursors form structures that fold back on themselves in self-complementary regions; they are then processed by the nuclease Dicer in animals or DCL1 in plants. miRNA molecules interrupt translation through precise or imprecise base-pairing with their targets.

Many miRNAs are conserved among diverse organisms, and this has led to the suggestion that miRNAs are involved in essential biological processes throughout the life span of an organism (Esquela-Kerscher and Slack, 2006). In particular, miRNAs have been implicated in regulating cell growth and cell and tissue differentiation; cellular processes that are associated with the development of cancer. For instance, lin-4 and let-7 both regulate passage from one larval state to another during C. *elegans* development (Ambros, 2001). miR-14 and bantam are *Drosophila* miRNAs that regulate cell death, apparently by regulating the expression of genes involved in apoptosis (Brennecke *et al.,* 2003, Xu *et al.,* 2003).

Research on miRNAs is increasing as scientists are beginning to appreciate the broad role that these molecules play in the regulation of eukaryotic gene expression. In particular, several recent studies have shown that expression levels of numerous miRNAs are associated with various cancers (reviewed in Esquela-Kerscher and Slack, 2006). Reduced expression of two miRNAs correlates strongly with chronic lymphocytic leukemia in human s, providing a possible link between miRNAs and cancer (Calin *et al.,* 2002). Others have evaluated the expression patterns of large numbers of miRNAs in multiple human cancers and observed differential expression of almost all miRNAs across numerous cancer types (Lu *et al.,* 2005). Most such studies link miRNAs to cancer only by indirect evidence. In contrast, a single study has provided more direct evidence that miRNAs may contribute directly to causing cancer. By forcing the over-expression of six miRNAs in mice, He *et al.* (2005) demonstrated a significant increase in B cell lymphomas.

Pancreatic cancer is a particularly challenging disease to diagnose and treat. Each year about 33,000 people in the United States are diagnosed with adenocarcinoma of the pancreas, and about 32,000 people die each year from pancreatic cancer (Jemal *et al.,* 2006). Pancreatic carcinoma ranks as the fourth leading cause of cancer deaths in the United States, and the five year survival rate (∼4%) is the lowest among all cancers (Jemal *et al.,* 2006).

Currently, effective diagnostic methods and/or treatments for pancreatic cancer are lacking (Monti *et al.,* 2004). Combinations of chemotherapy and radiation therapy may extend patient survival; but, only the surgical removal of part or all of the pancreas offers a potential cure for pancreatic cancer. Additional diagnostic methods and therapeutic interventions are needed to address this normally incurable disease.

Distinguishing between chronic pancreatitis and pancreatic cancer can be extremely difficult. Symptoms are frequently non-specific and limited to jaundice, weight loss and bruising. Many patients with chronic pancreatitis (non-cancerous condition) exhibit the same symptoms as patients with pancreatic cancer, which are mostly adenocarcinomas of the ductal epithelium (Freelove and Walling, 2006) - or pancreatic ductal adenocarcinomas (PDAC). Serum levels of certain proteins may be suggestive of pancreatic adenocarcinoma but are not diagnostic; and the serum tumor marker CA19-9 can help confirm pancreatic cancer diagnosis, but is ineffective as a patient screening tool (Freelove and Walling, 2006). A need exists for additional diagnostic assays that can assess the condition of the pancreas in general and distinguish chronic pancreatitis from pancreatic adenocarcinoma in particular.

### SUMMARY OF THE INVENTION

The present invention overcomes these problems in the art by identifying miRNAs that are differentially expressed or mis-regulated in various states of diseased, normal, cancerous, and/or abnormal tissues, including but not limited to normal pancreas, non-cancerous diseased pancreas, and pancreatic cancer (e.g., pancreatic ductal adenocarcinomas (PDAC)), as limited by the claims. Further, the invention describes a method for diagnosing diseased, normal, cancerous, and/or abnormal tissues, including but not limited to pancreatic cancer and chronic pancreatitis that is based on determining levels (increased or decreased) of selected miRNAs in patient-derived samples, as limited by the claims. The disclosure also describes genes that the inventors contemplate are influenced by the expression or lack of expression (mis-regulation) of miRNAs in biological samples. Samples obtained and/or analyzed from patients, including but not limited to patient having or suspected of having PDAC or chronic pancreatitis, or patient suspected of having one or the other condition. These genes and their regulatory pathways represent targets for therapeutic intervention by regulating their expression with miRNAs.

The term "miRNA" is used according to its ordinary and plain meaning and refers to a microRNA molecule found in eukaryotes that is involved in RNA-based gene regulation. See, *e.g., Carrington et al.,* 2003. The term will be used to refer to the single-stranded RNA molecule processed from a precursor. Individual miRNAs have been identified and sequenced in different organisms, and they have been given names. Names of miRNAs and their sequences related to the present invention are provided herein.

It is understood that a "synthetic nucleic acid" of the invention means that the nucleic acid does not have a chemical structure or sequence of a naturally occuring nucleic acid. Consequently, it will be understood that the term "synthetic miRNA" refers to a "synthetic nucleic acid" that functions in a cell or under physiological conditions as a naturally occurring miRNA.

While many of the embodiments of the invention involve synthetic miRNAs or synthetic nucleic acids, in some embodiments of the invention, the nucleic acid molecule(s) need not be "synthetic." In certain embodiments, a non-synthetic miRNA employed in methods and compositions of the invention may have the entire sequence and structure of a naturally occurring miRNA precursor or the mature miRNA. For example, non-synthetic miRNAs used in methods and compositions of the invention may not have one or more modified nucleotides or nucleotide analogs. In these embodiments, the non-synthetic miRNA may or may not be recombinantly produced. In particular embodiments, the nucleic acid in methods and/or compositions of the invention is specifically a synthetic miRNA and not a non-synthetic miRNA (that is, not a miRNA that qualifies as "synthetic"); though in other embodiments, the invention specifically involves a non-synthetic miRNA and not a synthetic miRNA. Any embodiments discussed with respect to the use of synthetic miRNAs can be applied with respect to non-synthetic miRNAs, and vice versa.

It will be understood that the term "naturally occurring" refers to something found in an organism without any intervention by a person; it could refer to a naturally-occurring wildtype or mutant molecule. In some embodiments a synthetic miRNA molecule does not have the sequence of a naturally occurring miRNA molecule. In other embodiments, a synthetic miRNA molecule may have the sequence of a naturally occurring miRNA molecule, but the chemical structure of the molecule, particularly in the part unrelated specifically to the precise sequence (non-sequence chemical structure) differs from chemical structure of the naturally occurring miRNA molecule with that sequence. In some cases, the synthetic miRNA has both a sequence and non-sequence chemical structure that are not found in a naturally-occurring miRNA. Moreover, the sequence of the synthetic molecules will identify which miRNA is effectively being provided or inhibited; the endogenous miRNA will be referred to as the "corresponding miRNA." Corresponding miRNA sequences that can be used in the context of the invention include, but are not limited to, all or a portion of those sequences in SEQ ID NOs: 1-350, as well as any other miRNA sequence, miRNA precursor sequence, or any sequence complementary thereof; as limited by the claims. In some embodiments, the sequence is or is derived from or contains all or part of a sequence identified in Table 1 below to target a particular miRNA (or set of miRNAs) that can be used with that sequence, as limited by the claims.

In some embodiments, it may be useful to know whether a cell expresses a particular miRNA endogenously or whether such expression is affected under particular conditions or when it is in a particular disease state. Thus, in some embodiments of the invention, methods include assaying a cell or a sample containing a cell for the presence of one or more miRNA. Consequently, in some embodiments, methods include a step of generating a miRNA profile for a sample. The term "miRNA profile" refers to a set of data regarding the expression pattern for a plurality of miRNAs (e.g., one or more miRNA from Table 1) in the sample; it is contemplated that the miRNA profile can be obtained using a set of miRNAs, using for example nucleic acid amplification or hybridization techniques well know to one of ordinary skill in the art.

In some embodiments of the invention, a miRNA profile is generated by steps that include: (a) labeling miRNA in the sample; b) hybridizing miRNA to a number of probes, or amplifying a number of miRNA, and c) determining miRNA hybridization to the probes or detection miRNA amplification products, wherein a miRNA profile is generated. See U.S. Provisional Patent Application 60/575,743 and the U.S. Provisional Patent Application 60/649,584, and U.S. Patent Application Serial No. 11/141,707.

Methods of the invention involve diagnosing a patient based on a miRNA expression profile, as limited by the claims. In certain embodiments, the elevation or reduction in the level of expression of a particular miRNA or set of miRNA in a cell is correlated with a disease state compared to the expression level of that miRNA or set of miRNA in a normal cell. This correlation allows for diagnostic methods to be carried out when that the expression level of a miRNA is measured in a biological sample being assessed and then compared to the expression level of a normal cell. It is specifically contemplated that miRNA profiles for patients, particularly those suspected of having a particular disease or condition such as pancreatits or pancreatic cancer, can be generated by evaluating any of or sets of the miRNAs discussed in this application. The miRNA profile that is generated from the patient will be one that provides information regarding the particular disease or condition. In many embodiments, the miRNA profile is generated using miRNA hybridization or amplification, (*e.g*., array hybridization or RT-PCR). In certain aspects, a miRNA profile can be used in conjunction with other diagnostic tests, such protein profiles in the serum, *e.g*., CA19-9 detection.

Embodiments of the invention include methods for diagnosing and/or assessing a condition in a patient comprising measuring an expression profile of one or more miRNAs in a sample from the patient, as limited by the claims. The difference in the expression profile in the sample from the patient and a reference expression profile, such as an expression profile from a normal or non-pathologic sample, is indicative of a pathologic, disease, or cancerous condition. A miRNA or probe set comprising or identifying a segment of a corresponding miRNA can include all or part of 1, 2, 3, 4, 5,6, 7, 8, 9, 10, 11, 12,13, 14, 15, 16, 17, 18, 19, 20, 21, 22, 23, 24, 25, 26, 27, 28, 29, 30, 35, 40, 45, 50, 55, 60, 65, 70, 75, 80, 85, 90, 95, 100, 125, 150, 175, 200, 250, 300, 350, or any integer or range derivable there between, of a miRNA or a probe listed in Table 1 below.

In certain aspects, the methods for diagnosing a condition in a patient comprise measuring an expression profile of one or more miRNAs in a sample from the patient, wherein a difference in the expression profile in the sample from the patient and an expression profile of a normal sample is indicative of a pathological condition; wherein the miRNA is one or more of hsa-let-7a, hsa-let-7b, hsa-let-7c, hsa-let-7d, hsa-let-7e, hsa-let-7f, hsa-let-7g, hsa-let-7i, hsa-miR-100, hsa-miR-101, hsa-miR-103, hsa-miR-106a, hsa-miR-106b, hsa-miR-107, hsa-iR-10a, hsa-miR-125a, hsa-miR-125b, hsa-miR-130a, hsa-miR-130b, hsa-miR-134, hsa-miR-140, hsa-miR-141, hsa-miR-143, hsa-miR-145, hsa-miR-146a, hsa-miR-148a, hsa-miR-148b, hsa-miR-150, hsa-miR-154, hsa-miR-155, hsa-miR-15b, hsa-miR-17-5p, hsa-miR-18a, hsa-miR-181b, hsa-miR-182, hsa-miR-186, hsa-miR-196a, hsa-miR-196b, hsa-miR-199a, hsa-miR-199a-AS, hsa-miR-19a, hsa-miR-19b, hsa-miR-200a, hsa-miR-200b, hsa-miR-200c, hsa-miR-203, hsa-miR-21, hsa-miR-210, hsa-miR-214, hsa-miR-216, hsa-miR-217, hsa-miR-221, hsa-miR-222, hsa-miR-223, hsa-miR-224, hsa-miR-23a, hsa-miR-24, hsa-miR-25, hsa-miR-26a, hsa-miR-26b, hsa-miR-27a, hsa-miR-27b, hsa-miR-28, hsa-miR-29a, hsa-miR-29b, hsa-miR-29c, hsa-miR-30a-3p, hsa-miR-30a-5p, hsa-miR-30b, hsa-miR-30c, hsa-miR-30d, hsa-miR-30e-3p, hsa-miR-30e-5p, hsa-miR-31, hsa-miR-331, hsa-miR-335, hsa-miR-365, hsa-miR-368, hsa-miR-374, hsa-miR-375, hsa-miR-376a, hsa-miR-377, hsa-miR-379, hsa-miR-429, hsa-miR-93, hsa-miR-95, hsa-miR-96, hsa-miR-98, hsa-miR-99a, hsa-miR-99b, hsa-miR-452, hsa-miR-494, hsa-miR-497, miR-205, or ambi-miR-7105, as limited by the claims.

In a further aspect, the miRNA is one or more of miR-205, miR-29c, miR-216, miR-217, miR-375, miR-143, miR-145, miR-146a, miR-148a, miR-196b, miR-93, miR-96, miR-31, miR-210, miR-148b, miR-196a, miR-141, miR-18a, miR-203, miR-150, miR-155, miR-130b, miR-221, miR-222, miR-223, or miR-224, as limited by the claims.

In still further aspects of the invention the miRNA is miR-196a, miR-217, or both miR-196a and miR-217, as limited by the claims.

Embodiments of the invention include methods wherein differential expression of one or more miRNA is indicative of pancreatitis, wherein the miRNA is one or more of hsa-let-7a, hsa-let-7b, hsa-let-7c, hsa-let-7d, hsa-let-7e, hsa-let-7f, hsa-let-7g, hsa-let-7i, hsa-miR-100, hsa-miR-101, hsa-miR-10a, hsa-miR-125a, hsa-miR-125b, hsa-miR-130a, hsa-miR-130b, hsa-miR-141, hsa-miR-143, hsa-miR-145, hsa-miR-146a, hsa-miR-148a, hsa-miR-148b, hsa-miR-150, hsa-miR-18a, hsa-miR-182, hsa-miR-186, hsa-miR-199a hsa-miR-199a-AS, hsa-miR-19a, hsa-miR-19b, hsa-miR-200a, hsa-miR-200b, hsa-miR-200c, hsa-miR-203, hsa-miR-210, hsa-miR-214, hsa-miR-216, hsa-miR-217, hsa-miR-222, hsa-miR-223, hsa-miR-24, hsa-miR-25, hsa-miR-26a, hsa-miR-26b, hsa-miR-27b, hsa-miR-28, hsa-miR-29a, hsa-miR-29b, hsa-miR-29c, hsa-miR-30a-3p, hsa-miR-30a-5p, hsa-miR-30b, hsa-miR-30c, hsa-miR-30d, hsa-miR-30e-3p, hsa-miR-30e-5p, hsa-miR-31, hsa-miR-335, hsa-miR-365, hsa-miR-368, hsa-miR-374, hsa-miR-376a, hsa-miR-429, hsa-miR-95, hsa-miR-96, hsa-miR-98, hsa-miR-99a, hsa-miR-497, or ambi-miR-7105, as limited by the claims.

In certain aspects, an increase in expression of one or more miRNA in a patient sample is indicative of pancreatitis, wherein the miRNA is one or more of hsa-let-7i, hsa-miR-100, hsa-iR-10a, hsa-miR-125a, hsa-miR-125b, hsa-miR-143, hsa-miR-145, hsa-miR-146a, hsa-miR-150, hsa-miR-18a, hsa-miR-199a, hsa-miR-199a-AS, hsa-miR-210, hsa-miR-214, hsa-miR-222, hsa-miR-223, hsa-miR-24, hsa-miR-31, hsa-miR-99a, or hsa-miR-497, as limited by the claims.

In further aspects, a decrease in expression of one or more miRNA in a patient sample is indicative of pancreatitis, wherein the miRNA is one or more of hsa-let-7a, hsa-let-7b, hsa-let-7c, hsa-let-7d, hsa-let-7e, hsa-let-7f, hsa-let-7g, hsa-miR-101, hsa-miR-130a, hsa-miR-130b, hsa-miR-141, hsa-miR-148a, hsa-miR-148b, hsa-miR-182, hsa-miR-186, hsa-miR-19a, hsa-miR-19b, hsa-miR-200a, hsa-miR-200b, hsa-miR-200c, hsa-miR-203, hsa-miR-216, hsa-miR-217, hsa-miR-25, hsa-miR-26a, hsa-miR-26b, hsa-miR-27b, hsa-miR-28, hsa-miR-29a, hsa-miR-29b, hsa-miR-29c, hsa-miR-30a-3p, hsa-miR-30a-5p, hsa-miR-30b, hsa-miR-30c, hsa-miR-30d, hsa-miR-30e-3p, hsa-miR-30e-5p, hsa-miR-335, hsa-miR-365, hsa-miR-368, hsa-miR-374, hsa-miR-376a, hsa-miR-429, hsa-miR-95, hsa-miR-96, hsa-miR-98, or ambi-miR-7105, as limited by the claims.

In still further aspects, differential expression of one or more miRNA is indicative of PDAC, wherein the miRNA is one or more of hsa-let-7a, hsa-let-7b, hsa-let-7c, hsa-let-7d, hsa-let-7f, hsa-let-7g, hsa-let-7i, hsa-miR-100, hsa-miR-101, hsa-miR-103, hsa-miR-106b, hsa-miR-107, hsa-iR-10a, hsa-miR-125a, hsa-miR-125b, hsa-miR-130a, hsa-miR-130b, hsa-miR-134, hsa-miR-140, hsa-miR-141, hsa-miR-143, hsa-miR-145, hsa-miR-146a, hsa-miR-148a, hsa-miR-148b, hsa-miR-150, hsa-miR-154, hsa-miR-155, hsa-miR-18a, hsa-miR-181b, hsa-miR-182, hsa-miR-186, hsa-miR-196a, hsa-miR-196b, hsa-miR-199a, hsa-miR-199a-AS, hsa-miR-19a, hsa-miR-19b, hsa-miR-200a, hsa-miR-200b, hsa-miR-200c, hsa-miR-203, hsa-miR-21, hsa-miR-210, hsa-miR-214, hsa-miR-216, hsa-miR-217, hsa-miR-221, hsa-miR-222, hsa-miR-223, hsa-miR-224, hsa-miR-23a, hsa-miR-24, hsa-miR-26a, hsa-miR-26b, hsa-miR-27a, hsa-miR-27b, hsa-miR-29c, hsa-miR-30a-3p, hsa-miR-30a-5p, hsa-miR-30b, hsa-miR-30c, hsa-miR-30d, hsa-miR-30e-3p, hsa-miR-30e-5p, hsa-miR-31, hsa-miR-331, hsa-miR-335, hsa-miR-365, hsa-miR-368, hsa-miR-374, hsa-miR-375, hsa-miR-376a, hsa-miR-377, hsa-miR-379, hsa-miR-429, hsa-miR-93, hsa-miR-95, hsa-miR-96, hsa-miR-99b, hsa-miR-452, hsa-miR-494, hsa-miR-497, miR-205, or ambi-miR-7105, as limited by the claims.

In certain aspects, an increase in expression of one or more miRNA is indicative of PDAC, wherein the miRNA is one or more of hsa-let-7i, hsa-miR-100, hsa-miR-103, hsa-miR-106b, hsa-miR-107, hsa-miR-10a, hsa-miR-125a, hsa-miR-125b, hsa-miR-140, hsa-miR-143, hsa-miR-145, hsa-miR-146a, hsa-miR-150, hsa-miR-155, hsa-miR-18a, hsa-miR-181b, hsa-miR-196a, hsa-miR-196b, hsa-miR-199a, hsa-miR-199a-AS, hsa-miR-203, hsa-miR-21, hsa-miR-210, hsa-miR-214, hsa-miR-221, hsa-miR-222, hsa-miR-223, hsa-miR-224, hsa-miR-23a, hsa-miR-24, hsa-miR-27a, hsa-miR-31, hsa-miR-331, hsa-miR-93, hsa-miR-99b, hsa-miR-452, hsa-miR-497, or ambi-miR-7105, as limited by the claims.

In a further aspect, a decrease in expression of one or more miRNA is indicative of PDAC, wherein the miRNA is one or more of hsa-let-7a, hsa-let-7b, hsa-let-7c, hsa-let-7d, hsa-let-7e, hsa-let-7f, hsa-let-7g, hsa-miR-101, hsa-miR-130a, hsa-miR-130b, hsa-miR-134, hsa-miR-141, hsa-miR-148a, hsa-miR-148b, hsa-miR-154, hsa-miR-182, hsa-miR-186, hsa-miR-19a, hsa-miR-19b, hsa-miR-200a, hsa-miR-200b, hsa-miR-200c, hsa-miR-216, hsa-miR-217, hsa-miR-26a, hsa-miR-26b, hsa-miR-27b, hsa-miR-29c, hsa-miR-30a-3p, hsa-miR-30a-5p, hsa-miR-30b, hsa-miR-30c, hsa-miR-30d, hsa-miR-30e-3p, hsa-miR-30e-5p, hsa-miR-335, hsa-miR-365, hsa-miR-368, hsa-miR-374, hsa-miR-375, hsa-miR-376a, hsa-miR-377, hsa-miR-379, hsa-miR-429, hsa-miR-95, hsa-miR-96, or hsa-miR-494, as limited by the claims.

In still another aspect, pancreatitis is distinguished from PDAC by differential expression of one or more of hsa-let-7b, hsa-let-7e, hsa-miR-103, hsa-miR-106a, hsa-miR-106b, hsa-miR-107, hsa-miR-125b, hsa-miR-130a, hsa-miR-130b, hsa-miR-141, hsa-miR-146a, hsa-miR-148a, hsa-miR-154, hsa-miR-155, hsa-miR-15b, hsa-miR-17-5p, hsa-miR-18a, hsa-miR-181b, hsa-miR-196a, hsa-miR-196b, hsa-miR-199a-AS, hsa-miR-203, hsa-miR-21, hsa-miR-210, hsa-miR-216, hsa-miR-217, hsa-miR-221, hsa-miR-222, hsa-miR-224, hsa-miR-23a, hsa-miR-24, hsa-miR-25, hsa-miR-27a, hsa-miR-28, hsa-miR-29a, hsa-miR-29c, hsa-miR-30a-3p, hsa-miR-30a-5p, hsa-miR-30b, hsa-miR-30c, hsa-miR-30d, hsa-miR-30e-5p, hsa-miR-331, hsa-miR-368, hsa-miR-375, hsa-miR-377, hsa-miR-379, hsa-miR-93, hsa-miR-452, hsa-miR-494, hsa-miR-497, or ambi-miR-7105, as limited by the claims.

A sample may be taken from a patient having or suspected of having a disease or pathological condition. In certain aspects, the sample can be, but is not limited to tissue (*e.g*., biopsy, particularly fine needle biopsy), blood, serum, plasma, or a pancreatic juice samples. The sample can be fresh, frozen, fixed (*e.g*., formalin fixed), or embedded (*e.g*., paraffin embedded). In a particular aspect, the sample can be a pancreatic sample.

Methods of the invention can be used to diagnose or assess a pathological condition, as limited by the claims. In certain aspect, the condition is pancreatitis or chronic pancreatitis. In other aspects the condition is pancreatic ductal adenocarcinoma (PDAC).

The methods can further comprise one or more of steps including: (a) obtaining a sample from the patient, (b) isolating nucleic acids from the sample, (c) labeling the nucleic acids isolated from the sample, and (d) hybridizing the labeled nucleic acids to one or more probes. Nucleic acids of the invention include one or more nucleic acid comprising at least one segment having a sequence or complementary sequence of one or more of the miRNA sequences in Table 1, as limited by the claims. In certain aspects, the nucleic acids identify one or more miRNAs listed in Table 1, as limited by the claims. Nucleic acids of the invention are typically coupled to a support. Such supports are well known to those of ordinary skill in the art and include, but are not limited to glass, plastic, metal, or latex. In particular aspects of the invention, the support can be planar or in the form of a bead or other geometric shapes or configurations.

Certain embodiments of the invention include determining expression of one or more miRNA by using an amplification assay or a hybridization assay, a variety of which are well known to one of ordinary skill in the art. In certain aspects, an amplification assay can be a quantitative amplification assay, such as quantitative RT-PCR or the like. In still further aspects, a hybridization assay can include array hybridization assays or solution hybridization assays.

Aspects of the invention can be used to diagnose or assess a patient's condition, as limited by the claims. For example, the methods can be used to screen for a pathological condition, assess prognosis of a pathological condition, stage a pathological condition, or assess response of a pathological condition to therapy.

Embodiments of the invention concern nucleic acids for use that perform the activities of or inhibit endogenous miRNAs when introduced into cells, as limited by the claims. In certain aspects, nucleic acids are synthetic or non-synthetic miRNA. Sequence-specific miRNA inhibitors can be used to inhibit sequentially or in combination the activities of one or more endogenous miRNAs in cells, as well those genes and associated pathways modulated by the endogenous miRNA.

The present invention concerns, in some embodiments, short nucleic acid molecules for use that function as miRNAs or as inhibitors of miRNA in a cell, as limited by the claims. The term "short" refers to a length of a single polynucleotide that is 25, 50, 100, or 150 nucleotides or fewer, including all integers or range derivable there between.

The nucleic acid molecules are typically synthetic. The term "synthetic" means the nucleic acid molecule is isolated and not identical in sequence (the entire sequence) and/or chemical structure to a naturally-occurring nucleic acid molecule, such as an endogenous precursor miRNA or miRNA molecule. While in some embodiments, nucleic acids of the invention do not have an entire sequence that is identical to a sequence of a naturally-occurring nucleic acid, such molecules may encompass all or part of a naturally-occurring sequence. It is contemplated, however, that a synthetic nucleic acid administered to a cell may subsequently be modified or altered in the cell such that its structure or sequence is the same as non-synthetic or naturally occurring nucleic acid, such as a mature miRNA sequence. For example, a synthetic nucleic acid may have a sequence that differs from the sequence of a precursor miRNA, but that sequence may be altered once in a cell to be the same as an endogenous, processed miRNA. The term "isolated" means that the nucleic acid molecules of the invention are initially separated from different (in terms of sequence or structure) and unwanted nucleic acid molecules such that a population of isolated nucleic acids is at least about 90% homogenous, and may be at least about 95, 96, 97, 98, 99, or 100% homogenous with respect to other polynucleotide molecules. In many embodiments of the invention, a nucleic acid is isolated by virtue of it having been *synthesized in vitro* separate from endogenous nucleic acids in a cell. It will be understood, however, that isolated nucleic acids may be subsequently mixed or pooled together.

In certain aspects, synthetic miRNA of the invention are RNA or RNA analogs. miRNA inhibitors may be DNA or RNA, or analogs thereof. miRNA and miRNA inhibitors of the invention are collectively referred to as "synthetic nucleic acids."

In some embodiments, there is a synthetic miRNA having a length of between 17 and 130 residues, as limited by the claims. The present invention concerns synthetic miRNA molecules that are, are at least, or are at most 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 140, 145, 150, 160, 170, 180, 190, 200 or more residues in length, including any integer or any range derivable therein.

In certain embodiments, synthetic miRNA have (a) a "miRNA region" whose sequence from 5' to 3' is identical to all or a segment of a mature miRNA sequence, and (b) a "complementary region" whose sequence from 5' to 3' is between 60% and 100% complementary to the miRNA sequence, as limited by the claims. In certain embodiments, these synthetic miRNA are also isolated, as defined above. The term "miRNA region" refers to a region on the synthetic miRNA that is at least 75, 80, 85, 90, 95, or 100% identical, including all integers there between, to the entire sequence of a mature, naturally occurring miRNA sequence. In certain embodiments, the miRNA region is or is at least 90, 91, 92, 93, 94, 95, 96, 97, 98, 99, 99.1, 99.2, 99.3, 99.4, 99.5, 99.6, 99.7, 99.8, 99.9 or 100% identical to the sequence of a naturally-occurring miRNA.

The term "complementary region" refers to a region of a synthetic miRNA that is or is at least 60% complementary to the mature, naturally occurring miRNA sequence that the miRNA region is identical to. The complementary region is or is at least 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 99.1, 99.2, 99.3, 99.4, 99.5, 99.6, 99.7, 99.8, 99.9 or 100% complementary, or any range derivable therein. With single polynucleotide sequences, there is a hairpin loop structure as a result of chemical bonding between the miRNA region and the complementary region. In other embodiments, the complementary region is on a different nucleic acid molecule than the miRNA region, in which case the complementary region is on the complementary strand and the miRNA region is on the active strand.

In other embodiments of the invention, there are synthetic nucleic acids for use that are miRNA inhibitors, as limited by the claims. A miRNA inhibitor is between about 17 to 25 nucleotides in length and comprises a 5' to 3' sequence that is at least 90% complementary to the 5' to 3' sequence of a mature miRNA. In certain embodiments, a miRNA inhibitor molecule is 17, 18, 19, 20, 21, 22, 23, 24, or 25 nucleotides in length, or any range derivable therein. Moreover, a miRNA inhibitor has a sequence (from 5' to 3') that is or is at least 90, 91, 92, 93, 94, 95, 96, 97, 98, 99, 99.1, 99.2, 99.3, 99.4, 99.5, 99.6, 99.7, 99.8, 99.9 or 100% complementary, or any range derivable therein, to the 5' to 3' sequence of a mature miRNA, particularly a mature, naturally occurring miRNA. Probe sequences for miRNAs are disclosed in Table 1. One of skill in the art could use a portion of the probe sequence that is complementary to the sequence of a mature miRNA as the sequence for a miRNA inhibitor. Table 1 indicates what the mature sequence of a miRNA. Moreover, that portion of the probe sequence can be altered so that it is still 90% complementary to the sequence of a mature miRNA.

In some embodiments, of the invention, a synthetic miRNA contains one or more design elements. These design elements include, but are not limited to: (i) a replacement group for the phosphate or hydroxyl of the nucleotide at the 5' terminus of the complementary region; (ii) one or more sugar modifications in the first or last 1 to 6 residues of the complementary region; or, (iii) noncomplementarity between one or more nucleotides in the last 1 to 5 residues at the 3' end of the complementary region and the corresponding nucleotides of the miRNA region.

In certain embodiments, a synthetic miRNA has a nucleotide at its 5' end of the complementary region in which the phosphate and/or hydroxyl group has been replaced with another chemical group (referred to as the "replacement design"). In some cases, the phosphate group is replaced, while in others, the hydroxyl group has been replaced. In particular embodiments, the replacement group is biotin, an amine group, a lower alkylamine group, an acetyl group, 2'O-Me (2'oxygen-methyl), DMTO (4,4'-dimethoxytrityl with oxygen), fluoroscein, a thiol, or acridine, though other replacement groups are well known to those of skill in the art and can be used as well. This design element can also be used with a miRNA inhibitor.

Additional embodiments concern a synthetic miRNA having one or more sugar modifications in the first or last 1 to 6 residues of the complementary region (referred to as the "sugar replacement design"). In certain cases, there is one or more sugar modifications in the first 1, 2, 3, 4, 5, 6 or more residues of the complementary region, or any range derivable therein. In additional cases, there is one or more sugar modifications in the last 1, 2, 3, 4, 5, 6 or more residues of the complementary region, or any range derivable therein, have a sugar modification. It will be understood that the terms "first" and "last" are with respect to the order of residues from the 5' end to the 3' end of the region. In particular embodiments, the sugar modification is a 2'O-Me modification. In further embodiments, there is one or more sugar modifications in the first or last 2 to 4 residues of the complementary region or the first or last 4 to 6 residues of the complementary region. This design element can also be used with a miRNA inhibitor. Thus, a miRNA inhibitor can have this design element and/or a replacement group on the nucleotide at the 5' terminus, as discussed above.

In other embodiments of the invention, there is a synthetic miRNA in which one or more nucleotides in the last 1 to 5 residues at the 3' end of the complementary region are not complementary to the corresponding nucleotides of the miRNA region ("noncomplementarity") (referred to as the "noncomplementarity design"). The noncomplementarity may be in the last 1, 2, 3, 4, and/or 5 residues of the complementary miRNA. In certain embodiments, there is noncomplementarity with at least 2 nucleotides in the complementary region.

It is contemplated that synthetic miRNA of the invention have one or more of the replacement, sugar modification, or noncomplementarity designs. In certain cases, synthetic RNA molecules have two of them, while in others these molecules have all three designs in place.

The miRNA region and the complementary region may be on the same or separate polynucleotides. In cases in which they are contained on or in the same polynucleotide, the miRNA molecule will be considered a single polynucleotide. In embodiments in which the different regions are on separate polynucleotides, the synthetic miRNA will be considered to be comprised of two polynucleotides.

When the RNA molecule is a single polynucleotide, there is a linker region between the miRNA region and the complementary region. In some embodiments, the single polynucleotide is capable of forming a hairpin loop structure as a result of bonding between the miRNA region and the complementary region. The linker constitutes the hairpin loop. It is contemplated that in some embodiments, the linker region is, is at least, or is at most 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 residues in length, or any range derivable therein. In certain embodiments, the linker is between 3 and 30 residues (inclusive) in length.

In addition to having a miRNA region and a complementary region, there may be flanking sequences as well at either the 5' or 3' end of the region. In some embodiments, there is or is at least 1, 2, 3, 4, 5, 6, 7, 8, 9, 10 nucleotides or more, or any range derivable therein, flanking one or both sides of these regions.

The invention includes a miRNA inhibitor for use reducing or eliminating activity of one or more miRNAs in a cell, as limited in the claims, comprising introducing into a cell a miRNA inhibitor; or supplying or enhancing the activity of one or more miRNAs in a cell. Disclosed are inducing certain cellular characteristics by providing to a cell a particular nucleic acid for use according to the claims, such as a specific synthetic miRNA molecule for use or a synthetic miRNA inhibitor molecule for use. However the miRNA molecule for use or miRNA inhibitor for use need not be synthetic. They may have a sequence that is identical to a naturally occurring miRNA or they may not have any design modifications. In certain embodiments, the miRNA molecule and/or a miRNA inhibitor for use are synthetic, as discussed above.

The particular nucleic acid molecule provided to the cell is understood to correspond to a particular miRNA in the cell, and thus, the miRNA in the cell is referred to as the "corresponding miRNA." In situations in which a named miRNA molecule is introduced into a cell, the corresponding miRNA will be understood to be the induced miRNA. It is contemplated, however, that the miRNA molecule introduced into a cell is not a mature miRNA but is capable of becoming a mature miRNA under the appropriate physiological conditions. In cases in which a particular corresponding miRNA is being inhibited by a miRNA inhibitor, the particular miRNA will be referred to as the targeted miRNA. It is contemplated that multiple corresponding miRNAs may be involved. In particular embodiments, more than one miRNA molecule is for use according to the claims and to be introduced into a cell. Moreover, in other embodiments, more than one miRNA inhibitor is for use according to the claims and to be introduced into a cell. Furthermore, a combination of miRNA molecule(s) and miRNA inhibitor(s) may be introduced into a cell.

Methods include identifying a cell or patient in need of inducing those cellular characteristics, as limited by the claims. Also, it will be understood that an amount of a synthetic nucleic acid that is provided to a cell or organism is an "effective amount," which refers to an amount needed to achieve a desired goal, such as inducing a particular cellular characteristic(s).

In certain embodiments of the miRNA molecule for use according to the claims are included providing or introducing to a cell a nucleic acid molecule corresponding to a mature miRNA in the cell in an amount effective to achieve a desired physiological result.

Moreover, methods can involve providing synthetic or nonsynthetic miRNA molecules. It is contemplated that in these embodiments, methods may or may not be limited to providing only one or more synthetic miRNA molecules or only on or more nonsynthetic miRNA molecules. Thus, in certain embodiments, methods may involve providing both synthetic and nonsynthetic miRNA molecules. In this situation, a cell or cells are most likely provided a synthetic miRNA molecule corresponding to a particular miRNA and a nonsynthetic miRNA molecule corresponding to a different miRNA. Furthermore, any method articulated a list of miRNAs using Markush group language may be articulated without the Markush group language and a disjunctive article *(i.e.,* or) instead, and vice versa.

In some embodiments, there is for reducing or inhibiting cell proliferation in a cell, introducing into or providing to the cell an effective amount of (i) a miRNA inhibitor molecule for use or (ii) a synthetic or nonsynthetic miRNA molecule for use that corresponds to a miRNA sequence, as limited by the claims. In certain embodiments introducing into the cell an effective amount of (i) a miRNA inhibitor molecule for use having a 5' to 3' sequence that is at least 90% complementary to the 5' to 3' sequence of one or more mature miRNA of Table 1 is involved, as limited by the claims.

Certain embodiments of the invention include a miRNA molecule or inhibitor for use in treating a pancreatic condition, as limited by the claims. In one aspect, contacting a pancreatic cell with one or more nucleic acid, synthetic miRNA, or miRNA for use comprising at least one nucleic acid segment having all or a portion of a miRNA sequence is involved, as limited by the claims. The segment may be 5, 6, 7, 8, 9, 10, 11, 12, 13, 14, 15, 16, 17, 18, 19, 20, 21, 22, 23, 24, 25, 30 or more nucleotides or nucleotide analog, including all integers there between. An aspect of the invention includes the modulation of a miRNA or a mRNA within a target cell, such as a pancreatic cell, as limited by the claims.

Typically, an endogenous gene, miRNA or mRNA is modulated in the cell. In particular embodiments, the nucleic acid sequence comprises at least one segment that is at least 70, 75, 80, 85, 90, 95, or 100% identical in nucleic acid sequence to one or more miRNA sequence listed in Table 1. Modulation of the expression or processing of an endogenous gene, miRNA, or mRNA can be through modulation of the processing of a mRNA, such processing including transcription, transportation and/or translation with in a cell. Modulation may also be effected by the inhibition or enhancement of miRNA activity with a cell, tissue, or organ. Such processing may effect the expression of an encoded product or the stability of the mRNA. In still other embodiments, a nucleic acid sequence can comprise a modified nucleic acid sequence.

In particular embodiments the pancreatic cell is a pancreatic ductal adenocarcinoma cell. Methods can further comprise administering a second therapy, such as chemotherapy, radiotherapy, surgery, or immunotherapy. The nucleic acid can be transcribed from a nucleic acid vector, such as a plasmid vector or a viral vector.

Method of treating a pancreatic condition include contacting or administering to a pancreatic cell with one or more nucleic acid comprise a miRNA sequence, wherein expression of an endogenous miRNA is modulated in the pancreatic cell; where the miRNA sequence is at least 70, 75, 80, 85% or more identical to one or more of hsa-let-7a, hsa-let-7b, hsa-let-7c, hsa-let-7d, hsa-let-7e, hsa-let-7f, hsa-let-7g, hsa-let-7i, hsa-miR-100, hsa-miR-101, hsa-miR-103, hsa-miR-106a, hsa-miR-106b, hsa-miR-107, hsa-iR-10a, hsa-miR-125a, hsa-miR-125b, hsa-miR-130a, hsa-miR-130b, hsa-miR-134, hsa-miR-140, hsa-miR-141, hsa-miR-143, hsa-miR-145, hsa-miR-146a, hsa-miR-148a, hsa-miR-148b, hsa-miR-150, hsa-miR-154, hsa-miR-155, hsa-miR-15b, hsa-miR-17-5p, hsa-miR-18a, hsa-miR-181b, hsa-miR-182, hsa-miR-186, hsa-miR-196a, hsa-miR-196b, hsa-miR-199a, hsa-miR-199a-AS, hsa-miR-19a, hsa-miR-19b, hsa-miR-200a, hsa-miR-200b, hsa-miR-200c, hsa-miR-203, hsa-miR-21, hsa-miR-210, hsa-miR-214, hsa-miR-216, hsa-miR-217, hsa-miR-221, hsa-miR-222, hsa-miR-223, hsa-miR-224, hsa-miR-23a, hsa-miR-24, hsa-miR-25, hsa-miR-26a, hsa-miR-26b, hsa-miR-27a, hsa-miR-27b, hsa-miR-28, hsa-miR-29a, hsa-miR-29b, hsa-miR-29c, hsa-miR-30a-3p, hsa-miR-30a-5p, hsa-miR-30b, hsa-miR-30c, hsa-miR-30d, hsa-miR-30e-3p, hsa-miR-30e-5p, hsa-miR-31, hsa-miR-331, hsa-miR-335, hsa-miR-365, hsa-miR-368, hsa-miR-374, hsa-miR-375, hsa-miR-376a, hsa-miR-377, hsa-miR-379, hsa-miR-429, hsa-miR-93, hsa-miR-95, hsa-miR-96, hsa-miR-98, hsa-miR-99a, hsa-miR-99b, hsa-miR-452, hsa-miR-494, hsa-miR-497, miR-205, or ambi-miR-7105.

In certain aspects, one or more miRNA sequence may include or comprise a modified nucleobase or nucleic acid sequence.

In other aspects, a pancreatic cell is a pancreatic cancer cell.

The methods may further comprise administering a second therapy. The second therapy can be, but is not limited to chemotherapy, radiotherapy, surgery, or immunotherapy.

In still further aspects, one or more miRNA are transcribed from a nucleic acid vector, such as a plasmid or viral vector.

Embodiments of the invention include a miRNA molecule or inhibitor for use in treating pancreatic ductal adenocarcinoma in a subject, the treating comprising administering to the subject an effective amount of one or more synthetic miRNA molecules or inhibitors having a nucleic acid segment having sequence identity to hsa-let-7a, hsa-let-7b, hsa-let-7c, hsa-let-7d, hsa-let-7f, hsa-let-7g, hsa-let-7i, hsa-miR-100, hsa-miR-101, hsa-miR-103, hsa-miR-106b, hsa-miR-107, hsa-iR-10a, hsa-miR-125a, hsa-miR-125b, hsa-miR-130a, hsa-miR-130b, hsa-miR-134, hsa-miR-140, hsa-miR-141, hsa-miR-143, hsa-miR-145, hsa-miR-146a, hsa-miR-148a, hsa-miR-148b, hsa-miR-150, hsa-miR-154, hsa-miR-155, hsa-miR-18a, hsa-miR-181b, hsa-miR-182, hsa-miR-186, hsa-miR-196a, hsa-miR-196b, hsa-miR-199a, hsa-miR-199a-AS, hsa-miR-19a, hsa-miR-19b, hsa-miR-200a, hsa-miR-200b, hsa-miR-200c, hsa-miR-203, hsa-miR-21, hsa-miR-210, hsa-miR-214, hsa-miR-216, hsa-miR-217, hsa-miR-221, hsa-miR-222, hsa-miR-223, hsa-miR-224, hsa-miR-23a, hsa-miR-24, hsa-miR-26a, hsa-miR-26b, hsa-miR-27a, hsa-miR-27b, hsa-miR-29c, hsa-miR-30a-3p, hsa-miR-30a-5p, hsa-miR-30b, hsa-miR-30c, hsa-miR-30d, hsa-miR-30e-3p, hsa-miR-30e-5p, hsa-miR-31, hsa-miR-331, hsa-miR-335, hsa-miR-365, hsa-miR-368, hsa-miR-374, hsa-miR-375, hsa-miR-376a, hsa-miR-377, hsa-miR-379, hsa-miR-429, hsa-miR-93, hsa-miR-95, hsa-miR-96, hsa-miR-99b, hsa-miR-452, hsa-miR-494, hsa-miR-497, miR-205, or ambi-miR-7105, as limited by the claims.

It is disclosed, a subject is administered: one or more miRNA inhibitors having a nucleic acid segment having at least 80% nucleic acid sequence identity to hsa-let-7i, hsa-miR-100, hsa-miR-103, hsa-miR-106b, hsa-miR-107, hsa-miR-10a, hsa-miR-125a, hsa-miR-125b, hsa-miR-140, hsa-miR-143, hsa-miR-145, hsa-miR-146a, hsa-miR-150, hsa-miR-155, hsa-miR-18a, hsa-miR-181b, hsa-miR-196a, hsa-miR-196b, hsa-miR-199a, hsa-miR-199a-AS, hsa-miR-203, hsa-miR-21, hsa-miR-210, hsa-miR-214, hsa-miR-221, hsa-miR-222, hsa-miR-223, hsa-miR-224, hsa-miR-23a, hsa-miR-24, hsa-miR-27a, hsa-miR-31, hsa-miR-331, hsa-miR-93, hsa-miR-99b, hsa-miR-452, hsa-miR-497, or ambi-miR-7105; and/or one or miRNAs having a nucleic acid segment having at least 80% nucleic acid sequence identity to hsa-let-7a, hsa-let-7b, hsa-let-7c, hsa-let-7d, hsa-let-7e, hsa-let-7f, hsa-let-7g, hsa-miR-101, hsa-miR-130a, hsa-miR-130b, hsa-miR-134, hsa-miR-141, hsa-miR-148a, hsa-miR-148b, hsa-miR-154, hsa-miR-182, hsa-miR-186, hsa-miR-19a, hsa-miR-19b, hsa-miR-200a, hsa-miR-200b, hsa-miR-200c, hsa-miR-216, hsa-miR-217, hsa-miR-26a, hsa-miR-26b, hsa-miR-27b, hsa-miR-29c, hsa-miR-30a-3p, hsa-miR-30a-5p, hsa-miR-30b, hsa-miR-30c, hsa-miR-30d, hsa-miR-30e-3p, hsa-miR-30e-5p, hsa-miR-335, hsa-miR-365, hsa-miR-368, hsa-miR-374, hsa-miR-375, hsa-miR-376a, hsa-miR-377, hsa-miR-379, hsa-miR-429, hsa-miR-95, hsa-miR-96, or hsa-miR-494.

In other aspects, a miRNA molecule or inhibitor is for use in treating chronic pancreatitis in a subject, the treating comprising administering to the subject an effective amount of one or more synthetic miRNA molecules or miRNA inhibitors comprising a nucleic acid segment havingnucleic acid sequence identity to hsa-let-7a, hsa-let-7b, hsa-let-7c, hsa-let-7d, hsa-let-7e, hsa-let-7f, hsa-let-7g, hsa-let-7i, hsa-miR-100, hsa-miR-101, hsa-miR-10a, hsa-miR-125a, hsa-miR-125b, hsa-miR-130a, hsa-miR-130b, hsa-miR-141, hsa-miR-143, hsa-miR-145, hsa-miR-146a, hsa-miR-148a, hsa-miR-148b, hsa-miR-150, hsa-miR-18a, hsa-miR-182, hsa-miR-186, hsa-miR-199a hsa-miR-199a-AS, hsa-miR-19a, hsa-miR-19b, hsa-miR-200a, hsa-miR-200b, hsa-miR-200c, hsa-miR-203, hsa-miR-210, hsa-miR-214, hsa-miR-216, hsa-miR-217, hsa-miR-222, hsa-miR-223, hsa-miR-24, hsa-miR-25, hsa-miR-26a, hsa-miR-26b, hsa-miR-27b, hsa-miR-28, hsa-miR-29a, hsa-miR-29b, hsa-miR-29c, hsa-miR-30a-3p, hsa-miR-30a-5p, hsa-miR-30b, hsa-miR-30c, hsa-miR-30d, hsa-miR-30e-3p, hsa-miR-30e-5p, hsa-miR-31, hsa-miR-335, hsa-miR-365, hsa-miR-368, hsa-miR-374, hsa-miR-376a, hsa-miR-429, hsa-miR-95, hsa-miR-96, hsa-miR-98, hsa-miR-99a, hsa-miR-497, or ambi-miR-7105, as limited by the claims.

It is disclosed that the subject is administered one or more miRNA inhibitors having a nucleic acid segment having at least 80% nucleic acid sequence identity to hsa-let-7i, hsa-miR-100, hsa-iR-10a, hsa-miR-125a, hsa-miR-125b, hsa-miR-143, hsa-miR-145, hsa-miR-146a, hsa-miR-150, hsa-miR-18a, hsa-miR-199a, hsa-miR-199a-AS, hsa-miR-210, hsa-miR-214, hsa-miR-222, hsa-miR-223, hsa-miR-24, hsa-miR-31, hsa-miR-99a, or hsa-miR-497; and/or one or miRNAs having a nucleic acid segment having at least 80% nucleic acid sequence identity to hsa-let-7a, hsa-let-7b, hsa-let-7c, hsa-let-7d, hsa-let-7e, hsa-let-7f, hsa-let-7g, hsa-miR-101, hsa-miR-130a, hsa-miR-130b, hsa-miR-141, hsa-miR-148a, hsa-miR-148b, hsa-miR-182, hsa-miR-186, hsa-miR-19a, hsa-miR-19b, hsa-miR-200a, hsa-miR-200b, hsa-miR-200c, hsa-miR-203, hsa-miR-216, hsa-miR-217, hsa-miR-25, hsa-miR-26a, hsa-miR-26b, hsa-miR-27b, hsa-miR-28, hsa-miR-29a, hsa-miR-29b, hsa-miR-29c, hsa-miR-30a-3p, hsa-miR-30a-5p, hsa-miR-30b, hsa-miR-30c, hsa-miR-30d, hsa-miR-30e-3p, hsa-miR-30e-5p, hsa-miR-335, hsa-miR-365, hsa-miR-368, hsa-miR-374, hsa-miR-376a, hsa-miR-429, hsa-miR-95, hsa-miR-96, hsa-miR-98, or ambi-miR-7105.

Synthetic nucleic acids can be administered to the subject or patient using modes of administration that are well known to those of skill in the art, particularly for therapeutic applications. It is particularly contemplated that a patient is human or any other mammal or animal having miRNA.

It will be understood in methods of the invention that a cell or other biological matter such as an organism (including patients) can be provided a miRNA or miRNA molecule corresponding to a particular miRNA by administering to the cell or organism a nucleic acid molecule that functions as the corresponding miRNA once inside the cell. The form of the molecule provided to the cell may not be the form that acts as a miRNA once inside the cell. Thus, it is contemplated that in some embodiments, biological matter is provided a synthetic miRNA or a nonsynthetic miRNA, such as one that becomes processed into a mature and active miRNA once it has access to the cell's miRNA processing machinery. In certain embodiments, it is specifically contemplated that the miRNA molecule provided to the biological matter is not a mature miRNA molecule but a nucleic acid molecule that can be processed into the mature miRNA once it is accessible to miRNA processing machinery. The term "nonsynthetic" in the context of miRNA means that the miRNA is not "synthetic," as defined herein. Furthermore, it is contemplated that in embodiments of the invention that concern the use of synthetic miRNAs, the use of corresponding nonsynthetic miRNAs is also considered an aspect of the invention, and vice versa.

In other embodiments, the methods involve reducing cell viability comprising introducing into or providing to the cell an effective amount of (i) a miRNA inhibitor molecule or (ii) a synthetic or nonsynthetic miRNA molecule that corresponds to a miRNA sequence. Methods for inducing apoptosis have a number of therapeutic applications including, but not limited to, the treatment of cancer.

Disclosed are miRNA compositions to treat diseases or conditions or to prepare therapeutics for the treatment of diseases or conditions. It is contemplated that 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 more miRNA (or any range derivable therein) may be used for these embodiments. In certain embodiments, methods involve one or more miRNA inhibitors and/or a miRNA molecules corresponding to any of these miRNAs, particularly for the treatment or prevention of cancer. Cancer includes, but is not limited to, malignant cancers, tumors, metastatic cancers, unresectable cancers, chemo- and/or radiation-resistant cancers, and terminal cancers.

It will be understood that shorthand notations are employed such that a generic description of a miRNA refers to any of its gene family members (distinguished by a number), unless otherwise indicated. It is understood by those of skill in the art that a "gene family" refers to a group of genes having the same miRNA coding sequence. Typically, members of a gene family are identified by a number following the initial designation. For example, miR-16-1 and miR-16-2 are members of the miR-16 gene family and "miR-7" refers to miR-7-1, miR-7-2 and miR-7-3. Moreover, unless otherwise indicated, a shorthand notation refers to related miRNAs (distinguished by a letter). Thus, "let-7," for example, refers to let-7a-1, let7-a-2, let-7b, let-7c, let-7d, let-7e, let-7f-1, and let-7f-2." Exceptions to these shorthand notations will be otherwise identified.

It will be understood that the term "providing" an agent is used to include "administering" the agent to a patient.

Methods also include targeting a miRNA to modulate in a cell or organism. The term "targeting a miRNA to modulate" means a nucleic acid of the invention will be employed so as to modulate the selected miRNA. In some disclosures the modulation is achieved with a synthetic or non-synthetic miRNA that corresponds to the targeted miRNA, which effectively provides the targeted miRNA to the cell or organism (positive modulation). In other disclosures, the modulation is achieved with a miRNA inhibitor, which effectively inhibits the targeted miRNA in the cell or organism (negative modulation).

In some disclosures, the miRNA targeted to be modulated is a miRNA that affects a disease, condition, or pathway. In certain disclosures, the miRNA is targeted because a treatment can be provided by negative modulation of the targeted miRNA. In other disclosures, the miRNA is targeted because a treatment can be provided by positive modulation of the targeted miRNA.

In certain methods, there is a further step of administering the selected miRNA modulator to a cell, tissue, organ, or organism (collectively "biological matter") in need of treatment related to modulation of the targeted miRNA or in need of the physiological or biological results discussed herein (such as with respect to a particular cellular pathway or result like decrease in cell viability). Consequently, in some methods there is a step of identifying a patient in need of treatment that can be provided by the miRNA modulator(s). It is contemplated that an effective amount of a miRNA modulator can be administered in some embodiments. In particular embodiments, there is a therapeutic benefit conferred on the biological matter, where a "therapeutic benefit" refers to an improvement in the one or more conditions or symptoms associated with a disease or condition or an improvement in the prognosis, duration, or status with respect to the disease. It is contemplated that a therapeutic benefit includes, but is not limited to, a decrease in pain, a decrease in morbidity, a decrease in a symptom. For example, with respect to cancer, it is contemplated that a therapeutic benefit can be inhibition of tumor growth, prevention of metastasis, reduction in number of metastases, inhibition of cancer cell proliferation, inhibition of cancer cell proliferation, induction of cell death in cancer cells, inhibition of angiogenesis near cancer cells, induction of apoptosis of cancer cells, reduction in pain, reduction in risk of recurrence, induction of chemo- or radiosensitivity in cancer cells, prolongation of life, and/or delay of death directly or indirectly related to cancer.

Furthermore, it is contemplated that the miRNA compositions may be provided as part of a therapy to a patient, in conjunction with traditional therapies or preventative agents. Moreover, it is contemplated that any method discussed in the context of therapy may be applied as preventatively, particularly in a patient identified to be potentially in need of the therapy or at risk of the condition or disease for which a therapy is needed.

In addition, miRNA molecules for use according to the invention concern employing one or more nucleic acids corresponding to a miRNA and a therapeutic drug, as limited by the claims. The nucleic acid can enhance the effect or efficacy of the drug, reduce any side effects or toxicity, modify its bioavailability, and/or decrease the dosage or frequency needed. In certain embodiments, the therapeutic drug is a cancer therapeutic. Consequently, in some embodiments comprise administering to the patient the cancer therapeutic and an effective amount of at least one miRNA molecule that improves the efficacy of the cancer therapeutic or protects non-cancer cells, as limited by the claims. Cancer therapies also include a variety of combination therapies with both chemical and radiation based treatments. Combination chemotherapies include but are not limited to, for example, bevacizumab, cisplatin (CDDP), carboplatin, EGFR inhibitors (gefitinib and cetuximab), procarbazine, mechlorethamine, cyclophosphamide, camptothecin, COX-2 inhibitors (e.g., celecoxib) ifosfamide, melphalan, chlorambucil, busulfan, nitrosurea, dactinomycin, daunorubicin, doxorubicin (adriamycin), bleomycin, plicomycin, mitomycin, etoposide (VP16), tamoxifen, raloxifene, estrogen receptor binding agents, taxol, taxotere, gemcitabien, navelbine, farnesyl-protein transferase inhibitors, transplatinum, 5-fluorouracil, vincristin, vinblastin and methotrexate, or any analog or derivative variant of the foregoing.

Alternatively or additionally, a miRNA molecule for use according to the invention protects non-cancer cells from the cancer therapeutic and is selected from the group consisting of miR-16, miR-24, miR-30a-3p, miR-125b, miR-152, miR-194, miR-197, miR-214, and miR-331, as limited by the claims.

Generally, inhibitors of miRNAs can be given to achieve the opposite effect as compared to when nucleic acid molecules corresponding to the mature miRNA are given. Similarly, nucleic acid molecules corresponding to the mature miRNA can be given to achieve the opposite effect as compared to when inhibitors of the miRNA are given. For example, miRNA molecules that increase cell proliferation can be provided to cells to increase proliferation or inhibitors of such molecules can be provided to cells to decrease cell proliferation. The present invention contemplates these embodiments in the context of the different physiological effects observed with the different miRNA molecules and miRNA inhibitors disclosed herein. These include, but are not limited to, the following physiological effects: increase and decreasing cell proliferation, increasing or decreasing apoptosis, increasing transformation, increasing or decreasing cell viability, activating ERK, activating/inducing or inhibiting hTert, inhibit stimulation of Stat3, reduce or increase viable cell number, and increase or decrease number of cells at a particular phase of the cell cycle. Methods of the invention are generally contemplated to include providing or introducing one or more different nucleic acid molecules corresponding to one or more different miRNA molecules. It is contemplated that the following, at least the following, or at most the following number of different nucleic acid molecules may be provided or introduced: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or any range derivable therein. This also applies to the number of different miRNA molecules that can be provided or introduced into a cell.

The present invention also concerns the use of kits containing compositions of the invention or compositions to implement methods of the invention, as limited by the claims. In some embodiments, kits can be used to evaluate one or more miRNA molecules. In certain embodiments, a kit contains, contains at least or contains at most 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 or more miRNA probes, synthetic miRNA molecules or miRNA inhibitors, or any range and combination derivable therein. In some embodiments, there are kits for evaluating miRNA activity in a cell.

Kits may comprise components, which may be individually packaged or placed in a container, such as a tube, bottle, vial, syringe, or other suitable container means.

Individual components may also be provided in a kit in concentrated amounts; in some embodiments, a component is provided individually in the same concentration as it would be in a solution with other components. Concentrations of components may be provided as 1x, 2x, 5x, 10x or 20x or more.

Kits for using miRNA probes, synthetic miRNAs, nonsynthetic, and/or miRNA inhibitors of the invention for therapeutic, prognostic, or diagnostic applications are included as part of the invention. Specifically contemplated are any such molecules corresponding to any miRNA reported to influence biological activity, such as those discussed herein.

In certain aspects, negative and/or positive control synthetic miRNAs and/or miRNA inhibitors are included in some kit embodiments. The Control molecules can be used to verify transfection efficiency and/or control for transfection-induced changes in cells.

It is contemplated that any method or composition described herein can be implemented with respect to any other method or composition described herein and that different embodiments may be combined. It is specifically contemplated that any methods and compositions discussed herein with respect to miRNA molecules or miRNA may be implemented with respect to synthetic miRNAs to the extent the synthetic miRNA is exposed to the proper conditions to allow it to become a mature miRNA under physiological circumstances. The claims originally filed are contemplated to cover claims that are multiply dependent on any filed claim or combination of filed claims.

Embodiments of the invention include the use of kits for analysis of a pathological sample by assessing miRNA profile for a sample comprising, in suitable container means, two or more miRNA probes, wherein the miRNA probes detect one or more of hsa-let-7a, hsa-let-7b, hsa-let-7c, hsa-let-7d, hsa-let-7e, hsa-let-7f, hsa-let-7g, hsa-let-7i, hsa-miR-100, hsa-miR-101, hsa-miR-103, hsa-miR-106a, hsa-miR-106b, hsa-miR-107, hsa-iR-10a, hsa-miR-125a, hsa-miR-125b, hsa-miR-130a, hsa-miR-130b, hsa-miR-134, hsa-miR-140, hsa-miR-141, hsa-miR-143, hsa-miR-145, hsa-miR-146a, hsa-miR-148a, hsa-miR-148b, hsa-miR-150, hsa-miR-154, hsa-miR-155, hsa-miR-15b, hsa-miR-17-5p, hsa-miR-18, hsa-miR-181b, hsa-miR-182, hsa-miR-186, hsa-miR-196a, hsa-miR-196b, hsa-miR-199a ,hsa-miR-199a-AS, hsa-miR-19a, hsa-miR-19b, hsa-miR-200a, hsa-miR-200b, hsa-miR-200c, hsa-miR-203, hsa-miR-21, hsa-miR-210, hsa-miR-214, hsa-miR-216, hsa-miR-217, hsa-miR-221, hsa-miR-222, hsa-miR-223, hsa-miR-224, hsa-miR-23a, hsa-miR-24, hsa-miR-25, hsa-miR-26a, hsa-miR-26b, hsa-miR-27a, hsa-miR-27b, hsa-miR-28, hsa-miR-29a, hsa-miR-29b, hsa-miR-29c, hsa-miR-30a-3p, hsa-miR-30a-5p, hsa-miR-30b, hsa-miR-30c, hsa-miR-30d, hsa-miR-30e-3p, hsa-miR-30e-5p, hsa-miR-31, hsa-miR-331, hsa-miR-335, hsa-miR-365, hsa-miR-368, hsa-miR-374, hsa-miR-375, hsa-miR-376a, hsa-miR-377, hsa-miR-379, hsa-miR-429, hsa-miR-93, hsa-miR-95, hsa-miR-96, hsa-miR-98, hsa-miR-99a, hsa-miR-99b, hsa-miR-452, hsa-miR-494, hsa-miR-497 or ambi-miR-7105, as limited by the claims. The kit can further comprise reagents for labeling miRNA in the sample. The kit may also include the labeling reagents include at least one amine-modified nucleotide, poly(A) polymerase, and poly(A) polymerase buffer. Labeling reagents can include an amine-reactive dye.

**Table 1. Listing of miRNA probes for pancreatic sample assessment.**

| **Probe Sequence** | **SEQ ID NO:** | **miRNA** | **miR Base Information** |
|---|---|---|---|
| UGAGGUAGUAGGUUGUAUAGUU | SEQ ID NO:1 | hsa-let-7a | >hsa-let-7a MIMA T0000062 |
| UGAGGUAGUAGGUUGUGUGGUU | SEQ ID NO:2 | hsa-let-7b | >hsa-let-7b MIMAT0000063 |
| UGAGGUAGUAGGUUGUAUGGUU | SEQ ID NO:3 | hsa-let-7c | >lisa-let-7c MIMAT0000064 |
| AGAGGUAGUAGGUUGCAUAGU | SEQ ID NO:4 | hsa-let-7d | >hsa-let-7d MIMAT0000065 |
| UGAGGUAGGAGGUUGUAUAGU | SEQ ID NO:5 | hsa-let-7e | >lisa-let-7e MIMAT0000066 |
| UGAGGUAGUAGAUUGUAUAGUU | SEQ ID NO:6 | hsa-let-7f | >hsa-let-7f MIMAT0000067 |
| UGAGGUAGUAGUUUGUACAGU | SEQ ID NO:7 | hsa-let-7g | >hsa-let-7g MIMAT0000414 |
| UGAGGUAGUAGUUUGUGCUGU | SEQ ID NO:8 | hsa-let-7i | >hsa-let-7i MIMAT0000415 |
| UGGAAUGUAAAGAAGUAUGUA | SEQ ID NO:9 | hsa-miR-1 | >hsa-miR-1 MIMAT0000416 |
| AACCCGUAGAUCCGAACUUGUG | SEQ ID NO:10 | hsa-miR-100 | >hsa-miR-100 MIMAT0000098 |
| UACAGUACUGUGAUAACUGAAG | SEQ ID NO:11 | hsa-miR-101 | >hsa-miR-101 MIMAT0000099 |
| AGCAGCAUUGUACAGGGCUAUGA | SEQ ID NO:12 | hsa-miR-103 | >hsa-miR-103 MIMAT0000101 |
| UCAAAUGCUCAGACUCCUGU | SEQ ID NO:13 | hsa-miR-105 | >hsa-miR-105 |
| | | | MIMAT0000102 |
| AAAAGUGCUUACAGUGCAGGUAGC | SEQ ID NO:14 | hsa-miR-106a | >hsa-miR-106a MIMAT0000103 |
| UAAAGUGCUGACAGUGCAGAU | SEQ ID NO:15 | hsa-miR-106b | MIMAT0000680 MIMA T0000680 |
| AGCAGCAUUGUACAGGGCUAUCA | SEQ ID NO:16 | hsa-miR-107 | MIMAT0000104 |
| UACCCUGUAGAUCCGAAUUUGUG | SEQ ID NO:17 | hsa-miR-10a | >hsa-miR-10a MIMAT0000253 |
| UACCCUGUAGAACCGAAUUUGU | SEQ ID NO:18 | hsa-miR-10b | >hsa-miR-10b MIMAT0000254 |
| UGGAGUGUGACAAUGGUGUUUGU | SEQ ID NO:19 | hsa-miR-122a | >hsa-mir-122a MIMAT0000421 |
| UUAAGGCACGCGUGAAUGCCA | SEQ ID NO:20 | hsa-miR-124a | >hsa-miR-124a MIMAT0000422 |
| UCCCUGAGACCCUUUAACCUGUG | SEQ ID NO:21 | hsa-miR-125a | >hsa-miR-125a MIMAT0000443 |
| UCCCUGAGACCCUAACUUGUGA | SEQ ID NO:22 | hsa-miR-125b | >hsa-mir-125b MIMAT0000423 |
| UCGUACCGUGAGUAAUAAUGC | SEQ ID NO:23 | hsa-miR-126 | >hsa-miR-126 MIMAT0000445 |
| CAUUAUUACUUUUGGUACGCG | SEQ ID NO:24 | hsa-miR-126-AS | >hsa-miR-126* MIMAT0000444 |
| UCGGAUCCGUCUGAGCUUGGCU | SEQ ID NO:25 | hsa-miR-127 | >hsa-mir-127 MlMAT0000446 |
| UCACAGUGAACCGGUCUCUUUU | SEQ ID NO:26 | hsa-miR-128a | >hsa-miR-128a MIMAT0000424 |
| CUUUUUGCGGUCUGGGCUUGC | SEQ ID NO:27 | hsa-miR-129 | >hsa-mir-129 MIMAT0000242 |
| CAGUGCAAUGUUAAAAGGGCAU | SEQ ID NO:28 | hsa-miR-130a | >hsa-mir-130a MIMAT0000425 |
| CAGUGCAAUGAUGAAAGGGCAU | SEQ ID NO:29 | hsa-miR-130b | >hsa-miR-130b MIMAT0000691 |
| UAACAGUCUACAGCCAUGGUCG | SEQ ID NO:30 | hsa-miR-132 | >hsa-mir-132 MIMAT0000426 |
| UUGGUCCCCUUCAACCAGCUGU | SEQ ID NO:31 | hsa-miR-133a | >hsa-mir-133a MIMAT0000427 |
| UGUGACUGGUUGACCAGAGGG | SEQ ID NO:32 | hsa-miR-134 | >hsa-mir-134 MIMAT0000447 |
| UAUGGCUUUUAUUCCUAUGUGA | SEQ ID NO:33 | hsa-miR-135a | >hsa-miR-135a MIMAT0000428 |
| UAUGGCUUUUCAUUCCUAUGUG | SEQ ID NO:34 | hsa-miR-135b | >hsa-miR-135b MIMAT0000758 |
| ACUCCAUUUGUUUUGAUGAUGGA | SEQ ID NO:35 | hsa-miR-136 | >hsa-miR-136 MIMAT0000448 |
| UAUUGCUUAAGAAUACGCGUAG | SEQIDNO:36 | hsa-miR-137 | >hsa-miR-137 MIMAT0000429 |
| AGCUGGUGUUGUGAAUC | SEQ ID NO:37 | hsa-miR-138 | >hsa-miR-138 MIMAT0000430 |
| UCUACAGUGCACGUGUCU | SEQ ID NO:38 | hsa-miR-139 | >hsa-miR-139 MIMAT0000250 |
| AGUGGUUUUACCCUAUGGUAG | SEQ ID NO:39 | hsa-miR-140 | >hsa-miR-140 MIMAT0000431 |
| UAACACUGUCUGGUAAAGAUGG | SEQ ID NO:40 | hsa-miR-141 | >hsa-miR-141 MIMAT0000432 |
| UGUAGUGUUUCCUACUUUAUGGA | SEQ ID NO:41 | hsa-miR-142-3p | >hsa-miR-142-3p MIMAT0000434 |
| CAUAAAGUAGAAAGCACUAC | SEQ ID NO:42 | hsa-miR-142-5p | >hsa-miR-142-5p MIMAT0000433 |
| UGAGAUGAAGCACUGUAGCUCA | SEQ ID NO:43 | hsa-miR-143 | >hsa-miR-143 MIMAT0000435 |
| UACAGUAUAGAUGAUGUACUAG | SEQ ID NO:44 | hsa-miR-144 | >hsa-miR-144 MIMAT0000436 |
| GUCCAGUUUUCCCAGGAAUCCCUU | SEQ ID NO:45 | hsa-miR-145 | >hsa-miR-145 MIMAT0000437 |
| UGAGAACUGAAUUCCAUGGGUU | SEQ ID NO:46 | hsa-miR-146a | >hsa-miR-146 MIMAT0000449 |
| GUGUGUGGAAAUGCUUCUGC | SEQ ID NO:47 | hsa-miR-147 | >hsa-miR-147 MIMAT0000251 |
| UCAGUGCACUACAGAACUUUGU | SEQ ID NO:48 | hsa-miR-148a | >hsa-miR-148 MIMAT0000243 |
| UCAGUGCAUCACAGAACUUUGU | SEQ ID NO:49 | hsa-miR-148b | >hsa-miR-148b MIMAT0000759 |
| UCUGGCUCCGUGUCUUCACUCC | SEQ ID NO:50 | hsa-miR-149 | >hsa-miR-149 MIMAT0000450 |
| UCUCCCAACCCUUGUACCAGUG | SEQ ID NO:51 | hsa-miR-150 | >hsa-miR-150-MIMAT0000451 |
| ACUAGACUGAAGCUCCUUGAGG | SEQ ID NO:52 | hsa-miR-151 | >hsa-miR-151 MIMAT0000757 |
| UCAGUGCAUGACAGAACUUGGG | SEQ ID NO:53 | hsa-miR-152 | >hsa-miR-152 MIMAT0000438 |
| UUGCAUAGUCACAAAAGUGA | SEQ ID NO:54 | hsa-miR-153 | >hsa-miR-153 MIMAT0000439 |
| UAGGUUAUCCGUGUUGCCUUCG | SEQ ID NO:55 | hsa-miR-154 | >hsa-miR-154 MIMAT0000452 |
| UUAAUGCUAAUCGUGAUAGGGG | SEQ ID NO:56 | hsa-miR-155 | >hsa-miR-155 MlMAT0000646 |
| UAGCAGCACAUAAUGGUUUGUG | SEQIDNO:57 | hsa-miR-15a | >hsa-miR-15a MIMAT0000068 |
| UAGCAGCACAUCAUGGUUUACA | SEQ ID NO:58 | hsa-miR-15b | >hsa-miR-15b MIMAT0000417 |
| UAGCAGCACGUAAAUAUUGGCG | SEQ ID NO:59 | hsa-miR-16 | >hsa-miR-16 MIMAT0000069 |
| ACUGCAGUGAAGGCACUUGU | SEQ ID NO:60 | hsa-miR-17-3p | >hsa-miR-17-3p MIMAT0000071 |
| CAAAGUGCUUACAGUGCAGGUAGU | SEQ ID NO:61 | hsa-miR-17-5p | >hsa-miR-17-5p MIMAT0000070 |
| UAAGGUGCAUCUAGUGCAGAUA | SEQ ID NO:62 | hsa-miR-18a | >hsa-miR-18A MIMAT0000072 |
| AACAUUCAACGCUGUCGGUGAGU | SEQ ID NO:63 | hsa-miR-181a | >hsa-miR-181a MIMAT0000256 |
| AACAUUCAUUGCUGUCGGUGGG | SEQ ID NO:64 | hsa-miR-181b | >hsa-miR-181b MIMAT0000257 |
| AACAUUCAACCUGUCGGUGAGU | SEQ ID NO:65 | hsa-miR-181c | >hsa-miR-181c MIMAT0000258 |
| UUUGGCAAUGGUAGAACUCACA | SEQ ID NO:66 | hsa-miR-182 | >hsa-miR-182 MIMAT0000259 |
| UGGUUCUAGACUUGCCAACUA | SEQ ID NO:67 | hsa-miR-182-AS | >hsa-miR-182* MIMAT0000260 |
| UAUGGCACUGGUAGAAUUCACUG | SEQ ID NO:68 | hsa-miR-183 | >hsa-miR-183 MIMAT0000261 |
| UGGACGGAGAACUGAUAAGGGU | SEQ ID NO:69 | hsa-miR-184 | >hsa-miR-184 MIMAT0000454 |
| UGGAGAGAAAGGCAGUUC | SEQ ID NO:70 | hsa-miR-185 | >hsa-miR-185 MIMAT0000455 |
| CAAAGAAUUCUCCUUUUGGGCUU | SEQ ID NO:71 | hsa-miR-186 | >hsa-miR-186 MIMAT0000456 |
| UCGUGUCUUGUGUUGCAGCCG | SEQ ID NO:72 | hsa-miR-187 | >hsa-miR-187 MIMAT0000262 |
| CAUCCCUUGCAUGGUGGAGGGU | SEQ ID NO:73 | hsa-miR-188 | >hsa-miR-188 MIMAT0000457 |
| GUGCCUACUGAGCUGAUAUCAGU | SEQ ID NO:74 | hsa-miR-189 | >hsa-miR-189 MIMAT0000079 |
| UGAUAUGUUUGAUAUAUUAGGU | SEQ ID NO:75 | hsa-miR-190 | >hsa-miR-190 MIMAT0000458 |
| CAACGGAAUCCCAAAAGCAGCU | SEQ ID NO:76 | hsa-miR-191 | >hsa-miR-191 MIMAT0000440 |
| CUGACCUAUGAAUUGACAGCC | SEQ ID NO:77 | hsa-miR-192 | >hsa-miR-192 MIMAT0000222 |
| AACUGGCCUACAAAGUCCCAG | SEQ ID NO:78 | hsa-miR-193a | >hsa-miR-193a MIMAT0000459 |
| UGUAACAGCAACUCCAUGUGGA | SEQ ID NO:79 | hsa-miR-194 | >hsa-miR-194 MIMAT0000460 |
| UAGCAGCACAGAAAUAUUGGC | SEQ ID NO:80 | hsa-miR-195 | >hsa-miR-195 MIMAT0000461 |
| UAGGUAGUUUCAUGUUGUUGG | SEQ ID NO:81 | hsa-miR-196a | >hsa-miR-196a MIMAT0000226 |
| UAGGUAGUUUCCUGUUGUUGG | SEQ ID NO:82 | hsa-miR-196b | >hsa-miR-196b MIMAT0001080 |
| UUCACCACCUUCUCCACCCAGC | SEQ ID NO:83 | hsa-miR-197 | >hsa-miR-197 MIMAT0000227 |
| GGUCCAGAGGGGAGAUAGG | SEQ ID NO:84 | hsa-miR-198 | >hsa-miR-198 MIMAT0000228 |
| CCCAGUGUUCAGACUACCUGUUC | SEQ ID NO:85 | hsa-miR-199a | >hsa-miR-199a MIMAT0000231 |
| UACAGUAGUCUGCACAUUGGUU | SEQ ID NO:86 | hsa-miR-199a-AS | >hsa-miR-199a* MIMAT0000232 |
| CCCAGUGUUUAGACUAUCUGUUC | SEQ ID NO:87 | hsa-miR-199b | >hsa-miR-199b MIMAT0000263 |
| UGUGCAAAUCUAUGCAAAACUGA | SEQ ID NO:88 | hsa-miR-19a | >hsa-miR-19a MIMAT0000073 |
| UGUGCAAAUCCAUGCAAAACUGA | SEQ ID NO:89 | hsa-miR-19b | >hsa-miR-19b MIMAT0000074 |
| UAAAGUGCUUAUAGUGCAGGUAG | SEQ ID NO:90 | hsa-miR-20a | >hsa-miR-20a MIMAT0000075 |
| UAACACUGUCUGGUAACGAUGU | SEQ ID NO:91 | hsa-miR-200a | >hsa-miR-200a MIMAT0000682 |
| UAAUACUGCCUGGUAAUGAUGAC | SEQ ID NO:92 | hsa-miR-200b | >hsa-miR-200b MIMAT0000318 |
| UAAUACUGCCGGGUAAUGAUGG | SEQ ID NO:93 | hsa-miR-200c | >hsa-miR-200c MIMAT0000617 |
| GUGAAAUGUUUAGGACCACUAG | SEQ ID NO:94 | hsa-miR-203 | >hsa-miR-203 MIMAT0000264 |
| UUCCCUUUGUCAUCCUAUGCCU | SEQ ID NO:95 | hsa-miR-204 | >hsa-miR-204 MIMAT0000265 |
| UCCUUCAUUCCACCGGAGUCUG | SEQ ID NO:96 | hsa-miR-205 | >hsa-miR-205 MIMAT0000266 |
| UGGAAUGUAAGGAAGUGUGUGG | SEQ ID NO:97 | hsa-miR-206 | >hsa-miR-206 MIMAT0000462 |
| AUAAGACGAGCAAAAAGCUUGU | SEQ ID NO:98 | hsa-miR-208 | >hsa-miR-208 MIMAT0000241 |
| UAGCUUAUCAGACUGAUGUUGA | SEQ ID NO:99 | hsa-miR-21 | >hsa-miR-21 MIMAT00000076 |
| CUGUGCGUGUGACAGCGGCUGA | SEQ ID NO:100 | hsa-miR-210 | >hsa-miR-210 MIMAT0000267 |
| UUCCCUUUGUCAUCCUUCGCCU | SEQ ID NO:101 | hsa-miR-211 | >hsa-miR-211 MIMAT0000268 |
| UAACAGUCUCCAGUCACGGCC | SEQ ID NO: 102 | hsa-miR-212 | >hsa-miR-212 MIMAT0000269 |
| ACCAUCGACCGUUGAUUGUACC | SEQ ID NO:103 | hsa-miR-213 | >hsa-miR-181a* MIMAT0000270 |
| ACAGCAGGCACAGACAGGCAG | SEQ ID NO:104 | hsa-miR-214 | >hsa-miR-214 MIMAT0000271 |
| AUGACCUAUGAAUUGACAGAC | SEQ ID NO:105 | hsa-miR-215 | >hsa-miR-215 MIMAT0000272 |
| UAAUCUCAGCUGGCAACUGUG | SEQ ID NO:106: | hsa-miR-216 | >hsa-miR-216 MIMAT0000273 |
| UACUGCAUCAGGAACUGAUUGGAU | SEQ ID NO:107 | hsa-miR-217 | >hsa-miR-217 MIMAT0000274 |
| UUGUGCUUGAUCUAACCAUGU | SEQ ID NO:108 | hsa-miR-218 | >hsa-miR-218 MIMAT0000275 |
| UGAUUGUCCAAACGCAAUUCU | SEQ ID NO:109 | hsa-miR-219 | >hsa-miR-219 MIMAT000276 |
| AAGCUGCCAGUUGAAGAACUGU | SEQ ID NO:110 | hsa-miR-22 | >hsa-miR-22 MIMAT0000077 |
| CCACACCGUAUCUGACACUUU | SEQ ID NO:111 | hsa-miR-220 | >hsa-miR-220 MIMAT0000277 |
| AGCUACAUUGUCUGCUGGGUUUC | SEQ ID NO:112 | hsa-miR-221 | >hsa-miR-221 MIMAT0000278 |
| AGCUACAUCUGGCUACUGGGUCUC | SEQ ID NO:113 | hsa-miR-222 | >hsa-miR-222 MIMAT0000279 |
| UGUCAGUUUGUCAAAUACCCC | SEQ ID NO:114 | hsa-miR-223 | >hsa-miR-223 MIMAT0000280 |
| CAAGUCACUAGUGGUUCCGUUUA | SEQ ID NO:115 | hsa-miR-224 | >hsa-miR-224 MIMAT0000281 |
| AUCACAUUGCCAGGGAUUUCC | SEQ ID NO:116 | hsa-miR-23a | >hsa-miR-23a MIMAT0000078 |
| AUCACAUUGCCAGGGAUUACC | SEQ ID NO:117 | hsa-miR-23b | >hsa-miR-23b MIMAT0000418 |
| UGGCUCAGUUCAGCAGGAACAG | SEQ ID NO:118 | hsa-miR-24 | >hsa-miR-24 MIMAT0000080 |
| CAUUGCACUUGUCUCGGUCUGA | SEQ ID NO:119 | hsa-miR-25 | >hsa-miR-25 MIMAT0000081 |
| UUCAAGUAAUCCAGGAUAGGC | SEQ ID NO:120 | hsa-miR-26a | >hsa-miR-26a MIMAT0000082 |
| UUCAAGUAAUUCAGGAUAGGUU | SEQ ID NO:121 | hsa-miR-26b | >hsa-miR-26b MIMAT0000083 |
| UUCACAGUGGCUAAGUUCCGC | SEQ ID NO:122 | hsa-miR-27a | >hsa-miR-27a MIMAT0000084 |
| UUCACAGUGGCUAAGUUCUGC | SEQ ID NO:123 | hsa-miR-27b | >hsa-miR-27b MIMAT0000419 |
| AAGGAGCUCACAGUCUAUUGAG | SEQ ID NO:124 | hsa-miR-28 | >hsa-miR-28 MIMAT0000085 |
| AGGGCCCCCCCUCAAUCCUGU | SEQ ID NO:125 | hsa-miR-296 | >hsa-miR-296 MIMAT0000690 |
| UGGUUUACCGUCCCACAUACAU | SEQ ID NO:126 | hsa-miR-299-5p | >hsa-miR-299-Sp MIMAT0002890 |
| UAGCACCAUCUGAAAUCGGUU | SEQ ID NO:127 | hsa-miR-29a | >hsa-miR-29a MIMAT0000086 |
| UAGCACCAUUUGAAAUCAGUGUU | SEQ ID NO:128 | hsa-miR-29b | >hsa-miR-29b MIMAT0000100 |
| UAGCACCAUUUGAAAUCGGU | SEQ ID NO:129 | hsa-miR-29c | >hsa-miR-29c MIMAT0000681 |
| CAGUGCAAUAGUAUUGUCAAAGC | SEQ ID NO:130 | hsa-miR-301 | >hsa-miR-301 MIMAT0000688 |
| UAAGUGCUUCCAUGUUUUGGUGA | SEQ ID NO:131 | hsa-miR-302a | >hsa-miR-302a MIMAT0000684 |
| UAAGUGCUUCCAUGUUUUAGUAG | SEQ ID NO:132 | hsa-miR-302b | >hsa-miR-302b MIMAT0000715 |
| ACUUUAACAUGGAAGUGCUUUCU | SEQ ID NO:133 | hsa-miR-302b-AS | >hsa-miR-302b* MIMAT0000714 |
| UAAGUGCUUCCAUGUUUCAGUGG | SEQ ID NO:134 | hsa-miR-302c | >hsa-miR-302c MIMAT0000717 |
| UUUAACAUGGGGGUACCUGCUG | SEQ ID NO:135 | hsa-miR-302c-AS | >hsa-miR-302c* MIMAT0000716 |
| UAAGUGCUUCCAUGUUUGAGUGU | SEQ ID NO:136 | hsa-miR-302d | >hsa-miR-302d MIMAT0000718 |
| CUUUCAGUCGGAUGUUUGCAGC | SEQ ID NO:137 | hsa-miR-30a-3p | >hsa-miR-30a-3p MIMAT0000088 |
| UGUAAACAUCCUCGACUGGAAG | SEQ ID NO:138 | hsa-miR-30a-5p | >hsa-miR-30a-5p MIMAT0000087 |
| UGUAAACAUCCUACACUCAGCU | SEQ ID NO:139 | hsa-miR-30b | >hsa-miR-30b MIMAT0000420 |
| UGUAAACAUCCUACACUCUCAGC | SEQ ID NO:140 | hsa-miR-30c | >hsa-miR-30c MIMAT0000244 |
| UGUAAACAUCCCCGACUGGAAG | SEQ ID NO:141 | hsa-miR-30d | >hsa-miR-30d MIMAT0000245 |
| CUUUCAGUCGGAUGUUUACAGC | SEQ ID NO:142 | hsa-miR-30e-3p | >hsa-miR-30e-3p MIMAT0000693 |
| UGUAAACAUCCUUGACUGGA | SEQ ID NO:143 | hsa-miR-30e-5p | >hsa-miR-30e-5p MIMAT0000692 |
| GGCAAGAUGCUGGCAUAGCUG | SEQ ID NO:144 | hsa-miR-31 | >hsa-miR-31 MIMAT0000089 |
| UAUUGGACAUUACUAAGUUGC | SEQ ID NO:145 | hsa-miR-32 | >hsa-miR-32 MIMAT0000090 |
| AAAAGCUGGGUUGAGAGGGCGAA | SEQ ID NO:146 | hsa-miR-320 | >hsa-miR-320 MIMAT0000510 |
| GCACAUUACACGGUCGACCUCU | SEQ ID NO:147 | hsa-miR-323 | >hsa-miR-323 MIMAT0000755 |
| CCACUGCCCCAGGUGCUGCUGG | SEQ ID NO: 148 | hsa-miR-324-3p | >hsa-miR-324-3p MIMAT0000762 |
| CGCAUCCCCUAGGGCAUUGGUGU | SEQ ID NO: 149 | hsa-miR-324+-5p | >hsa-miR-324-5p MIMAT0000761 |
| CCUAGUAGGUGUCCAGUAAGUGU | SEQ ID NO:150 | hsa-miR-325 | >hsa-miR-325 MIMAT0000771 |
| CCUCUGGGCCCUUCCUCCAG | SEQ ID NO:151 | hsa-miR-326 | >hsa-miR-326 MIMAT0000756 |
| CUGGCCCUCUCUGCCCUUCCGU | SEQ ID NO:152 | hsa-miR-328 | >hsa-miR-328 MIMAT0000752 |
| GUGCAUUGUAGUUGCAUUG | SEQ ID NO:153 | hsa-miR-33 | >hsa-miR-33 MIMAT0000091 |
| GCAAAGCACACGGCCUGCAGAGA | SEQ ID NO:154 | hsa-miR-330 | >hsa-miR-330 MIMAT0000751 |
| GCCCCUGGGCCUAUCCUAGAA | SEQ ID NO:155 | hsa-miR-331 | >hsa-miR-331 MIMAT0000760 |
| UCAAGAGCAAUAACGAAAAAUGU | SEQ ID NO:156 | hsa-miR-335 | >hsa-miR-335 MIMAT0000765 |
| UCCAGCUCCUAUAUGAUGCCUUU | SEQ ID NO:157 | hsa-miR-337 | >hsa-miR-337 MIMAT0000754 |
| UCCAGCAUCAGUGAUUUUGUUGA | SEQ ID NO:158 | hsa-miR-338 | >hsa-miR-338 MIMAT0000763 |
| UCCCUGUCCUCCAGGAGCUCA | SEQ ID NO:159 | hsa-miR-339 | >hsa-miR-339 MIMAT0000764 |
| UCCGUCUCAGUUACUUUAUAGCC | SEQ ID NO:160- | hsa-miR-340 | >hsa-miR-340 MIMAT0000750 |
| UCUCACACAGAAAUCGCACCCGUC | SEQ ID NO:161 | hsa-miR-342 | >hsa-miR-342 MIMAT0000753 |
| UGCUGACUCCUAGUCCAGGGC | SEQ ID NO:162 | hsa-miR-345 | >hsa-miR-345 MIMAT0000772 |
| UGUCUGCCCGCAUGCCUGCCUCU | SEQ ID NO:163 | hsa-miR-346 | >hsa-miR-346 MIMAT0000773 |
| UGGCAGUGUCUUAGCUGGUUGUU | SEQ ID NO:164 | hsa-miR-34a | >hsa-miR-34a MIMAT0000255 |
| UAGGCAGUGUCAUUAGCUGAUUG | SEQ ID NO:165 | hsa-miR-34b | >hsa-miR-34b MIMAT0000685 |
| AGGCAGUGUAGUUAGCUGAUUGC | SEQ ID NO:166 | hsa-miR-34c | >hsa-miR-34c MIMAT0000686 |
| UUAUCAGAAUCUCCAGGGGUAC | SEQ ID NO:167 | hsa-miR-361 | >hsa-miR-361 MIMAT0000703 |
| UAAUGCCCCUAAAAAUCCUUAU | SEQ ID NO:168 | hsa-miR-365 | >hsa-miR-365 MIMAT0000710 |
| AAUUGCACUUUAGCAAUGGUGA | SEQ ID NO:169 | hsa-miR-367 | >hsa-miR-367 MIMAT0000719 |
| ACAUAGAGGAAAUUCCACGUUU | SEQ ID NO:170 | hsa-miR-368 | >hsa-miR-368 MIMAT0000720 |
| AAUAAUACAUGGUUGAUCUUU | SEQ ID NO:171 | hsa-miR-369-3p | >hsa-miR-369-3p MIMAT0000721 |
| GCCUGCUGGGGUGGAACCUGG | SEQ ID NO:172 | hsa-miR-370 | >hsa-miR-370 MIMAT0000722 |
| GUGCCGCCAUCUUUUGAGUGU | SEQ ID NO:173 | hsa-miR-371 | >hsa-miR-371 MlMAT0000723 |
| AAAGUGCUGCGACAUUUGAGCGU | SEQ ID NO:174 | hsa-miR-372 | >hsa-miR-372 MIMAT0000724 |
| GAAGUGCUUCGAUUUUGGGGUGU | SEQ ID NO:175 | hsa-miR-373 | >hsa-miR-373 MIMAT0000726 |
| ACUCAAAAUGGGGGCGCUUUCC | SEQ ID NO:176 | hsa-miR-373-AS | >hsa-miR-373* |
| UUAUAAUACAACCUGAUAAGUG | SEQ ID NO:177 | hsa-miR-374 | >hsa-miR-374 MlMAT0000727 |
| UUUGUUCGUUCGGCUCGCGUGA | SEQ ID NO:178 | hsa-miR-375 | >hsa-miR-375 MIMAT0000728 |
| AUCAUAGAGGAAAAUCCACGU | SEQ ID NO:179 | hsa-miR-376a | >hsa-miR-376a MIMAT0000729 |
| AUCACACAAAGGCAACUUUUGU | SEQ ID NO:180 | hsa-miR-377 | >hsa-miR-377 MIMAT0000730 |
| CUCCUGACUCCAGGUCCUGUGU | SEQ ID NO:181 | hsa-miR-378 | >hsa-miR-378 MIMAT0000731 |
| UGGUAGACUAUGGAACGUA | SEQ ID NO:182 | hsa-miR-379 | >hsa-miR-379 MIMAT0000733 |
| UAUGUAAUAUGGUCCACAUCUU | SEQ ID NO:183 | hsa-miR-380-3p | >hsa-miR-380-3p MIMAT0000735 |
| UGGUUGACCAUAGAACAUGCGC | SEQ ID NO:184 | hsa-miR-380-5p | >hsa-miR-380-5p MIMAT0000734 |
| UAUACAAGGGCAAGCUCUCUGU | SEQ ID NO:185 | hsa-miR-381 | >hsa-miR-381 MIMAT0000736 |
| GAAGUUGUUCGUGGUGGAUUCG | SEQ ID NO:186 | hsa-miR-382 | >hsa-miR-385 MIMAT0000737 |
| AGAUCAGAAGGUGAUUGUGGCU | SEQ ID NO:187 | hsa-miR-383 | >hsa-miR-383 MIMAT0000738 |
| AUUCCUAGAAAUUGUUCAUA | SEQ ID NO:188 | hsa-miR-384 | >hsa-miR-384 MIMAT0001075 |
| CUGGACUUAGGGUCAGAAGGCC | SEQ ID NO:189 | hsa-miR-422a | MIMAT0001339 |
| CUGGACUUGGAGUCAGAAGGCC | SEQ ID NO:190 | hsa-miR-422b | >hsa-miR-422b MIMAT0000732 |
| AGCUCGGUCUGAGGCCCCUCAG | SEQ ID NO:191 | hsa-miR-423 | >hsa-miR-423 MIMAT0001340 |
| CAGCAGCAAUUCAUGUUUUGAA | SEQ ID NO:192 | hsa-miR-424 | >hsa-miR-424 MIMAT0001341 |
| AUCGGGAAUGUCGUGUCCGCC | SEQ ID NO:193 | hsa-miR-425 | >hsa-miR-425-3p MIMAT0001343 |
| UAAUACUGUCUGGUAAAACCGU | SEQ ID NO:194 | hsa-miR-429 | >hsa-miR-429 MIMAT0001536 |
| UUGCAUAUGUAGGAUGUCCCAU | SEQ ID NO:195 | hsa-miR-448 | >hsa-miR-448 MIMAT0001532 |
| UGGCAGUGUAUUGUUAGCUGGU | SEQ ID NO:196 | hsa-miR-449 | >hsa-miR-449 MIMAT0001541 |
| UUUUUGCGAUGUGUUCCUAAUA | SEQ ID NO:197 | hsa-miR-450 | >hsa-miR-450 MIMAT0001545 |
| UGGAAGACUAGUGAUUUUGUUG | SEQ ID NO:198 | hsa-miR-7 | >hsa-miR-7 MIMAT0000252 |
| UCUUUGGUUAUCUAGCUGUAUGA | SEQ ID NO:199 | hsa-miR-9 | >hsa-miR-9 MIMAT0000441 |
| UAAAGCUAGAUAACCGAAAGU | SEQ ID NO:200 | hsa-miR-9-AS | >hsa-miR-9* MIMAT0000442 |
| UAUUGCACUUGUCCCGGCCUG | SEQ ID NO:201 | hsa-miR-92 | >hsa-miR-92 MIMAT0000092 |
| AAAGUGCUGUUCGUGCAGGUAG | SEQ ID NO:202 | hsa-miR-93 | >hsa-miR-93 MIMAT0000093 |
| UUCAACGGGUAUUUAUUGAGCA | SEQ ID NO:203 | hsa-miR-95 | >hsa-miR-95 MIMAT0000094 |
| UUUGGCACUAGCACAUUUUUGC | SEQ ID NO:204 | hsa-miR-96 | >hsa-miR-96 MIMAT0000095 |
| UGAGGUAGUAAGUUGUAUUGUU | SEQ ID NO:205 | hsa-miR-98 | >hsa-miR-98 MIMAT0000096 |
| AACCCGUAGAUCCGAUCUUGUG | SEQ ID NO: 206 | hsa-miR-99a | >hsa-miR-99a MIMAT0000097 |
| CACCCGUAGAACCGACCUUGCG | SEQ ID NO:207 | hsa-miR-99b | >hsa-miR-99b MIMAT0000689 |
| CUAUACGACCUGCUGCCUUUCU | SEQ ID NO:208 | mmu-let-7d-AS | >mmu-let-7d* MIMAT0000384 |
| UACAGUACUGUGAUAGCUGAAG | SEQ ID NO:209 | mmu-miR-101b | >mmu-miR-101b MIMAT0000616 |
| CAAAGUGCUAACAGUGCAGGUA | SEQ ID NO:210 | mmu-miR-106a | >mmu-miR-106a MIMAT0000385 |
| AAGCCCUUACCCCAAAAAGCAU | SEQ ID NO:211 | mmu-miR-129-3p | >mmu-miR-129-3p MIMAT0000544 |
| UACCACAGGGUAGAACCACGGA | SEQ ID NO:212 | mmu-miR-140-AS | >mmu-miR-140* MIMAT0000152 |
| CUAGACUGAGGCUCCUUGAGG | SEQ ID NO:213 | mmu-miR-151 | >mmu-miR-151 MIMAT0000161 |
| UUAAUGCUAAUUGUGAUAGGGG | SEQ ID NO:214 | mmu-miR-155 | >mmu-miR-155 MIMAT0000165 |
| ACUGCAGUGAGGGCACUUGUA | SEQ ID NO:215 | mmu-miR-17-3p | >mmu-miR-17-3p MIMAT0000650 |
| CUGACCUAUGAAUUGACA | SEQ ID NO:216 | mmu-miR-192 | >mmu-miR-192 MIMAT0000517 |
| CCCAGUGUUUAGACUACCUGUUC | SEQ ID NO:217 | mmu-miR-199b | >mmu-miR-199b MIMAT0000672 |
| UACUCAGUAAGGCAUUGUUCU | SEQ ID NO:218 | mmu-miR-201 | >mmu-miR-201 MIMAT0000234 |
| AGAGGUAUAGCGCAUGGGAAGA | SEQ ID NO:219 | mmu-miR-202 | >mmu-miR-202 MIMAT0000235 |
| GCUUCUCCUGGCUCUCCUCCCUC | SEQ ID NO:220 | mmu-miR-207 | >mmu-miR-207 MIMAT0000240 |
| UUCCCUUUGUCAUCCUUUGCCU | SEQ ID NO:221 | mmu-miR-211 | >mmu-miR-211 MIMAT0000668 |
| AUGACCUAUGAUUUGACAGAC | SEQ ID NO:222 | mmu-miR-215 | >mmu-miR-215 MIMAT0000904 |
| UACUGCAUCAGGAACUGACUGGAU | SEQ ID NO:223 | mmu-miR-217 | >mmu-miR-217 MIMAT0000679 |
| CUCAAACUAUGGGGGCACUUUUU | SEQ ID NO:224 | mmu-miR-290 | >mmu-miR-290 MIMAT0000366 |
| AAAGUGCUUCCACUUUGUGUGCC | SEQ ID NO:225 | mmu-miR-291-3p | >mmu-miR-291a-3p MIMAT0000368 |
| CAUCAAAGUGGAGGCCCUCUCU | SEQ ID NO:226 | mmu-miR-291-5p | >mmu-miR-291a-5p MIMAT0000367 |
| AAGUGCCGCCAGGUUUUGAGUGU | SEQ ID NO:227 | mmu-miR-292-3p | >mmu-miR-292-3p MIMAT0000370 |
| ACUCAAACUGGGGGCUCUUUUG | SEQ ID NO:228 | mmu-miR-292-5p | >mmu-miR-292-5p MIMAT0000369 |
| AGUGCCGCAGAGUUUGUAGUGU | SEQ ID NO:229 | mmu-miR-293 | >mmu-miR-293 MIMAT0000371 |
| AAAGUGCUUCCCUUUUGUGUGU | SEQ ID NO:230 | mmu-miR-294 | >mmu-miR-294 MIMAT0000372 |
| AAAGUGCUACUACUUUUGAGUCU | SEQ ID NO:231 | mmu-miR-295 | >mmu-miR-295 MIMAT0000373 |
| AUGUAUGUGUGCAUGUGCAUG | SEQ ID NO:232 | mmu-miR-297 | >mmu-miR-297 MIMAT0000375 |
| GGCAGAGGAGGGCUGUUCUUCC | SEQ ID NO:233 | mmu-miR-298 | >mmu-miR-298 MIMAT0000376 |
| UAUGCAAGGGCAAGCUCUCUUC | SEQ ID NO:234 | mmu-miR-300 | >mmu-miR-300 MIMAT0000378 |
| AAACAUGAAGCGCUGCAACA | SEQ ID NO:235 | mmu-miR-322 | >mmu-miR-322 MIMAT0000549 |
| CAGCAGCAAUUCAUGUUUUGGA | SEQ ID NO:236 | mmu-miR-424 | >mmu-miR-424 MIMAT0000548 |
| CCUAGUAGGUGCUCAGUAAGUGU | SEQ ID NO:237 | mmu-miR-325 | >mmu-miR-325 MIMAT0000558 |
| AACACACCCAGCUAACCUUUUU | SEQ ID NO:238 | mmu-miR-329 | >mmu-miR-329 MIMAT0000567 |
| GCAAAGCACAGGGCCUGCAGAGA | SEQ ID NO:239 | mmu-miR-330 | >mmu-miR-330 MIMAT0000569 |
| UUCAGCUCCUAUAUGAUGCCUUU | SEQ ID NO:240 | mmu-miR-337 | >mmu-miR-337 MIMAT0000578 |
| UCGAUCGGUCGGUCGGUCAGU | SEQ ID NO:241 | mmu-miR-341 | >mmu-miR-341 MIMAT0000588 |
| UGAUCUAGCCAAAGCCUGACUGU | SEQ ID NO:242 | mmu-miR-344 | >mmu-miR-344 MIMAT0000593 |
| UGCUGACCCCUAGUCCAGUGC | SEQ ID NO:243 | mmu-miR-345 | >mmu-miR-345 MIMAT0000595 |
| UGUCUGCCCGAGUGCCUGCCUCU | SEQ ID NO:244 | mmu-miR-346 | >mmu-miR-346 MIMAT0000597 |
| UAGGCAGUGUAAUUAGCUGAUUG | SEQ ID NO:245 | mmu-miR-34b | >mmu-miR-34b MIMAT0000382 |
| UUCACAAAGCCCAUACACUUUCA | SEQ ID NO:246 | mmu-miR-350 | >mmu-miR-350 MIMAT0000605 |
| UCCCUGAGGAGCCCUUUGAGCCUG | SEQ ID NO:247 | mmu-miR-351 | >mmu-miR-351 MIMAT0000609 |
| AUCGUAGAGGAAAAUCCACGU | SEQ ID NO:248 | mmu-miR-376a | >mmu-miR-376a MIMAT0000740 |
| AUCAUAGAGGAACAUCCACUUU | SEQ ID NO:249 | mmu-miR-376b | >mmu-miR-376b MIMAT0001092 |
| UAUGUAGUAUGGUCCACAUCUU | SEQ ID NO:250 | mmu-miR-380-3p | >mmu-miR-380-3p MIMAT0000745 |
| AGAUCAGAAGGUGACUGUGGCU | SEQ ID NO:251 | mmu-miR-383 | >mmu-miR-383 MIMAT0000748 |
| AUUCCUAGAAAUUGUUCACA | SEQ ID NO:252 | mmu-miR-384 | >mmu-miR-384 MIMAT0001076 |
| GAAUGUUGCUCGGUGAACCCCUU | SEQ ID NO:253 | mmu-miR-409 | >mmu-miR-409 MIMAT0001090 |
| AAUAUAACACAGAUGGCCUGU | SEQ ID NO:254 | hsa-miR-410 | >hsa-miR-410 MIMAT0002171 |
| AACACGGUCCACUAACCCUCAGU | SEQ ID NO:255 | mmu-miR-411 | >mmu-miR-411 MIMAT0001093 |
| ACUUCACCUGGUCCACUAGCCGU | SEQ ID NO:256 | hsa-miR-412 | >hsa-miR-412 MIMAT0002170 |
| UAAUACUGUCUGGUAAUGCCGU | SEQ ID NO:257 | mmu-miR-429 | >mmu-miR-429 MIMAT0001537 |
| UGGAAGACUUGUGAUUUUGUU | SEQ ID NO:258 | mmu-miR-7b | >mmu-miR-7b MIMAT0000678 |
| UCGAGGAGCUCACAGUCUAGUA | SEQ ID NO:259 | rno-miR-151-AS | >rno-miR-151* MIMAT0000613 |
| ACUGCAUUACGAGCACUUACA | SEQ ID NO:260 | rno-miR-20-AS | >rno-miR-20a* MIMAT0000603 |
| AUGUAUGUGUGCAUGUAUGCAUG | SEQ ID NO:261 | rno-miR-297 | >rno-miR-297 MIMAT0000899 |
| CCUUGAGGGGCAUGAGGGU | SEQ ID NO:262 | rno-miR-327 | >rno-miR-327 MIMAT0000561 |
| GUGGUGUGCUAGUUACUUUU | SEQ ID NO:263 | rno-miR-333 | >rno-miR-333 MIMAT0000572 |
| UCACCCUUCCAUAUCUAGUCU | SEQ ID N0:264 | rno-miR-336 | >rno-miR-336 MIMAT0000576 |
| UCUCCCUCCGUGUGCCCAGA | SEQ ID NO:265 | rno-miR-343 | >rno-miR-343 MIMAT0000591 |
| UGAUCUAGCCAAAGCCUGACCGU | SEQ ID NO:266 | rno-miR-344 | >rno-miR-344 MIMAT0000592 |
| UGUCUGCCUGAGUGCCUGCCUCU | SEQ ID NO:267 | rno-miR-346 | >rno-miR-346 MIMAT0000596 |
| UGUCCCUCUGGGUCGCCCA | SEQ ID NO:268 | rno-miR-347 | >rno-miR-347 MIMAT0000598 |
| CAGCCCUGCUGUCUUAACCUCU | SEQ ID NO:269 | rno-miR-349 | >rno-miR-349 MIMAT0000599 |
| AGAGUAGUAGGUUGCAUAGUA | SEQ ID NO:270 | rno-miR-352 | >rno-miR-352 MIMAT0000610 |
| GGCCUCAUUAAAUGUUUGUUG | SEQ ID NO:271 | rno-miR-421 | >rno-miR-421 MIMAT0001320 |
| CAACAAAUCACAGUCUGCCAUA | SEQ ID NO:272 | rno-miR-7-AS | >mo-miR-7* MIMAT0000607 |
| AAAAUGGUUCCCUUUAGAGUGUU | SEQ ID NO:273 | hsa-miR-522 | >hsa-miR-522 MIMAT0002868 |
| AAAGUGCAUCCUUUUAGAGGUUU | SEQ ID NO:274 | hsa-miR-519b | >hsa-miR-519b MIMAT0002837 |
| AAAGUGCUUCCUUUUAGAGGGUU | SEQ ID NO:275 | hsa-miR-520c | >bsa-miR-520c MIMAT0002846 |
| AAAGUGCCUCCUUUUAGAGUGU | SEQ ID NO:276 | hsa-miR-519e | >hsa-miR-519e MIMAT0002829 |
| CAAAGUGCCUCCCUUUAGAGUGU | SEQ ID NO:277 | hsa-miR-519d | >hsa-miR-519d MIMAT0002853 |
| AAAGUGCUUCCUUUUAGAGGG | SEQ ID NO:278 | hsa-miR-520b | >hsa-miR-520b MIMAT0002843 |
| AAAGUGCAUCUUUUUAGAGGAU | SEQ ID NO:279 | hsa-miR-519c | >hsa-miR-519c MIMAT0002832 |
| AAAGUGCUUCCUUUUAGAGGC | SEQ ID NO:280 | hsa-miR-526b-AS | >hsa-miR-526b* MIMAT0002836 |
| AAAGUGCUUCCUUUUUGAGGG | SEQ ID NO:281 | hsa-miR-520e | >hsa-miR-520e MIMAT0002825 |
| AAAGUGCUUCCCUUUGGACUGU | SEQ ID NO:282 | hsa-miR-520a | >hsa-miR-520a MIMAT0002834 |
| AAAGUGCUUCUCUUUGGUGGGUU | SEQ ID NO:283 | hsa-miR-520d | >hsa-miR-520d MIMAT0002856 |
| ACAAAGUGCUUCCCUUUAGAGU | SEQ ID NO:284 | hsa-miR-520h | >hsa-miR-520h MIMAT0002867 |
| AUCGUGCAUCCCUUUAGAGUGUU | SEQ ID NO:285 | hsa-miR-517a | >hsa-miR-517a MIMAT0002852 |
| AAAGCGCUUCCCUUCAGAGUGU | SEQ ID NO:286 | hsa-miR-518e | >hsa-miR-518e MIMAT0002861 |
| AACGCACUUCCCUUUAGAGUGU | SEQ ID NO:287 | hsa-miR-521 | >hsa-miR-521 MIMAT0002854 |
| AACGCGCUUCCCUAUAGAGGG | SEQ ID NO:288 | hsa-miR-523 | >hsa-miR-523 MMAT0002940 |
| AAAGCGCUUCUCUUUAGAGGA | SEQ ID NO:289 | hsa-miR-518f | >hsa-miR-518f MIMAT0002842 |
| CAAAGCGCUUCUCUUUAGAGUG | SEQ ID NO:290 | hsa-miR-518c | >hsa-miR-518c MIMAT0002848 |
| CAAAGCGCUCCCCUUUAGAGGU | SEQ ID NO:291 | hsa-miR-518b | >hsa-miR-518b MIMAT0002844 |
| CAAAGCGCUUCCCUUUGGAGC | SEQ ID NO:292 | hsa-miR-518d | >hsa-miR-518d MIMAT0002864 |
| GAAGGCGCUUCCCUUUAGAGC | SEQ ID NO:293 | hsa-miR-525-AS | >hsa-miR-525* MIMAT0002839 |
| GAAGGCGCUUCCCUUUGGAGU | SEQ ID NO:294 | hsa-miR-524 | >hsa-miR-524 MIMAT0002850 |
| AAAGCGCUUCCCUUUGCUGGA | SEQ ID NO:295 | hsa-miR-518a | >hsa-miR-518a MIMAT0002863 |
| GAGUGCCUUCUUUUGGAGCGU | SEQ ID NO:296 | hsa-miR-515-3p | >hsa-miR-515-3p MIMAT0002827 |
| UGCUUCCUUUCAGAGGGU | SEQ ID NO:297 | hsa-miR-516-3p | >hsa-miR-516-3p MIMAT0002860 |
| AAGUGCUGUCAUAGCUGAGGUC | SEQ ID NO:298 | hsa-miR-512-3p | >hsa-miR-512-3p MIMAT0002823 |
| GGAAACCGUUACCAUUACUGAGU | SEQ ID NO:299 | ambi-miR-7029 | |
| AGUGGGGAACCCUUCCAUGAGGA | SEQ ID NO:300 | hsa-miR-491 | >hsa-miR-491 MIMAT0002807 |
| UAAGGCACCCUUCUGAGUAGA | SEQ ID NO:301 | hsa-miR-506 | >hsa-miR-506 MIMAT0002878 |
| AUUGACACUUCUGUGAGUAG | SEQ ID NO:302 | hsa-miR-514 | >hsa-miR-514 MIMAT0002883 |
| UGAUUGGUACGUCUGUGGGUAGA | SEQ ID NO:303 | hsa-miR-509 | >hsa-miR-509 MIMAT0002881 |
| UGAUUGUAGCCUUUUGGAGUAGA | SEQ ID NO:304 | hsa-miR-508 | >hsa-miR-508 MIMAT0002880 |
| UUUUGCACCUUUUGGAGUGAA | SEQ ID NO:305 | hsa-miR-507 | >hsa-miR-507 MIMAT0002879 |
| AACUGGCCCUCAAAGUCCCGCUUU | SEQ ID NO:306 | hsa-miR-193b | >hsa-miR-193b MIMAT0002819 |
| GCCGAGACUAGAGUCACAUCCUG | SEQ ID NO:307 | ambi-miR-7039 | |
| CCCAGAUAAUGGCACUCUCAA | SEQ ID NO:308 | hsa-miR-488 | >hsa-miR-488 MIMAT0002804 |
| UACUCAGGAGAGUGGCAAUCACA | SEQ ID NO:309 | hsa-miR-510 | >hsa-miR-510 MIMAT0002882 |
| CCUCUAGAUGGAAGCACUGUCU | SEQ ID NO:310 | hsa-miR-517-AS | >hsa-miR-517* MIMAT0002851 |
| CUCUAGAGGGAAGCACUUUCUCU | SEQ ID NO:311 | hsa-miR-518f-AS | >hsa-miR-518f* MIMAT0002841 |
| UCUCUGGAGGGAAGCACUUUCUG | SEQ ID NO:312 | hsa-miR-518c-AS | >hsa-miR-518c* MIMAT0002847 |
| CUCUAGAGGGAAGCGCUUUCUGUU | SEQ ID NO:313 | hsa-miR-526c | >hsa-miR-526c MIMAT0002831 |
| CUCUUGAGGGAAGCACUUUCUGUU | SEQ ID NO:314 | hsa-miR-526b | >hsa-miR-526b MIMAT0002835 |
| CUCCAGAGGGAAGUACUUUCU | SEQ ID NO:315 | hsa-miR-520a-AS | >hsa-miR-520a* MIMAT0002833 |
| CUCCAGAGGGAUGCACUUUCU | SEQ ID NO:316 | hsa-miR-525 | >hsa-miR-525 MIMAT0002838 |
| CUACAAAGGGAAGCACUUUCUC | SEQ ID NO:317 | hsa-miR-524-AS | >hsa-miR-524* MIMAT0002849 |
| UCUACAAAGGGAAGCCCUUUCUG | SEQ ID NO:318 | hsa-miR-520d-AS | >hsa-miR-520d* MIMAT0002855 |
| CUGCAAAGGGAAGCCCUUUCU | SEQ ID NO:319 | hsa-miR-527 | >hsa-miR-527 MIMAT0002862 |
| UUCUCCAAAAGAAAGCACUUUCUG | SEQ ID NO:320 | hsa-miR-515-5p | >hsa-miR-515-5p MIMAT0002826 |
| UUCUCCAAAAGGGAGCACUUUC | SEQ ID NO:321 | hsa-miR-519e-AS | >hsa-miR-519e* MIMAT0002828 |
| UUUCAAGCCAGGGGGCGUUUUUC | SEQ ID No:322 | hsa-miR-498 | >hsa-miR-498 MIMAT0002824 |
| UUCACAGGGAGGUGUCAUUUAU | SEQ ID NO:323 | hsa-miR-513 | >hsa-miR-513 MIMAT0002877 |
| UGAGCGGCAGAGAGCGAGACUUU | SEQ ID NO:324 | ambi-miR-7058 | |
| UGUUUGCAGAGGAAACUGAGAC | SEQ ID NO:325 | hsa-miR-452 | >hsa-miR-452 MIMAT0001635 |
| UUGUACAUGGUAGGCUUUCAUU | SEQ ID NO:326 | hsa-miR-493 | >hsa-miR-493-5p MIMAT0002813 |
| UCUUGGAGUAGGUCAUUGGGUGG | SEQ ID NO:327 | hsa-miR-432 | >hsa-miR-432 MIMAT0002814 |
| AAACAAACAUGGUGCACUUCUUU | SEQ ID NO:328 | hsa-miR-495 | >hsa-miR-495 MIMAT0002817 |
| UGAAACAUACACGGGAAACCUCUU | SEQ ID NO:329 | hsa-miR-494 | >hsa-miR-494 MIMAT0002816 |
| AUUACAUGGCCAAUCUC | SEQ ID NO:330 | hsa-miR-496 | >hsa-miR-496 MIMAT0002818 |
| AGGACCUGCGGGACAAGAUUCUU | SEQ ID NO:331 | hsa-miR-492 | >hsa-miR-492 MIMAT0002812 |
| CAACCUGGAGGACUCCAUGCUG | SEQ ID NO:332 | hsa-miR-490 | >hsa-miR-490 MIMAT0002806 |
| CAGCAGCACACUGUGGUUUGU | SEQ ID NO:333 | hsa-miR-497 | >hsa-miR-497 MIMAT0002820 |
| AAUCCUUGGAACCUAGGUGUGAGU | SEQ ID NO:334 | ambi-miR-7076 | >hsa-miR-362 MIMAT0000705 |
| AAUCCUUUGUCCCUGGGUGAGA | SEQ ID NO:335 | hsa-miR-501 | >hsa-miR-501 MIMAT0002872 |
| AUCCUUGCUAUCUGGGUGCUA | SEQ ID NO:336 | hsa-miR-502 | >hsa-miR-502 MIMAT0002873 |
| UUUCCUAUGCAUAUACUUCUUU | SEQ ID NO:337 | lisa-miR-202-AS | >hsa-miR-202* MIMAT0002810 |
| GCAGUCCAUGGGCAUAUACAC | SEQ ID NO:338 | ambi-miR-7083 | |
| CACUCAGCCUUGAGGGCACUUUC | SEQ ID NO:339 | hsa-miR-512-5p | >hsa-miR-512-5p MIMAT0002822 |
| AGACCCUGGUCUGCACUCUAU | SEQ ID NO:340 | hsa-miR-504 | >hsa-miR-504 MIMAT0002875 |
| GUGUCUUUUGCUCUGCAGUCA | SEQ ID NO:341 | hsa-miR-511 | >hsa-miR-511 MIMAT0002808 |
| UCAGUCUCAUCUGCAAAGAAG | SEQ ID NO:342 | hsa-miR-452-AS | >hsa-miR-452* MIMAT0001636 |
| UAGCAGCGGGAACAGUUCUGCAG | SEQ ID NO:343 | hsa-miR-503 | >hsa-miR-503 MIMAT0002874 |
| AGAGGCUGGCCGUGAUGAAUUC | SEQ ID NO:344 | hsa-miR-485-5p | >hsa-miR-485-5p MIMAT0002175 |
| UUAAGACUUGCAGUGAUGUUUAA | SEQ ID NO:345 | hsa-miR-499 | >hsa-miR-499 MIMAT0002870 |
| GUCAACACUUGCUGGUUUCCUC | SEQ ID NO:346 | hsa-miR-505 | >hsa-miR-505 MIMAT0002876 |
| AGUGACAUCACAUAUACGGCAGC | SEQ ID NO:347 | hsa-miR-489 | >hsa-miR-489 MIMAT0002805 |
| CUGGAUGGCUCCUCCAUGUCU | SEQ ID NO:348 | hsa-miR-432-AS | >hsa-miR-432* MIMAT0002815 |
| AUGCACCUGGGCAAGGAUUCUG | SEQ ID NO:349 | hsa-miR-500 | >hsa-miR-500 MIMAT0002871 |
| UCCCCCAGGUGUGAUUCUGAUUU | SEQ ID NO:350 | ambi-miR-7105 | |

Other embodiments of the invention are discussed throughout this application. Any embodiment discussed with respect to one aspect of the invention applies to other aspects of the invention as well and vice versa. The embodiments in the Example section are understood to be embodiments of the invention that are applicable to all aspects of the invention.

The terms "inhibiting," "reducing," or "prevention," or any variation of these terms, when used in the claims and/or the specification includes any measurable decrease or complete inhibition to achieve a desired result.

The use of the word "a" or "an" when used in conjunction with the term "comprising" in the claims and/or the specification may mean "one," but it is also consistent with the meaning of "one or more," "at least one," and "one or more than one."

It is contemplated that any embodiment discussed herein can be implemented with respect to any method or composition of the invention, and vice versa. Any embodiment discussed with respect to a particular pancreatic disorder can be applied or implemented with respect to a different pancreatic disorder. Furthermore, compositions and kits of the invention can be used to achieve methods of the invention.

Throughout this application, the term "about" is used to indicate that a value includes the standard deviation of error for the device or method being employed to determine the value.

The use of the term "or" in the claims is used to mean "and/or" unless explicitly indicated to refer to alternatives only or the alternatives are mutually exclusive, although the disclosure supports a definition that refers to only alternatives and "and/or."

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

### DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.
**FIG. 1.** Integrity of total RNA from pancreatic tissues. About 1 µg of total RNA isolated from each of 24 individual pancreatic tissue samples was analyzed on a 1% denaturing formaldehyde agarose gel stained with ethidium bromide.
**FIG. 2.** Characterization of the expressed miRNome in normal pancreatic tissues. After global normalization of the raw array data from the 38 samples, the average miRNA expression in the 5 normal pancreatic tissues (N) was compared against the average miRNA expression in the 33 tissue reference set (Ref).
**FIG. 3.** Comparison of the expressed miRNome in normal and PDAC tissues. After normalization of the raw array data, the average miRNA expression in the 5 normal pancreatic tissues (N) was compared against the average miRNA expression in the 8 PDAC samples (Ca).
**FIGs. 4A and 4B.** miRNAs differentially expressed in normal (N), chronic pancreatitis (Ch) and PDAC samples (Ca). (**FIG. 4A**) Venn diagrams illustrating the relationships between sets of differentially expressed miRNAs. Circles include the total number of differentially expressed miRNAs in the direct pair-wise comparison indicated. Intersection areas correspond to the number of differentially expressed miRNAs shared between each comparison. (**FIG. 4B**) Principal Component Analysis on the 94 differentially expressed miRNAs from Table 6.
**FIG. 5.** Top 20 miRNAs differentially expressed in normal and diseased pancreatic tissues. miRNA candidates were selected for a |Δh|>1.6 and p-value<0.0001 between at least 2 out of the 3 primary tissue types. The graph shows mean normalized expression values and standard deviations for the 20 indicated miRNAs in the 3 sample types. (N), normal; (Ch), chronic pancreatitis; (Ca), PDAC.
**FIG. 6.** Principal Component Analysis of miRNAs differentially expressed in normal (N), chronic pancreatitis (Ch), PDAC (Ca) and cell line (CL) samples.
**FIG. 7.** miRNAs over-expressed in PDAC and cell line samples. Individual normalized miRNA expression levels and associated p-values in 5 normal (N), 6 chronic pancreatic (Ch), 8 PDAC (Ca) and 6 cell lines (CL) samples.
**FIG. 8.** Comparison between array and qRT-PCR data. Real time RT-PCR were performed using 25 ng of total RNA input from the 19 tissue samples previously profiled plus 2 normal (N), 2 PDAC (Ca) and 1 chronic pancreatic (Ch) samples. miRNA expression data obtained with primer sets specific for the indicated miRNAs were normalized to 5S rRNA expression level for each sample (miRNA Ct - 5S Ct). The graphs show the individual normalized miRNA expression levels determined by array (19 samples) or qRT-PCR (24 samples) method, and associated p-values.
**FIGs. 9A and 9B.** Expression of miR-196a and miR-217 classifies normal and diseased pancreatic tissues. (**FIG. 9A**) Real time RT-PCR were performed with primer sets specific for miR-196a and -217 using the indicated total RNA input amount from one normal (N5), one PDAC (Ca3) or one chronic pancreatic (Chl) sample. Raw Ct values were directly used to calculate the ratio of miR-196a to miR-217 expression, *i*.*e*., miR-196a Ct - miR217 Ct in the logarithmic space. (**FIG. 9B**) Same study as in (**FIG. 9A**) with the indicated 24 individual tissue samples and 25 ng of total RNA input.
**FIG. 10.** Expression of miR-196a and miR-217 classifies normal and diseased pancreatic tissues. TaqMan® MicroRNA Assays (Applied Biosystems; Foster City, CA, USA) were performed on a subset of 20 frozen pancreatic tissue samples (6 N, 6 Ch and 8 Ca, Example 1). qRT-PCR reactions utilized 10 ng RNA input. RT reactions were carried out using random primers, while for PCR reactions gene-specific priming was used. The graph shows the raw Ct difference between miR-196a and miR-217 in individual samples and the associated p-value (ANOVA).
**FIGs. 11A. and 11B. (FIG. 11A.)** qRT-PCR expression data for four genes reported in the literature as differentially expressed between normal pancreas, chronic pancreatitis and pancreatic adenocarcinoma. The same sample set as in **Fig. 10** (20 frozen pancreatic tissue samples; 6 N, 6 Ch and 8 Ca) was interrogated using TaqMan® Gene Expression Assays (Applied Biosystems). qRT-PCR reactions were performed with random priming for RT reactions and gene-specific priming for PCR reactions, using 5 ng of total RNA input. For each sample, mRNA expression data were normalized to the expression of GAPDH (mRNA-X Ct - GAPDH Ct). The graphs show the individual normalized mRNA expression levels determined by qRT-PCR and associated p-values (ANOVA). **(FIG. 11B.)** Combinations of miR-196a, miR-217 and mRNA gene expression signatures improve segregation of normal pancreas, chronic pancreatitis and pancreatic cancer. These graphs show separation between the experimental groups achieved through combining the individual Ct values for markers normalized to miR-24 (miRNAs) or GAPDH (mRNAs), as well as associated p-values (ANOVA).
**FIG. 12.** A comparison of differential expression data for six miRNA and two mRNA genes between frozen pancreatic tissue samples (6 N, 6C h and 8 Ca) and pancreatic fine needle aspirates (FNAs, n=13). Ten FNA samples were pathologically described as PDAC (Ca FNA, solid diamonds). Of the remaining three samples (FNA), two had a non-definitive pathological description (FNA-8- solid circle and FNA-13- star), and one was consistent with neoplasia of endocrine pancreas (FNA-12, solid triangle). Real time RT-PCR reactions were performed and normalized as in FIGs. 10 and 11.
**FIG. 13.** Performance comparison of combinations of miR-196a, miR-217 and mRNA gene expression signatures between frozen normal pancreas, chronic pancreatitis, pancreatic cancer, pancreatic cancer FNAs (Ca FNAs, solid diamonds) and other FNAs (FNAs, FNA-8- solid circle, FNA-12- solid triangle and FNA-13-star). The graphs show the separation between the experimental groups achieved using the combination of the individual miRNA/mRNA expression signatures normalized to miR-24 (miRNAs) or GAPDH (mRNAs).

### DETAILED DESCRIPTION OF THE INVENTION

The present invention is directed to compositions and methods relating to preparation and characterization of miRNAs, as well as use of miRNAs for therapeutic, prognostic, and diagnostic applications, particularly those methods and compositions related to assessing and/or identifying pancreatic disease, as limited by the claims.

### .I miRNA Molecules

MicroRNA molecules ("miRNAs") are generally 21 to 22 nucleotides in length, though lengths of 19 and up to 23 nucleotides have been reported. The miRNAs are each processed from a longer precursor RNA molecule ("precursor miRNA"). Precursor miRNAs are transcribed from non-protein-encoding genes. The precursor miRNAs have two regions of complementarity that enables them to form a stem-loop- or fold-back-like structure, which is cleaved in animals by a ribonuclease III-like nuclease enzyme called Dicer. The processed miRNA is typically a portion of the stem.

The processed miRNA (also referred to as "mature miRNA") become part of a large complex to down-regulate a particular target gene. Examples of animal miRNAs include those that imperfectly basepair with the target, which halts translation (Olsen *et al.,* 1999; Seggerson *et al.,* 2002). siRNA molecules also are processed by Dicer, but from a long, double-stranded RNA molecule. siRNAs are not naturally found in animal cells, but they can direct the sequence-specific cleavage of an mRNA target through a RNA-induced silencing complex (RISC) (Denli *et al.,* 2003).

### .A Nucleic Acids

The present invention concerns miRNAs that can be labeled, used in array analysis, or employed in diagnostic, therapeutic, or prognostic applications, particularly those related to pathological conditions of the pancreas, as limited by the claims. The RNA may have been endogenously produced by a cell, or been synthesized or produced chemically or recombinantly. They may be isolated and/or purified. The term "miRNA," unless otherwise indicated, refers to the processed RNA, after it has been cleaved from its precursor. Table 1 indicates which SEQ ID NO correspond to the mature sequence. The name of the miRNA is often abbreviated and referred to without a hsa-, mmu-, or rno- prefix and will be understood as such, depending on the context. Unless otherwise indicated, miRNAs referred to in the application are human sequences identified as miR-X or let-X, where X is a number and/or letter.

In certain experiments, a miRNA probe designated by a suffix "5P" or "3P" can be used. "5P" indicates that the mature miRNA derives from the 5' end of the precursor and a corresponding "3P" indicates that it derives from the 3' end of the precursor, as described on the World Wide Web at sanger.ac.uk. Moreover, in some embodiments, a miRNA probe is used that does not correspond to a known human miRNA. It is contemplated that these non-human miRNA probes may be used in embodiments of the invention or that there may exist a human miRNA that is homologous to the non-human miRNA. While the invention is not limited to human miRNA, in certain embodiments, miRNA from human cells or a human biological sample is evaluated. In other embodiments, any mammalian cell, biological sample, or preparation thereof may be employed.

In some embodiments of the invention, methods and compositions involving miRNA may concern miRNA and/or other nucleic acids, as limited by the claims. Nucleic acids may be, be at least, or be at most 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 441, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000 nucleotides, or any range derivable therein, in length. Such lengths cover the lengths of processed miRNA, miRNA probes, precursor miRNA, miRNA containing vectors, control nucleic acids, and other probes and primers. In many embodiments, miRNA are 19-24 nucleotides in length, while miRNA probes are 19-35 nucleotides in length, depending on the length of the processed miRNA and any flanking regions added. miRNA precursors are generally between 62 and 110 nucleotides in human s.

Nucleic acids of the invention may have regions of identity or complementarity to another nucleic acid. It is contemplated that the region of complementarity or identity can be at least 5 contiguous residues, though it is specifically contemplated that the region is, is at least, or is at most 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 441, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000 contiguous nucleotides. It is further understood that the length of complementarity within a precursor miRNA or between a miRNA probe and a miRNA or a miRNA gene are such lengths. Moreover, the complementarity may be expressed as a percentage, meaning that the complementarity between a probe and its target is 90% or greater over the length of the probe. In some embodiments, complementarity is or is at least 90%, 95% or 100%, as limited by the claims. In particular, such lengths may be applied to any nucleic acid comprising a nucleic acid sequence identified in any of SEQ ID NO:1 through. SEQ ID NO:350 or any other sequence disclosed herein. Each of these SEQ ID NOs is disclosed herein. The commonly used name of the miRNA is given (with its identifying source in the prefix, for example, "hsa" for human sequences) and the processed miRNA sequence. Unless otherwise indicated, a miRNA without a prefix will be understood to refer to a human miRNA. A miRNA designated, for example, as miR-1-2 in the application will be understood to refer to hsa-miR-1-2. Moreover, a lowercase letter in the table below may or may not be lowercase; for example, hsa-mir-130b can also be referred to as miR-130B. In addition, miRNA sequences with a "mu" or "mmu" sequence will be understood to refer to a mouse miRNA and miRNA sequences with a "rno" sequence will be understood to refer to a rat miRNA. The term "miRNA probe" refers to a nucleic acid probe that can identify a particular miRNA or structurally related miRNAs.

It is understood that a miRNA is derived from genomic sequences or a gene. In this respect, the term "gene" is used for simplicity to refer to the genomic sequence encoding the precursor miRNA for a given miRNA. However, embodiments of the invention may involve genomic sequences of a miRNA that are involved in its expression, such as a promoter or other regulatory sequences.

The term "recombinant" may be used and this generally refers to a molecule that has been manipulated *in vitro* or that is a replicated or expressed product of such a molecule.

The term "nucleic acid" is well known in the art. A "nucleic acid" as used herein will generally refer to a molecule (one or more strands) of DNA, RNA or a derivative or analog thereof, comprising a nucleobase. A nucleobase includes, for example, a naturally occurring purine or pyrimidine base found in DNA (*e*.*g*., an adenine "A," a guanine "G," a thymine "T" or a cytosine "C") or RNA (*e.g*., an A, a G, an uracil "U" or a C). The term "nucleic acid" encompasses the terms "oligonucleotide" and "polynucleotide," each as a subgenus of the term "nucleic acid."

The term "miRNA" generally refers to a single-stranded molecule, but in specific embodiments, molecules implemented in the invention will also encompass a region or an additional strand that is partially (between 10 and 50% complementary across length of strand), substantially (greater than 50% but less than 100% complementary across length of strand) or fully complementary to another region of the same single-stranded molecule or to another nucleic acid. Thus, nucleic acids may encompass a molecule that comprises one or more complementary or self-complementary strand(s) or "complement(s)" of a particular sequence comprising a molecule. For example, precursor miRNA may have a self-complementary region, which is up to 100% complementary. miRNA probes or nucleic acids of the invention can include, can be or can be at least 60, 65, 70, 75, 80, 85, 90, 95, 96, 97, 98, 99 or 100% complementary to their target, as limited by the claims.

As used herein, "hybridization", "hybridizes" or "capable of hybridizing" is understood to mean the forming of a double or triple stranded molecule or a molecule with partial double or triple stranded nature. The term "anneal" as used herein is synonymous with "hybridize." The term "hybridization", "hybridize(s)" or "capable of hybridizing" encompasses the terms "stringent condition(s)" or "high stringency" and the terms "low stringency" or "low stringency condition(s)."

As used herein "stringent condition(s)" or "high stringency" are those conditions that allow hybridization between or within one or more nucleic acid strand(s) containing complementary sequence(s), but preclude hybridization of random sequences. Stringent conditions tolerate little, if any, mismatch between a nucleic acid and a target strand. Such conditions are well known to those of ordinary skill in the art, and are preferred for applications requiring high selectivity. Non-limiting applications include isolating a nucleic acid, such as a gene or a nucleic acid segment thereof, or detecting at least one specific mRNA transcript or a nucleic acid segment thereof, and the like.

Stringent conditions may comprise low salt and/or high temperature conditions, such as provided by about 0.02 M to about 0.5 M NaCl at temperatures of about 42°C to about 70°C. It is understood that the temperature and ionic strength of a desired stringency are determined in part by the length of the particular nucleic acid(s), the length and nucleobase content of the target sequence(s), the charge composition of the nucleic acid(s), and to the presence or concentration of formamide, tetramethylammonium chloride or other solvent(s) in a hybridization mixture.

It is also understood that these ranges, compositions and conditions for hybridization are mentioned by way of non-limiting examples only, and that the desired stringency for a particular hybridization reaction is often determined empirically by comparison to one or more positive or negative controls. Depending on the application envisioned it is preferred to employ varying conditions of hybridization to achieve varying degrees of selectivity of a nucleic acid towards a target sequence. In a non-limiting example, identification or isolation of a related target nucleic acid that does not hybridize to a nucleic acid under stringent conditions may be achieved by hybridization at low temperature and/or high ionic strength. Such conditions are termed "low stringency" or "low stringency conditions," and non-limiting examples of low stringency include hybridization performed at about 0.15 M to about 0.9 M NaCl at a temperature range of about 20°C to about 50°C. Of course, it is within the skill of one in the art to further modify the low or high stringency conditions to suite a particular application.

### .1 Nucleobases

As used herein a "nucleobase" refers to a heterocyclic base, such as for example a naturally occurring nucleobase (*i*.*e*., an A, T, G, C or U) found in at least one naturally occurring nucleic acid (*i*.*e*., DNA and RNA), and naturally or non-naturally occurring derivative(s) and analogs of such a nucleobase. A nucleobase generally can form one or more hydrogen bonds ("anneal" or "hybridize") with at least one naturally occurring nucleobase in manner that may substitute for naturally occurring nucleobase pairing (*e*.*g*., the hydrogen bonding between A and T, G and C, and A and U).

"Purine" and/or "pyrimidine" nucleobase(s) encompass naturally occurring purine and/or pyrimidine nucleobases and also derivative(s) and analog(s) thereof, including but not limited to, those a purine or pyrimidine substituted by one or more of an alkyl, caboxyalkyl, amino, hydroxyl, halogen (*i*.*e*., fluoro, chloro, bromo, or iodo), thiol or alkylthiol moiety. Preferred alkyl (*e*.*g*., alkyl, caboxyalkyl, *etc*.) moieties comprise of from about 1, about 2, about 3, about 4, about 5, to about 6 carbon atoms. Other non-limiting examples of a purine or pyrimidine include a deazapurine, a 2,6-diaminopurine, a 5-fluorouracil, a xanthine, a hypoxanthine, a 8-bromoguanine, a 8-chloroguanine, a bromothymine, a 8-aminoguanine, a 8-hydroxyguanine, a 8-methylguanine, a 8-thioguanine, an azaguanine, a 2-aminopurine, a 5-ethylcytosine, a 5-methylcyosine, a 5-bromouracil, a 5-ethyluracil, a 5-iodouracil, a 5-chlorouracil, a 5-propyluracil, a thiouracil, a 2-methyladenine, a methylthioadenine, a N,N-diemethyladenine, an azaadenines, a 8-bromoadenine, a 8-hydroxyadenine, a 6-hydroxyaminopurine, a 6-thiopurine, a 4-(6-aminohexyl/cytosine), and the like. Other examples are well known to those of skill in the art.

A nucleobase may be comprised in a nucleoside or nucleotide, using any chemical or natural synthesis method described herein or known to one of ordinary skill in the art. Such nucleobase may be labeled or it may be part of a molecule that is labeled and contains the nucleobase.

### .2 Nucleosides

As used herein, a "nucleoside" refers to an individual chemical unit comprising a nucleobase covalently attached to a nucleobase linker moiety. A non-limiting example of a "nucleobase linker moiety" is a sugar comprising 5-carbon atoms (*i*.*e*., a "5-carbon sugar"), including but not limited to a deoxyribose, a ribose, an arabinose, or a derivative or an analog of a 5-carbon sugar. Non-limiting examples of a derivative or an analog of a 5-carbon sugar include a 2'-fluoro-2'-deoxyribose or a carbocyclic sugar where a carbon is substituted for an oxygen atom in the sugar ring.

Different types of covalent attachment(s) of a nucleobase to a nucleobase linker moiety are known in the art. By way of non-limiting example, a nucleoside comprising a purine (*i*.*e*., A or G) or a 7-deazapurine nucleobase typically covalently attaches the 9 position of a purine or a 7-deazapurine to the 1'-position of a 5-carbon sugar. In another non-limiting example, a nucleoside comprising a pyrimidine nucleobase (*i*.*e*., C, T or U) typically covalently attaches a 1 position of a pyrimidine to a 1'-position of a 5-carbon sugar (Kornberg and Baker, 1992).

### .3 Nucleotides

As used herein, a "nucleotide" refers to a nucleoside further comprising a "backbone moiety". A backbone moiety generally covalently attaches a nucleotide to another molecule comprising a nucleotide, or to another nucleotide to form a nucleic acid. The "backbone moiety" in naturally occurring nucleotides typically comprises a phosphorus moiety, which is covalently attached to a 5-carbon sugar. The attachment of the backbone moiety typically occurs at either the 3'- or 5'-position of the 5-carbon sugar. However, other types of attachments are known in the art, particularly when a nucleotide comprises derivatives or analogs of a naturally occurring 5-carbon sugar or phosphorus moiety.

### .4 Nucleic Acid Analogs

A nucleic acid may comprise, or be composed entirely of, a derivative or analog of a nucleobase, a nucleobase linker moiety and/or backbone moiety that may be present in a naturally occurring nucleic acid. RNA with nucleic acid analogs may also be labeled according to methods of the invention. As used herein a "derivative" refers to a chemically modified or altered form of a naturally occurring molecule, while the terms "mimic" or "analog" refer to a molecule that may or may not structurally resemble a naturally occurring molecule or moiety, but possesses similar functions. As used herein, a "moiety" generally refers to a smaller chemical or molecular component of a larger chemical or molecular structure. Nucleobase, nucleoside and nucleotide analogs or derivatives are well known in the art, and have been described (see for example, Scheit, 1980).

Additional non-limiting examples of nucleosides, nucleotides or nucleic acids comprising 5-carbon sugar and/or backbone moiety derivatives or analogs, include those in: U.S. Patent 5,681,947, which describes oligonucleotides comprising purine derivatives that form triple helixes with and/or prevent expression of dsDNA; U.S. Patents 5,652,099 and 5,763,167, which describe nucleic acids incorporating fluorescent analogs of nucleosides found in DNA or RNA, particularly for use as fluorescent nucleic acids probes; U.S. Patent 5,614,617, which describes oligonucleotide analogs with substitutions on pyrimidine rings that possess enhanced nuclease stability; U.S. Patents 5,670,663, 5,872,232 and 5,859,221, which describe oligonucleotide analogs with modified 5-carbon sugars (*i*.*e*., modified 2'-deoxyfuranosyl moieties) used in nucleic acid detection; U.S. Patent 5,446,137, which describes oligonucleotides comprising at least one 5-carbon sugar moiety substituted at the 4' position with a substituent other than hydrogen that can be used in hybridization assays; U.S. Patent 5,886,165, which describes oligonucleotides with both deoxyribonucleotides with 3'-5' internucleotide linkages and ribonucleotides with 2'-5' internucleotide linkages; U.S. Patent 5,714,606, which describes a modified internucleotide linkage wherein a 3'-position oxygen of the internucleotide linkage is replaced by a carbon to enhance the nuclease resistance of nucleic acids; U.S. Patent 5,672,697, which describes oligonucleotides containing one or more 5' methylene phosphonate internucleotide linkages that enhance nuclease resistance; U.S. Patents 5,466,786 and 5,792,847, which describe the linkage of a substituent moiety which may comprise a drug or label to the 2' carbon of an oligonucleotide to provide enhanced nuclease stability and ability to deliver drugs or detection moieties; U.S. Patent 5,223,618, which describes oligonucleotide analogs with a 2 or 3 carbon backbone linkage attaching the 4' position and 3' position of adjacent 5-carbon sugar moiety to enhanced cellular uptake, resistance to nucleases and hybridization to target RNA; U.S. Patent 5,470,967, which describes oligonucleotides comprising at least one sulfamate or sulfamide internucleotide linkage that are useful as nucleic acid hybridization probe; U.S. Patents 5,378,825, 5,777,092, 5,623,070, 5,610,289 and 5,602,240, which describe oligonucleotides with three or four atom linker moiety replacing phosphodiester backbone moiety used for improved nuclease resistance, cellular uptake, and regulating RNA expression; U.S. Patent 5,858,988, which describes hydrophobic carrier agent attached to the 2'-O position of oligonucleotides to enhanced their membrane permeability and stability; U.S. Patent 5,214,136, which describes oligonucleotides conjugated to anthraquinone at the 5' terminus that possess enhanced hybridization to DNA or RNA; enhanced stability to nucleases; U.S. Patent 5,700,922, which describes PNA-DNA-PNA chimeras wherein the DNA comprises 2'-deoxy-erythro-pentofuranosyl nucleotides for enhanced nuclease resistance, binding affinity, and ability to activate RNase H; and U.S. Patent 5,708,154, which describes RNA linked to a DNA to form a DNA-RNA hybrid; U.S. Patent 5,728,525, which describes the labeling of nucleoside analogs with a universal fluorescent label.

Additional teachings for nucleoside analogs and nucleic acid analogs are U.S. Patent 5,728,525, which describes nucleoside analogs that are end-labeled; U.S. Patent 5,637,683, 6,251,666 (L-nucleotide substitutions), and 5,480,980 (7-deaza-2'deoxyguanosine nucleotides and nucleic acid analogs thereof).

### .5 Modified Nucleotides

Labeling methods and kits specifically contemplate the use of nucleotides that are both modified for attachment of a label and can be incorporated into a miRNA molecule. Such nucleotides include those that can be labeled with a dye, including a fluorescent dye, or with a molecule such as biotin. Labeled nucleotides are readily available; they can be acquired commercially or they can be synthesized by reactions known to those of skill in the art.

Modified nucleotides for use in the invention are not naturally occurring nucleotides, but instead, refer to prepared nucleotides that have a reactive moiety on them. Specific reactive functionalities of interest include: amino, sulfhydryl, sulfoxyl, aminosulfhydryl, azido, epoxide, isothiocyanate, isocyanate, anhydride, monochlorotriazine, dichlorotriazine, mono-or dihalogen substituted pyridine, mono-or disubstituted diazine, maleimide, epoxide, aziridine, sulfonyl halide, acid halide, alkyl halide, aryl halide, alkylsulfonate, N-hydroxysuccinimide ester, imido ester, hydrazine, azidonitrophenyl, azide, 3-(2-pyridyl dithio)-propionamide, glyoxal, aldehyde, iodoacetyl, cyanomethyl ester, p-nitrophenyl ester, o-nitrophenyl ester, hydroxypyridine ester, carbonyl imidazole, and the other such chemical groups. In some embodiments, the reactive functionality may be bonded directly to a nucleotide, or it may be bonded to the nucleotide through a linking group. The functional moiety and any linker cannot substantially impair the ability of the nucleotide to be added to the miRNA or to be labeled. Representative linking groups include carbon containing linking groups, typically ranging from about 2 to 18, usually from about 2 to 8 carbon atoms, where the carbon containing linking groups may or may not include one or more heteroatoms, *e*.*g*. S, O, N etc., and may or may not include one or more sites of unsaturation. Of particular interest in many embodiments are alkyl linking groups, typically lower alkyl linking groups of 1 to 16, usually 1 to 4 carbon atoms, where the linking groups may include one or more sites of unsaturation. The functionalized nucleotides (or primers) used in the above methods of functionalized target generation may be fabricated using known protocols or purchased from commercial vendors, *e*.*g*., Sigma, Roche, Ambion, Biosearch Technologies and NEN. Functional groups may be prepared according to ways known to those of skill in the art, including the representative information found in U.S. Patents 4,404,289; 4,405,711; 4,337,063 and 5,268,486, and U.K. Patent 1,529,202.

Amine-modified nucleotides are used in several embodiments of the invention. The amine-modified nucleotide is a nucleotide that has a reactive amine group for attachment of the label. It is contemplated that any ribonucleotide (G, A, U, or C) or deoxyribonucleotide (G, A, T, or C) can be modified for labeling. Examples include, but are not limited to, the following modified ribo- and deoxyribo-nucleotides: 5-(3-aminoallyl)-UTP; 8-[(4-amino)butyl]-amino-ATP and 8-[(6-amino)butyl]-amino-ATP; N6-(4-amino)butyl-ATP, N6-(6-amino)butyl-ATP, N4-[2,2-oxy-bis-(ethylamine)]-CTP; N6-(6-Amino)hexyl-ATP; 8-[(6-Amino)hexyl]-amino-ATP; 5-propargylamino-CTP, 5-propargylamino-UTP; 5-(3-aminoallyl)-dUTP; 8-[(4-amino)butyl]-amino-dATP and 8-[(6-amino)butyl]-amino-dATP; N6-(4-amino)butyl-dATP, N6-(6-amino)butyl-dATP, N4-[2,2-oxy-bis-(ethylamine)]-dCTP; N6-(6-Amino)hexyl-dATP; 8-[(6-Amino)hexyl]-amino-dATP; 5-propargylamino-dCTP, and 5-propargylamino-dUTP. Such nucleotides can be prepared according to methods known to those of skill in the art. Moreover, a person of ordinary skill in the art could prepare other nucleotide entities with the same amine-modification, such as a 5-(3-aminoallyl)-CTP, GTP, ATP, dCTP, dGTP, dTTP, or dUTP in place of a 5-(3-aminoallyl)-UTP.

### .B Preparation of Nucleic Acids

A nucleic acid may be made by any technique known to one of ordinary skill in the art, such as for example, chemical synthesis, enzymatic production or biological production. It is specifically contemplated that miRNA probes of the invention are chemically synthesized.

In some embodiments of the invention, miRNAs are recovered or isolated from a biological sample. The miRNA may be recombinant or it may be natural or endogenous to the cell (produced from the cell's genome). It is contemplated that a biological sample may be treated in a way so as to enhance the recovery of small RNA molecules such as miRNA. U.S. Patent Application Serial No. 10/667,126 describes such methods. Generally, methods involve lysing cells with a solution having guanidinium and a detergent.

Alternatively, nucleic acid synthesis is performed according to standard methods. See, for example, Itakura and Riggs (1980). Additionally, U.S. Patents 4,704,362, 5,221,619, and 5,583,013 each describe various methods of preparing synthetic nucleic acids. Non-limiting examples of a synthetic nucleic acid (*e*.*g*., a synthetic oligonucleotide), include a nucleic acid made by *in vitro* chemically synthesis using phosphotriester, phosphite, or phosphoramidite chemistry and solid phase techniques such as described in EP 266,032, or via deoxynucleoside H-phosphonate intermediates as described by Froehler *et al*., 1986 and U.S. Patent 5,705,629. In the methods of the present invention, one or more oligonucleotide may be used. Various different mechanisms of oligonucleotide synthesis have been disclosed in for example, U.S. Patents 4,659,774, 4,816,571, 5,141,813, 5,264,566, 4,959,463, 5,428,148, 5,554,744, 5,574,146, 5,602,244.

A non-limiting example of an enzymatically produced nucleic acid include one produced by enzymes in amplification reactions such as PCR™ (see for example, U.S. Patents 4,683,202 and 4,682,195), or the synthesis of an oligonucleotide described in U.S. Patent 5,645,897. A non-limiting example of a biologically produced nucleic acid includes a recombinant nucleic acid produced (*i*.*e*., replicated) in a living cell, such as a recombinant DNA vector replicated in bacteria (see for example, Sambrook *et al*., 2001).

Oligonucleotide synthesis is well known to those of skill in the art. Various different mechanisms of oligonucleotide synthesis have been disclosed in for example, U.S. Patents 4,659,774, 4,816,571, 5,141,813, 5,264,566, 4,959,463, 5,428,148, 5,554,744, 5,574,146, 5,602,244.

Basically, chemical synthesis can be achieved by the diester method, the triester method polynucleotides phosphorylase method and by solid-phase chemistry. The diester method was the first to be developed to a usable state, primarily by Khorana and co-workers. (Khorana, 1979). The basic step is the joining of two suitably protected deoxynucleotides to form a dideoxynucleotide containing a phosphodiester bond.

The main difference between the diester and triester methods is the presence in the latter of an extra protecting group on the phosphate atoms of the reactants and products (Itakura *et al*., 1975). Purification's are typically done in chloroform solutions. Other improvements in the method include (i) the block coupling of trimers and larger oligomers, (ii) the extensive use of high-performance liquid chromatography for the purification of both intermediate and final products, and (iii) solid-phase synthesis.

Polynucleotide phosphorylase method is an enzymatic method of DNA synthesis that can be used to synthesize many useful oligonucleotides (Gillam *et al*., 1978; Gillam *et al*., 1979). Under controlled conditions, polynucleotide phosphorylase adds predominantly a single nucleotide to a short oligonucleotide. Chromatographic purification allows the desired single adduct to be obtained. At least a trimer is required to start the procedure, and this primer must be obtained by some other method. The polynucleotide phosphorylase method works and has the advantage that the procedures involved are familiar to most biochemists.

Solid-phase methods draw on technology developed for the solid-phase synthesis of polypeptides, it has been possible to attach the initial nucleotide to solid support material and proceed with the stepwise addition of nucleotides. All mixing and washing steps are simplified, and the procedure becomes amenable to automation. These syntheses are now routinely carried out using automatic nucleic acid synthesizers.

Phosphoramidite chemistry (Beaucage and Lyer, 1992) has become by far the most widely used coupling chemistry for the synthesis of oligonucleotides. Phosphoramidite synthesis of oligonucleotides involves activation of nucleoside phosphoramidite monomer precursors by reaction with an activating agent to form activated intermediates, followed by sequential addition of the activated intermediates to the growing oligonucleotide chain (generally anchored at one end to a suitable solid support) to form the oligonucleotide product.

Recombinant methods for producing nucleic acids in a cell are well known to those of skill in the art. These include the use of vectors (viral and non-viral), plasmids, cosmids, and other vehicles for delivering a nucleic acid to a cell, which may be the target cell (*e*.*g*., a cancer cell) or simply a host cell (to produce large quantities of the desired RNA molecule). Alternatively, such vehicles can be used in the context of a cell free system so long as the reagents for generating the RNA molecule are present. Such methods include those described in Sambrook, 2003, Sambrook, 2001 and Sambrook, 1989.

In certain embodiments, the present invention concerns nucleic acid molecules that are not synthetic. In some embodiments, the nucleic acid molecule has a chemical structure of a naturally occurring nucleic acid and a sequence of a naturally occurring nucleic acid, such as the exact and entire sequence of a single stranded primary miRNA (see Lee 2002), a single-stranded precursor miRNA, or a single-stranded mature miRNA. In addition to the use of recombinant technology, such non-synthetic nucleic acids may be generated chemically, such as by employing technology used for creating oligonucleotides.

### .C Isolation of Nucleic Acids

Nucleic acids may be isolated using techniques well known to those of skill in the art, though in particular embodiments, methods for isolating small nucleic acid molecules, and/or isolating RNA molecules can be employed. Chromatography is a process often used to separate or isolate nucleic acids from protein or from other nucleic acids. Such methods can involve electrophoresis with a gel matrix, filter columns, alcohol precipitation, and/or other chromatography. If miRNA from cells is to be used or evaluated, methods generally involve lysing the cells with a chaotropic (*e*.*g*., guanidinium isothiocyanate) and/or detergent (e.g., N-lauroyl sarcosine) prior to implementing processes for isolating particular populations of RNA.

In particular methods for separating miRNA from other nucleic acids, a gel matrix is prepared using polyacrylamide, though agarose can also be used. The gels may be graded by concentration or they may be uniform. Plates or tubing can be used to hold the gel matrix for electrophoresis. Usually one-dimensional electrophoresis is employed for the separation of nucleic acids. Plates are used to prepare a slab gel, while the tubing (glass or rubber, typically) can be used to prepare a tube gel. The phrase "tube electrophoresis" refers to the use of a tube or tubing, instead of plates, to form the gel. Materials for implementing tube electrophoresis can be readily prepared by a person of skill in the art or purchased, such as from C.B.S. Scientific Co., Inc. or Scie-Plas.

Methods may involve the use of organic solvents and/or alcohol to isolate nucleic acids, particularly miRNA used in methods and compositions of the invention. Some embodiments are described in U.S. Patent Application Serial No. 10/667,126. Generally, this disclosure provides methods for efficiently isolating small RNA molecules from cells comprising: adding an alcohol solution to a cell lysate and applying the alcohol/lysate mixture to a solid support before eluting the RNA molecules from the solid support. In some embodiments, the amount of alcohol added to a cell lysate achieves an alcohol concentration of about 55% to 60%. While different alcohols can be employed, ethanol works well. A solid support may be any structure, and it includes beads, filters, and columns, which may include a mineral or polymer support with electronegative groups. A glass fiber filter or column has worked particularly well for such isolation procedures.

In specific embodiments, miRNA isolation processes include: a) lysing cells in the sample with a lysing solution comprising guanidinium, wherein a lysate with a concentration of at least about 1 M guanidinium is produced; b) extracting miRNA molecules from the lysate with an extraction solution comprising phenol; c) adding to the lysate an alcohol solution for form a lysate/alcohol mixture, wherein the concentration of alcohol in the mixture is between about 35% to about 70%; d) applying the lysate/alcohol mixture to a solid support; e) eluting the miRNA molecules from the solid support with an ionic solution; and, f) capturing the miRNA molecules. Typically the sample is dried down and resuspended in a liquid and volume appropriate for subsequent manipulation.

### .II Labels and Labeling Techniques

In some embodiments, the present invention concerns miRNA that are labeled. It is contemplated that miRNA may first be isolated and/or purified prior to labeling. This may achieve a reaction that more efficiently labels the miRNA, as opposed to other RNA in a sample in which the miRNA is not isolated or purified prior to labeling. In many embodiments of the invention, the label is non-radioactive. Generally, nucleic acids may be labeled by adding labeled nucleotides (one-step process) or adding nucleotides and labeling the added nucleotides (two-step process).

### .A Labeling Techniques

In some embodiments, nucleic acids are labeled by catalytically adding to the nucleic acid an already labeled nucleotide or nucleotides. One or more labeled nucleotides can be added to miRNA molecules. See U.S Patent 6,723,509.

In other embodiments, an unlabeled nucleotide or nucleotides is catalytically added to a miRNA, and the unlabeled nucleotide is modified with a chemical moiety that enables it to be subsequently labeled. In embodiments of the invention, the chemical moiety is a reactive amine such that the nucleotide is an amine-modified nucleotide. Examples of amine-modified nucleotides are well known to those of skill in the art, many being commercially available such as from Ambion, Sigma, Jena Bioscience, and TriLink.

In contrast to labeling of cDNA during its synthesis, the issue for labeling miRNA is how to label the already existing molecule. The present invention concerns the use of an enzyme capable of using a di- or tri-phosphate ribonucleotide or deoxyribonucleotide as a substrate for its addition to a miRNA. Moreover, in specific embodiments, it involves using a modified di- or tri-phosphate ribonucleotide, which is added to the 3' end of a miRNA. The source of the enzyme is not limiting. Examples of sources for the enzymes include yeast, gram-negative bacteria such as *E. coli*, *lactococcus lactis*, and sheep pox virus.

Enzymes capable of adding such nucleotides include, but are not limited to, poly(A) polymerase, terminal transferase, and polynucleotide phosphorylase. In specific embodiments of the invention, a ligase is contemplated as not being the enzyme used to add the label, and instead, a non-ligase enzyme is employed.

Terminal transferase catalyzes the addition of nucleotides to the 3' terminus of a nucleic acid.

Polynucleotide phosphorylase can polymerize nucleotide diphosphates without the need for a primer.

### .B Labels

Labels on miRNA or miRNA probes may be colorimetric (includes visible and UV spectrum, including fluorescent), luminescent, enzymatic, or positron emitting (including radioactive). The label may be detected directly or indirectly. Radioactive labels include ¹²⁵I, ³²P, ³³P, and ³⁵S. Examples of enzymatic labels include alkaline phosphatase, luciferase, horseradish peroxidase, and β-galactosidase. Labels can also be proteins with luminescent properties, e.g., green fluorescent protein and phicoerythrin.

The colorimetric and fluorescent labels contemplated for use as conjugates include, but are not limited to, Alexa Fluor dyes, BODIPY dyes, such as BODIPY FL; Cascade Blue; Cascade Yellow; coumarin and its derivatives, such as 7-amino-4-methylcoumarin, aminocoumarin and hydroxycoumarin; cyanine dyes, such as Cy3 and Cy5; eosins and erythrosins; fluorescein and its derivatives, such as fluorescein isothiocyanate; macrocyclic chelates of lanthanide ions, such as Quantum Dye™; Marina Blue; Oregon Green; rhodamine dyes, such as rhodamine red, tetramethylrhodamine and rhodamine 6G; Texas Red; , fluorescent energy transfer dyes, such as thiazole orange-ethidium heterodimer; and, TOTAB.

Specific examples of dyes include, but are not limited to, those identified above and the following: Alexa Fluor 350, Alexa Fluor 405, Alexa Fluor 430, Alexa Fluor 488, Alexa Fluor 500. Alexa Fluor 514, Alexa Fluor 532, Alexa Fluor 546, Alexa Fluor 555, Alexa Fluor 568, Alexa Fluor 594, Alexa Fluor 610, Alexa Fluor 633, Alexa Fluor 647, Alexa Fluor 660, Alexa Fluor 680, Alexa Fluor 700, and, Alexa Fluor 750; amine-reactive BODIPY dyes, such as BODIPY 493/503, BODIPY 530/550, BODIPY 558/568, BODIPY 564/570, BODIPY 576/589, BODIPY 581/591, BODIPY 630/650, BODIPY 650/655, BODIPY FL, BODIPY R6G, BODIPY TMR, and, BODIPY-TR; Cy3, Cy5, 6-FAM, Fluorescein Isothiocyanate, HEX, 6-JOE, Oregon Green 488, Oregon Green 500, Oregon Green 514, Pacific Blue, REG, Rhodamine Green, Rhodamine Red, Renographin, ROX, SYPRO, TAMRA, 2',4',5',7'-Tetrabromosulfonefluorescein, and TET.

Specific examples of fluorescently labeled ribonucleotides are available from Molecular Probes, and these include, Alexa Fluor 488-5-UTP, Fluorescein-12-UTP, BODIPY FL-14-UTP, BODIPY TMR-14-UTP, Tetramethylrhodamine-6-UTP, Alexa Fluor 546-14-UTP, Texas Red-5-UTP, and BODIPY TR-14-UTP. Other fluorescent ribonucleotides are available from Amersham Biosciences, such as Cy3-UTP and Cy5-UTP.

Examples of fluorescently labeled deoxyribonucleotides include Dinitrophenyl (DNP)-11-dUTP, Cascade Blue-7-dUTP, Alexa Fluor 488-5-dUTP, Fluorescein-12-dUTP, Oregon Green 488-5-dUTP, BODIPY FL-14-dUTP, Rhodamine Green-5-dUTP, Alexa Fluor 532-5-dUTP, BODIPY TMR-14-dUTP, Tetramethylrhodamine-6-dUTP, Alexa Fluor 546-14-dUTP, Alexa Fluor 568-5-dUTP, Texas Red-12-dUTP, Texas Red-5-dUTP, BODIPY TR-14-dUTP, Alexa Fluor 594-5-dUTP, BODIPY 630/650-14-dUTP, BODIPY 650/665-14-dUTP; Alexa Fluor 488-7-OBEA-dCTP, Alexa Fluor 546-16-OBEA-dCTP, Alexa Fluor 594-7-OBEA-dCTP, Alexa Fluor 647-12-OBEA-dCTP.

It is contemplated that nucleic acids may be labeled with two different labels. Furthermore, fluorescence resonance energy transfer (FRET) may be employed in methods of the invention (*e*.*g*., Klostermeier *et al*., 2002; Emptage, 2001; Didenko, 2001).

Alternatively, the label may not be detectable *per se*, but indirectly detectable or allowing for the isolation or separation of the targeted nucleic acid. For example, the label could be biotin, digoxigenin, polyvalent cations, chelator groups and the other ligands, include ligands for an antibody.

### .C Visualization Techniques

A number of techniques for visualizing or detecting labeled nucleic acids are readily available. Such techniques include, microscopy, arrays, Fluorometry, Light cyclers or other real time PCR machines, FACS analysis, scintillation counters, Phosphoimagers, Geiger counters, MRI, CAT, antibody-based detection methods (Westerns, immunofluorescence, immunohistochemistry), histochemical techniques, HPLC (Griffey *et al*., 1997), spectroscopy, capillary gel electrophoresis (Cummins *et al*., 1996), spectroscopy; mass spectroscopy; radiological techniques; and mass balance techniques.

When two or more differentially colored labels are employed, fluorescent resonance energy transfer (FRET) techniques may be employed to characterize association of one or more nucleic acid. Furthermore, a person of ordinary skill in the art is well aware of ways of visualizing, identifying, and characterizing labeled nucleic acids, and accordingly, such protocols may be used as part of the invention. Examples of tools that may be used also include fluorescent microscopy, a BioAnalyzer, a plate reader, Storm (Molecular Dynamics), Array Scanner, FACS (fluorescent activated cell sorter), or any instrument that has the ability to excite and detect a fluorescent molecule.

### .III Array Preparation and Screening

### .A Array Preparation

Disclosed is the preparation and use of miRNA arrays or miRNA probe arrays, which are ordered macroarrays or microarrays of nucleic acid molecules (probes) that are fully or nearly complementary or identical to a plurality of miRNA molecules or precursor miRNA molecules and that are positioned on a support or support material in a spatially separated organization. Microarrays are typically sheets of nitrocellulose or nylon upon which probes have been spotted. Microarrays position the nucleic acid probes more densely such that up to 10,000 nucleic acid molecules can be fit into a region typically 1 to 4 square centimeters. Microarrays can be fabricated by spotting nucleic acid molecules, *e*.*g*., genes, oligonucleotides, *etc*., onto substrates or fabricating oligonucleotide sequences *in situ* on a substrate. Spotted or fabricated nucleic acid molecules can be applied in a high density matrix pattern of up to about 30 non-identical nucleic acid molecules per square centimeter or higher, e.g. up to about 100 or even 1000 per square centimeter. Microarrays typically use coated glass as the solid support, in contrast to the nitrocellulose-based material of filter arrays. By having an ordered array of miRNA-complementing nucleic acid samples, the position of each sample can be tracked and linked to the original sample. A variety of different array devices in which a plurality of distinct nucleic acid probes are stably associated with the surface of a solid support are known to those of skill in the art. Useful substrates for arrays include nylon, glass, metal, plastic, and silicon. Such arrays may vary in a number of different ways, including average probe length, sequence or types of probes, nature of bond between the probe and the array surface, e.g. covalent or non-covalent, and the like. The labeling and screening methods of the present invention and the arrays are not limited in its utility with respect to any parameter except that the probes detect miRNA; consequently, methods and compositions may be used with a variety of different types of miRNA arrays.

Representative methods and apparatus for preparing a microarray have been described, for example, in U.S. Patents 5,143,854; 5,202,231; 5,242,974; 5,288,644; 5,324,633; 5,384,261; 5,405,783; 5,412,087; 5,424,186; 5,429,807; 5,432,049; 5,436,327; 5,445,934; 5,468,613; 5,470,710; 5,472,672; 5,492,806; 5,525,464; 5,503,980; 5,510,270; 5,525,464; 5,527,681; 5,529,756; 5,532,128; 5,545,531; 5,547,839; 5,554,501; 5,556,752; 5,561,071; 5,571,639; 5,580,726; 5,580,732; 5,593,839; 5,599,695; 5,599,672; 5,610;287; 5,624,711; 5,631,134; 5,639,603; 5,654,413; 5,658,734; 5,661,028; 5,665,547; 5,667,972; 5,695,940; 5,700,637; 5,744,305; 5,800,992; 5,807,522; 5,830,645; 5,837,196; 5,871,928; 5,847,219; 5,876,932; 5,919,626; 6,004,755; 6,087,102; 6,368,799; 6,383,749; 6,617,112; 6,638,717; 6,720,138, as well as WO 93/17126; WO 95/11995; WO 95/21265; WO 95/21944; WO 95/35505; WO 96/31622; WO 97/10365; WO 97/27317; WO 99/35505; WO 09923256; WO 09936760; WO0138580; WO 0168255; WO 03020898; WO 03040410; WO 03053586; WO 03087297; WO 03091426; WO03100012; WO 04020085; WO 04027093; EP 373 203; EP 785 280; EP 799 897 and UK 8 803 000.

It is contemplated that the arrays can be high density arrays, such that they contain 2, 20, 25, 50, 80, 100 or more different probes. It is contemplated that they may contain 1000, 16,000, 65,000, 250,000 or 1,000,000 or more different probes. The probes can be directed to targets in one or more different organisms or cell types. The oligonucleotide probes range from 5 to 50, 5 to 45, 10 to 40, 9 to 34, or 15 to 40 nucleotides in length in some embodiments. In certain embodiments, the oligonucleotide probes are 5, 10, 15, 20 to 20, 25, 30, 35, 40 nucleotides in length including all integers and ranges there between.

The location and sequence of each different probe sequence in the array are generally known. Moreover, the large number of different probes can occupy a relatively small area providing a high density array having a probe density of generally greater than about 60, 100, 600, 1000, 5,000, 10,000, 40,000, 100,000, or 400,000 different oligonucleotide probes per cm². The surface area of the array can be about or less than about 1, 1.6, 2, 3, 4, 5, 6, 7, 8, 9, or 10 cm².

Moreover, a person of ordinary skill in the art could readily analyze data generated using an array. Such protocols are disclosed above, and include information found in WO 9743450; WO 03023058; WO 03022421; WO 03029485; WO 03067217; WO 03066906; WO 03076928; WO 03093810; WO 03100448A1.

### .B Sample Preparation

It is contemplated that the miRNA of a wide variety of samples can be analyzed using the arrays, index of miRNA probes, or array technology. While endogenous miRNA is contemplated for use with compositions and methods of the invention, recombinant miRNA - including nucleic acids that are complementary or identical to endogenous miRNA or precursor miRNA - can also be handled and analyzed as described herein. Samples may be biological samples, in which case, they can be from biopsy, fine needle aspirates, exfoliates, blood, tissue, organs, semen, saliva, tears, other bodily fluid, hair follicles, skin, or any sample containing or constituting biological cells. In certain embodiments, samples may be, but are not limited to, fresh, frozen, fixed, formalin fixed, paraffin embedded, or formalin fixed and paraffin embedded. Alternatively, the sample may not be a biological sample, but be a chemical mixture, such as a cell-free reaction mixture (which may contain one or more biological enzymes).

### .C Hybridization

After an array or a set of miRNA probes is prepared and the miRNA in the sample is labeled, the population of target nucleic acids is contacted with the array or probes under hybridization conditions, where such conditions can be adjusted, as desired, to provide for an optimum level of specificity in view of the particular assay being performed. Suitable hybridization conditions are well known to those of skill in the art and reviewed in Sambrook *et al.* (2001) and WO 95/21944. Of particular interest in many embodiments is the use of stringent conditions during hybridization. Stringent conditions are known to those of skill in the art.

It is specifically contemplated that a single array or set of probes may be contacted with multiple samples. The samples may be labeled with different labels to distinguish the samples. For example, a single array can be contacted with a tumor tissue sample labeled with Cy3, and normal tissue sample labeled with Cy5. Differences between the samples for particular miRNAs corresponding to probes on the array can be readily ascertained and quantified.

The small surface area of the array permits uniform hybridization conditions, such as temperature regulation and salt content. Moreover, because of the small area occupied by the high density arrays, hybridization may be carried out in extremely small fluid volumes (e.g., about 250 µl or less, including volumes of about or less than about 5, 10, 25, 50, 60, 70, 80 ,90, 100 µl, or any range derivable therein). In small volumes, hybridization may proceed very rapidly.

### .D Differential Expression Analyses

Arrays can be used to detect differences between two samples. Specifically contemplated applications include identifying and/or quantifying differences between miRNA from a sample that is normal and from a sample that is not normal, between a cancerous condition and a non-cancerous condition, or between two differently treated samples. Also, miRNA may be compared between a sample believed to be susceptible to a particular disease or condition and one believed to be not susceptible or resistant to that disease or condition. A sample that is not normal is one exhibiting phenotypic trait(s) of a disease or condition or one believed to be not normal with respect to that disease or condition. It may be compared to a cell that is normal with respect to that disease or condition. Phenotypic traits include symptoms of, or susceptibility to, a disease or condition of which a component is or may or may not be genetic or caused by a hyperproliferative or neoplastic cell or cells.

An array comprises a solid support with nucleic acid probes attached to the support. Arrays typically comprise a plurality of different nucleic acid probes that are coupled to a surface of a substrate in different, known locations. These arrays, also described as "microarrays" or colloquially "chips" have been generally described in the art, for example, U.S. Pat. Nos. 5,143,854, 5,445,934, 5,744,305, 5,677,195, 6,040,193, 5,424,186 and Fodor *et al.* (1991). These arrays may generally be produced using mechanical synthesis methods or light directed synthesis methods which incorporate a combination of photolithographic methods and solid phase synthesis methods. Techniques for the synthesis of these arrays using mechanical synthesis methods are described in, e.g., U.S. Pat. No. 5,384,261. Although a planar array surface is used in certain aspects, the array may be fabricated on a surface of virtually any shape or even a multiplicity of surfaces. Arrays may be nucleic acids on beads, gels, polymeric surfaces, fibers such as fiber optics, glass or any other appropriate substrate, see U.S. Pat. Nos. 5,770,358, 5,789,162, 5,708,153, 6,040,193 and 5,800,992. Arrays may be packaged in such a manner as to allow for diagnostics or other manipulation of an all inclusive device, see for example, U.S. Pat. Nos. 5,856,174 and 5,922,591. See also U.S. patent application Ser. No. 09/545,207, filed Apr. 7, 2000 for additional information concerning arrays, their manufacture, and their characteristics.

Particularly arrays can be used to evaluate samples with respect to diseases or conditions that include, but are not limited to: chronic pancreatitis; pancreatic cancer; AIDS, autoimmune diseases (rheumatoid arthritis, multiple sclerosis, diabetes-insulin-dependent and non-independent, systemic lupus erythematosus and Graves disease); cancer (*e*.*g*., malignant, benign, metastatic, precancer); cardiovascular diseases (heart disease or coronary artery disease, stroke-ischemic and hemorrhagic, and rheumatic heart disease); diseases of the nervous system; and infection by pathogenic microorganisms (Athlete's Foot, Chickenpox, Common cold, Diarrheal diseases, Flu, Genital herpes, Malaria, Meningitis, Pneumonia, Sinusitis, Skin diseases, Strep throat, Tuberculosis, Urinary tract infections, Vaginal infections, Viral hepatitis); inflammation (allergy, asthma); prion diseases (*e*.*g*., CJD, kuru, GSS, FFI).

Moreover, miRNA can be evaluated with respect to the following diseases, conditions, and disorders: pancreatitis, chronic pancreatitis, and/or pancreatic cancer.

Cancers that may be evaluated by methods and compositions of the invention include cancer cells particularly from the pancreas, including pancreatic ductal adenocarcinoma (PDAC), but may also include cells and cancer cells from the bladder, blood, bone, bone marrow, brain, breast, colon, esophagus, gastrointestine, gum, head, kidney, liver, lung, nasopharynx, neck, ovary, prostate, skin, stomach, testis, tongue, or uterus. In addition, the cancer may specifically be of the following histological type, though it is not limited to these: neoplasm, malignant; carcinoma; carcinoma, undifferentiated; giant and spindle cell carcinoma; small cell carcinoma; papillary carcinoma; squamous cell carcinoma; lymphoepithelial carcinoma; basal cell carcinoma; pilomatrix carcinoma; transitional cell carcinoma; papillary transitional cell carcinoma; adenocarcinoma; gastrinoma, malignant; cholangiocarcinoma; hepatocellular carcinoma; combined hepatocellular carcinoma and cholangiocarcinoma; trabecular adenocarcinoma; adenoid cystic carcinoma; adenocarcinoma in adenomatous polyp; adenocarcinoma, familial polyposis coli; solid carcinoma; carcinoid tumor, malignant; branchiolo-alveolar adenocarcinoma; papillary adenocarcinoma; chromophobe carcinoma; acidophil carcinoma; oxyphilic adenocarcinoma; basophil carcinoma; clear cell adenocarcinoma; granular cell carcinoma; follicular adenocarcinoma; papillary and follicular adenocarcinoma; nonencapsulating sclerosing carcinoma; adrenal cortical carcinoma; endometroid carcinoma; skin appendage carcinoma; apocrine adenocarcinoma; sebaceous adenocarcinoma; ceruminous adenocarcinoma; mucoepidermoid carcinoma; cystadenocarcinoma; papillary cystadenocarcinoma; papillary serous cystadenocarcinoma; mucinous cystadenocarcinoma; mucinous adenocarcinoma; signet ring cell carcinoma; infiltrating duct carcinoma; medullary carcinoma; lobular carcinoma; inflammatory carcinoma; paget's disease, mammary; acinar cell carcinoma; adenosquamous carcinoma; adenocarcinoma w/squamous metaplasia; thymoma, malignant; ovarian stromal tumor, malignant; thecoma, malignant; granulosa cell tumor, malignant; androblastoma, malignant; sertoli cell carcinoma; leydig cell tumor, malignant; lipid cell tumor, malignant; paraganglioma, malignant; extra-mammary paraganglioma, malignant; pheochromocytoma; glomangiosarcoma; malignant melanoma; amelanotic melanoma; superficial spreading melanoma; malig melanoma in giant pigmented nevus; epithelioid cell melanoma; blue nevus, malignant; sarcoma; fibrosarcoma; fibrous histiocytoma, malignant; myxosarcoma; liposarcoma; leiomyosarcoma; rhabdomyosarcoma; embryonal rhabdomyosarcoma; alveolar rhabdomyosarcoma; stromal sarcoma; mixed tumor, malignant; mullerian mixed tumor; nephroblastoma; hepatoblastoma; carcinosarcoma; mesenchymoma, malignant; brenner tumor, malignant; phyllodes tumor, malignant; synovial sarcoma; mesothelioma, malignant; dysgerminoma; embryonal carcinoma; teratoma, malignant; struma ovarii, malignant; choriocarcinoma; mesonephroma, malignant; hemangiosarcoma; hemangioendothelioma, malignant; kaposi's sarcoma; hemangiopericytoma, malignant; lymphangiosarcoma; osteosarcoma; juxtacortical osteosarcoma; chondrosarcoma; chondroblastoma, malignant; mesenchymal chondrosarcoma; giant cell tumor of bone; ewing's sarcoma; odontogenic tumor, malignant; ameloblastic odontosarcoma; ameloblastoma, malignant; ameloblastic fibrosarcoma; pinealoma, malignant; chordoma; glioma, malignant; ependymoma; astrocytoma; protoplasmic astrocytoma; fibrillary astrocytoma; astroblastoma; glioblastoma; oligodendroglioma; oligodendroblastoma; primitive neuroectodermal; cerebellar sarcoma; ganglioneuroblastoma; neuroblastoma; retinoblastoma; olfactory neurogenic tumor; meningioma, malignant; neurofibrosarcoma; neurilemmoma, malignant; granular cell tumor, malignant; malignant lymphoma; Hodgkin's disease; Hodgkin's lymphoma; paragranuloma; malignant lymphoma, small lymphocytic; malignant lymphoma, large cell, diffuse; malignant lymphoma, follicular; mycosis fungoides; other specified non-Hodgkin's lymphomas; malignant histiocytosis; multiple myeloma; mast cell sarcoma; immunoproliferative small intestinal disease; leukemia; lymphoid leukemia; plasma cell leukemia; erythroleukemia; lymphosarcoma cell leukemia; myeloid leukemia; basophilic leukemia; eosinophilic leukemia; monocytic leukemia; mast cell leukemia; megakaryoblastic leukemia; myeloid sarcoma; and hairy cell leukemia, as limited by the claims. Moreover, miRNA can be evaluated in precancers, such as metaplasia, dysplasia, and hyperplasia.

It is specifically contemplated that the invention can be used to evaluate differences between stages of disease, such as between hyperplasia, neoplasia, precancer and cancer, or between a primary tumor and a metastasized tumor.

Moreover, it is contemplated that samples that have differences in the activity of certain pathways may also be compared. These pathways include the following and those involving the following factors: antibody response, apoptosis, calcium/NFAT signaling, cell cycle, cell migration, cell adhesion, cell division, cytokines and cytokine receptors, drug metabolism, growth factors and growth factor receptors, inflammatory response, insulin signaling, NFκ-B signaling, angiogenesis, adipogenesis, cell adhesion, viral infecton, bacterial infection, senescence, motility, glucose transport, stress response, oxidation, aging, telomere extension, telomere shortening, neural transmission, blood clotting, stem cell differentiation, G-Protein Coupled Receptor (GPCR) signaling, and p53 activation.

Cellular pathways that may be profiled also include but are not limited to the following: any adhesion or motility pathway including but not limited to those involving cyclic AMP, protein kinase A, G-protein couple receptors, adenylyl cyclase, L-selectin, E-selectin, PECAM, VCAM-1, α-actinin, paxillin, cadherins, AKT, integrin-α, integrin-β, RAF-1, ERK, PI-3 kinase, vinculin, matrix metalloproteinases, Rho GTPases, p85, trefoil factors, profilin, FAK, MAP kinase, Ras, caveolin, calpain-1, calpain-2, epidermal growth factor receptor, ICAM-1, ICAM-2, cofilin, actin, gelsolin, RhoA, RAC1, myosin light chain kinase, platelet-derived growth factor receptor or ezrin; any apoptosis pathway including but not limited to those involving AKT, Fas ligand, NFκB, caspase-9, PI3 kinase, caspase-3, caspase-7, ICAD, CAD, EndoG, Granzyme B, Bad, Bax, Bid, Bak, APAF-1, cytochrome C, p53, ATM, Bcl-2, PARP, Chkl, Chk2, p21, c-Jun, p73, Rad51, Mdm2, Rad50, c-Abl, BRCA-1, perforin, caspase-4, caspase-8, caspase-6, caspase-1, caspase-2, caspase-10, Rho, Jun kinase, Jun kinase kinase, Rip2, lamin-A, lamin-B1, lamin-B2, Fas receptor, H₂O₂, Granzyme A, NADPH oxidase, HMG2, CD4, CD28, CD3, TRADD, IKK, FADD, GADD45, DR3 death receptor, DR4/5 death receptor, FLIPs, APO-3, GRB2, SHC, ERK, MEK, RAF-1, cyclic AMP, protein kinase A, E2F, retinoblastoma protein, Smac/Diablo, ACH receptor, 14-3-3, FAK, SODD, TNF receptor, RIP, cyclin-D1, PCNA, Bcl-XL, PIP2, PIP3, PTEN, ATM, Cdc2, protein kinase C, calcineurin, IKKα, IKKβ, IKKγ, SOS-1, c-FOS, Traf-1, Traf-2, IκBβ□ or the proteasome; any cell activation pathway including but not limited to those involving protein kinase A, nitric oxide, caveolin-1, actin, calcium, protein kinase C, Cdc2, cyclin B, Cdc25, GRB2, SRC protein kinase, ADP-ribosylation factors (ARFs), phospholipase D, AKAP95, p68, Aurora B, CDK1, Eg7, histone H3, PKAc, CD80, PI3 kinase, WASP, Arp2, Arp3, p16, p34, p20, PP2A, angiotensin, angiotensin-converting enzyme, protease-activated receptor-1, protease-activated receptor-4, Ras, RAF-1, PLCβ, PLCγ, COX-1, G-protein-coupled receptors, phospholipase A2, IP3, SUMO1, SUMO 2/3, ubiquitin, Ran, Ran-GAP, Ran-GEF, p53, glucocorticoids, glucocorticoid receptor, components of the SWI/SNF complex, RanBP1, RanBP2, importins, exportins, RCC1, CD40, CD40 ligand, p38, IKKα, IKKβ, NFκB, TRAF2, TRAF3, TRAF5, TRAF6, IL-4, IL-4 receptor, CDK5, AP-1 transcription factor, CD45, CD4, T cell receptors, MAP kinase, nerve growth factor, nerve growth factor receptor, c-Jun, c-Fos, Jun kinase, GRB2, SOS-1, ERK-1, ERK, JAK2, STAT4, IL-12, IL-12 receptor, nitric oxide synthase, TYK2, IFNγ, elastase, IL-8, epithelins, IL-2, IL-2 receptor, CD28, SMAD3, SMAD4, TGFβ or TGFβ receptor; any cell cycle regulation, signaling or differentiation pathway including but not limited to those involving TNFs, SRC protein kinase, Cdc2, cyclin B, Grb2, Sos-1, SHC, p68, Aurora kinases, protein kinase A, protein kinase C, Eg7, p53, cyclins, cyclin-dependent kinases, neural growth factor, epidermal growth factor, retinoblastoma protein, ATF-2, ATM, ATR, AKT, CHK1, CHK2, 14-3-3, WEE1, CDC25 CDC6, Origin Recognition Complex proteins, p15, p16, p27, p21, ABL, c-ABL, SMADs, ubiquitin, SUMO, heat shock proteins, Wnt, GSK-3, angiotensin, p73 any PPAR, TGFα, TGFβ, p300, MDM2, GADD45, Notch, cdc34, BRCA-1, BRCA-2, SKP1, the proteasome, CUL1, E2F, p107, steroid hormones, steroid hormone receptors, IκBα, IκBβ, Sin3A, heat shock proteins, Ras, Rho, ERKs, IKKs, PI3 kinase, Bcl-2, Bax, PCNA, MAP kinases, dynein, RhoA, PKAc, cyclin AMP, FAK, PIP2, PIP3, integrins, thrombopoietin, Fas, Fas ligand, PLK3, MEKs, JAKs, STATs, acetylcholine, paxillin calcineurin, p38, importins, exportins, Ran, Rad50, Rad51, DNA polymerase, RNA polymerase, Ran-GAP, Ran-GEF, NuMA, Tpx2, RCC1, Sonic Hedgehog, Crm1, Patched (Ptc-1), MPF, CaM kinases, tubulin, actin, kinetochore-associated proteins, centromere-binding proteins, telomerase, TERT, PP2A, c-MYC, insulin, T cell receptors, B cell receptors, CBP, IKβ, NFκB, RAC1, RAF1, EPO, diacylglycerol, c-Jun, c-Fos, Jun kinase, hypoxia-inducible factors, GATA4, β-catenin, α-catenin, calcium, arrestin, survivin, caspases, procaspases, CREB, CREM, cadherins, PECAMs, corticosteroids, colony-stimulating factors, calpains, adenylyl cyclase, growth factors, nitric oxide, transmembrane receptors, retinoids, G-proteins, ion channels, transcriptional activators, transcriptional coactivators, transcriptional repressors, interleukins, vitamins, interferons, transcriptional corepressors, the nuclear pore, nitrogen, toxins, proteolysis, or phosphorylation; or any metabolic pathway including but not limited to those involving the biosynthesis of amino acids, oxidation of fatty acids, biosynthesis of neurotransmitters and other cell signaling molecules, biosynthesis of polyamines, biosynthesis of lipids and sphingolipids, catabolism of amino acids and nutrients, nucleotide synthesis, eicosanoids, electron transport reactions, ER-associated degradation, glycolysis, fibrinolysis, formation of ketone bodies, formation of phagosomes, cholesterol metabolism, regulation of food intake, energy homeostasis, prothrombin activation, synthesis of lactose and other sugars, multi-drug resistance, biosynthesis of phosphatidylcholine, the proteasome, amyloid precursor protein, Rab GTPases, starch synthesis, glycosylation, synthesis of phoshoglycerides, vitamins, the citric acid cycle, IGF-1 receptor, the urea cycle, vesicular transport, or salvage pathways. Additional cellular pathways include pathways that include genes and their related mRNAs identified in Table 10. It is further contemplated that nucleic acids molecules of the invention can be employed in diagnostic and therapeutic methods with respect to any of the above pathways or factors. Thus, in some embodiments of the invention, a miRNA may be differentially expressed with respect to one or more of the above pathways or factors, as limited by the claims.

Phenotypic traits also include characteristics such as longevity, morbidity, appearance (*e*.*g*., baldness, obesity), strength, speed, endurance, fertility, susceptibility or receptivity to particular drugs or therapeutic treatments (drug efficacy), and risk of drug toxicity. Samples that differ in these phenotypic traits may also be evaluated using the arrays and methods described.

In certain embodiments, miRNA profiles may be generated to evaluate and correlate those profiles with pharmacokinetics. For example, miRNA profiles may be created and evaluated for patient tumor and blood samples prior to the patient's being treated or during treatment to determine if there are miRNAs whose expression correlates with the outcome of the patient. Identification of differential miRNAs can lead to a diagnostic assay involving them that can be used to evaluate tumor and/or blood samples to determine what drug regimen the patient should be provided. In addition, it can be used to identify or select patients suitable for a particular clinical trial. If a miRNA profile is determined to be correlated with drug efficacy or drug toxicity, that may be relevant to whether that patient is an appropriate patient for receiving the drug or for a particular dosage of the drug.

In addition to the above prognostic assay, blood samples from patients with a variety of diseases can be evaluated to determine if different diseases can be identified based on blood miRNA levels. A diagnostic assay can be created based on the profiles that doctors can use to identify individuals with a disease or who are at risk to develop a disease. Alternatively, treatments can be designed based on miRNA profiling. Examples of such methods and compositions are described in the U.S. Provisional Patent Application entitled "Methods and Compositions Involving miRNA and miRNA Inhibitor Molecules" filed on May 23, 2005 in the names of David Brown, Lance Ford, Angie Cheng and Rich Jarvis.

### .E Other Assays

In addition to the use of arrays and microarrays, it is contemplated that a number of difference assays could be employed to analyze miRNAs, their activities, and their effects. Such assays include, but are not limited to, nucleic amplification, polymerase chain reaction, quantitative PCR, RT-PCR, *in situ* hybridization, Northern hybridization, hybridization protection assay (HPA)(GenProbe), branched DNA (bDNA) assay (Chiron), rolling circle amplification (RCA), single molecule hybridization detection (US Genomics), Invader assay (ThirdWave Technologies), and/or Bridge Litigation Assay (Genaco),.

### .IV Kits

Any of the compositions described herein may be comprised in a kit. In a non-limiting example, reagents for isolating miRNA, labeling miRNA, and/or evaluating a miRNA population using an array, nucleic acid amplification, and/or hybridization can be included in a kit, as well reagents for preparation of samples from pancreatic samples. The kit may further include reagents for creating or synthesizing miRNA probes. The kits will thus comprise, in suitable container means, an enzyme for labeling the miRNA by incorporating labeled nucleotide or unlabeled nucleotides that are subsequently labeled. In certain aspects, the kit can include amplification reagents. In other aspects, the kit may include various supports, such as glass, nylon, polymeric beads, and the like, and/or reagents for coupling any probes and/or target nucleic acids. It may also include one or more buffers, such as reaction buffer, labeling buffer, washing buffer, or a hybridization buffer, compounds for preparing the miRNA probes, and components for isolating miRNA. Other kits of may include components for making a nucleic acid array comprising miRNA, and thus, may include, for example, a solid support.

Kits for implementing methods of the invention described herein are specifically contemplated. In some embodiments, there are kits for preparing miRNA for multi-labeling and kits for preparing miRNA probes and/or miRNA arrays. In these embodiments, kit comprise, in suitable container means, 1, 2, 3, 4, 5, 6, 7, 8, 9, 10, 11, 12 or more of the following: 1) poly(A) polymerase; 2) unmodified nucleotides (G, A, T, C, and/or U); 3) a modified nucleotide (labeled or unlabeled); 4) poly(A) polymerase buffer; and, 5) at least one microfilter; 6) label that can be attached to a nucleotide; 7) at least one miRNA probe; 8) reaction buffer; 9) a miRNA array or components for making such an array; 10) acetic acid; 11) alcohol; 12) solutions for preparing, isolating, enriching, and purifying miRNAs or miRNA probes or arrays. Other reagents include those generally used for manipulating RNA, such as formamide, loading dye, ribonuclease inhibitors, and DNase.

In specific embodiments, kits include an array containing miRNA probes, as described in the application. An array may have probes corresponding to all known miRNAs of an organism or a particular tissue or organ in particular conditions, or to a subset of such probes. The subset of probes on arrays may be or include those identified as relevant to a particular diagnostic, therapeutic, or prognostic application. For example, the array may contain one or more probes that is indicative or suggestive of 1) a disease or condition (chronic pancreatitis and/or pancreatic cancer), 2) susceptibility or resistance to a particular drug or treatment; 3) susceptibility to toxicity from a drug or substance; 4) the stage of development or severity of a disease or condition (prognosis); and 5) genetic predisposition to a disease or condition.

For any kit embodiment, including an array, there can be nucleic acid molecules that contain or can be used to amplify a sequence that is a variant of, identical to or complementary to all or part of any of SEQ ID NOS: 1-350. In certain embodiments, a kit or array can contain one or more probes for the miRNAs identified by SEQ ID NOS:1-350. Any nucleic acid discussed above may be implemented as part of a kit.

The components of the kits may be packaged either in aqueous media or in lyophilized form. The container means of the kits will generally include at least one vial, test tube, flask, bottle, syringe or other container means, into which a component may be placed, and preferably, suitably aliquotted. Where there is more than one component in the kit (labeling reagent and label may be packaged together), the kit also will generally contain a second, third or other additional container into which the additional components may be separately placed. However, various combinations of components may be comprised in a vial. The kits also will typically include a means for containing the nucleic acids, and any other reagent containers in close confinement for commercial sale. Such containers may include injection or blow molded plastic containers into which the desired vials are retained.

When the components of the kit are provided in one and/or more liquid solutions, the liquid solution is an aqueous solution, with a sterile aqueous solution being particularly preferred.

However, the components of the kit may be provided as dried powder(s). When reagents and/or components are provided as a dry powder, the powder can be reconstituted by the addition of a suitable solvent. It is envisioned that the solvent may also be provided in another container means. In some embodiments, labeling dyes are provided as a dried power. It is contemplated that 10, 20, 30, 40, 50, 60, 70, 80, 90, 100, 120, 120, 130, 140, 150, 160, 170, 180, 190, 200, 300, 400, 500, 600, 700, 800, 900, 1000 µg or at least or at most those amounts of dried dye are provided in kits. The dye may then be resuspended in any suitable solvent, such as DMSO.

The container means will generally include at least one vial, test tube, flask, bottle, syringe and/or other container means, into which the nucleic acid formulations are placed, preferably, suitably allocated. The kits may also comprise a second container means for containing a sterile, pharmaceutically acceptable buffer and/or other diluent.

The kits will also typically include a means for containing the vials in close confinement for commercial sale, such as, e.g., injection and/or blow-molded plastic containers into which the desired vials are retained.

Such kits may also include components that facilitate isolation of the labeled miRNA. It may also include components that preserve or maintain the miRNA or that protect against its degradation. Such components may be RNAse-free or protect against RNAses. Such kits generally will comprise, in suitable means, distinct containers for each individual reagent or solution.

A kit will also include instructions for employing the kit components as well the use of any other reagent not included in the kit. Instructions may include variations that can be implemented.

Kits may also include one or more of the following: Control RNA; nuclease-free water; RNase-free containers, such as 1.5 ml tubes; RNase-free elution tubes; PEG or dextran; ethanol; acetic acid; sodium acetate; ammonium acetate; guanidinium; detergent; nucleic acid size marker; RNase-free tube tips; and RNase or DNase inhibitors.

### .V EXAMPLES

Unless otherwise designated, catalog numbers refer to products available by that number from Ambion, Inc.®, The RNA Company.

### EXAMPLE 1

### SAMPLE COLLECTION AND RNA ISOLATION

Six pancreatic primary ductal adenocarcinoma cell lines (IMIMPC2, PT45, PL45, SKPC1, PancTuI, PaCa44) and tissue samples from normal pancreas (N, n=7), chronic pancreatitis (Ch, n=7), and pancreatic ductal adenocarcinomas (PDAC) (Ca, n=10) were collected, flash frozen and stored at -80°C. The latter two diseased tissues were macro-dissected to remove as much normal pancreatic tissue as possible. Complete pathologic analyses were performed on all 24 tissue samples (Table 2). Thirteen fine needle aspirate (FNA) samples of diseased pancreatic tissue were collected during surgery and placed in RNARetain™ (Asuragen, Inc.; Austin, TX) within 30 minutes of collection and stored at 4°C.

RNA isolation was performed using the mirVana™ miRNA Isolation Kit (Ambion) according to the manufacturer's protocol. As isolation of high quality RNA from organs containing high levels of nucleases such as pancreas can be challenging, the integrity of the isolated RNA was verified on a standard 1% formaldehyde agarose gel (FIG. 1). FNA samples were centrifuged at 3,000 rpm for five minutes at 4°C prior to RNA isolation to recover diseased pancreatic tissue from RNARetain™ solution. Purified total RNA was quantified using a Nanodrop® ND-1000 (Nanodrop Technologies).

**Table 2. Tissue Sample Pathology Report**

| **ID** | **Sex** | **Age** | **Assay** | **Diagnosis** | **Comment** | **Features of tissue block** |
|---|---|---|---|---|---|---|
| Ca1 | M | 63 | Array PCR | pancreatic ductal adenocarcinoma | Grade 3, T4N1M0 | tumor content: 100%, desmoplasia: 40%, inflammatory cells: strongly neutrophils, lymphocytes |
| Ca2 | M | 62 | Array PCR | pancreatic ductal adenocarcinoma | Grade 3, T4N1M0 | tumor content: 100%, desmoplasia: 80%, inflammatory cells: weakly neutrophils |
| Ca3 | F | 69 | Array PCR | pancreatic ductal adenocarcinoma | Grade 2, T3N0M0 | tumor content: 100%, desmoplasia: 60%, inflammatory cells: weakly neutrophils |
| Ca4 | M | 71 | Array PCR | pancreatic ductal adenocarcinoma | Grade 3, T3N1M0 | tumor content: 100%, desmoplasia:50%, inflammatory cells: moderately lymphocytes |
| Ca5 | F | 70 | Array PCR | pancreatic ductal adenocarcinoma | Grade 3, T4N1M0 | tumor content: 100%, desmoplasia: 40%, inflammatory cells: moderately neutrophils, lymphocytes, plasma cells |
| Ca6 | F | 63 | Array PCR | pancreatic ductal adenocarcinoma | Grade 3, T3N1M0 | tumor content: 80%, desmoplasia: 70%, inflammatory cells: weakly neutrophils |
| Ca7 | F | 71 | Array PCR | pancreatic ductal adenocarcinoma | Grade 2, T3N0M0 | tumor content: 100%, desmoplasia: 70%, inflammatory cells: weakly lymphocytes |
| Ca8 | F | 51 | Array PCR | pancreatic ductal adenocarcinoma | Grade 2, T3N1M0 | tumor content: 80%, desmoplasia: 80%, inflammatory cells: weakly neutrophils, lymphocytes |
| Ca9 | F | 77 | PCR | pancreatic ductal adenocarcinoma | Grade 3, T3N1M0 | tumor content: 90%, desmoplasia: 80%, inflammatory cells: weakly neutrophils |
| Ca10 | M | 80 | PCR | pancreatic ductal adenocarcinoma | Grade 3, T3N0M0 | tumor content: 100%, desmoplasia: 30%, no inflammatory cells |
| Ch1 | M | 58 | Array PCR | chronic pancreatitis | pronounced fibrosis, formation of pseudocysts and numerous calculi | fibrosis app. 90%, moderate inflammatory activity |
| Ch2 | M | 37 | Array PCR | chronic pancreatitis | scattered small pseudocyst, fibrosis, calculi | fibrosis app. 75%, moderate inflammatory activity |
| Ch3 | F | 43 | Array PCR | chronic pancreatitis | pronounced fibrosis, calculi | fibrosis app. 30%, low inflammatory activity |
| Ch4 | F | 56 | Array PCR | chronic pancreatitis | moderate fibrosis, few calculi | fibrosis app. 80%, low inflammatory activity, CAVE: small fragments of lymph node as well |
| Ch5 | M | 37 | Array PCR | chronic pancreatitis | pronounced fibrosis, focal acute inflammation and numerous calculi | fibrosis app. 50%, low inflammatory activity |
| Ch6 | F | 58 | Array PCR | chronic pancreatitis | pronounced fibrosis, calculi | fibrosis app. 50%, low inflammatory activity |
| Ch7 | F | 70 | PCR | chronic pancreatitis | moderate fibrosis, no calculi | fibrosis app. 25%, moderate inflammatory activity |
| N1 | F | 35 | Array PCR | histologically normal pancreas | resection because of solid pseudopapillary neoplasm | Normal |
| N2 | M | 73 | Array PCR | histologically normal pancreas | resection because of ampullary carcinoma | Normal |
| N3 | F | 58 | Array PCR | histologically normal pancreas | resection because of solid pseudopapillary neoplasm | fibrosis 10% |
| N4 | M | 61 | Array PCR | histologically normal pancreas | resection because of bile duct carcinoma | fibrosis 10% |
| N5 | F | 47 | Array PCR | histologically normal pancreas | resection because of bile duct carcinoma | fibrosis 10% |
| N6 | M | 58 | PCR | histologically normal pancreas | resection because of serious cystadenoma of the pancreas | Normal |
| N7 | F | 76 | PCR | histologically normal pancreas | resection because of ductal adenocarcinom a of the tail of the pancreas | Normal |
| FNA-1 | F | 74 | PCR | pancreatic ductal adenocarcinoma | MX; | N/A |
| FNA-2 | F | 72 | PCR | pancreatic ductal adenocarcinoma | MX; | N/A |
| FNA-3 | F | 72 | PCR | pancreatic ductal adenocarcinoma | MX; | N/A |
| FNA-4 | F | 65 | PCR | pancreatic ductal adenocarcinoma | MX; extensive necrosis | N/A |
| FNA-5 | F | 68 | PCR | pancreatic ductal adenocarcinoma | M0; | N/A |
| FNA-6 | F | 84 | PCR | pancreatic ductal adenocarcinoma | M0; | N/A |
| FNA-7 | M | 84 | PCR | pancreatic ductal adenocarcinoma | MX; | N/A |
| FNA-8 | F | 59 | PCR | suspect for pancreatic ductal ademocarcinoma | M0; | N/A |
| FNA-9 | M | 72 | PCR | pancreatic ductal adenocarcinoma | M1; | N/A |
| FNA-11 | F | 57 | PCR | pancreatic ductal adenocarcinoma | M1; | N/A |
| FNA-12 | F | 46 | PCR | neoplasm of endocrine pancreas | MX; | N/A |
| FNA-13 | M | 59 | PCR | Atypical | MX; | N/A |
| FNA-14 | F | 85 | PCR | pancreatic ductal adenocarcinoma | MX; extensive necrosis | N/A |

| | | | | | | |
|---|---|---|---|---|---|---|
| Ca = PDAC; Ch = chronic pancreatitis; N = normal; FNA = fine needle aspirate; MX = presence of distant metastasis cannot be assessed. | | | | | | |

### EXAMPLE 2:

### MIRNA EXPRESSION PROFILING IN NORMAL AND DISEASED PANCREATIC SAMPLES

miRNA expression profiling was performed as previously described (Shingara *et al.,* 2005), except that the miRNA fractions recovered from 10-15 µg total RNA were labeled with Cy5 fluorescent dye (GE Healthcare Life Sciences) and hybridized to mirVana miRNA Bioarrays (Ambion) containing 377 individual miRNA probes, including 281 human miRNAs from the mirBase Sequence Database (on the world wide wed at microrna.sanger.ac.uk/) (Griffiths-Jones *et al.,* 2006), 33 new human miRNAs (Ambi-miRs) and 63 mouse or rat miRNAs from the mirBase Sequence Database. Following hybridization, the arrays were scanned using the Axon® GenePix 4000B scanner and associated GenePix software. Raw array data were normalized with the variance stabilization method (Huber *et al.,* 2002).

Six pancreatic primary ductal adenocarcinoma cell lines, five normal pancreas tissue samples, six chronic pancreatitis tissue samples, and eight PDAC tissue samples were profiled. Following array processing and normalization, the expressed miRNomes of the 25 different samples were established (Tables 3 and 4). On average, 200 miRNAs were detected above background signal in the tissue samples and 140 in the cell lines, corresponding to 54 and 38% respectively, of the miRNA probes present on the microarray. Unsupervised clustering of samples and miRNA expression levels showed a clear segregation between the four sample types (normal, chronic, cancer, and cell line), indicating that miRNA expression profiles were highly reproducible within each sample type.

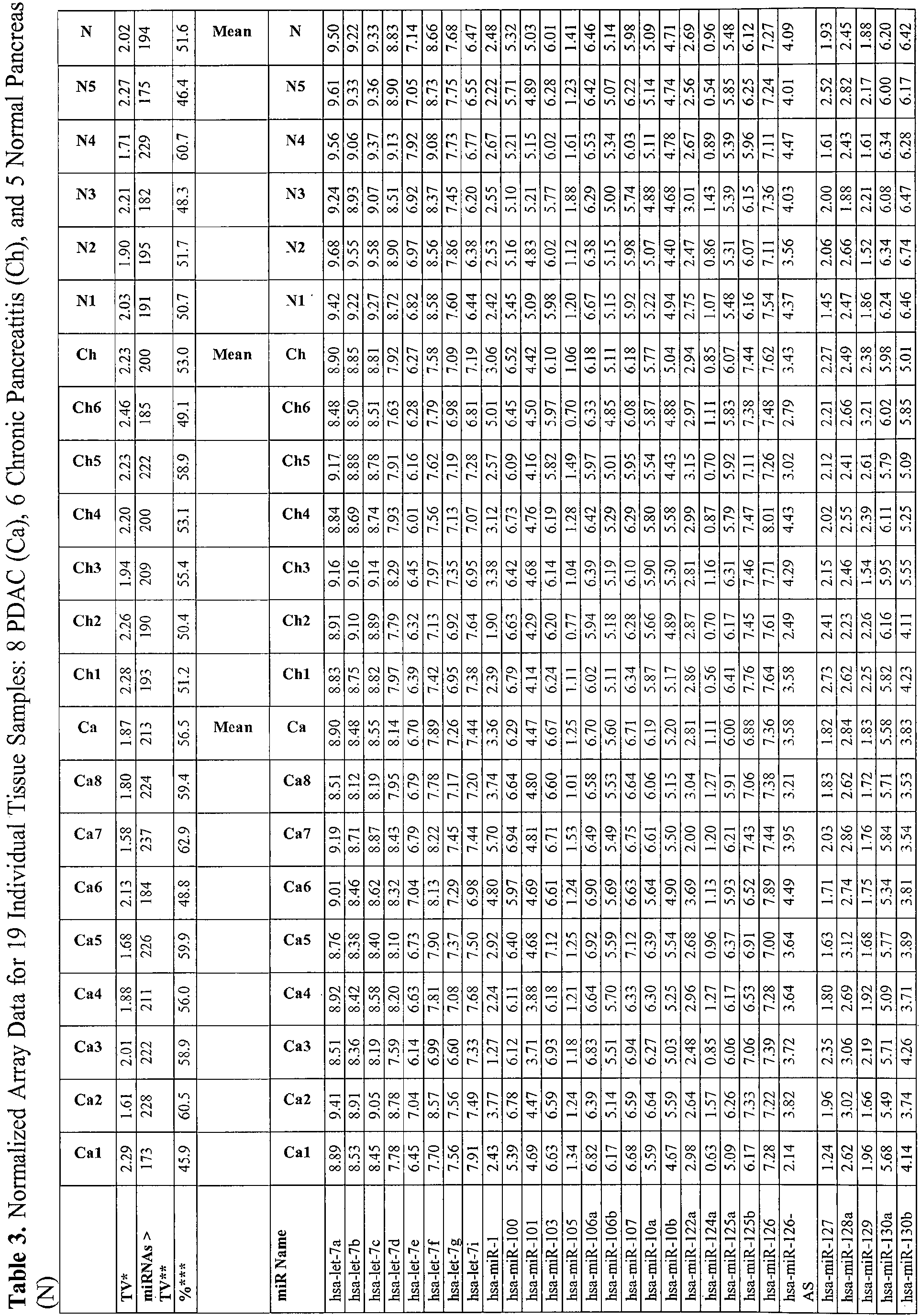

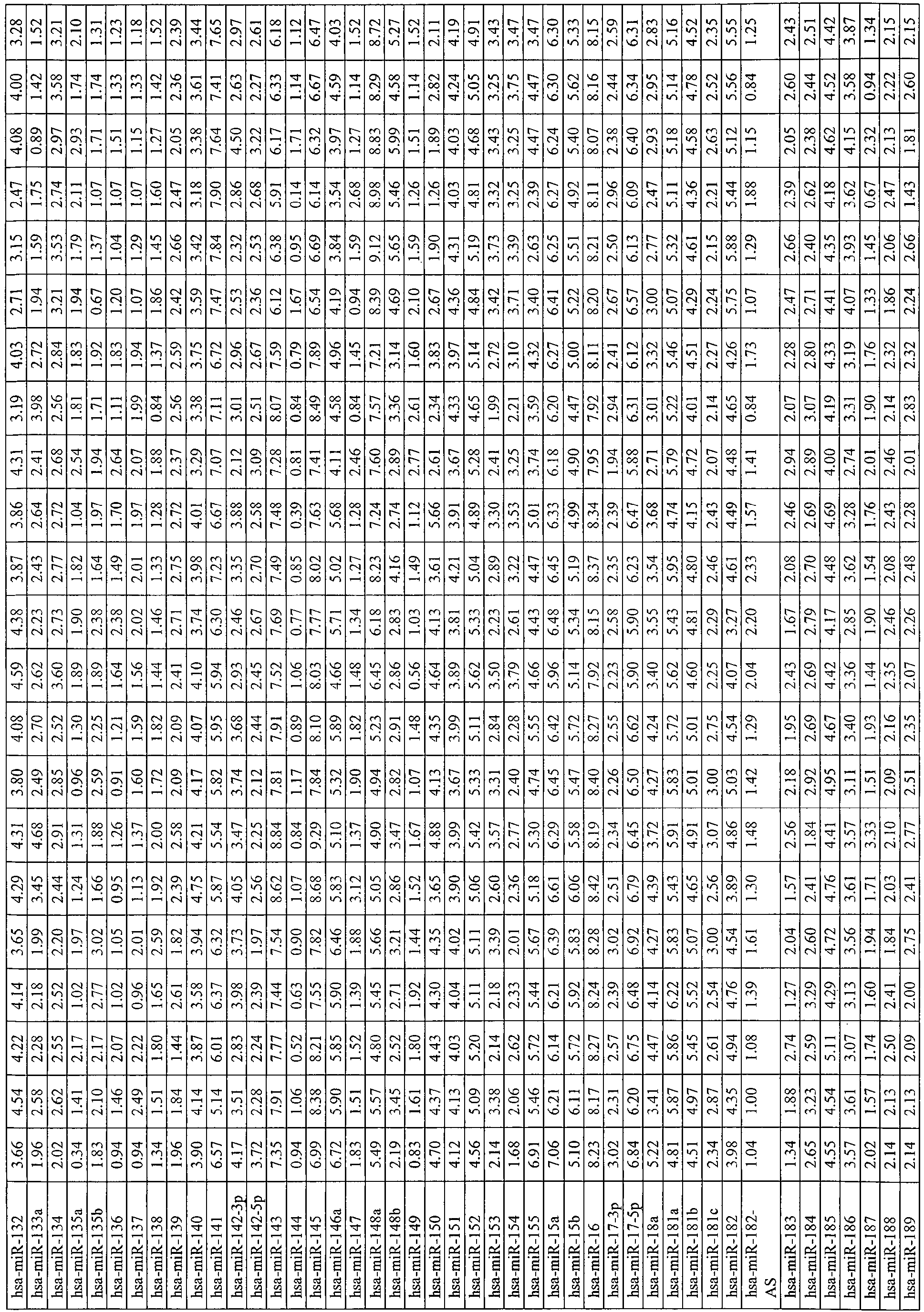

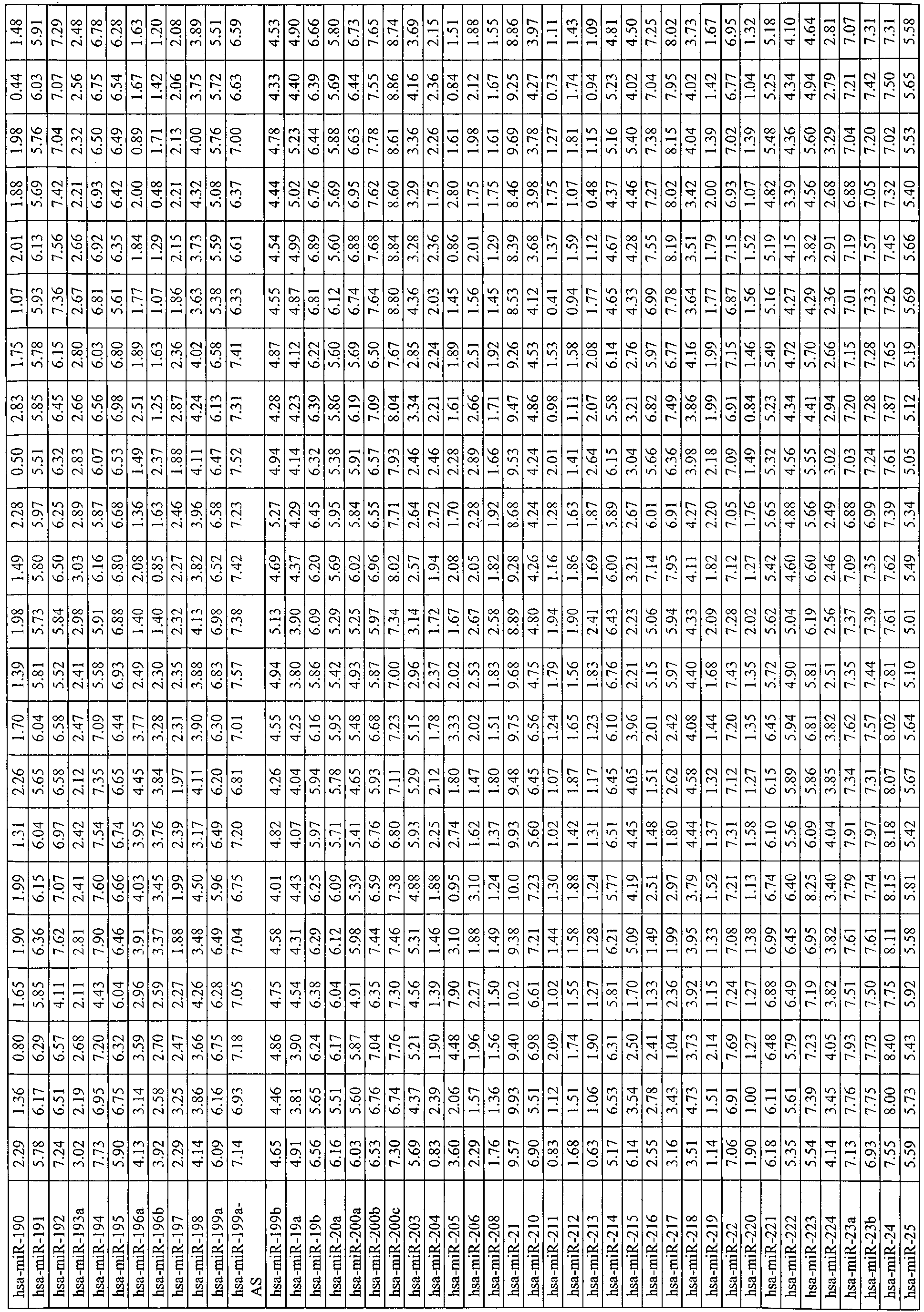

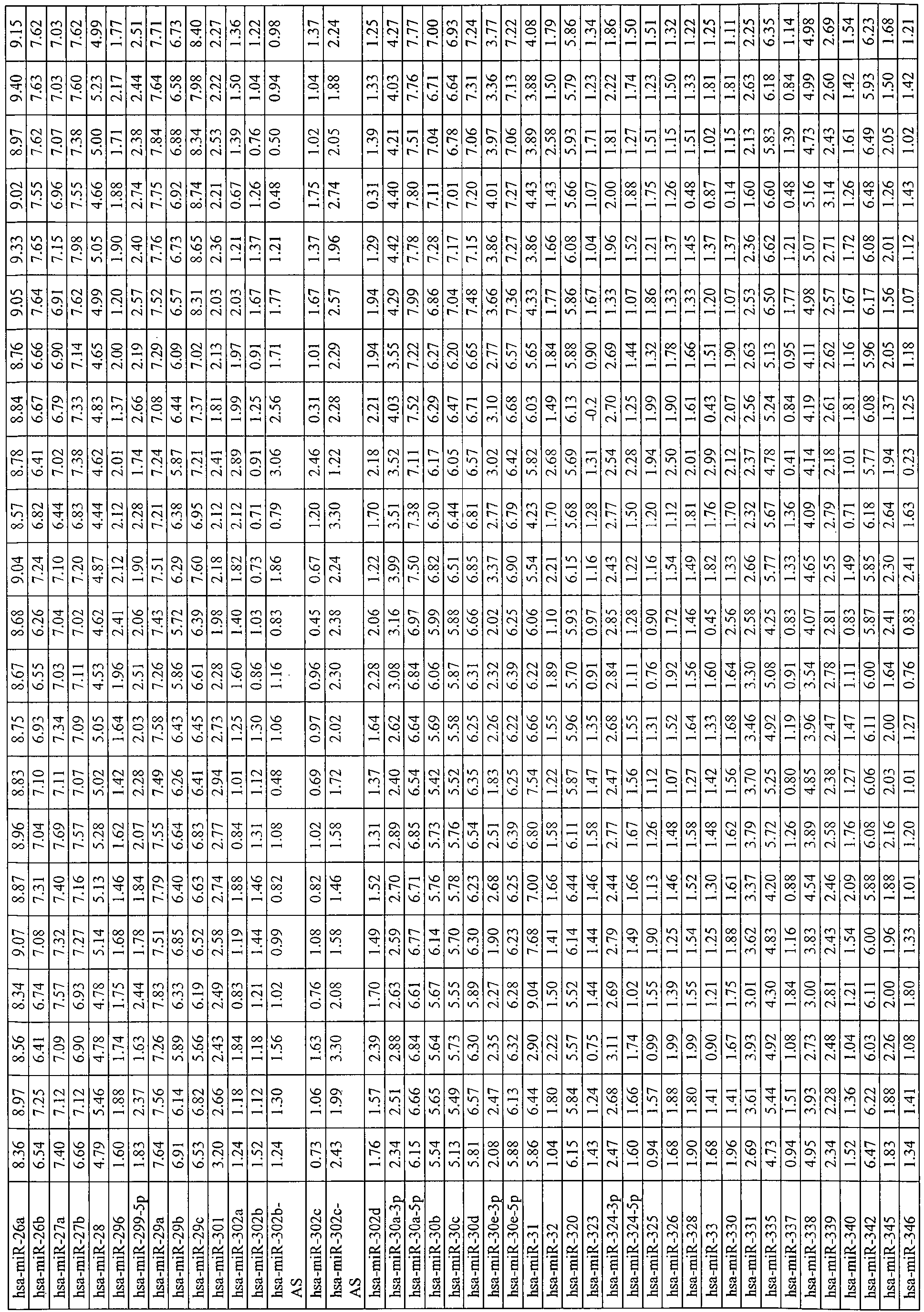

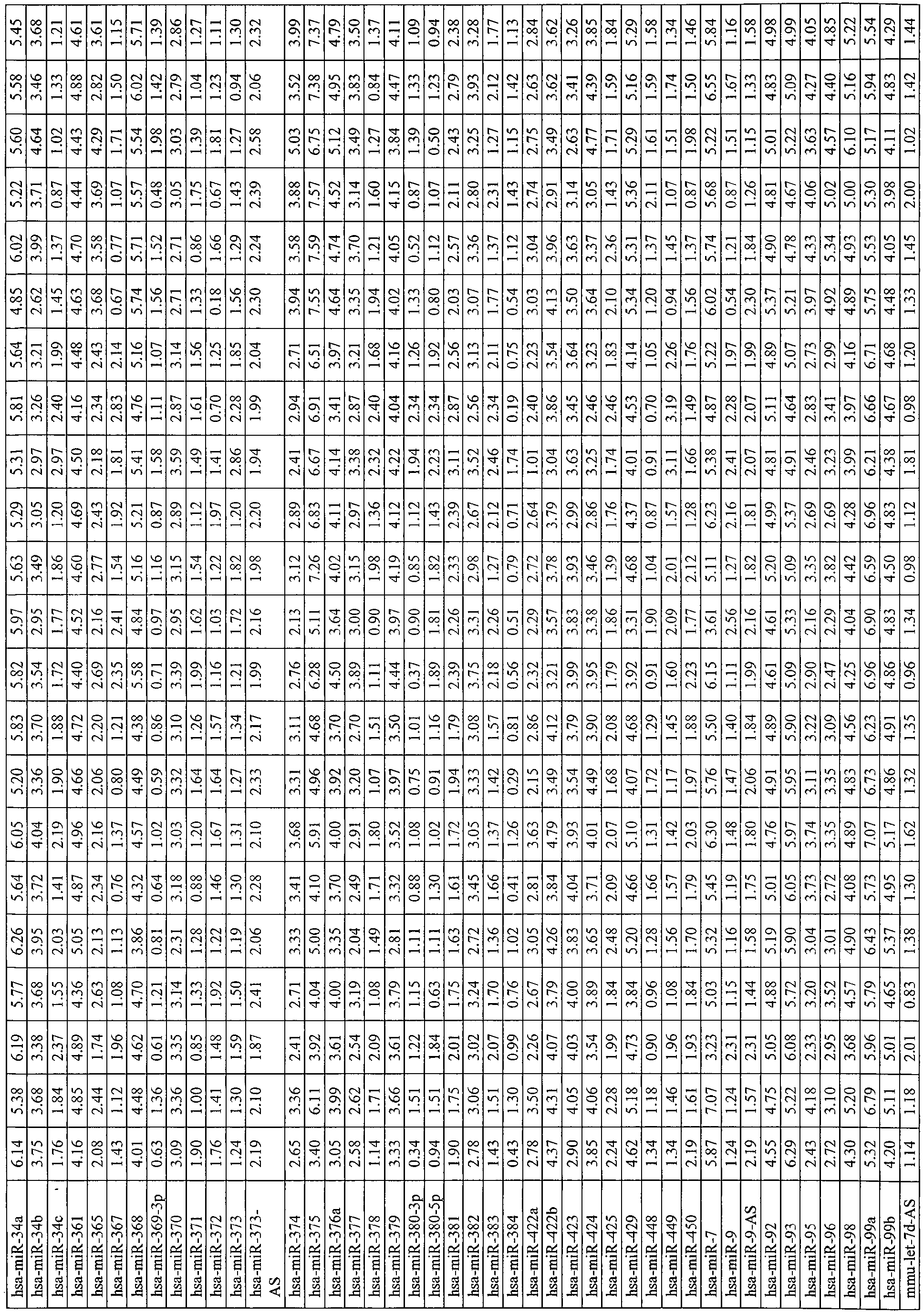

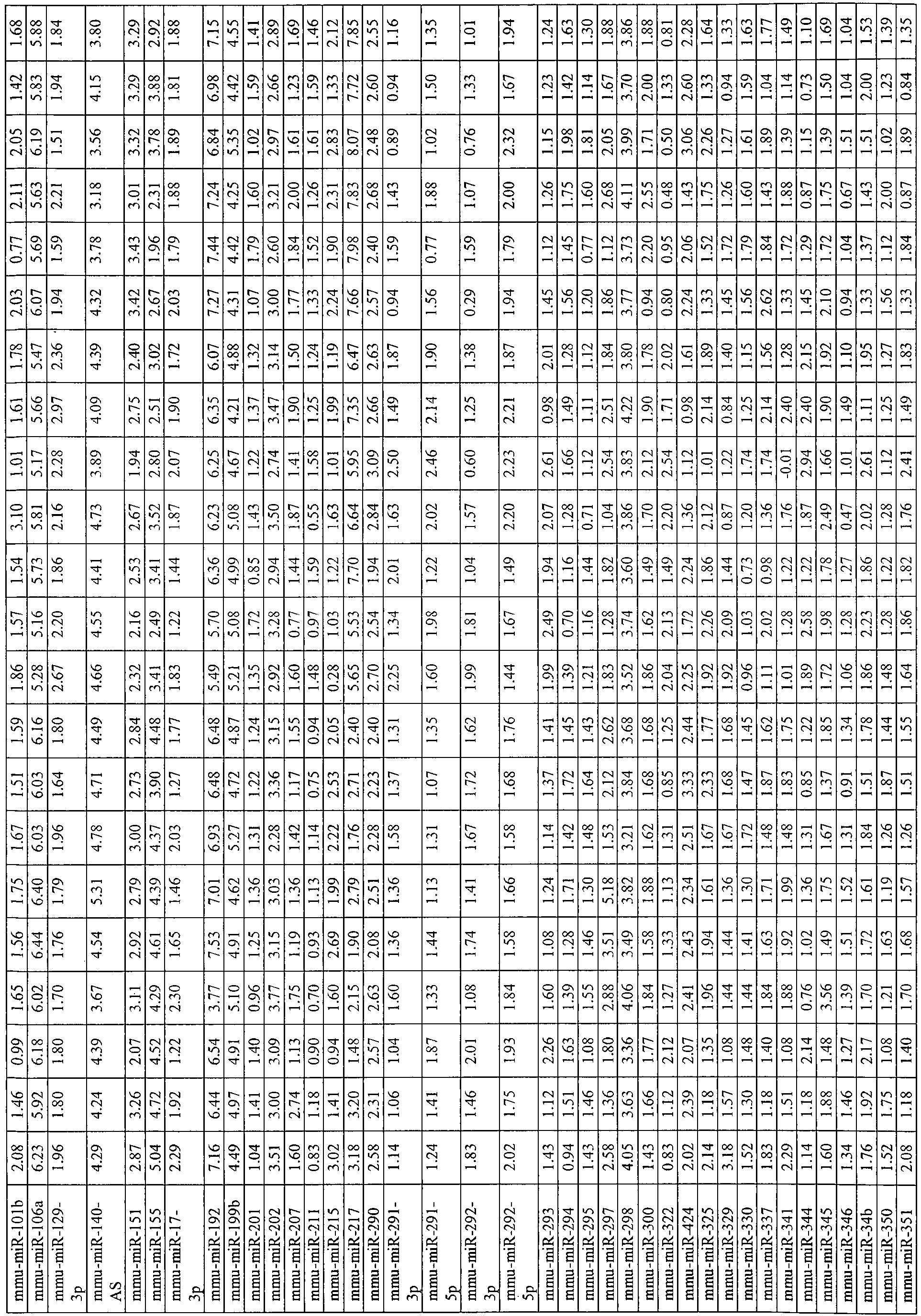

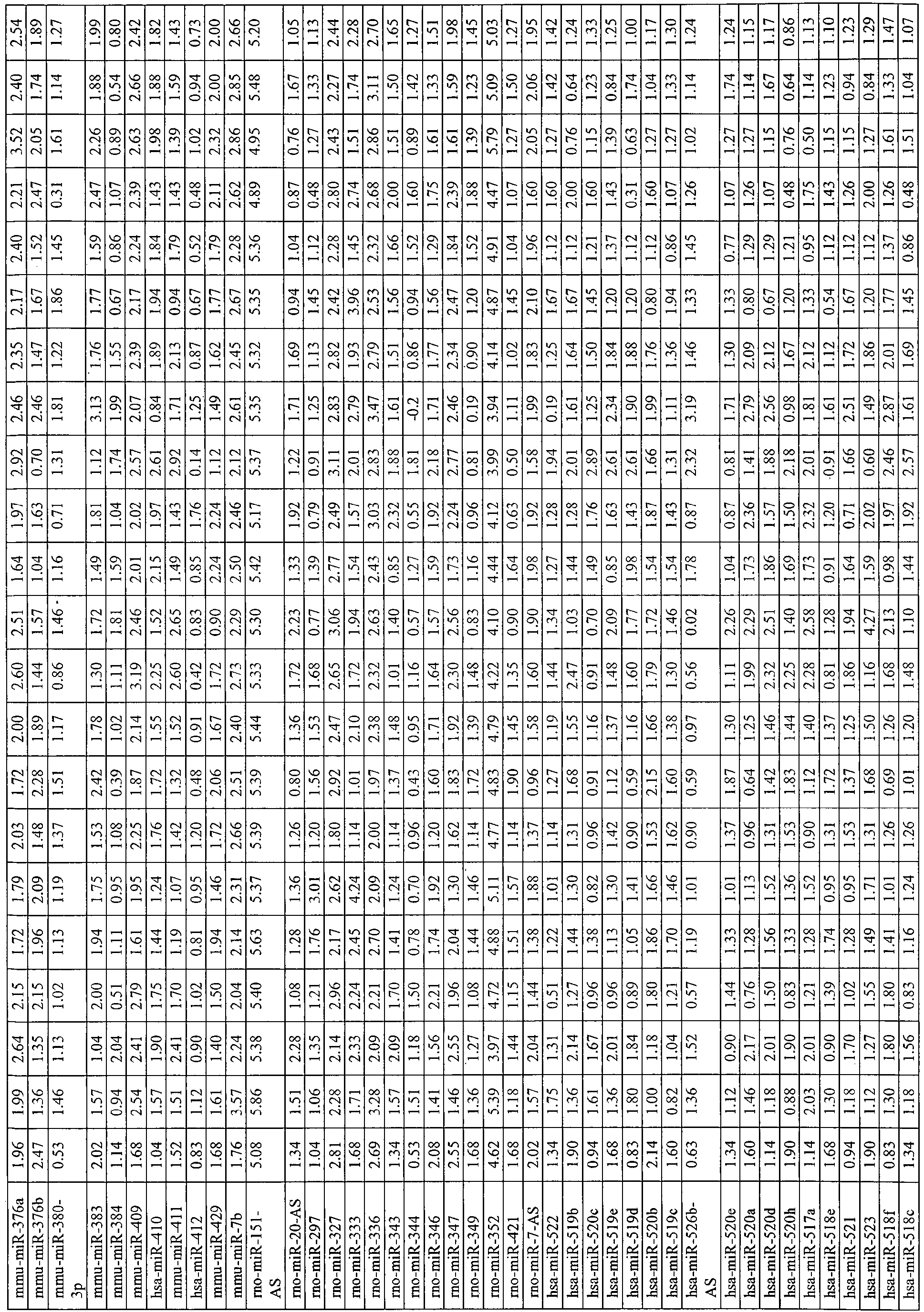

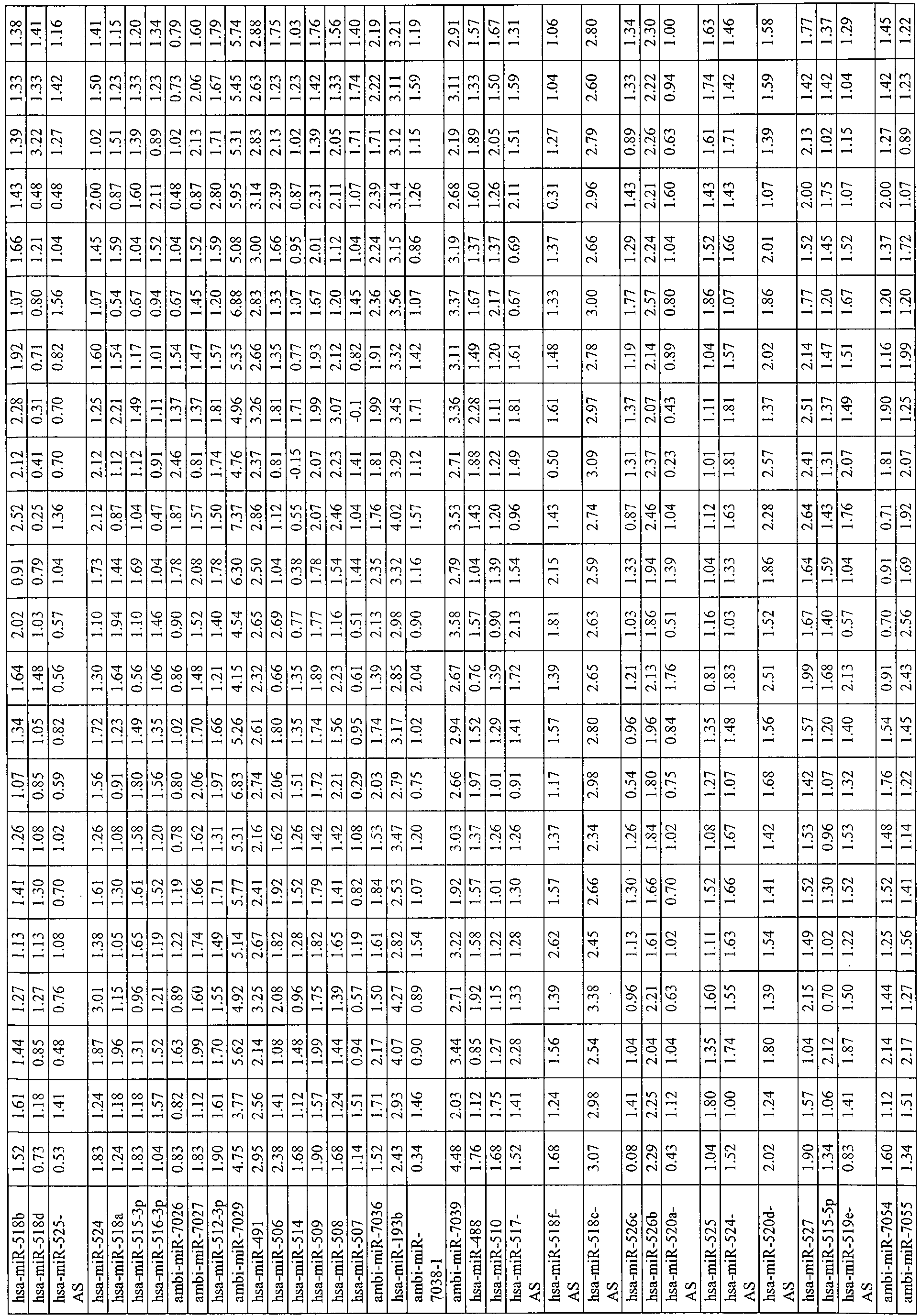

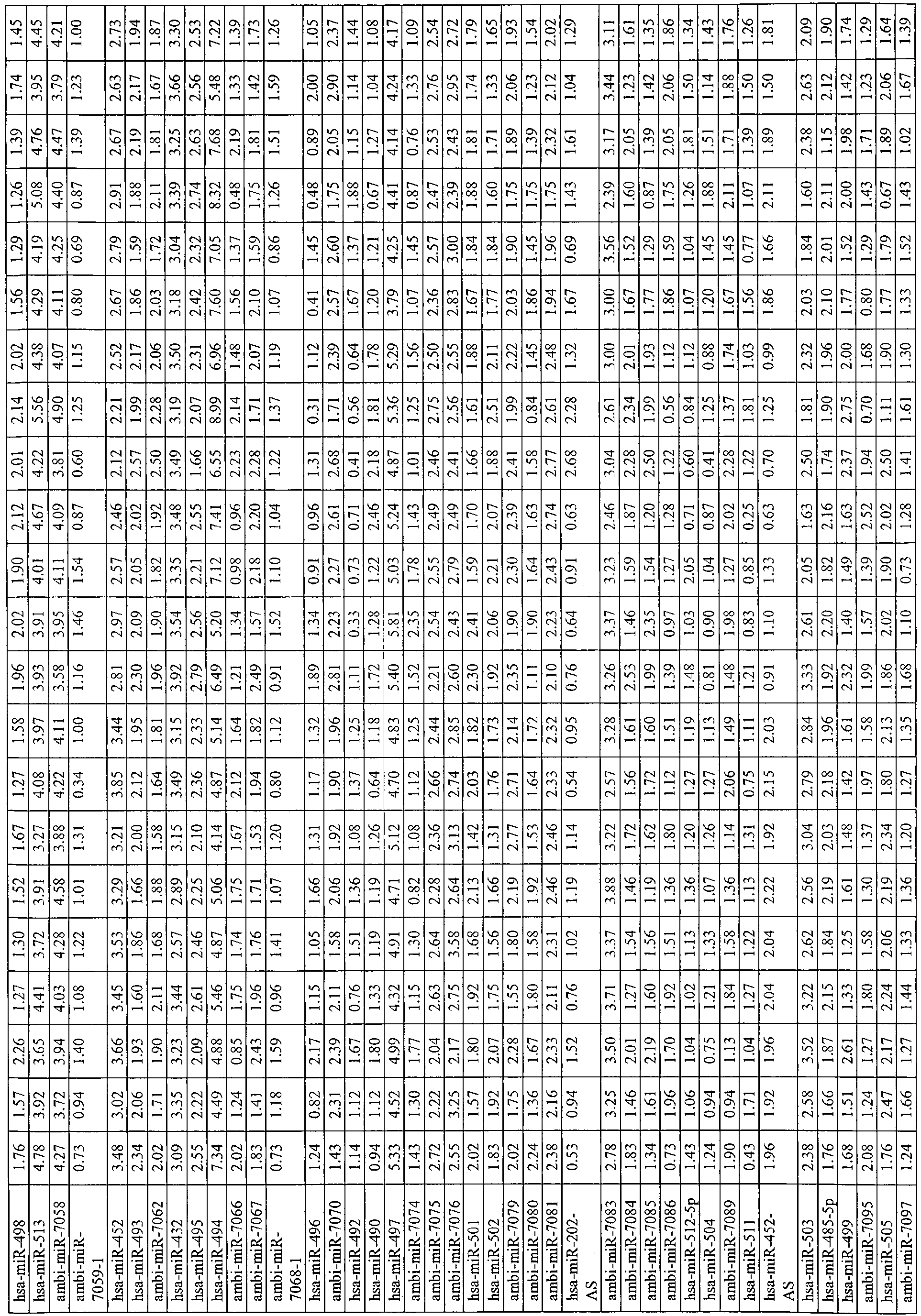

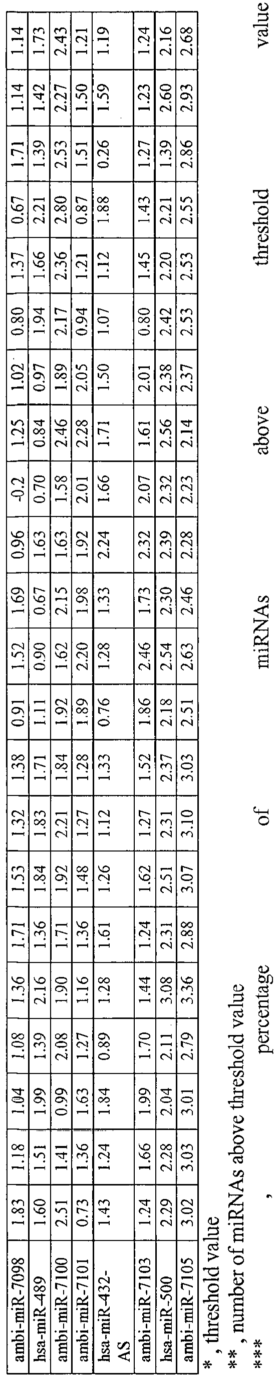

**Table 4. Normalized Array Data for Six Individual Pancreatic Cancer Cell Lines and Three Tissue Types**

| | **Cell Lines** | | | | | | **Mean** | **%*** | **Mean** | **%*** | **Mean** | **%*** | **Mean** | **%*** |
|---|---|---|---|---|---|---|---|---|---|---|---|---|---|---|
| **miR Name** | **IMIMPC2** | **PT45** | **SKPC1** | **PL45** | **PancTul** | **PaCa44** | **CL** | **CL** | **Ca** | **Ca** | **Ch** | **Ch** | **N** | **N** |
| hsa-let-7a | 8.95 | 8.40 | 9.43 | 8.46 | 8.65 | 8.32 | 8.70 | 100 | 8.95 | 100 | 8.83 | 100 | 9.35 | 100 |
| hsa-let-7b | 8.40 | 7.72 | 8.99 | 7.72 | 7.33 | 7.63 | 7.96 | 100 | 8.53 | 100 | 8.78 | 100 | 9.06 | 100 |
| hsa-let-7c | 8.44 | 7.87 | 8.93 | 8.17 | 7.77 | 7.29 | 8.08 | 100 | 8.60 | 100 | 8.74 | 100 | 9.18 | 100 |
| hsa-let-7d | 8.05 | 7.73 | 8.34 | 8.31 | 8.07 | 7.53 | 8.01 | 100 | 8.19 | 100 | 7.85 | 100 | 8.68 | 100 |
| hsa-let-7e | 6.58 | 6.52 | 6.97 | 6.82 | 6.03 | 5.86 | 6.46 | 100 | 6.75 | 100 | 6.19 | 100 | 6.98 | 100 |
| hsa-let-7f | 7.26 | 7.08 | 7.23 | 8.15 | 7.69 | 6.72 | 7.36 | 100 | 7.94 | 100 | 7.51 | 100 | 8.51 | 100 |
| hsa-let-7g | 6.53 | 6.19 | 6.76 | 6.60 | 6.61 | 5.99 | 6.45 | 100 | 7.31 | 100 | 7.01 | 100 | 7.52 | 100 |
| hsa-let-7i | 7.52 | 6.59 | 8.91 | 6.91 | 8.88 | 8.95 | 7.96 | 100 | 7.49 | 100 | 7.12 | 100 | 6.31 | 100 |
| hsa-miR-1 | 0.92 | 0.17 | 1.05 | 0.28 | 1.01 | 1.22 | 0.78 | 0 | 3.23 | 88 | 2.84 | 83 | 2.19 | 80 |
| hsa-miR-100 | 7.14 | 6.49 | 6.32 | 7.23 | 7.73 | 7.76 | 7.11 | 100 | 6.34 | 100 | 6.44 | 100 | 5.16 | 100 |
| hsa-miR-101 | 1.97 | 2.77 | 2.16 | 2.72 | 2.60 | 2.69 | 2.48 | 83 | 4.49 | 100 | 4.33 | 100 | 4.87 | 100 |
| hsa-miR-103 | 6.14 | 7.33 | 7.36 | 6.75 | 6.62 | 6.63 | 6.80 | 100 | 6.72 | 100 | 6.02 | 100 | 5.86 | 100 |
| hsa-miR-105 | -0.03 | 2.33 | 2.27 | 0.92 | 1.47 | 1.59 | 1.42 | 33 | 0.50 | 0 | 0.22 | 0 | 0.81 | 0 |
| hsa-miR-106a | 7.09 | 8.19 | 7.55 | 7.68 | 8.46 | 8.50 | 7.91 | 100 | 6.74 | 100 | 6.10 | 100 | 6.30 | 100 |
| hsa-miR-106b | 5.88 | 6.06 | 5.97 | 6.28 | 5.88 | 6.06 | 6.02 | 100 | 5.65 | 100 | 5.02 | 100 | 4.98 | 100 |
| hsa-miR-107 | 6.12 | 7.41 | 7.41 | 6.73 | 6.66 | 6.68 | 6.83 | 100 | 6.76 | 100 | 6.10 | 100 | 5.82 | 100 |
| hsa-miR-10a | 5.22 | 4.76 | 5.79 | 6.49 | 6.45 | 6.49 | 5.87 | 100 | 6.23 | 100 | 5.70 | 100 | 4.92 | 100 |
| hsa-miR-10b | 2.87 | 4.54 | 3.61 | 4.53 | 4.29 | 4.00 | 3.97 | 100 | 5.24 | 100 | 4.96 | 100 | 4.54 | 100 |
| hsa-miR-122a | 2.62 | 2.92 | 1.52 | 1.81 | 2.63 | 2.80 | 2.38 | 67 | 2.71 | 100 | 2.76 | 100 | 2.43 | 100 |
| hsa-miR-124a | -0.15 | 0.85 | 0.59 | 0.92 | 0.44 | -0.03 | 0.44 | 0 | 0.29 | 0 | -0.06 | 0 | 0.21 | 0 |
| hsa-miR-125a | 6.38 | 6.65 | 7.11 | 6.37 | 5.21 | 5.53 | 6.21 | 100 | 6.04 | 100 | 6.00 | 100 | 5.32 | 100 |
| hsa-miR-125b | 6.01 | 5.23 | 5.26 | 6.47 | 6.54 | 6.85 | 6.06 | 100 | 6.93 | 100 | 7.37 | 100 | 5.96 | 100 |
| hsa-miR-126 | 3.07 | 2.20 | 3.79 | 4.56 | 4.49 | 4.52 | 3.77 | 100 | 7.41 | 100 | 7.54 | 100 | 7.12 | 100 |
| hsa-miR-126-AS | 1.63 | -0.20 | 1.24 | 1.50 | 0.88 | 0.35 | 0.90 | 0 | 3.55 | 88 | 3.27 | 100 | 3.91 | 100 |
| hsa-miR-127 | 0.92 | 0.17 | -0.18 | 0.84 | 1.14 | 0.35 | 0.54 | 0 | 1.42 | 50 | 1.97 | 50 | 1.52 | 40 |
| hsa-miR-128a | 3.26 | 3.17 | 4.19 | 3.55 | 3.25 | 3.61 | 3.50 | 100 | 2.77 | 100 | 2.23 | 83 | 2.16 | 80 |
| hsa-miR-129 | 1.16 | 1.41 | 0.90 | 0.75 | 1.47 | 1.59 | 1.21 | 0 | 1.46 | 50 | 2.07 | 50 | 1.44 | 0 |
| hsa-miR-130a | 5.39 | 6.66 | 6.46 | 4.07 | 2.67 | 2.36 | 4.60 | 100 | 5.62 | 100 | 5.90 | 100 | 6.04 | 100 |
| hsa-miR-130b | 4.56 | 5.00 | 5.77 | 4.42 | 5.08 | 5.06 | 4.98 | 100 | 3.84 | 100 | 4.93 | 100 | 6.27 | 100 |
| hsa-miR-132 | 2.36 | 2.79 | 2.24 | 1.61 | 2.45 | 1.80 | 2.21 | 67 | 4.09 | 100 | 3.92 | 100 | 3.06 | 100 |
| hsa-miR-133a | 1.27 | 0.59 | 0.67 | 0.84 | 0.74 | 0.62 | 0.79 | 0 | 2.55 | 88 | 2.49 | 83 | 0.97 | 0 |
| hsa-miR-134 | 1.16 | 1.21 | 0.75 | 0.92 | 1.88 | 0.62 | 1.09 | 0 | 2.37 | 88 | 2.65 | 100 | 2.99 | 100 |
| hsa-miR-135a | -0.48 | 1.10 | 1.11 | 0.92 | 0.88 | 1.00 | 0.76 | 0 | 0.62 | 25 | 1.35 | 17 | 1.72 | 20 |
| hsa-miR-135b | 0.10 | 0.45 | 1.42 | 1.81 | 1.26 | 1.80 | 1.14 | 0 | 2.01 | 75 | 1.49 | 17 | 0.72 | 0 |
| hsa-miR-136 | 0.65 | 0.04 | 0.19 | 0.01 | 1.01 | 0.75 | 0.44 | 0 | 0.42 | 13 | 1.32 | 33 | 0.57 | 0 |
| hsa-miR-137 | 1.71 | 0.98 | 0.04 | 0.01 | -0.13 | 0.62 | 0.54 | 0 | 1.02 | 38 | 1.51 | 17 | 0.51 | 0 |
| hsa-miR-138 | 3.05 | 2.24 | 4.38 | 1.16 | 0.59 | 0.62 | 2.01 | 50 | 1.41 | 25 | 0.67 | 0 | 0.96 | 0 |
| hsa-miR-139 | 1.78 | 0.98 | 1.36 | 2.34 | 1.26 | 0.49 | 1.37 | 17 | 1.81 | 75 | 2.36 | 100 | 2.09 | 100 |
| hsa-miR-140 | 2.40 | 1.87 | 1.79 | 1.37 | 1.88 | 1.32 | 1.77 | 33 | 4.08 | 100 | 3.63 | 100 | 3.24 | 100 |
| hsa-miR-141 | 5.81 | 6.28 | 6.46 | 6.53 | 6.33 | 6.63 | 6.34 | 100 | 6.00 | 100 | 6.65 | 100 | 7.50 | 100 |
| hsa-miR-142-3p | 0.79 | 0.72 | 0.67 | 0.92 | 0.88 | 1.22 | 0.87 | 0 | 3.68 | 100 | 2.76 | 83 | 2.71 | 100 |
| hsa-miR-142-5p | 1.37 | 0.17 | 0.35 | 0.47 | 1.14 | 0.75 | 0.71 | 0 | 2.28 | 100 | 2.44 | 100 | 2.34 | 80 |
| hsa-miR-143 | 0.79 | 0.59 | 0.27 | 0.37 | 0.59 | 0.35 | 0.49 | 0 | 7.96 | 100 | 7.52 | 100 | 6.02 | 100 |
| hsa-miR-144 | 0.23 | 1.31 | 1.05 | 0.10 | 0.59 | -0.15 | 0.52 | 0 | -0.05 | 0 | -0.14 | 0 | 0.48 | 0 |
| hsa-miR-145 | 1.37 | 0.31 | 0.51 | 0.75 | 0.59 | 0.35 | 0.65 | 0 | 8.15 | 100 | 7.82 | 100 | 6.32 | 100 |
| hsa-miR-146a | 3.01 | 2.37 | 5.33 | 3.95 | 2.01 | 2.09 | 3.13 | 67 | 5.93 | 100 | 4.87 | 100 | 3.85 | 100 |
| hsa-miR-147 | 0.10 | 0.59 | 0.75 | 1.24 | 1.14 | 0.22 | 0.67 | 0 | 1.38 | 38 | 0.76 | 17 | 0.94 | 20 |
| hsa-miR-148a | 1.37 | 2.96 | 5.02 | 1.31 | 2.53 | 2.09 | 2.55 | 50 | 5.27 | 100 | 7.14 | 100 | 8.57 | 100 |
| hsa-miR-148b | 2.87 | 2.77 | 3.76 | 2.29 | 3.05 | 2.57 | 2.88 | 100 | 2.82 | 88 | 2.97 | 100 | 5.11 | 100 |
| hsa-miR-149 | 1.97 | 2.59 | 2.34 | 1.72 | 1.47 | 1.93 | 2.00 | 33 | 0.90 | 25 | 0.94 | 33 | 0.96 | 20 |
| hsa-miR-150 | 1.16 | 0.04 | 1.05 | 1.61 | 1.94 | 1.32 | 1.19 | 0 | 4.38 | 100 | 3.68 | 83 | 1.73 | 60 |
| hsa-miR-151 | 4.24 | 4.35 | 4.87 | 4.65 | 4.65 | 4.94 | 4.62 | 100 | 4.00 | 100 | 3.86 | 100 | 4.02 | 100 |
| hsa-miR-152 | 2.94 | 4.12 | 2.88 | 2.06 | 3.07 | 2.32 | 2.90 | 83 | 5.15 | 100 | 5.05 | 100 | 4.75 | 100 |
| hsa-miR-153 | 1.16 | 0.59 | 1.11 | 1.66 | 2.12 | 1.59 | 1.37 | 0 | 2.73 | 88 | 2.47 | 67 | 3.23 | 100 |
| hsa-miR-154 | 0.92 | 0.72 | 0.19 | 0.56 | -0.13 | 1.50 | 0.63 | 0 | 2.07 | 88 | 2.92 | 83 | 3.27 | 100 |
| hsa-miR-155 | 5.08 | 2.05 | 5.30 | 5.44 | 6.19 | 5.77 | 4.97 | 100 | 5.59 | 100 | 4.22 | 100 | 3.25 | 100 |
| hsa-miR-15a | 5.19 | 5.05 | 5.77 | 5.66 | 6.01 | 5.94 | 5.60 | 100 | 6.47 | 100 | 6.19 | 100 | 6.14 | 100 |
| hsa-miR-15b | 6.89 | 7.59 | 7.95 | 7.87 | 7.87 | 7.63 | 7.63 | 100 | 5.77 | 100 | 4.92 | 100 | 5.17 | 100 |
| hsa-miR-16 | 8.08 | 8.20 | 8.98 | 8.49 | 8.50 | 8.66 | 8.48 | 100 | 8.32 | 100 | 8.04 | 100 | 7.99 | 100 |
| hsa-miR-17-3p | 2.58 | 3.59 | 3.44 | 2.84 | 3.40 | 4.12 | 3.33 | 100 | 2.43 | 100 | 2.13 | 67 | 2.31 | 100 |
| hsa-miR-17-5p | 6.95 | 8.11 | 7.53 | 7.65 | 8.40 | 8.52 | 7.86 | 100 | 6.66 | 100 | 6.04 | 100 | 6.15 | 100 |
| hsa-miR-18a | 4.16 | 5.72 | 4.94 | 4.38 | 5.69 | 5.98 | 5.14 | 100 | 4.26 | 100 | 3.17 | 100 | 2.58 | 100 |
| hsa-miR-181a | 6.31 | 6.53 | 6.75 | 5.41 | 6.09 | 6.31 | 6.23 | 100 | 5.76 | 100 | 5.38 | 100 | 5.00 | 100 |
| hsa-miR-181b | 5.86 | 6.54 | 6.47 | 4.59 | 6.24 | 6.24 | 5.99 | 100 | 5.05 | 100 | 4.42 | 100 | 4.35 | 100 |
| hsa-miR-181c | 2.62 | 3.41 | 2.48 | 2.16 | 2.56 | 1.80 | 2.51 | 67 | 2.65 | 100 | 1.97 | 50 | 2.04 | 80 |
| hsa-miR-182 | 4.95 | 5.23 | 5.12 | 5.84 | 4.72 | 4.64 | 5.08 | 100 | 4.57 | 100 | 4.16 | 100 | 5.39 | 100 |
| hsa-miR-182-AS | 1.55 | 0.04 | 0.67 | 0.84 | 0.88 | 0.75 | 0.79 | 0 | 0.57 | 0 | 1.22 | 17 | 0.58 | 0 |
| hsa-miR-183 | 2.55 | 3.59 | 3.35 | 3.56 | 3.43 | 2.88 | 3.23 | 100 | 1.57 | 63 | 1.94 | 67 | 2.14 | 100 |
| hsa-miR-184 | 2.71 | 3.01 | 1.97 | 2.02 | 2.01 | 2.57 | 2.38 | 67 | 2.57 | 100 | 2.61 | 100 | 2.22 | 100 |
| hsa-miR-185 | 4.16 | 4.65 | 5.09 | 3.80 | 4.44 | 4.22 | 4.39 | 100 | 4.70 | 100 | 4.23 | 100 | 4.25 | 100 |
| hsa-miR-186 | 2.77 | 3.33 | 3.06 | 1.86 | 2.56 | 2.14 | 2.62 | 67 | 3.39 | 100 | 3.04 | 100 | 3.69 | 100 |
| hsa-miR-187 | 0.23 | 1.31 | 1.18 | 1.16 | 0.59 | 0.22 | 0.78 | 0 | 1.55 | 25 | 1.25 | 0 | 0.73 | 20 |
| hsa-miR-188 | 2.62 | 2.52 | 1.83 | 2.45 | 2.56 | 2.60 | 2.43 | 83 | 1.92 | 75 | 2.03 | 83 | 1.80 | 60 |
| hsa-miR-189 | 0.37 | 0.85 | 1.71 | 4.80 | 1.26 | 0.88 | 1.65 | 17 | 2.17 | 88 | 2.02 | 50 | 1.78 | 80 |
| hsa-miR-190 | 0.92 | 0.85 | 0.51 | 0.37 | 0.14 | 0.09 | 0.48 | 0 | 1.23 | 25 | 1.23 | 33 | 0.91 | 40 |
| hsa-miR-191 | 6.13 | 6.37 | 5.86 | 5.68 | 5.20 | 4.97 | 5.70 | 100 | 6.08 | 100 | 5.70 | 100 | 5.75 | 100 |
| hsa-miR-192 | 2.19 | 2.24 | 2.50 | 2.32 | 2.06 | 2.04 | 2.23 | 67 | 6.63 | 100 | 6.07 | 100 | 7.13 | 100 |
| hsa-miR-193a | 3.13 | 3.30 | 2.39 | 3.31 | 2.79 | 3.61 | 3.09 | 100 | 2.33 | 100 | 2.60 | 100 | 2.20 | 80 |
| hsa-miR-194 | 1.85 | 2.05 | 2.52 | 2.40 | 2.45 | 2.36 | 2.27 | 83 | 7.13 | 100 | 5.95 | 100 | 6.63 | 100 |
| hsa-miR-195 | 3.17 | 3.94 | 3.13 | 3.75 | 3.97 | 2.97 | 3.49 | 100 | 6.49 | 100 | 6.72 | 100 | 6.12 | 100 |
| hsa-miR-196a | 2.97 | 4.37 | 3.84 | 2.45 | 2.23 | 1.22 | 2.85 | 83 | 3.77 | 100 | 1.41 | 50 | 1.13 | 0 |
| hsa-miR-196b | 3.11 | 4.38 | 3.38 | 2.42 | 0.59 | 0.22 | 2.35 | 67 | 3.24 | 100 | 1.04 | 33 | 0.57 | 0 |
| hsa-miR-197 | 4.32 | 4.25 | 4.79 | 2.29 | 3.68 | 3.13 | 3.74 | 100 | 2.11 | 75 | 2.07 | 83 | 1.72 | 40 |
| hsa-miR-198 | 4.02 | 3.82 | 2.29 | 3.65 | 3.64 | 4.09 | 3.59 | 100 | 3.91 | 100 | 3.92 | 100 | 3.70 | 100 |
| hsa-miR-199a | 1.37 | 0.59 | 0.04 | 0.37 | 1.01 | -0.03 | 0.56 | 0 | 6.35 | 100 | 6.51 | 100 | 5.35 | 100 |
| hsa-miR-199a-AS | 1.04 | 1.10 | 1.05 | 0.10 | -0.13 | 1.12 | 0.71 | 0 | 7.06 | 100 | 7.33 | 100 | 6.43 | 100 |
| hsa-miR-199b | 0.51 | 0.98 | 0.35 | 0.92 | 0.29 | 1.32 | 0.73 | 0 | 4.58 | 100 | 4.79 | 100 | 4.36 | 100 |
| hsa-miR-19a | 3.60 | 4.70 | 4.11 | 3.98 | 4.90 | 4.82 | 4.35 | 100 | 4.27 | 100 | 4.02 | 100 | 4.74 | 100 |
| hsa-miR-19b | 5.90 | 6.89 | 6.51 | 6.42 | 6.82 | 7.04 | 6.60 | 100 | 6.20 | 100 | 6.14 | 100 | 6.50 | 100 |
| hsa-miR-20a | 6.02 | 7.04 | 6.46 | 6.92 | 7.46 | 7.67 | 6.93 | 100 | 5.99 | 100 | 5.52 | 100 | 5.64 | 100 |
| hsa-miR-200a | 5.79 | 5.32 | 6.37 | 5.27 | 5.28 | 5.74 | 5.63 | 100 | 5.52 | 100 | 5.61 | 100 | 6.57 | 100 |
| hsa-miR-200b | 7.34 | 6.77 | 7.76 | 7.01 | 6.76 | 6.94 | 7.10 | 100 | 6.72 | 100 | 6.43 | 100 | 7.50 | 100 |
| hsa-miR-200c | 8.15 | 8.60 | 8.45 | 8.67 | 8.53 | 8.38 | 8.46 | 100 | 7.28 | 100 | 7.60 | 100 | 8.59 | 100 |
| hsa-miR-203 | 0.37 | 1.66 | 1.90 | 2.52 | 4.52 | 5.13 | 2.68 | 50 | 5.19 | 100 | 2.66 | 100 | 3.50 | 100 |
| hsa-miR-204 | 1.16 | 0.17 | 1.05 | 0.28 | 0.59 | 0.75 | 0.67 | 0 | 1.34 | 38 | 1.90 | 50 | 1.80 | 60 |
| hsa-miR-205 | 5.82 | 8.76 | 8.41 | 8.75 | 6.24 | 6.11 | 7.35 | 100 | 3.12 | 75 | 1.44 | 33 | 0.90 | 20 |
| hsa-miR-206 | 1.85 | 1.50 | 1.11 | 1.37 | 1.37 | 1.80 | 1.50 | 0 | 1.69 | 50 | 2.26 | 100 | 1.47 | 40 |
| hsa-miR-208 | 0.65 | 0.04 | 0.35 | 1.16 | 1.14 | 0.75 | 0.68 | 0 | 0.92 | 0 | 1.49 | 17 | 1.02 | 0 |
| hsa-miR-21 | 10.06 | 9.69 | 10.33 | 9.66 | 9.40 | 9.51 | 9.77 | 100 | 9.80 | 100 | 9.18 | 100 | 8.71 | 100 |
| hsa-miR-210 | 5.38 | 4.58 | 5.65 | 4.00 | 4.43 | 4.25 | 4.71 | 100 | 6.61 | 100 | 4.43 | 100 | 3.79 | 100 |
| hsa-miR-211 | 0.51 | 0.85 | 0.51 | 1.16 | 1.56 | 1.12 | 0.95 | 0 | 0.47 | 13 | 0.91 | 0 | 0.43 | 0 |
| hsa-miR-212 | 1.04 | 0.85 | 1.47 | 0.56 | 1.01 | -0.46 | 0.75 | 0 | 1.18 | 13 | 1.00 | 0 | 0.87 | 20 |
| hsa-miR-213 | 1.63 | 0.98 | 1.18 | 1.01 | 0.59 | 1.87 | 1.21 | 0 | 0.48 | 0 | 1.70 | 33 | 0.41 | 0 |
| hsa-miR-214 | 1.78 | 2.05 | 1.18 | 2.06 | 2.06 | 2.36 | 1.91 | 33 | 6.14 | 100 | 6.06 | 100 | 4.65 | 100 |
| hsa-miR-215 | 0.10 | 0.98 | 0.98 | 0.47 | 1.56 | 1.66 | 0.96 | 0 | 3.91 | 88 | 2.54 | 67 | 4.33 | 100 |
| hsa-miR-216 | 1.91 | 1.31 | 0.75 | 0.56 | 1.26 | 0.49 | 1.05 | 0 | 1.64 | 50 | 5.90 | 100 | 7.09 | 100 |
| hsa-miR-217 | 0.92 | 1.50 | 1.30 | 1.44 | 1.37 | 1.12 | 1.27 | 0 | 2.19 | 88 | 6.70 | 100 | 7.86 | 100 |
| hsa-miR-218 | 2.94 | 2.77 | 1.90 | 1.81 | 2.45 | 1.59 | 2.24 | 50 | 4.10 | 100 | 4.06 | 100 | 3.54 | 100 |
| hsa-miR-219 | 0.92 | 0.59 | 0.59 | 0.10 | -0.48 | 0.88 | 0.43 | 0 | 0.80 | 13 | 1.59 | 0 | 1.17 | 0 |
| hsa-miR-22 | 5.38 | 6.48 | 5.91 | 4.58 | 5.50 | 5.72 | 5.59 | 100 | 7.25 | 100 | 7.07 | 100 | 6.79 | 100 |
| hsa-miR-220 | 0.65 | 0.04 | 0.83 | 0.92 | 1.01 | 0.49 | 0.66 | 0 | 0.66 | 0 | 0.81 | 0 | 0.69 | 0 |
| hsa-miR-221 | 7.66 | 7.21 | 8.08 | 8.47 | 7.85 | 8.12 | 7.90 | 100 | 6.50 | 100 | 5.41 | 100 | 5.02 | 100 |
| hsa-miR-222 | 7.14 | 6.81 | 7.70 | 7.62 | 7.12 | 6.90 | 7.22 | 100 | 5.99 | 100 | 4.63 | 100 | 3.92 | 100 |
| hsa-miR-223 | 0.51 | 1.50 | 0.90 | 1.61 | 1.88 | 1.74 | 1.36 | 0 | 6.86 | 100 | 5.62 | 100 | 4.47 | 100 |
| hsa-miR-224 | 2.14 | 2.24 | 5.47 | 5.07 | 5.14 | 5.12 | 4.20 | 100 | 3.83 | 100 | 2.44 | 100 | 2.56 | 100 |
| hsa-miR-23a | 7.92 | 8.35 | 7.93 | 7.89 | 7.96 | 7.65 | 7.95 | 100 | 7.67 | 100 | 7.08 | 100 | 6.91 | 100 |
| hsa-miR-23b | 7.67 | 7.99 | 7.87 | 7.45 | 7.86 | 7.60 | 7.74 | 100 | 7.62 | 100 | 7.21 | 100 | 7.16 | 100 |
| hsa-miR-24 | 7.06 | 7.71 | 7.21 | 7.47 | 7.26 | 6.87 | 7.26 | 100 | 8.07 | 100 | 7.58 | 100 | 7.15 | 100 |
| hsa-miR-25 | 5.83 | 5.58 | 5.90 | 5.98 | 5.32 | 5.37 | 5.66 | 100 | 5.69 | 100 | 5.10 | 100 | 5.42 | 100 |
| hsa-miR-26a | 7.13 | 7.40 | 8.08 | 7.55 | 7.06 | 7.19 | 7.40 | 100 | 8.80 | 100 | 8.69 | 100 | 9.00 | 100 |
| hsa-miR-26b | 5.15 | 4.70 | 5.27 | 6.18 | 5.50 | 4.89 | 5.28 | 100 | 6.98 | 100 | 6.58 | 100 | 7.46 | 100 |
| hsa-miR-27a | 7.15 | 7.25 | 6.94 | 7.42 | 7.47 | 7.57 | 7.30 | 100 | 7.39 | 100 | 6.83 | 100 | 6.87 | 100 |
| hsa-miR-27b | 6.36 | 6.17 | 6.78 | 6.38 | 7.20 | 7.57 | 6.74 | 100 | 7.13 | 100 | 7.07 | 100 | 7.47 | 100 |
| hsa-miR-28 | 4.60 | 4.71 | 4.92 | 5.09 | 5.16 | 5.20 | 4.95 | 100 | 5.08 | 100 | 4.56 | 100 | 4.82 | 100 |
| hsa-miR-296 | 1.78 | 0.98 | 1.05 | 1.31 | 1.94 | 1.32 | 1.40 | 0 | 1.16 | 25 | 1.59 | 33 | 1.32 | 0 |
| hsa-miR-299-5p | 1.04 | 1.31 | 1.05 | 1.44 | 1.14 | 1.80 | 1.30 | 0 | 1.73 | 63 | 1.85 | 50 | 2.22 | 100 |
| hsa-miR-29a | 6.90 | 7.09 | 7.52 | 8.80 | 8.02 | 8.08 | 7.73 | 100 | 7.63 | 100 | 7.22 | 100 | 7.55 | 100 |
| hsa-miR-29b | 5.40 | 5.13 | 5.49 | 6.87 | 6.74 | 6.12 | 5.96 | 100 | 6.48 | 100 | 6.02 | 100 | 6.58 | 100 |
| hsa-miR-29c | 4.27 | 4.59 | 4.78 | 5.68 | 4.44 | 3.84 | 4.60 | 100 | 6.49 | 100 | 6.95 | 100 | 8.25 | 100 |
| hsa-miR-301 | 2.58 | 3.83 | 3.01 | 2.77 | 3.79 | 3.64 | 3.27 | 100 | 2.64 | 100 | 1.77 | 33 | 1.95 | 40 |
| hsa-miR-302a | 0.65 | 0.59 | 0.90 | 0.37 | -0.26 | 1.12 | 0.56 | 0 | 0.50 | 0 | 1.50 | 17 | 0.77 | 0 |
| hsa-miR-302b | 0.79 | 0.98 | 0.98 | 1.31 | 1.14 | 1.00 | 1.03 | 0 | 0.58 | 0 | 0.01 | 0 | 0.56 | 0 |
| hsa-miR-302b-AS | 1.04 | 0.72 | 0.51 | 0.37 | 0.29 | 0.49 | 0.57 | 0 | 0.20 | 0 | 1.10 | 33 | 0.28 | 0 |
| hsa-miR-302c | 0.23 | 0.04 | 0.43 | -0.23 | 0.74 | 1.12 | 0.39 | 0 | 0.07 | 0 | 0.18 | 17 | 0.75 | 0 |
| hsa-miR-302c-AS | 2.14 | 1.66 | 1.52 | 3.84 | 2.23 | 2.09 | 2.25 | 50 | 1.67 | 50 | 1.95 | 67 | 1.89 | 80 |
| hsa-miR-302d | 1.16 | 0.59 | 0.98 | 0.66 | 1.26 | 0.75 | 0.90 | 0 | 1.13 | 13 | 1.51 | 0 | 0.65 | 0 |
| hsa-miR-30a-3p | 4.90 | 2.05 | 4.65 | 4.09 | 3.82 | 3.69 | 3.87 | 100 | 2.51 | 100 | 3.42 | 100 | 4.10 | 100 |
| hsa-miR-30a-Sp | 7.60 | 6.25 | 7.34 | 7.32 | 6.47 | 6.52 | 6.92 | 100 | 6.69 | 100 | 7.15 | 100 | 7.61 | 100 |
| hsa-miR-30b | 4.96 | 5.16 | 5.25 | 6.07 | 5.27 | 5.49 | 5.37 | 100 | 5.74 | 100 | 6.20 | 100 | 6.84 | 100 |
| hsa-miR-30c | 6.94 | 5.60 | 7.05 | 6.44 | 5.88 | 5.91 | 6.30 | 100 | 5.63 | 100 | 6.13 | 100 | 6.77 | 100 |
| hsa-miR-30d | 6.75 | 6.01 | 6.60 | 6.90 | 6.16 | 6.05 | 6.41 | 100 | 6.29 | 100 | 6.58 | 100 | 7.08 | 100 |
| hsa-miR-30e-3p | 3.93 | 2.20 | 3.67 | 3.09 | 2.73 | 2.60 | 3.04 | 100 | 2.06 | 88 | 2.54 | 83 | 3.58 | 100 |
| hsa-miR-30e-5p | 6.78 | 5.09 | 5.99 | 6.30 | 5.62 | 5.50 | 5.88 | 100 | 6.26 | 100 | 6.50 | 100 | 7.06 | 100 |
| hsa-miR-31 | 1.55 | 7.35 | 1.57 | 8.66 | 8.42 | 8.81 | 6.06 | 67 | 6.69 | 100 | 5.57 | 100 | 3.90 | 100 |
| hsa-miR-32 | -0.15 | 1.10 | 0.51 | 0.92 | 1.14 | 0.35 | 0.64 | 0 | 1.00 | 25 | 1.33 | 33 | 1.32 | 20 |
| hsa-miR-320 | 6.53 | 6.11 | 7.04 | 5.89 | 6.60 | 6.78 | 6.49 | 100 | 6.00 | 100 | 5.80 | 100 | 5.70 | 100 |
| hsa-miR-323 | 0.23 | 0.04 | 0.27 | 1.66 | 1.94 | 0.88 | 0.84 | 0 | 0.69 | 0 | 0.06 | 0 | 0.73 | 0 |
| hsa-miR-324-3p | 2.52 | 3.84 | 3.12 | 3.27 | 2.56 | 2.95 | 3.04 | 100 | 2.58 | 100 | 2.47 | 100 | 1.44 | 40 |
| hsa-miR-324-5p | 1.46 | 2.05 | 1.24 | 0.75 | 1.56 | 1.50 | 1.43 | 17 | 1.01 | 25 | 0.73 | 17 | 0.95 | 0 |
| hsa-miR-325 | 0.23 | 0.59 | 0.67 | 0.10 | 1.01 | 1.12 | 0.62 | 0 | 0.60 | 13 | 0.58 | 0 | 0.94 | 0 |
| hsa-miR-326 | 0.92 | 1.58 | 1.11 | 1.09 | 1.37 | 0.49 | 1.09 | 0 | 0.95 | 13 | 1.28 | 17 | 0.71 | 0 |
| hsa-miR-328 | 0.51 | 1.58 | 1.18 | 1.09 | 1.37 | 0.75 | 1.08 | 0 | 1.15 | 13 | 1.09 | 0 | 0.60 | 0 |
| hsa-miR-33 | 0.79 | 1.31 | 0.67 | 0.56 | 0.74 | 0.88 | 0.83 | 0 | 0.64 | 0 | 0.89 | 17 | 0.64 | 0 |
| hsa-miR-330 | 1.97 | 2.20 | 2.50 | 0.92 | 2.18 | 1.59 | 1.89 | 50 | 1.23 | 25 | 1.45 | 17 | 0.49 | 0 |
| hsa-miR-331 | 3.95 | 4.06 | 4.08 | 3.73 | 4.30 | 3.11 | 3.87 | 100 | 3.45 | 100 | 2.40 | 100 | 1.91 | 80 |
| hsa-miR-335 | 4.70 | 0.31 | 3.60 | 3.58 | 3.92 | 4.09 | 3.37 | 83 | 4.96 | 100 | 5.05 | 100 | 6.19 | 100 |
| hsa-miR-337 | 0.92 | 0.45 | 1.05 | -0.08 | 0.14 | 0.75 | 0.54 | 0 | 0.40 | 0 | 0.08 | 0 | 0.47 | 0 |
| hsa-miR-338 | 0.79 | -0.08 | 0.04 | 3.40 | -0.13 | 1.74 | 0.96 | 17 | 3.97 | 100 | 4.01 | 100 | 4.82 | 100 |
| hsa-miR-339 | 2.74 | 4.16 | 3.74 | 3.61 | 3.05 | 3.54 | 3.47 | 100 | 2.33 | 100 | 2.39 | 83 | 2.43 | 100 |
| hsa-miR-340 | 0.37 | 0.45 | 1.18 | 0.75 | 0.44 | -0.26 | 0.49 | 0 | 0.88 | 13 | 0.37 | 0 | 1.00 | 0 |
| hsa-miR-342 | 5.37 | 5.66 | 4.09 | 5.17 | 5.64 | 5.49 | 5.24 | 100 | 6.15 | 100 | 5.88 | 100 | 6.07 | 100 |
| hsa-miR-345 | 2.71 | 1.21 | 2.13 | 2.13 | 1.47 | 0.88 | 1.75 | 33 | 1.71 | 75 | 1.64 | 50 | 1.19 | 40 |
| hsa-miR-346 | 0.79 | -0.31 | 0.35 | 0.75 | 1.14 | 0.88 | 0.60 | 0 | 0.53 | 0 | 0.42 | 17 | 0.55 | 0 |
| hsa-miR-34a | 4.04 | 3.35 | 3.95 | 1.44 | 2.87 | 4.22 | 3.31 | 83 | 5.87 | 100 | 5.56 | 100 | 5.29 | 100 |
| hsa-miR-34b | 2.03 | 2.05 | 2.07 | 1.24 | 1.94 | 2.40 | 1.95 | 50 | 3.70 | 100 | 3.06 | 100 | 3.48 | 100 |
| hsa-miR-34c | 0.51 | 1.80 | 1.87 | 0.19 | 0.74 | -0.03 | 0.85 | 17 | 1.51 | 63 | 1.53 | 17 | 0.56 | 0 |
| hsa-miR-361 | 4.92 | 5.41 | 5.56 | 5.26 | 5.03 | 5.13 | 5.22 | 100 | 4.76 | 100 | 4.39 | 100 | 4.45 | 100 |
| hsa-miR-365 | 2.62 | 2.89 | 2.91 | 2.97 | 2.87 | 2.44 | 2.78 | 100 | 1.97 | 75 | 2.16 | 50 | 3.41 | 100 |
| hsa-miR-367 | -0.03 | 0.31 | 0.98 | 1.31 | 1.01 | 0.49 | 0.68 | 0 | 0.43 | 0 | 1.78 | 50 | 0.48 | 0 |
| hsa-miR-368 | 0.51 | 0.31 | 0.59 | 0.92 | 1.14 | 0.75 | 0.71 | 0 | 4.41 | 100 | 5.08 | 100 | 5.56 | 100 |
| hsa-miR-369-3p | -0.27 | 1.21 | 1.71 | 0.01 | 0.29 | 1.22 | 0.69 | 0 | -0.08 | 0 | 0.21 | 0 | 0.83 | 20 |
| hsa-miR-370 | 2.74 | 3.13 | 2.34 | 2.68 | 2.85 | 2.92 | 2.78 | 100 | 3.05 | 100 | 2.98 | 100 | 2.62 | 100 |
| hsa-miR-371 | 0.37 | 0.45 | 0.75 | 1.01 | 0.74 | 0.22 | 0.59 | 0 | 0.52 | 0 | 0.97 | 0 | 0.62 | 0 |
| hsa-miR-372 | -0.38 | 0.98 | 0.51 | 0.92 | 0.74 | 1.42 | 0.70 | 0 | 1.03 | 25 | 0.48 | 0 | 0.48 | 20 |
| hsa-miR-373 | 0.23 | 0.17 | 1.11 | 0.28 | 0.59 | 0.75 | 0.52 | 0 | 0.64 | 0 | 1.32 | 17 | 0.66 | 0 |
| hsa-miR-373-AS | 2.03 | 2.33 | 1.42 | 1.61 | 1.94 | 1.99 | 1.89 | 17 | 1.94 | 75 | 1.67 | 17 | 2.00 | 80 |
| hsa-miR-374 | 1.91 | 1.93 | 1.83 | 0.92 | 1.88 | 1.00 | 1.58 | 17 | 3.06 | 100 | 2.48 | 83 | 3.80 | 100 |
| hsa-miR-375 | 0.51 | 0.72 | 0.19 | 1.76 | 0.59 | 1.32 | 0.85 | 0 | 4.70 | 100 | 6.43 | 100 | 7.21 | 100 |
| hsa-miR-376a | 0.37 | 0.45 | 0.67 | 0.92 | 1.37 | 1.12 | 0.82 | 0 | 3.70 | 100 | 3.86 | 100 | 4.63 | 100 |
| hsa-miR-377 | 0.65 | -0.08 | 0.19 | 2.34 | 1.01 | 1.00 | 0.85 | 17 | 2.59 | 100 | 3.05 | 100 | 3.30 | 100 |
| hsa-miR-378 | 2.09 | 0.72 | 2.04 | 2.06 | 2.56 | 2.75 | 2.04 | 50 | 0.94 | 38 | 1.08 | 33 | 0.75 | 0 |
| hsa-miR-379 | 2.48 | 1.99 | 1.18 | 1.81 | 2.23 | 2.44 | 2.02 | 67 | 3.49 | 100 | 4.06 | 100 | 3.93 | 100 |
| hsa-miR-380-3p | 0.37 | 0.45 | 1.24 | 0.84 | 1.56 | 0.49 | 0.82 | 0 | 0.14 | 0 | 0.50 | 0 | 0.40 | 0 |
| hsa-miR-380-5p | 1.16 | 0.17 | 0.35 | 0.47 | 1.26 | -0.37 | 0.51 | 0 | 0.36 | 0 | 1.48 | 0 | 0.22 | 0 |
| hsa-miR-381 | 0.92 | 0.98 | 0.75 | 0.75 | 1.14 | 1.22 | 0.96 | 0 | 1.39 | 38 | 2.31 | 83 | 2.08 | 60 |
| hsa-miR-382 | 1.37 | 1.31 | 0.43 | 0.56 | 1.47 | 0.88 | 1.00 | 0 | 3.04 | 100 | 2.96 | 100 | 3.07 | 100 |
| hsa-miR-383 | 1.04 | 0.45 | 1.11 | 0.47 | 1.01 | 1.42 | 0.92 | 0 | 1.02 | 13 | 1.73 | 17 | 1.29 | 20 |
| hsa-miR-384 | -0.15 | 1.10 | 1.05 | 0.01 | -0.13 | 0.62 | 0.41 | 0 | -0.12 | 0 | -0.15 | 0 | 0.46 | 0 |
| hsa-miR-422a | 2.90 | 2.37 | 3.94 | 3.86 | 3.89 | 4.26 | 3.54 | 100 | 2.77 | 100 | 1.89 | 67 | 2.60 | 100 |
| hsa-miR-422b | 3.87 | 2.99 | 4.84 | 4.37 | 4.51 | 5.00 | 4.26 | 100 | 4.13 | 100 | 3.41 | 100 | 3.43 | 100 |
| hsa-miR-423 | 4.90 | 5.07 | 5.35 | 4.99 | 5.10 | 4.98 | 5.07 | 100 | 3.79 | 100 | 3.51 | 100 | 3.05 | 100 |
| hsa-miR-424 | 2.62 | 2.94 | 2.01 | 0.84 | 0.59 | 0.35 | 1.56 | 50 | 3.91 | 100 | 3.07 | 83 | 3.66 | 100 |
| hsa-miR-425 | 2.44 | 2.89 | 2.24 | 1.50 | 1.73 | 2.04 | 2.14 | 50 | 1.82 | 38 | 1.35 | 0 | 1.39 | 40 |
| hsa-miR-429 | 4.74 | 4.43 | 4.83 | 4.03 | 4.42 | 4.09 | 4.42 | 100 | 4.71 | 100 | 4.03 | 100 | 5.13 | 100 |
| hsa-miR-448 | 0.65 | 0.45 | 0.27 | 0.47 | 0.88 | 0.09 | 0.47 | 0 | 0.58 | 0 | 0.24 | 0 | 1.04 | 0 |
| hsa-miR-449 | 0.79 | 0.85 | 2.01 | 1.81 | 1.01 | 1.32 | 1.30 | 17 | 0.82 | 0 | 1.89 | 50 | 0.75 | 0 |
| hsa-miR-450 | 0.92 | 0.72 | 1.66 | 1.31 | 1.26 | 1.74 | 1.27 | 0 | 1.54 | 38 | 1.25 | 17 | 0.90 | 20 |
| hsa-miR-7 | 5.00 | 5.41 | 4.64 | 4.71 | 5.04 | 3.91 | 4.78 | 100 | 5.54 | 100 | 5.14 | 100 | 5.68 | 100 |
| hsa-miR-9 | 0.23 | 0.59 | 0.51 | 1.24 | 0.00 | 1.22 | 0.63 | 0 | 0.73 | 13 | 1.50 | 33 | 0.52 | 0 |
| hsa-miR-9-AS | 1.37 | 2.65 | 1.47 | 2.68 | 1.47 | 1.93 | 1.93 | 33 | 1.45 | 38 | 1.59 | 0 | 1.04 | 20 |
| hsa-miR-92 | 5.42 | 6.69 | 6.45 | 6.22 | 6.65 | 6.65 | 6.35 | 100 | 4.92 | 100 | 4.80 | 100 | 4.82 | 100 |
| hsa-miR-93 | 6.58 | 6.97 | 6.65 | 7.36 | 6.76 | 6.39 | 6.78 | 100 | 5.94 | 100 | 4.99 | 100 | 4.83 | 100 |
| hsa-miR-95 | 1.04 | 2.05 | 1.66 | 0.92 | 1.47 | 1.22 | 1.39 | 17 | 3.17 | 100 | 2.51 | 83 | 3.87 | 100 |
| hsa-miR-96 | 2.68 | 3.20 | 3.49 | 3.96 | 2.76 | 2.51 | 3.10 | 100 | 3.04 | 100 | 2.79 | 100 | 4.69 | 100 |
| hsa-miR-98 | 3.99 | 4.72 | 4.00 | 4.57 | 4.73 | 3.30 | 4.22 | 100 | 4.58 | 100 | 4.05 | 100 | 5.05 | 100 |
| hsa-miR-99a | 6.15 | 5.38 | 4.67 | 6.30 | 6.79 | 6.54 | 5.97 | 100 | 6.27 | 100 | 6.64 | 100 | 5.38 | 100 |
| hsa-miR-99b | 5.43 | 5.99 | 6.01 | 4.95 | 4.61 | 4.30 | 5.22 | 100 | 4.95 | 100 | 4.59 | 100 | 4.12 | 100 |
| mmu-let-7d-AS | -0.15 | 0.04 | 0.90 | 0.37 | 0.44 | -0.64 | 0.16 | 0 | 0.67 | 13 | 0.40 | 0 | 0.86 | 0 |
| mmu-miR-101b | 0.51 | 0.04 | 0.98 | 1.66 | 0.44 | 1.42 | 0.84 | 0 | 1.07 | 13 | 1.24 | 17 | 1.16 | 20 |
| mmu-miR-106a | 6.53 | 7.67 | 7.00 | 7.20 | 7.92 | 7.90 | 7.37 | 100 | 6.20 | 100 | 5.39 | 100 | 5.72 | 100 |
| mmu-miR-129-3p | 1.46 | 0.45 | 1.62 | 1.44 | 1.80 | 1.80 | 1.43 | 0 | 1.42 | 38 | 2.06 | 50 | 1.40 | 0 |
| mmu-miR-140-AS | 3.09 | 2.15 | 3.15 | 1.24 | 2.01 | 2.23 | 2.31 | 50 | 4.52 | 100 | 4.29 | 100 | 3.61 | 100 |
| mmu-miR-151 | 3.54 | 3.56 | 3.91 | 4.01 | 4.10 | 4.06 | 3.86 | 100 | 2.76 | 100 | 2.11 | 67 | 3.08 | 100 |
| mmu-miR-155 | 3.26 | 0.45 | 2.91 | 3.99 | 4.60 | 2.90 | 3.02 | 83 | 4.51 | 100 | 2.84 | 100 | 2.65 | 100 |
| mmu-miR-17-3p | 2.48 | 2.65 | 2.32 | 1.81 | 2.60 | 3.01 | 2.48 | 83 | 1.33 | 38 | 1.18 | 0 | 1.46 | 20 |
| mmu-miR-192 | 1.91 | 2.24 | 2.04 | 2.06 | 2.45 | 2.19 | 2.15 | 50 | 6.52 | 100 | 5.99 | 100 | 7.00 | 100 |
| mmu-miR-199b | 0.51 | 0.59 | 0.67 | 0.84 | 0.59 | 0.88 | 0.68 | 0 | 4.91 | 100 | 4.79 | 100 | 4.38 | 100 |
| mmu-miR-201 | -0.03 | -0.51 | 0.67 | 0.47 | 0.88 | 0.49 | 0.33 | 0 | 0.49 | 0 | 0.60 | 0 | 0.84 | 0 |
| mmu-miR-202 | 2.65 | 2.68 | 2.22 | 2.57 | 2.76 | 3.27 | 2.69 | 100 | 3.11 | 100 | 2.98 | 100 | 2.65 | 100 |
| mmu-miR-207 | 2.14 | 1.21 | 1.79 | 1.37 | 1.88 | 1.50 | 1.65 | 17 | 0.95 | 13 | 0.86 | 0 | 1.19 | 0 |
| mmu-miR-211 | 1.04 | -0.08 | 0.75 | 0.28 | -0.13 | 1.00 | 0.48 | 0 | 0.02 | 0 | 0.49 | 0 | 0.90 | 0 |
| mmu-miR-215 | -0.03 | 0.45 | 0.90 | -0.23 | -0.13 | 0.35 | 0.22 | 0 | 1.72 | 50 | 0.43 | 0 | 1.72 | 60 |
| mmu-miR-217 | 1.04 | 0.17 | -0.04 | 1.16 | 1.47 | 0.22 | 0.67 | 0 | 2.17 | 88 | 6.40 | 100 | 7.70 | 100 |
| mmu-miR-290 | 3.33 | 2.84 | 2.99 | 2.68 | 2.87 | 2.54 | 2.87 | 100 | 2.24 | 100 | 2.39 | 83 | 2.27 | 100 |
| mmu-miR-291-3p | 0.65 | 0.31 | 0.04 | 1.44 | 1.94 | 0.09 | 0.74 | 0 | 0.61 | 0 | 1.38 | 33 | 0.48 | 0 |
| mmu-miR-291-5p | 1.55 | 0.85 | 0.59 | 1.16 | 1.01 | 1.59 | 1.13 | 0 | 0.66 | 0 | 1.44 | 17 | 0.73 | 0 |
| mmu-miR-292-3p | 0.92 | 1.31 | 1.36 | 1.01 | 1.56 | 1.12 | 1.21 | 0 | 1.11 | 25 | 0.72 | 0 | 0.33 | 0 |
| mmu-miR-292-5p | 1.04 | 1.31 | 0.51 | 1.56 | 0.59 | 1.50 | 1.09 | 0 | 1.34 | 13 | 1.39 | 0 | 1.53 | 20 |
| mmu-miR-293 | 1.63 | -0.20 | 0.98 | 0.84 | 1.14 | 0.09 | 0.75 | 0 | 0.73 | 13 | 1.59 | 33 | 0.59 | 0 |
| mmu-miR-294 | 0.65 | -0.31 | 0.43 | 0.92 | 0.74 | 2.88 | 0.89 | 17 | 0.84 | 0 | 0.53 | 0 | 1.11 | 20 |
| mmu-miR-295 | -0.15 | 0.45 | 0.83 | 0.10 | 0.44 | 1.00 | 0.44 | 0 | 0.80 | 0 | 0.31 | 0 | 0.66 | 20 |
| mmu-miR-297 | 0.79 | 1.10 | 0.43 | 1.24 | 0.59 | 0.62 | 0.79 | 0 | 2.37 | 63 | 1.32 | 33 | 1.41 | 40 |
| mmu-miR-298 | 3.75 | 3.62 | 2.41 | 3.11 | 3.35 | 3.56 | 3.30 | 100 | 3.68 | 100 | 3.68 | 100 | 3.68 | 100 |
| mmu-miR-300 | 0.92 | 0.85 | 1.24 | 1.50 | 0.74 | 1.00 | 1.04 | 0 | 1.22 | 25 | 1.28 | 0 | 1.45 | 40 |
| mmu-miR-322 | 1.04 | 1.41 | 0.51 | 1.01 | 1.80 | 0.49 | 1.04 | 0 | 0.49 | 13 | 1.61 | 17 | 0.07 | 0 |
| mmu-miR-424 | 0.92 | 1.50 | 1.36 | 0.10 | 0.74 | 0.75 | 0.89 | 0 | 2.27 | 88 | 1.04 | 17 | 1.93 | 80 |
| mmu-miR-325 | 0.65 | 1.41 | 0.51 | 1.01 | 0.29 | 0.62 | 0.75 | 0 | 1.34 | 50 | 1.45 | 0 | 1.11 | 20 |
| mmu-miR-329 | 0.51 | 0.17 | 0.59 | 1.01 | 1.47 | 4.55 | 1.38 | 17 | 1.16 | 25 | 0.75 | 0 | 0.71 | 0 |
| mmu-miR-330 | 0.37 | 1.80 | 0.83 | 1.09 | 1.01 | -0.03 | 0.85 | 17 | 0.84 | 13 | 0.33 | 0 | 1.13 | 0 |
| mmu-miR-337 | -0.03 | 0.59 | 0.43 | 1.01 | 0.88 | 0.49 | 0.56 | 0 | 1.11 | 13 | 0.94 | 0 | 1.27 | 40 |
| mmu-miR-341 | 0.37 | 0.72 | 0.67 | 0.84 | 0.88 | 1.00 | 0.75 | 0 | 1.32 | 25 | 0.58 | 0 | 0.92 | 0 |
| mmu-miR-344 | 0.79 | -0.31 | -0.04 | 0.56 | 0.14 | 0.22 | 0.23 | 0 | 0.45 | 13 | 1.76 | 33 | 0.40 | 0 |
| mmu-miR-345 | 1.46 | 2.20 | 1.30 | 1.81 | 2.12 | 2.09 | 1.83 | 17 | 1.40 | 38 | 1.48 | 17 | 1.20 | 20 |
| mmu-miR-346 | 1.55 | 0.72 | 0.59 | 1.09 | -0.26 | 1.00 | 0.78 | 0 | 0.65 | 0 | 0.29 | 0 | 0.33 | 0 |
| mmu-miR-34b | 1.91 | 1.58 | 1.83 | 1.98 | 1.65 | 1.42 | 1.73 | 0 | 1.37 | 50 | 1.51 | 17 | 0.99 | 0 |
| mmu-miR-350 | 0.92 | 0.45 | 0.59 | 0.47 | 0.29 | 1.87 | 0.76 | 0 | 0.81 | 25 | 0.52 | 0 | 0.77 | 0 |
| mmu-miR-351 | 0.79 | 1.41 | 1.05 | 1.24 | 1.26 | 1.32 | 1.18 | 0 | 0.99 | 0 | 1.34 | 17 | 0.75 | 20 |
| mmu-miR-376a | 0.92 | 0.31 | 0.04 | 0.56 | 0.00 | 0.62 | 0.41 | 0 | 1.70 | 63 | 2.04 | 50 | 2.25 | 80 |
| mmu-miR-376b | 0.51 | 0.72 | 0.75 | 0.92 | 1.14 | 1.74 | 0.96 | 0 | 1.51 | 50 | 0.83 | 0 | 1.45 | 40 |
| mmu-miR-380-3p | 1.55 | 1.31 | 1.11 | 0.10 | 1.94 | 0.62 | 1.11 | 0 | 0.39 | 0 | 0.45 | 0 | 0.67 | 0 |
| mmu-miR-383 | 0.37 | 0.59 | 0.90 | 0.75 | 1.01 | 1.22 | 0.81 | 0 | 1.36 | 38 | 1.21 | 17 | 1.59 | 40 |
| mmu-miR-384 | 0.10 | 0.31 | 1.05 | 1.09 | 0.88 | -0.46 | 0.49 | 0 | 0.15 | 13 | 0.93 | 0 | 0.02 | 0 |
| mmu-miR-409 | 0.79 | 1.31 | 1.24 | 0.92 | 1.14 | 0.35 | 0.96 | 0 | 1.86 | 63 | 2.09 | 67 | 2.12 | 100 |
| hsa-miR-410 | 1.16 | 0.85 | 1.83 | 0.28 | -0.26 | 1.00 | 0.81 | 0 | 1.00 | 13 | 1.41 | 33 | 1.38 | 20 |
| mmu-miR-411 | 0.37 | 0.72 | 1.11 | 1.44 | 1.37 | 0.09 | 0.85 | 0 | 0.92 | 13 | 1.73 | 50 | 0.86 | 0 |
| hsa-miR-412 | 0.51 | 0.85 | 1.36 | 0.47 | 0.59 | 0.88 | 0.78 | 0 | -0.02 | 0 | 0.01 | 0 | -0.06 | 0 |
| mmu-miR-429 | 1.63 | 0.31 | 1.36 | 1.01 | 1.01 | 1.42 | 1.12 | 0 | 1.20 | 38 | 1.02 | 33 | 1.61 | 20 |
| mmu-miR-7b | 1.63 | 1.21 | 0.75 | 1.44 | 1.47 | 1.32 | 1.30 | 0 | 2.21 | 88 | 2.19 | 83 | 2.39 | 100 |
| mo-miR-151-AS | 5.52 | 5.77 | 6.30 | 5.95 | 5.91 | 6.20 | 5.94 | 100 | 5.48 | 100 | 5.24 | 100 | 5.04 | 100 |
| rno-miR-20-AS | 1.04 | 1.10 | 0.83 | 0.92 | 1.26 | 0.75 | 0.98 | 0 | 0.67 | 13 | 1.15 | 0 | 0.37 | 0 |
| rno-miR-297 | 0.37 | -0.60 | 0.51 | 0.37 | -0.13 | -0.03 | 0.08 | 0 | 0.90 | 25 | 0.34 | 0 | 0.47 | 0 |
| rno-miR-327 | 2.68 | 2.05 | 1.71 | 2.16 | 1.80 | 2.75 | 2.19 | 50 | 2.30 | 100 | 2.62 | 100 | 2.14 | 80 |
| rno-miR-333 | 1.37 | 0.85 | 0.59 | 0.92 | 1.37 | 1.66 | 1.13 | 0 | 1.71 | 63 | 1.48 | 17 | 1.86 | 40 |
| rno-miR-336 | 2.36 | 2.15 | 1.97 | 1.98 | 2.06 | 2.60 | 2.19 | 67 | 2.20 | 88 | 2.58 | 100 | 2.44 | 100 |
| rno-miR-343 | 0.92 | 0.98 | 0.51 | 1.44 | 0.29 | 1.50 | 0.94 | 0 | 0.87 | 13 | 0.85 | 17 | 1.14 | 0 |
| rno-miR-344 | 1.04 | 0.85 | 1.11 | 0.92 | 1.26 | 1.00 | 1.03 | 0 | 0.07 | 0 | 0.04 | 0 | 0.64 | 0 |
| rno-miR-346 | 0.37 | 1.21 | 0.51 | 0.75 | 1.47 | 0.09 | 0.73 | 0 | 1.25 | 25 | 1.26 | 0 | 0.94 | 0 |
| rno-miR-347 | 2.28 | 1.31 | 1.52 | 0.75 | 1.80 | 2.28 | 1.66 | 17 | 1.55 | 75 | 2.05 | 67 | 1.56 | 40 |
| rno-miR-349 | 0.10 | 0.04 | 0.83 | 1.94 | 1.65 | 0.88 | 0.91 | 0 | 0.73 | 0 | 0.06 | 0 | 0.86 | 0 |
| rno-miR-352 | 4.13 | 4.33 | 4.52 | 5.34 | 5.13 | 4.36 | 4.63 | 100 | 4.82 | 100 | 4.03 | 100 | 4.86 | 100 |
| rno-miR-421 | -0.38 | 0.45 | 0.98 | 0.56 | 1.37 | 0.35 | 0.55 | 0 | 0.82 | 13 | 0.20 | 0 | 0.63 | 0 |
| rno-miR-7-AS | 2.62 | 1.66 | 2.19 | 1.50 | 2.37 | 2.19 | 2.09 | 50 | 1.05 | 13 | 1.36 | 17 | 1.56 | 60 |
| hsa-miR-522 | -0.03 | 0.17 | 0.59 | 0.66 | 1.14 | 1.32 | 0.64 | 0 | 0.41 | 13 | 0.54 | 0 | 0.82 | 0 |
| hsa-miR-519b | 1.16 | 1.21 | 1.11 | 0.47 | 0.14 | 1.22 | 0.89 | 0 | 0.98 | 13 | 1.05 | 17 | 0.58 | 0 |
| hsa-miR-520c | -0.15 | 0.59 | 0.98 | 0.92 | 0.29 | 1.66 | 0.71 | 0 | 0.35 | 0 | 0.80 | 17 | 0.70 | 0 |
| hsa-miR-519e | 1.04 | -0.60 | 0.19 | 0.84 | 0.44 | 1.12 | 0.50 | 0 | 0.70 | 0 | 1.33 | 17 | 0.59 | 0 |
| hsa-miR-519d | 0.79 | 0.85 | 0.83 | 1.66 | 1.14 | 0.88 | 1.03 | 0 | 0.38 | 13 | 1.41 | 33 | 0.33 | 0 |
| hsa-miR-520b | -0.03 | 0.04 | 0.90 | 1.01 | 0.44 | 1.32 | 0.61 | 0 | 1.18 | 25 | 1.26 | 0 | 0.48 | 0 |
| hsa-miR-519c | 0.10 | 0.17 | 0.67 | 1.37 | -0.26 | 0.35 | 0.40 | 0 | 0.74 | 25 | 0.65 | 0 | 0.66 | 0 |
| hsa-miR-526b-AS | 0.65 | 0.72 | 1.11 | 0.37 | 1.37 | 0.49 | 0.79 | 0 | 0.08 | 0 | 0.80 | 33 | 0.58 | 0 |
| hsa-miR-520e | 0.23 | 0.31 | 0.35 | 0.01 | 0.00 | 0.88 | 0.30 | 0 | 0.58 | 13 | 0.58 | 0 | 0.60 | 0 |
| hsa-miR-520a | 0.92 | 0.72 | 0.98 | 0.75 | 0.88 | 1.50 | 0.96 | 0 | 0.50 | 13 | 1.71 | 50 | 0.47 | 0 |
| hsa-miR-520d | 0.92 | 0.85 | 0.67 | 0.56 | 1.37 | -0.15 | 0.70 | 0 | 0.84 | 0 | 1.76 | 50 | 0.52 | 0 |
| hsa-miR-520h | 1.04 | 0.59 | 1.05 | 0.66 | 0.29 | 0.75 | 0.73 | 0 | 0.82 | 13 | 1.10 | 0 | 0.11 | 0 |
| hsa-miR-517a | 1.27 | 1.10 | 0.83 | 1.31 | 1.47 | 1.22 | 1.20 | 0 | 0.73 | 13 | 1.76 | 33 | 0.45 | 0 |
| hsa-miR-518e | 0.51 | 0.85 | 0.67 | 0.66 | -0.91 | 0.88 | 0.44 | 0 | 0.71 | 13 | 0.30 | 0 | 0.41 | 0 |
| hsa-miR-521 | 0.79 | 0.17 | 0.43 | 0.10 | 0.59 | 1.12 | 0.53 | 0 | 0.50 | 0 | 1.21 | 17 | 0.56 | 0 |
| hsa-miR-523 | 0.92 | 0.72 | 1.24 | 0.75 | 0.59 | 1.32 | 0.92 | 0 | 0.92 | 0 | 1.29 | 17 | 0.63 | 0 |
| hsa-miR-518f | -0.15 | 0.72 | 1.11 | 0.66 | 1.47 | 0.35 | 0.69 | 0 | 0.53 | 0 | 1.57 | 33 | 0.90 | 0 |
| hsa-miR-518c | 1.63 | 0.85 | 0.27 | 0.01 | 1.65 | 0.88 | 0.88 | 0 | 0.41 | 0 | 1.09 | 17 | 0.38 | 0 |
| hsa-miR-518b | 1.27 | 0.31 | 1.30 | 1.37 | 0.88 | 0.75 | 0.98 | 0 | 0.64 | 0 | 1.45 | 17 | 0.78 | 0 |
| hsa-miR-518d | 0.37 | -0.41 | 0.98 | 0.75 | 0.29 | 1.32 | 0.55 | 0 | 0.19 | 0 | -0.17 | 0 | 0.80 | 20 |
| hsa-miR-525-AS | 1.97 | 0.17 | 0.98 | 0.19 | 0.29 | 0.75 | 0.73 | 0 | -0.12 | 0 | -0.09 | 0 | 0.51 | 0 |
| hsa-miR-524 | 0.37 | 0.98 | 1.47 | 0.37 | 1.01 | -0.03 | 0.70 | 0 | 1.21 | 13 | 0.98 | 0 | 0.82 | 0 |
| hsa-miR-518a | -0.03 | 0.72 | 0.27 | 0.01 | 1.01 | 1.32 | 0.55 | 0 | 0.46 | 0 | 0.94 | 0 | 0.50 | 0 |
| hsa-miR-515-3p | 1.04 | 0.72 | 0.59 | 0.47 | 1.01 | 0.22 | 0.68 | 0 | 0.91 | | 0.37 | | 0.55 | 0 |
| hsa-miR-516-3p | 1.27 | 1.58 | 0.11 | 1.44 | 1.47 | 0.62 | 1.08 | 0 | 0.66 | 0 | 0.16 | 0 | 0.72 | 0 |
| ambi-miR-7026 | 1.04 | 0.31 | 0.67 | 0.56 | 0.29 | 0.75 | 0.60 | 0 | 0.13 | 0 | 0.88 | 17 | 0.02 | 0 |
| ambi-miR-7027 | -0.15 | 0.72 | 1.47 | 0.84 | 0.74 | -0.03 | 0.60 | 0 | 1.25 | 38 | 0.83 | 17 | 1.11 | 20 |
| hsa-miR-512-3p | 1.16 | 1.66 | 0.83 | 1.01 | 0.59 | 0.75 | 1.00 | 0 | 1.17 | 13 | 0.96 | 0 | 1.31 | 20 |
| ambi-miR-7029 | 1.16 | 1.31 | 0.75 | 1.16 | 1.14 | 0.49 | 1.00 | 0 | 5.30 | 100 | 5.26 | 100 | 5.58 | 100 |
| hsa-miR-491 | 3.15 | 2.71 | 2.48 | 2.13 | 2.49 | 2.44 | 2.57 | 83 | 2.49 | 100 | 2.44 | 100 | 2.64 | 100 |
| hsa-miR-506 | 0.79 | 1.50 | 0.83 | 1.16 | 1.14 | 1.00 | 1.07 | 0 | 1.39 | 63 | 0.65 | 17 | 1.25 | 40 |
| hsa-miR-514 | -0.03 | 0.59 | 0.67 | 0.47 | -0.13 | 0.35 | 0.32 | 0 | 0.66 | 0 | -0.08 | 0 | 0.31 | 0 |
| hsa-miR-509 | 1.04 | 1.58 | 0.83 | 0.19 | 1.56 | 1.66 | 1.14 | 0 | 1.32 | 13 | 1.50 | 0 | 1.28 | 40 |
| hsa-miR-508 | 0.10 | 1.50 | 0.75 | 0.75 | -0.13 | 0.88 | 0.64 | 0 | 0.99 | 13 | 1.69 | 33 | 1.01 | 20 |
| hsa-miR-507 | 0.10 | -0.20 | 0.75 | 0.84 | 0.88 | 1.50 | 0.64 | 0 | 0.05 | 0 | -0.04 | 0 | 0.82 | 0 |
| ambi-miR-7036 | 1.04 | 0.17 | 0.83 | 1.44 | 0.59 | 0.22 | 0.72 | 0 | 1.30 | 38 | 1.45 | 17 | 1.84 | 60 |
| hsa-miR-193b | 5.10 | 4.62 | 6.00 | 4.76 | 4.67 | 5.32 | 5.08 | 100 | 3.11 | 100 | 3.17 | 100 | 3.00 | 100 |
| ambi-miR-7038-1 | 1.27 | 0.85 | 0.90 | 1.44 | 0.59 | 1.12 | 1.03 | 0 | 0.18 | 0 | 0.74 | 0 | 0.52 | 0 |
| ambi-miR-7039 | 2.97 | 3.89 | 3.45 | 2.84 | 2.49 | 2.57 | 3.04 | 100 | 2.84 | 88 | 2.94 | 100 | 2.66 | 100 |
| hsa-miR-488 | 0.37 | 1.10 | 0.59 | 1.01 | -0.37 | 0.62 | 0.55 | 0 | 0.95 | 25 | 0.83 | 0 | 1.03 | 20 |
| hsa-miR-510 | 0.37 | 1.10 | 0.90 | 1.16 | 1.37 | 0.35 | 0.88 | 0 | 0.56 | 13 | 0.41 | 0 | 1.16 | 40 |
| hsa-miR-517-AS | 1.37 | 0.72 | 0.59 | 1.01 | 0.29 | 1.32 | 0.88 | 0 | 0.75 | 13 | 1.05 | 0 | 0.70 | 0 |
| hsa-miR-518fAS | 1.04 | 0.98 | 1.30 | 0.92 | 0.29 | 0.35 | 0.81 | 0 | 1.01 | 13 | 0.87 | 17 | 0.39 | 0 |
| hsa-miR-518c-AS | 2.71 | 2.33 | 1.66 | 2.29 | 2.49 | 2.54 | 2.34 | 83 | 2.71 | 100 | 2.58 | 100 | 2.55 | 100 |
| hsa-miR-526c | 1.04 | 1.41 | 1.05 | 0.66 | 0.29 | 0.35 | 0.80 | 0 | 0.12 | 0 | 0.39 | 0 | 0.72 | 0 |
| hsa-miR-526b | 1.71 | 2.10 | 1.47 | 1.01 | 1.47 | 1.87 | 1.60 | 17 | 1.64 | 50 | 1.78 | 33 | 1.98 | 60 |
| hsa-miR-520a-AS | 0.79 | 0.85 | 0.75 | 0.56 | 1.14 | -0.26 | 0.64 | 0 | -0.12 | 0 | 0.09 | 0 | 0.27 | 0 |
| hsa-miR-525 | 1.55 | 1.31 | 0.83 | 0.66 | 1.37 | 1.66 | 1.23 | 0 | 0.65 | 13 | 0.17 | 0 | 1.13 | 0 |
| hsa-miR-524-AS | 1.16 | 0.98 | 0.35 | 0.37 | 1.37 | 0.09 | 0.72 | 0 | 0.89 | 13 | 0.97 | 0 | 0.90 | 0 |
| hsa-miR-520d-AS | 0.10 | 1.21 | 0.51 | 1.09 | 1.14 | 2.09 | 1.02 | 0 | 1.02 | 0 | 1.58 | 50 | 1.07 | 20 |
| hsa-miR-527 | 1.16 | 0.85 | 0.43 | 1.24 | 0.59 | 1.00 | 0.88 | 0 | 1.04 | 13 | 1.77 | 50 | 1.29 | 20 |
| hsa-miR-515-5p | 0.23 | 1.21 | 0.67 | 0.84 | 1.26 | 0.49 | 0.78 | 0 | 0.40 | 13 | 0.82 | 0 | 0.76 | 0 |
| hsa-miR-519e-AS | 1.16 | 0.17 | 0.27 | 1.09 | 1.14 | 0.49 | 0.72 | 0 | 0.76 | 0 | 0.88 | 0 | 0.65 | 0 |
| ambi-miR-7054 | 1.04 | 0.85 | 0.83 | 0.28 | 0.74 | 0.75 | 0.75 | 0 | 0.97 | 13 | 0.36 | 0 | 0.87 | 0 |
| ambi-miR-7055 | -0.15 | 0.59 | 0.67 | 0.75 | 0.88 | 1.12 | 0.64 | 0 | 0.82 | 13 | 1.56 | 33 | 0.58 | 0 |
| hsa-miR-498 | 1.55 | 1.41 | 1.11 | 1.01 | 1.01 | 1.42 | 1.25 | 0 | 1.03 | 25 | 1.64 | 0 | 0.88 | 0 |
| hsa-miR-513 | 4.04 | 3.76 | 2.62 | 4.17 | 3.49 | 3.74 | 3.64 | 100 | 3.98 | 100 | 4.29 | 100 | 4.28 | 100 |
| ambi-miR-7058 | 4.64 | 4.76 | 5.44 | 4.61 | 4.86 | 4.81 | 4.85 | 100 | 4.13 | 100 | 3.97 | 100 | 4.03 | 100 |
| ambi-miR-7059-1 | 1.04 | 0.59 | 0.11 | 0.37 | 1.14 | 0.88 | 0.69 | 0 | 0.14 | 0 | 0.36 | 0 | 0.28 | 0 |
| hsa-miR-452 | 1.46 | 1.66 | 3.89 | 2.34 | 2.73 | 2.36 | 2.41 | 67 | 3.42 | 100 | 2.27 | 67 | 2.48 | 100 |
| hsa-miR-493 | 1.91 | 1.58 | 0.19 | 0.37 | 0.88 | 0.88 | 0.97 | 0 | 1.63 | 63 | 1.83 | 50 | 1.53 | 20 |
| ambi-miR-7062 | 1.85 | 1.80 | 1.30 | 1.09 | 1.80 | 1.42 | 1.54 | 17 | 1.43 | 25 | 1.68 | 17 | 1.43 | 20 |
| hsa-miR-432 | 2.58 | 2.10 | 1.52 | 2.09 | 2.37 | 2.57 | 2.21 | 67 | 3.11 | 100 | 3.36 | 100 | 3.09 | 100 |
| hsa-miR-495 | -0.03 | 0.85 | 0.35 | 1.01 | 1.80 | -0.37 | 0.60 | 0 | 2.15 | 100 | 2.00 | 67 | 2.25 | 100 |
| hsa-miR-494 | 4.62 | 4.75 | 3.41 | 5.40 | 4.32 | 4.49 | 4.50 | 100 | 5.17 | 100 | 6.89 | 100 | 7.07 | 100 |
| ambi-miR-7066 | 1.04 | -0.08 | 0.04 | 0.84 | 0.14 | 0.49 | 0.41 | 0 | 1.16 | 38 | 0.80 | 0 | 0.81 | 20 |
| ambi-miR-7067 | 0.79 | 1.10 | 0.98 | 0.84 | 1.37 | 1.59 | 1.11 | 0 | 1.42 | 50 | 1.67 | 50 | 1.25 | 40 |
| ambi-miR-7068-1 | 1.16 | 0.72 | 1.18 | -0.08 | -0.26 | 0.88 | 0.60 | 0 | 0.30 | 0 | 0.40 | 0 | 0.62 | 0 |
| hsa-miR-496 | 1.55 | 0.72 | 0.83 | 1.01 | 0.29 | 1.32 | 0.95 | 0 | 0.61 | 13 | 0.37 | 0 | 0.41 | 0 |
| ambi-miR-7070 | -0.15 | 0.59 | 0.35 | 0.47 | 1.14 | 1.66 | 0.68 | 0 | 1.63 | 63 | 2.09 | 67 | 2.06 | 80 |
| hsa-miR-492 | 0.37 | 0.59 | -0.18 | 1.31 | 1.01 | 1.00 | 0.68 | 0 | 0.50 | 0 | -0.30 | 0 | 0.85 | 0 |
| hsa-miR-490 | 0.65 | 1.21 | 0.98 | 0.56 | 0.44 | 1.22 | 0.84 | 0 | 0.40 | 0 | 1.24 | 17 | 0.38 | 0 |
| hsa-miR-497 | 1.78 | 2.10 | 1.30 | 1.44 | 2.45 | 2.54 | 1.94 | 50 | 4.86 | 100 | 5.20 | 100 | 3.99 | 100 |
| ambi-miR-7074 | 0.10 | 0.04 | 0.83 | 1.16 | 1.56 | 0.62 | 0.72 | 0 | 0.48 | 0 | 0.96 | 17 | 0.42 | 0 |
| ambi-miR-7075 | 2.23 | 2.15 | 2.16 | 2.34 | 1.65 | 1.50 | 2.01 | 67 | 2.29 | 100 | 2.25 | 83 | 2.26 | 100 |
| ambi-miR-7076 | 2.74 | 3.38 | 2.50 | 3.70 | 2.73 | 2.60 | 2.94 | 100 | 2.77 | 100 | 2.31 | 100 | 2.46 | 100 |
| hsa-miR-501 | 1.16 | 1.99 | 1.71 | 1.37 | 0.88 | 1.80 | 1.48 | 17 | 1.43 | 25 | 1.43 | 33 | 1.34 | 20 |
| hsa-miR-502 | 1.55 | 1.80 | 1.42 | 1.81 | 1.80 | 1.66 | 1.67 | 17 | 1.29 | 25 | 1.75 | 33 | 1.15 | 0 |
| ambi-miR-7079 | 1.27 | 1.10 | 1.24 | 2.61 | 1.47 | 1.12 | 1.47 | 17 | 1.87 | 75 | 1.89 | 67 | 1.52 | 20 |
| ambi-miR-7080 | 1.71 | 0.59 | 1.05 | 0.92 | 1.73 | 0.75 | 1.12 | 0 | 1.27 | 0 | 0.80 | 0 | 0.98 | 0 |
| ambi-miR-7081 | 1.55 | 1.41 | 0.98 | 0.92 | 0.88 | 1.22 | 1.16 | 0 | 2.15 | 100 | 2.22 | 67 | 1.64 | 40 |
| hsa-miR-202-AS | 0.65 | 0.31 | 0.35 | 0.75 | 0.59 | 0.62 | 0.54 | 0 | 0.06 | 0 | 0.57 | 17 | 0.65 | 0 |
| ambi-miR-7083 | 2.40 | 4.03 | 0.35 | 0.56 | 3.05 | 2.40 | 2.13 | 67 | 3.25 | 100 | 2.82 | 100 | 2.89 | 100 |
| ambi-miR-7084 | 0.79 | 0.72 | 1.05 | 1.31 | 0.59 | 1.00 | 0.91 | 0 | 1.09 | 13 | 1.59 | 33 | 1.09 | 20 |
| ambi-miR-7085 | 1.71 | 0.98 | 0.90 | 1.44 | 0.59 | 1.50 | 1.19 | 0 | 1.08 | 25 | 1.48 | 33 | 0.75 | 0 |
| ambi-miR-7086 | 2.09 | 2.77 | 1.79 | 0.66 | 1.56 | 1.22 | 1.68 | 17 | 0.95 | 38 | 0.31 | 0 | 1.44 | 20 |
| hsa-miR-512-5p | 0.51 | 0.98 | 0.83 | 1.56 | 0.44 | 0.35 | 0.78 | 0 | 0.39 | 0 | 0.34 | 17 | 0.72 | 20 |
| hsa-miR-504 | 0.23 | 0.17 | 0.51 | 0.10 | 0.14 | 0.35 | 0.25 | 0 | 0.32 | 0 | -0.03 | 0 | 0.84 | 0 |
| ambi-miR-7089 | 0.37 | 0.85 | 1.18 | 1.37 | 1.37 | 0.75 | 0.98 | 0 | 0.89 | 13 | 1.19 | 17 | 1.30 | 0 |
| hsa-miR-511 | 0.92 | 1.10 | 1.30 | 0.75 | 0.29 | 0.22 | 0.76 | 0 | 0.31 | 13 | 0.20 | 0 | 0.63 | 0 |
| hsa-miR-452-AS | 1.27 | 0.45 | 2.34 | 1.66 | 1.94 | 1.12 | 1.46 | 17 | 1.75 | 75 | 0.12 | 0 | 1.35 | 20 |
| hsa-miR-503 | 3.60 | 4.94 | 3.82 | 1.50 | 1.37 | 0.22 | 2.57 | 50 | 2.76 | 100 | 1.99 | 67 | 1.73 | 40 |
| hsa-miR-485-5p | 0.79 | 1.41 | 0.90 | 0.92 | 1.01 | 1.74 | 1.13 | 0 | 1.64 | 75 | 1.55 | 0 | 1.47 | 40 |
| hsa-miR-499 | 1.27 | 0.85 | 0.98 | 1.01 | 1.37 | -0.03 | 0.91 | 0 | 1.07 | 13 | 1.55 | 50 | 1.25 | 20 |
| ambi-miR-7095 | 1.16 | 1.21 | 0.83 | 0.47 | 1.47 | 1.32 | 1.08 | 0 | 1.03 | 13 | 1.14 | 17 | 0.67 | 0 |
| hsa-miR-505 | 2.58 | 3.81 | 2.93 | 2.09 | 1.94 | 1.66 | 2.50 | 50 | 1.88 | 75 | 1.45 | 17 | 1.16 | 20 |
| ambi-miR-7097 | 1.16 | 1.10 | 0.98 | 1.56 | 1.26 | 1.00 | 1.17 | 0 | 0.65 | 13 | 0.57 | 0 | 0.81 | 0 |
| ambi-miR-7098 | 2.19 | 0.04 | 0.98 | 0.92 | 2.23 | 0.62 | 1.16 | 33 | 0.73 | 0 | 0.24 | 0 | 0.48 | 0 |
| hsa-miR-489 | 0.92 | 1.41 | 0.04 | 0.84 | 0.00 | -0.46 | 0.46 | 0 | 1.25 | 38 | 0.11 | 0 | 1.23 | 0 |
| ambi-miR-7100 | 0.23 | 0.04 | 0.11 | 0.84 | 1.01 | 1.12 | 0.56 | 0 | 1.45 | 63 | 1.44 | 17 | 2.13 | 80 |
| ambi-miR-7101 | 1.16 | 0.72 | 0.83 | 0.66 | -0.26 | 0.88 | 0.66 | 0 | 0.57 | 0 | 1.67 | 17 | 0.56 | 0 |
| hsa-miR-432-AS | 0.79 | 1.31 | 0.11 | 0.75 | 1.37 | 0.62 | 0.82 | 0 | 0.64 | 0 | 0.83 | 17 | 0.55 | 0 |
| ambi-miR-7103 | 1.71 | 0.31 | 1.94 | 1.16 | 1.01 | 1.12 | 1.21 | 0 | 0.95 | 25 | 1.60 | 33 | 0.59 | 0 |
| hsa-miR-500 | 1.78 | 2.55 | 2.24 | 2.29 | 1.65 | 1.59 | 2.02 | 50 | 2.19 | 88 | 2.11 | 83 | 1.80 | 60 |
| ambi-miR-7105 | 2.40 | 2.37 | 3.47 | 2.45 | 2.70 | 3.27 | 2.78 | 100 | 2.99 | 100 | 2.10 | 67 | 2.42 | 100 |
| **TV**** | 2.09 | 1.66 | 1.94 | 2.1 6 | 2.12 | 2.28 | 2.04 | | | | | | | |
| **miRNAs > TV***** | 144 | 161 | 144 | 129 | 137 | 133 | 141 | | | | | | | |
| **%*** | 38.2 | 42.7 | 38.2 | 34. 2 | 36.3 | 35.3 | 37.5 | | | | | | | |

| | | | | | | | | | | | | | | |
|---|---|---|---|---|---|---|---|---|---|---|---|---|---|---|
| *, percentage of miRNAs above threshold value; **, threshold value; ***, number of miRNAs above threshold value; CL = Cell Line; Ca = PDAC; Ch = chronic pancreatitis; N = normal | | | | | | | | | | | | | | |

### EXAMPLE 3:

### THE PANCREATIC MIRNOME

As no comprehensive data set about miRNA expression in normal pancreas is currently available, the inventors first characterized the normal pancreatic miRNome. About 190 miRNAs were reproducibly detected above background signal in all five normal pancreas samples (Table 3, Mean (N)>2). These included 158 well characterized huma miRNAs and 21 miRNAs previously identified in mouse or rat. In addition, six new human miRNAs (Ambi-miR-7029, -7039, -7058, -7076, -7083 and -7105) were detected. Ambi-miR-7029 and -7058 had a highly significant expression level (Mean (N)>4). The inventors next performed a global comparison of expression data between the five normal pancreatic tissue samples and a reference set consisting of 33 different human tissues analyzed on the same array platform. The data are summarized in a global graphical representation of mean expression levels within each sample set (FIG. 2). The human reference set consisted of FirstChoice® Total RNA samples (Ambion) isolated from 33 distinct tissues: adipose, adrenal, aorta, bladder, bone marrow, brain, breast, cervix, colon, duodenum, esophagus, fallopian tube, heart, ileum, jejunum, kidney, liver, lung, lymph node, muscle, ovary, pituitary, placenta, prostate, small intestine, spleen, stomach, testis, thymus, thyroid, trachea, uterus, and vena cava.

Statistical analyses indicated that most miRNAs had similar mean expression levels in both sample sets and that many miRNAs known to be highly expressed in all tissue types, (e.g., hsa-miR-16, -21, -24, -26 and the let-7 family members) were also very abundant in pancreas (Table 5). However, several miRNAs (hsa-miR-141, - 148a, -200a, -200b, -200c, -216, -217, and -375) were found clearly enriched in pancreas, whereas others (hsa-miR-133a, -143, -145, and -150) were present at lower levels in our normal pancreatic tissue set (Table 5). Notably, miR-133a was detected at significant expression levels in all of the tissues in our reference set but not in any of the five normal pancreatic tissues. Furthermore, miR-216 and 217 were found to be essentially specific to pancreas with low mean expression levels and standard deviations within the reference set (Table 5). The only other tissue where both miRNAs were detected at significant levels was duodenum, albeit at an expression level 15 to 25 times lower than in pancreas.

**Table 5. Normalized Array Data for Five Normal Pancreas Samples (N) and 33 Reference Tissue Samples (Ref)**

| **miR Name** | **Ref** | | | **N** | | | **Δ**□ |
|---|---|---|---|---|---|---|---|
| | **%*** | **Mean** | **S.D.** | **%*** | **Mean** | **S.D.** | **(N-Ref)** |
| hsa-miR-133a | 100 | 7.49 | 2.09 | 0 | 2.86 | 0.83 | -4.63 |
| hsa-miR-150 | 97 | 6.75 | 1.42 | 60 | 3.77 | 0.84 | -2.97 |
| hsa-miR-145 | 100 | 10.83 | 0.91 | 100 | 8.32 | 0.36 | -2.51 |
| hsa-miR-378 | 94 | 4.98 | 1.04 | 0 | 2.50 | 0.95 | -2.49 |
| hsa-miR-143 | 100 | 10.46 | 0.95 | 100 | 8.02 | 0.31 | -2.43 |
| hsa-miR-1 | 97 | 6.58 | 2.40 | 80 | 4.25 | 0.15 | -2.34 |
| hsa-miR-324-3p | 100 | 5.72 | 0.40 | 40 | 3.48 | 0.50 | -2.24 |
| hsa-miR-422b | 100 | 7.64 | 1.24 | 100 | 5.45 | 0.56 | -2.19 |
| ambi-miR-7079 | 100 | 5.74 | 1.03 | 20 | 3.59 | 0.20 | -2.15 |
| hsa-miR-205 | 42 | 4.63 | 2.91 | 20 | 2.54 | 1.43 | -2.09 |
| hsa-miR-422a | 97 | 6.66 | 1.34 | 100 | 4.64 | 0.23 | -2.02 |
| hsa-miR-505 | 97 | 5.10 | 0.65 | 20 | 3.10 | 0.98 | -2.00 |
| hsa-miR-128a | 100 | 6.20 | 1.18 | 80 | 4.22 | 0.47 | -1.98 |
| hsa-miR-222 | 100 | 7.90 | 0.82 | 100 | 5.94 | 0.50 | -1.96 |
| hsa-miR-187 | 58 | 4.40 | 1.76 | 20 | 2.46 | 1.09 | -1.94 |
| hsa-miR-196b | 48 | 4.16 | 1.98 | 0 | 2.31 | 0.85 | -1.86 |
| mmu-miR-140-AS | 100 | 7.41 | 0.70 | 100 | 5.63 | 0.54 | -1.78 |
| hsa-miR-100 | 100 | 8.88 | 0.82 | 100 | 7.17 | 0.34 | -1.71 |
| hsa-miR-99a | 100 | 9.07 | 1.01 | 100 | 7.38 | 0.40 | -1.70 |
| hsa-miR-139 | 97 | 5.81 | 1.24 | 100 | 4.15 | 0.29 | -1.66 |
| hsa-miR-328 | 76 | 4.02 | 0.72 | 0 | 2.39 | 0.79 | -1.63 |
| hsa-miR-125b | 100 | 9.59 | 0.77 | 100 | 7.96 | 0.18 | -1.63 |
| hsa-miR-331 | 94 | 5.54 | 0.68 | 80 | 3.98 | 0.54 | -1.56 |
| hsa-miR-197 | 97 | 5.35 | 0.63 | 40 | 3.79 | 0.16 | -1.56 |
| hsa-miR-125a | 100 | 8.87 | 0.72 | 100 | 7.33 | 0.29 | -1.55 |
| hsa-miR-124a | 27 | 3.12 | 1.72 | 0 | 1.62 | 0.93 | -1.50 |
| hsa-miR-423 | 100 | 6.54 | 0.50 | 100 | 5.08 | 0.48 | -1.46 |
| hsa-miR-193b | 100 | 6.48 | 1.03 | 100 | 5.03 | 0.21 | -1.45 |
| hsa-miR-221 | 100 | 8.46 | 0.83 | 100 | 7.02 | 0.32 | -1.44 |
| hsa-miR-345 | 91 | 4.64 | 0.61 | 40 | 3.23 | 0.48 | -1.41 |
| ambi-miR-7029 | 100 | 8.94 | 1.58 | 100 | 7.58 | 0.66 | -1.36 |
| hsa-miR-223 | 100 | 7.82 | 1.11 | 100 | 6.48 | 0.67 | -1.34 |
| hsa-miR-204 | 91 | 5.18 | 1.44 | 60 | 3.88 | 0.37 | -1.30 |
| hsa-miR-497 | 100 | 7.28 | 0.64 | 100 | 6.00 | 0.26 | -1.27 |
| hsa-miR-99b | 100 | 7.40 | 0.62 | 100 | 6.13 | 0.44 | -1.27 |
| hsa-miR-10b | 94 | 7.80 | 1.71 | 100 | 6.55 | 0.19 | -1.25 |
| hsa-miR-155 | 100 | 6.52 | 1.10 | 100 | 5.29 | 1.06 | -1.23 |
| ambi-miR-7085 | 64 | 3.83 | 0.56 | 0 | 2.62 | 0.58 | -1.21 |
| hsa-miR-132 | 100 | 6.30 | 0.99 | 100 | 5.10 | 0.81 | -1.20 |
| hsa-miR-500 | 88 | 5.03 | 0.65 | 60 | 3.84 | 0.68 | -1.19 |
| hsa-miR-15b | 100 | 8.34 | 0.64 | 100 | 7.18 | 0.39 | -1.17 |
| hsa-miR-224 | 94 | 5.77 | 1.16 | 100 | 4.61 | 0.38 | -1.16 |
| ambi-miR-7081 | 97 | 4.83 | 0.60 | 40 | 3.71 | 0.28 | -1.13 |
| hsa-miR-126 | 100 | 10.24 | 0.48 | 100 | 9.12 | 0.17 | -1.13 |
| hsa-miR-23a | 100 | 10.03 | 0.56 | 100 | 8.91 | 0.26 | -1.12 |
| hsa-miR-181a | 100 | 8.11 | 0.84 | 100 | 7.01 | 0.20 | -1.11 |
| hsa-miR-330 | 30 | 3.25 | 0.89 | 0 | 2.16 | 1.12 | -1.09 |
| hsa-miR-93 | 100 | 7.92 | 0.56 | 100 | 6.83 | 0.30 | -1.09 |
| hsa-miR-503 | 85 | 4.88 | 1.25 | 40 | 3.79 | 0.54 | -1.09 |
| hsa-miR-146a | 100 | 6.93 | 0.96 | 100 | 5.86 | 0.49 | -1.07 |
| ambi-miR-7105 | 100 | 5.52 | 0.49 | 100 | 4.47 | 0.26 | -1.05 |
| hsa-miR-23b | 100 | 10.21 | 0.59 | 100 | 9.16 | 0.32 | -1.05 |
| hsa-miR-425 | 94 | 4.50 | 0.34 | 40 | 3.46 | 0.52 | -1.04 |
| hsa-miR-206 | 64 | 4.57 | 2.20 | 40 | 3.54 | 0.32 | -1.04 |
| hsa-miR-517a | 15 | 3.03 | 1.69 | 0 | 2.01 | 1.07 | -1.02 |
| hsa-miR-34c | 30 | 3.37 | 1.95 | 0 | 2.35 | 0.49 | -1.02 |
| hsa-miR-107 | 100 | 8.83 | 0.38 | 100 | 7.82 | 0.29 | -1.01 |
| ambi-miR-7083 | 94 | 5.94 | 1.01 | 100 | 4.93 | 0.57 | -1.01 |
| hsa-miR-149 | 67 | 3.90 | 0.79 | 20 | 2.90 | 0.63 | -1.00 |
| hsa-miR-103 | 100 | 8.86 | 0.38 | 100 | 7.86 | 0.30 | -1.00 |
| hsa-miR-339 | 100 | 5.46 | 0.77 | 100 | 4.48 | 0.25 | -0.99 |
| hsa-miR-189 | 94 | 4.80 | 0.73 | 80 | 3.85 | 0.70 | -0.95 |
| hsa-miR-92 | 100 | 7.76 | 0.54 | 100 | 6.82 | 0.25 | -0.94 |
| ambi-miR-7055 | 24 | 3.31 | 1.37 | 0 | 2.37 | 0.64 | -0.94 |
| mmu-miR-322 | 0 | 2.32 | 0.42 | 0 | 1.40 | 0.74 | -0.92 |
| ambi-miR-7103 | 39 | 3.30 | 0.51 | 0 | 2.38 | 0.57 | -0.91 |
| hsa-miR-212 | 52 | 3.69 | 0.76 | 20 | 2.78 | 0.71 | -0.91 |
| hsa-miR-361 | 100 | 7.36 | 0.46 | 1.00 | 6.45 | 0.30 | -0.90 |
| hsa-miR-199a | 100 | 8.24 | 0.89 | 100 | 7.35 | 0.38 | -0.90 |
| hsa-miR-191 | 100 | 8.64 | 0.32 | 100 | 7.75 | 0.30 | -0.89 |
| hsa-miR-9-AS | 61 | 3.87 | 1.11 | 20 | 2.98 | 0.76 | -0.89 |
| hsa-miR-214 | 100 | 7.54 | 0.89 | 100 | 6.65 | 0.45 | -0.89 |
| ambi-miR-7101 | 24 | 3.15 | 0.92 | 0 | 2.33 | 0.58 | -0.82 |
| hsa-miR-196a | 48 | 3.85 | 1.92 | 0 | 3.05 | 0.89 | -0.80 |
| hsa-miR-213 | 18 | 2.76 | 0.67 | 0 | 1.97 | 0.91 | -0.79 |
| hsa-miR-520h | 6 | 2.25 | 0.90 | 0 | 1.48 | 0.76 | -0.77 |
| hsa-miR-195 | 100 | 8.89 | 0.74 | 100 | 8.12 | 0.42 | -0.77 |
| hsa-let-7i | 100 | 9.07 | 0.49 | 100 | 8.31 | 0.28 | -0.76 |
| hsa-miR-126-AS | 100 | 6.64 | 0.54 | 100 | 5.92 | 0.33 | -0.72 |
| hsa-miR-16 | 100 | 10.69 | 0.46 | 100 | 9.99 | 0.17 | -0.70 |
| hsa-miR-18a | 94 | 5.32 | 0.92 | 100 | 4.63 | 0.29 | -0.70 |
| hsa-miR-514 | 18 | 2.59 | 1.59 | 0 | 1.90 | 0.39 | -0.69 |
| hsa-miR-498 | 36 | 3.52 | 0.96 | 0 | 2.83 | 0.41 | -0.69 |
| hsa-miR-24 | 100 | 9.84 | 0.55 | 100 | 9.15 | 0.28 | -0.69 |
| mmu-miR-291-5p | 15 | 3.14 | 0.61 | 0 | 2.45 | 1.08 | -0.69 |
| hsa-miR-140 | 100 | 5.94 | 0.51 | 100 | 5.26 | 0.27 | -0.68 |
| ambi-miR-7076 | 91 | 5.20 | 0.66 | 100 | 4.52 | 0.40 | -0.68 |
| hsa-miR-324-5p | 64 | 3.55 | 0.50 | 0 | 2.88 | 0.58 | -0.67 |
| hsa-miR-519d | 6 | 2.52 | 1.31 | 0 | 1.85 | 1.13 | -0.67 |
| mmu-miR-384 | 3 | 1.95 | 0.56 | 0 | 1.29 | 0.65 | -0.66 |
| ambi-miR-7068-1 | 18 | 3.03 | 1.07 | 0 | 2.38 | 0.82 | -0.65 |
| hsa-miR-320 | 100 | 8.35 | 0.47 | 100 | 7.70 | 0.25 | -0.65 |
| ambi-miR-7026 | 6 | 1.93 | 1.01 | 0 | 1.28 | 0.40 | -0.65 |
| hsa-miR-181b | 100 | 7.01 | 0.80 | 100 | 6.36 | 0.31 | -0.65 |
| hsa-miR-181c | 94 | 4.74 | 0.73 | 80 | 4.10 | 0.26 | -0.64 |
| hsa-miR-10a | 94 | 7.54 | 1.54 | 100 | 6.93 | 0.22 | -0.61 |
| rno-miR-151-AS | 100 | 7.65 | 0.47 | 100 | 7.05 | 0.38 | -0.61 |
| hsa-miR-27a | 100 | 9.47 | 0.54 | 100 | 8.87 | 0.20 | -0.60 |
| hsa-miR-490 | 21 | 2.64 | 0.78 | 0 | 2.04 | 0.46 | -0.60 |
| hsa-miR-502 | 76 | 3.77 | 0.49 | 0 | 3.17 | 0.35 | -0.60 |
| mmu-miR-346 | 3 | 2.48 | 0.59 | 0 | 1.89 | 0.60 | -0.59 |
| hsa-miR-199a-AS | 100 | 8.99 | 0.84 | 100 | 8.43 | 0.33 | -0.55 |
| hsa-miR-9 | 21 | 2.75 | 1.30 | 0 | 2.19 | 0.96 | -0.55 |
| ambi-miR-7075 | 91 | 4.87 | 0.58 | 100 | 4.32 | 0.23 | -0.55 |
| hsa-miR-495 | 97 | 4.85 | 0.99 | 100 | 4.31 | 0.14 | -0.55 |
| hsa-miR-22 | 100 | 9.33 | 0.66 | 100 | 8.79 | 0.20 | -0.54 |
| ambi-miR-7058 | 100 | 6.58 | 0.32 | 100 | 6.04 | 0.21 | -0.54 |
| hsa-miR-412 | 0 | 1.69 | 0.66 | 0 | 1.15 | 0.55 | -0.54 |
| hsa-miR-31 | 94 | 6.44 | 1.69 | 100 | 5.91 | 0.19 | -0.53 |
| hsa-miR-326 | 27 | 3.14 | 0.41 | 0 | 2.61 | 0.29 | -0.53 |
| hsa-miR-520d | 9 | 2.74 | 1.03 | 0 | 2.23 | 0.77 | -0.51 |
| hsa-miR-185 | 100 | 6.73 | 0.61 | 100 | 6.26 | 0.25 | -0.47 |
| mmu-miR-34b | 27 | 3.44 | 1.31 | 0 | 2.97 | 0.47 | -0.47 |
| mmu-miR-292-3p | 0 | 2.34 | 0.40 | 0 | 1.89 | 1.09 | -0.44 |
| hsa-miR-152 | 100 | 7.18 | 0.48 | 100 | 6.75 | 0.31 | -0.43 |
| hsa-miR-449 | 18 | 3.03 | 1.80 | 0 | 2.62 | 0.61 | -0.41 |
| hsa-miR-138 | 42 | 3.34 | 1.21 | 0 | 2.93 | 0.40 | -0.40 |
| hsa-miR-142-5p | 88 | 4.79 | 0.89 | 80 | 4.39 | 0.37 | -0.40 |
| hsa-miR-17-5p | 100 | 8.54 | 0.60 | 100 | 8.15 | 0.24 | -0.40 |
| hsa-miR-509 | 24 | 3.67 | 1.64 | 40 | 3.29 | 0.55 | -0.38 |
| hsa-miR-29a | 100 | 9.92 | 0.52 | 100 | 9.55 | 0.17 | -0.38 |
| hsa-miR-380-5p | 0 | 2.08 | 0.59 | 0 | 1.71 | 0.70 | -0.37 |
| hsa-miR-106b | 100 | 7.35 | 0.46 | 100 | 6.98 | 0.19 | -0.37 |
| hsa-miR-106a | 100 | 8.66 | 0.62 | 100 | 8.30 | 0.20 | -0.36 |
| hsa-miR-342 | 100 | 8.43 | 0.57 | 100 | 8.07 | 0.15 | -0.36 |
| hsa-miR-373-AS | 94 | 4.40 | 0.57 | 80 | 4.06 | 0.17 | -0.35 |
| ambi-miR-7074 | 0 | 2.44 | 0.34 | 0 | 2.10 | 0.64 | -0.34 |
| hsa-miR-218 | 100 | 5.90 | 0.76 | 100 | 5.56 | 0.35 | -0.34 |
| hsa-miR-517-AS | 6 | 2.64 | 0.77 | 0 | 2.32 | 1.34 | -0.33 |
| mmu-miR-106a | 100 | 8.05 | 0.61 | 100 | 7.72 | 0.27 | -0.33 |
| hsa-miR-515-3p | 12 | 2.53 | 1.05 | 0 | 2.23 | 0.84 | -0.30 |
| mmu-miR-351 | 3 | 2.83 | 0.33 | 20 | 2.54 | 1.03 | -0.29 |
| hsa-miR-17-3p | 85 | 4.66 | 0.54 | 100 | 4.36 | 0.20 | -0.29 |
| hsa-miR-337 | 3 | 2.36 | 0.46 | 0 | 2.07 | 1.02 | -0.29 |
| ambi-miR-7059-1 | 3 | 2.09 | 0.67 | 0 | 1.80 | 0.80 | -0.28 |
| hsa-miR-452 | 97 | 4.81 | 0.86 | 100 | 4.53 | 0.11 | -0.28 |
| hsa-miR-137 | 15 | 2.57 | 1.32 | 0 | 2.31 | 0.18 | -0.27 |
| hsa-miR-370 | 97 | 4.91 | 0.41 | 100 | 4.66 | 0.12 | -0.26 |
| hsa-miR-25 | 100 | 7.68 | 0.54 | 100 | 7.43 | 0.23 | -0.25 |
| mmu-miR-17-3p | 64 | 3.77 | 0.64 | 20 | 3.52 | 0.12 | -0.24 |
| hsa-miR-373 | 9 | 2.70 | 0.64 | 0 | 2.45 | 0.64 | -0.24 |
| mmu-miR-199b | 97 | 6.63 | 0.94 | 100 | 6.39 | 0.47 | -0.24 |
| mo-miR-20-AS | 0 | 2.16 | 0.52 | 0 | 1.93 | 0.80 | -0.23 |
| mmu-miR-155 | 85 | 4.92 | 1.16 | 100 | 4.69 | 0.97 | -0.23 |
| mmu-miR-345 | 58 | 3.45 | 0.35 | 20 | 3.22 | 0.41 | -0.22 |
| ambi-miR-7038-1 | 9 | 2.45 | 1.57 | 0 | 2.23 | 0.76 | -0.22 |
| hsa-miR-496 | 0 | 2.13 | 0.56 | 0 | 1.92 | 1.32 | -0.21 |
| ambi-miR-7027 | 33 | 3.27 | 0.57 | 20 | 3.06 | 0.82 | -0.21 |
| hsa-miR-323 | 15 | 2.75 | 1.18 | 0 | 2.54 | 0.72 | -0.21 |
| mmu-miR-202 | 91 | 4.88 | 0.80 | 100 | 4.69 | 0.18 | -0.20 |
| hsa-miR-30c | 100 | 8.97 | 0.56 | 100 | 8.77 | 0.23 | -0.20 |
| hsa-miR-26a | 100 | 11.18 | 0.35 | 100 | 11.00 | 0.31 | -0.19 |
| hsa-miR-518f-AS | 6 | 2.22 | 0.59 | 0 | 2.03 | 0.85 | -0.18 |
| hsa-miR-519b | 6 | 2.29 | 1.04 | 0 | 2.12 | 1.29 | -0.18 |
| hsa-miR-523 | 6 | 2.48 | 1.03 | 0 | 2.31 | 0.91 | -0.17 |
| mo-miR-327 | 88 | 4.37 | 0.47 | 80 | 4.20 | 0.16 | -0.17 |
| hsa-miR-520a-AS | 3 | 1.86 | 0.59 | 0 | 1.70 | 0.85 | -0.16 |
| hsa-miR-367 | 0 | 2.25 | 0.37 | 0 | 2.09 | 1.08 | -0.15 |
| hsa-miR-28 | 100 | 6.98 | 0.43 | 100 | 6.83 | 0.33 | -0.15 |
| hsa-miR-20a | 100 | 7.78 | 0.63 | 100 | 7.64 | 0.20 | -0.14 |
| hsa-miR-485-5p | 64 | 3.61 | 0.57 | 40 | 3.47 | 0.73 | -0.14 |
| hsa-miR-19b | 100 | 8.64 | 0.53 | 100 | 8.50 | 0.24 | -0.14 |
| mmu-miR-101b | 24 | 3.11 | 0.59 | 20 | 2.97 | 1.24 | -0.14 |
| hsa-miR-151 | 100 | 6.16 | 0.36 | 100 | 6.03 | 0.25 | -0.13 |
| mmu-miR-300 | 58 | 3.52 | 0.36 | 40 | 3.40 | 0.96 | -0.12 |
| hsa-miR-34a | 100 | 7.40 | 0.67 | 100 | 7.30 | 0.53 | -0.10 |
| hsa-miR-452-AS | 45 | 3.49 | 0.88 | 20 | 3.39 | 0.30 | -0.10 |
| mmu-miR-409 | 82 | 4.28 | 0.94 | 100 | 4.18 | 0.27 | -0.10 |
| hsa-miR-127 | 45 | 3.66 | 0.98 | 40 | 3.57 | 0.59 | -0.09 |
| hsa-miR-491 | 100 | 4.78 | 0.61 | 100 | 4.69 | 0.16 | -0.09 |
| hsa-miR-380-3p | 6 | 2.13 | 1.08 | 0 | 2.04 | 1.00 | -0.09 |
| hsa-miR-519e-AS | 6 | 2.56 | 0.79 | 0 | 2.47 | 0.60 | -0.09 |
| hsa-miR-489 | 33 | 3.32 | 0.94 | 0 | 3.23 | 0.50 | -0.08 |
| hsa-miR-518c | 3 | 2.02 | 0.78 | 0 | 1.93 | 0.95 | -0.08 |
| ambi-miR-7098 | 0 | 2.23 | 0.43 | 0 | 2.14 | 0.79 | -0.08 |
| ambi-miR-7086 | 58 | 3.56 | 0.70 | 20 | 3.49 | 0.32 | -0.07 |
| hsa-miR-372 | 6 | 2.17 | 1.00 | 20 | 2.11 | 1.31 | -0.06 |
| mmu-miR-294 | 27 | 3.17 | 0.37 | 20 | 3.12 | 0.36 | -0.05 |
| hsa-miR-184 | 82 | 4.33 | 0.48 | 100 | 4.28 | 0.14 | -0.05 |
| hsa-miR-499 | 24 | 3.32 | 1.59 | 20 | 3.27 | 0.39 | -0.05 |
| ambi-miR-7080 | 9 | 2.98 | 0.98 | 0 | 2.93 | 0.48 | -0.05 |
| mmu-miR-329 | 3 | 2.59 | 0.35 | 0 | 2.55 | 0.69 | -0.04 |
| hsa-miR-27b | 100 | 9.51 | 0.64 | 100 | 9.47 | 0.31 | -0.04 |
| hsa-miR-210 | 97 | 5.84 | 0.73 | 100 | 5.80 | 0.28 | -0.04 |
| hsa-miR-346 | 3 | 2.34 | 0.56 | 0 | 2.31 | 0.52 | -0.04 |
| hsa-miR-340 | 24 | 3.05 | 0.55 | 0 | 3.01 | 0.29 | -0.03 |
| hsa-miR-193a | 82 | 4.29 | 0.70 | 80 | 4.26 | 0.30 | -0.03 |
| hsa-miR-527 | 21 | 3.34 | 0.98 | 20 | 3.31 | 0.44 | -0.03 |
| hsa-miR-30d | 100 | 9.11 | 0.46 | 100 | 9.08 | 0.21 | -0.03 |
| hsa-miR-188 | 73 | 3.88 | 0.42 | 60 | 3.86 | 0.24 | -0.02 |
| mmu-miR-380-3p | 9 | 2.44 | 0.60 | 0 | 2.42 | 1.11 | -0.02 |
| hsa-miR-211 | 0 | 1.99 | 0.48 | 0 | 1.97 | 1.17 | -0.02 |
| hsa-miR-302b-AS | 3 | 1.74 | 0.73 | 0 | 1.72 | 1.09 | -0.02 |
| mmu-miR-424 | 61 | 4.01 | 1.24 | 80 | 4.00 | 0.74 | -0.02 |
| hsa-miR-30b | 100 | 8.86 | 0.50 | 100 | 8.84 | 0.24 | -0.02 |
| hsa-miR-302d | 3 | 2.41 | 0.59 | 0 | 2.39 | 1.08 | -0.02 |
| mmu-miR-290 | 88 | 4.32 | 0.47 | 100 | 4.32 | 0.11 | 0.00 |
| rno-miR-7-AS | 55 | 3.62 | 0.49 | 60 | 3.63 | 0.29 | 0.01 |
| hsa-miR-296 | 30 | 3.34 | 0.78 | 0 | 3.35 | 0.57 | 0.01 |
| mmu-miR-129-3p | 33 | 3.41 | 1.05 | 0 | 3.43 | 0.42 | 0.01 |
| hsa-miR-521 | 3 | 2.26 | 1.04 | 0 | 2.27 | 0.70 | 0.01 |
| hsa-miR-518c-AS | 91 | 4.58 | 0.49 | 100 | 4.60 | 0.12 | 0.02 |
| hsa-miR-520d-AS | 15 | 3.05 | 1.07 | 20 | 3.07 | 0.59 | 0.02 |
| hsa-miR-129 | 33 | 3.45 | 0.80 | 0 | 3.47 | 0.47 | 0.02 |
| hsa-miR-144 | 3 | 2.03 | 0.86 | 0 | 2.06 | 1.24 | 0.03 |
| hsa-miR-524-AS | 15 | 2.83 | 1.01 | 0 | 2.86 | 0.44 | 0.03 |
| hsa-miR-526c | 9 | 2.51 | 0.73 | 0 | 2.55 | 0.71 | 0.03 |
| hsa-miR-432 | 97 | 5.09 | 0.87 | 100 | 5.12 | 0.28 | 0.03 |
| hsa-miR-186 | 100 | 5.66 | 0.45 | 100 | 5.70 | 0.28 | 0.04 |
| ambi-miR-7097 | 9 | 2.68 | 0.42 | 0 | 2.72 | 0.54 | 0.05 |
| mmu-miR-151 | 100 | 5.07 | 0.52 | 100 | 5.12 | 0.26 | 0.05 |
| hsa-miR-122a | 82 | 4.42 | 1.48 | 100 | 4.47 | 0.14 | 0.05 |
| hsa-miR-15a | 100 | 8.08 | 0.51 | 100 | 8.14 | 0.14 | 0.06 |
| hsa-miR-432-AS | 3 | 2.12 | 0.66 | 0 | 2.18 | 1.26 | 0.06 |
| hsa-miR-511 | 6 | 2.32 | 1.03 | 0 | 2.38 | 0.91 | 0.06 |
| hsa-miR-302a | 0 | 2.52 | 0.57 | 0 | 2.58 | 0.89 | 0.06 |
| hsa-let-7g | 100 | 9.46 | 0.52 | 100 | 9.52 | 0.28 | 0.06 |
| hsa-miR-504 | 15 | 2.67 | 0.63 | 0 | 2.74 | 0.53 | 0.07 |
| hsa-miR-199b | 97 | 6.30 | 1.23 | 100 | 6.37 | 0.18 | 0.07 |
| hsa-miR-520b | 6 | 2.06 | 0.83 | 0 | 2.13 | 0.67 | 0.08 |
| hsa-miR-518a | 6 | 2.12 | 0.81 | 0 | 2.20 | 0.90 | 0.08 |
| hsa-miR-450 | 18 | 2.73 | 0.72 | 20 | 2.82 | 0.66 | 0.09 |
| ambi-miR-7084 | 12 | 2.98 | 1.05 | 20 | 3.07 | 0.49 | 0.09 |
| ambi-miR-7067 | 30 | 3.18 | 0.80 | 40 | 3.28 | 0.38 | 0.09 |
| hsa-let-7a | 100 | 11.25 | 0.38 | 100 | 11.35 | 0.30 | 0.10 |
| hsa-miR-219 | 21 | 3.08 | 1.01 | 0 | 3.19 | 0.40 | 0.11 |
| hsa-miR-381 | 70 | 4.04 | 0.73 | 60 | 4.15 | 0.42 | 0.11 |
| hsa-miR-299-5p | 76 | 4.17 | 0.88 | 100 | 4.28 | 0.12 | 0.11 |
| hsa-miR-383 | 30 | 3.15 | 0.77 | 20 | 3.26 | 0.72 | 0.11 |
| ambi-miR-7066 | 15 | 2.51 | 1.09 | 20 | 2.63 | 1.06 | 0.11 |
| hsa-miR-516-3p | 9 | 2.34 | 0.59 | 0 | 2.46 | 0.90 | 0.12 |
| ambi-miR-7095 | 6 | 2.34 | 0.65 | 0 | 2.46 | 0.69 | 0.12 |
| hsa-miR-135b | 6 | 2.39 | 0.64 | 0 | 2.52 | 0.91 | 0.13 |
| hsa-miR-220 | 6 | 2.42 | 0.50 | 0 | 2.55 | 0.53 | 0.13 |
| ambi-miR-7036 | 61 | 3.77 | 0.98 | 60 | 3.90 | 0.36 | 0.13 |
| rno-miR-347 | 39 | 3.47 | 0.61 | 40 | 3.60 | 0.52 | 0.13 |
| hsa-miR-198 | 100 | 5.58 | 0.44 | 100 | 5.72 | 0.21 | 0.14 |
| hsa-miR-519e | 6 | 2.22 | 0.93 | 0 | 2.36 | 0.69 | 0.14 |
| hsa-miR-520a | 3 | 2.05 | 0.51 | 0 | 2.19 | 0.46 | 0.15 |
| rno-miR-346 | 6 | 2.75 | 0.47 | 0 | 2.89 | 0.40 | 0.15 |
| hsa-miR-506 | 18 | 3.07 | 1.20 | 40 | 3.22 | 0.71 | 0.15 |
| hsa-miR-147 | 0 | 2.60 | 0.38 | 20 | 2.75 | 1.01 | 0.15 |
| ambi-miR-7062 | 30 | 3.32 | 0.51 | 20 | 3.48 | 0.24 | 0.16 |
| ambi-miR-7070 | 67 | 3.97 | 0.93 | 80 | 4.13 | 0.60 | 0.16 |
| hsa-miR-525-AS | 3 | 2.02 | 0.97 | 0 | 2.19 | 0.88 | 0.17 |
| hsa-miR-136 | 3 | 2.20 | 0.67 | 0 | 2.36 | 0.50 | 0.17 |
| mmu-miR-298 | 100 | 5.52 | 0.47 | 100 | 5.69 | 0.09 | 0.17 |
| mmu-miR-293 | 0 | 2.21 | 0.48 | 0 | 2.38 | 0.41 | 0.17 |
| hsa-miR-522 | 9 | 2.53 | 1.20 | 0 | 2.70 | 0.57 | 0.17 |
| hsa-miR-512-5p | 9 | 2.37 | 1.16 | 20 | 2.54 | 0.71 | 0.18 |
| hsa-miR-203 | 82 | 5.34 | 1.80 | 100 | 5.52 | 0.58 | 0.18 |
| hsa-let-7c | 100 | 10.97 | 0.54 | 100 | 11.17 | 0.30 | 0.20 |
| mmu-miR-291-3p | 0 | 1.94 | 0.60 | 0 | 2.14 | 0.78 | 0.20 |
| hsa-miR-518e | 6 | 1.82 | 1.32 | 0 | 2.02 | 0.77 | 0.20 |
| hsa-let-7b | 100 | 10.86 | 0.62 | 100 | 11.06 | 0.36 | 0.21 |
| hsa-miR-515-5p | 6 | 2.41 | 1.12 | 0 | 2.63 | 0.55 | 0.22 |
| mmu-miR-411 | 18 | 2.56 | 0.66 | 0 | 2.79 | 0.61 | 0.22 |
| hsa-miR-519c | 3 | 2.17 | 0.69 | 0 | 2.41 | 0.91 | 0.23 |
| hsa-miR-30a-5p | 100 | 9.36 | 0.48 | 100 | 9.61 | 0.21 | 0.26 |
| mmu-miR-341 | 15 | 2.60 | 0.83 | 0 | 2.85 | 0.55 | 0.26 |
| hsa-let-7e | 100 | 8.73 | 0.44 | 100 | 8.98 | 0.46 | 0.26 |
| ambi-miR-7039 | 79 | 4.45 | 0.61 | 100 | 4.71 | 0.57 | 0.26 |
| rno-miR-336 | 91 | 4.23 | 0.69 | 100 | 4.49 | 0.36 | 0.26 |
| mmu-miR-344 | 3 | 1.77 | 0.59 | 0 | 2.04 | 0.74 | 0.26 |
| hsa-miR-520c | 6 | 2.27 | 0.77 | 0 | 2.54 | 0.46 | 0.27 |
| hsa-miR-142-3p | 85 | 4.48 | 1.09 | 100 | 4.75 | 0.88 | 0.27 |
| hsa-miR-512-3p | 9 | 3.02 | 1.23 | 20 | 3.30 | 0.74 | 0.28 |
| hsa-miR-510 | 18 | 2.87 | 1.19 | 40 | 3.15 | 0.62 | 0.29 |
| hsa-miR-508 | 15 | 2.58 | 1.45 | 20 | 2.87 | 0.83 | 0.29 |
| hsa-miR-182-AS | 0 | 1.97 | 0.50 | 0 | 2.26 | 0.83 | 0.29 |
| hsa-miR-302b | 0 | 1.98 | 0.55 | 0 | 2.27 | 0.82 | 0.29 |
| hsa-miR-30e-3p | 100 | 5.30 | 0.41 | 100 | 5.60 | 0.22 | 0.31 |
| mmu-miR-295 | 0 | 2.06 | 0.42 | 20 | 2.38 | 1.00 | 0.32 |
| hsa-miR-302c-AS | 70 | 3.62 | 0.46 | 80 | 3.94 | 0.40 | 0.32 |
| mmu-miR-350 | 3 | 2.22 | 0.60 | 0 | 2.55 | 0.79 | 0.33 |
| hsa-miR-325 | 3 | 2.51 | 0.63 | 0 | 2.85 | 0.60 | 0.33 |
| hsa-miR-526b | 64 | 3.71 | 0.70 | 60 | 4.05 | 0.18 | 0.34 |
| mmu-miR-292-5p | 52 | 3.25 | 0.62 | 20 | 3.59 | 0.29 | 0.34 |
| hsa-let-7d | 100 | 10.32 | 0.42 | 100 | 10.67 | 0.32 | 0.36 |
| hsa-miR-384 | 0 | 1.75 | 0.62 | 0 | 2.11 | 0.84 | 0.36 |
| rno-miR-421 | 0 | 2.08 | 0.41 | 0 | 2.44 | 0.57 | 0.36 |
| hsa-miR-29b | 100 | 8.21 | 0.48 | 100 | 8.58 | 0.13 | 0.37 |
| hsa-miR-525 | 9 | 2.78 | 0.85 | 0 | 3.16 | 0.28 | 0.39 |
| hsa-miR-524 | 3 | 2.28 | 0.77 | 0 | 2.67 | 0.71 | 0.39 |
| hsa-miR-526b-AS | 6 | 2.00 | 1.14 | 0 | 2.40 | 0.41 | 0.39 |
| hsa-miR-130a | 100 | 7.64 | 0.69 | 100 | 8.04 | 0.21 | 0.40 |
| hsa-miR-21 | 100 | 10.30 | 0.66 | 100 | 10.71 | 0.61 | 0.40 |
| hsa-miR-492 | 9 | 2.31 | 0.67 | 0 | 2.71 | 0.64 | 0.40 |
| hsa-miR-33 | 0 | 2.03 | 0.47 | 0 | 2.44 | 0.68 | 0.40 |
| rno-miR-352 | 100 | 6.46 | 0.59 | 100 | 6.87 | 0.53 | 0.41 |
| hsa-miR-518b | 3 | 2.30 | 0.75 | 0 | 2.71 | 0.38 | 0.41 |
| hsa-miR-202-AS | 9 | 1.91 | 1.12 | 0 | 2.34 | 1.10 | 0.44 |
| rno-miR-297 | 0 | 1.72 | 0.49 | 0 | 2.16 | 0.76 | 0.44 |
| mmu-let-7d-AS | 3 | 2.30 | 0.49 | 0 | 2.74 | 0.64 | 0.44 |
| hsa-miR-34b | 94 | 5.06 | 1.14 | 100 | 5.51 | 0.76 | 0.45 |
| mmu-miR-330 | 12 | 2.72 | 0.63 | 0 | 3.17 | 0.12 | 0.45 |
| hsa-miR-501 | 27 | 2.95 | 0.68 | 20 | 3.40 | 0.09 | 0.46 |
| hsa-miR-520e | 0 | 1.87 | 0.41 | 0 | 2.34 | 0.93 | 0.46 |
| hsa-miR-30e-5p | 100 | 8.58 | 0.42 | 100 | 9.06 | 0.15 | 0.49 |
| rno-miR-344 | 0 | 1.91 | 0.63 | 0 | 2.43 | 0.73 | 0.52 |
| hsa-miR-518d | 0 | 1.91 | 0.45 | 20 | 2.43 | 1.57 | 0.52 |
| hsa-miR-371 | 0 | 1.79 | 0.54 | 0 | 2.32 | 0.89 | 0.53 |
| mmu-miR-383 | 27 | 3.09 | 0.85 | 40 | 3.63 | 0.43 | 0.54 |
| hsa-miR-30a-3p | 100 | 5.56 | 0.62 | 100 | 6.11 | 0.16 | 0.55 |
| hsa-miR-105 | 0 | 2.10 | 0.52 | 0 | 2.65 | 0.64 | 0.55 |
| hsa-miR-410 | 21 | 2.90 | 1.03 | 20 | 3.44 | 0.31 | 0.55 |
| hsa-miR-424 | 88 | 5.12 | 1.34 | 100 | 5.68 | 0.77 | 0.56 |
| ambi-miR-7054 | 6 | 2.22 | 0.53 | 0 | 2.78 | 0.49 | 0.56 |
| hsa-miR-369-3p | 6 | 2.11 | 0.84 | 20 | 2.68 | 1.00 | 0.57 |
| hsa-miR-26b | 100 | 8.88 | 0.50 | 100 | 9.46 | 0.16 | 0.58 |
| mmu-miR-207 | 9 | 2.58 | 0.59 | 0 | 3.19 | 0.50 | 0.61 |
| hsa-miR-382 | 85 | 4.49 | 0.94 | 100 | 5.11 | 0.53 | 0.62 |
| hsa-miR-98 | 100 | 6.42 | 0.54 | 100 | 7.06 | 0.51 | 0.64 |
| hsa-miR-32 | 15 | 2.71 | 0.56 | 20 | 3.36 | 0.57 | 0.65 |
| ambi-miR-7100 | 36 | 3.53 | 0.97 | 80 | 4.18 | 0.21 | 0.65 |
| mmu-miR-325 | 0 | 2.43 | 0.59 | 20 | 3.10 | 0.55 | 0.67 |
| hsa-miR-302c | 3 | 1.86 | 0.58 | 0 | 2.55 | 0.75 | 0.69 |
| hsa-miR-183 | 55 | 3.51 | 1.00 | 100 | 4.20 | 0.34 | 0.69 |
| hsa-miR-493 | 15 | 2.88 | 0.88 | 20 | 3.58 | 0.37 | 0.70 |
| hsa-miR-365 | 94 | 4.73 | 0.86 | 100 | 5.43 | 0.49 | 0.70 |
| mmu-miR-211 | 6 | 2.19 | 0.70 | 0 | 2.89 | 0.23 | 0.70 |
| hsa-miR-95 | 97 | 5.18 | 1.28 | 100 | 5.89 | 0.36 | 0.71 |
| mmu-miR-201 | 3 | 1.99 | 0.79 | 0 | 2.73 | 0.69 | 0.74 |
| hsa-miR-154 | 76 | 4.55 | 0.96 | 100 | 5.29 | 0.32 | 0.75 |
| hsa-miR-379 | 97 | 5.18 | 0.78 | 100 | 5.94 | 0.30 | 0.76 |
| hsa-miR-507 | 3 | 1.90 | 0.83 | 0 | 2.68 | 0.75 | 0.78 |
| hsa-miR-518f | 3 | 2.05 | 0.69 | 0 | 2.85 | 0.37 | 0.80 |
| hsa-miR-19a | 100 | 5.91 | 0.87 | 100 | 6.74 | 0.27 | 0.83 |
| hsa-miR-190 | 0 | 1.80 | 0.61 | 40 | 2.66 | 1.45 | 0.86 |
| mo-miR-343 | 6 | 2.26 | 0.53 | 0 | 3.16 | 0.28 | 0.90 |
| hsa-let-7f | 100 | 9.59 | 0.51 | 100 | 10.51 | 0.32 | 0.92 |
| hsa-miR-513 | 100 | 5.37 | 0.83 | 100 | 6.29 | 0.35 | 0.92 |
| hsa-miR-208 | 0 | 2.06 | 0.62 | 0 | 2.99 | 0.39 | 0.93 |
| hsa-miR-448 | 3 | 2.06 | 0.44 | 0 | 3.00 | 0.52 | 0.94 |
| hsa-miR-134 | 64 | 4.08 | 1.03 | 100 | 5.03 | 0.48 | 0.95 |
| mmu-miR-376b | 3 | 2.51 | 0.61 | 40 | 3.48 | 0.47 | 0.97 |
| ambi-miR-7089 | 3 | 2.35 | 0.55 | 0 | 3.32 | 0.39 | 0.97 |
| hsa-miR-488 | 0 | 1.99 | 0.56 | 20 | 3.01 | 0.41 | 1.02 |
| hsa-miR-368 | 100 | 6.52 | 0.94 | 100 | 7.56 | 0.29 | 1.04 |
| mmu-miR-376a | 39 | 3.26 | 0.82 | 80 | 4.31 | 0.59 | 1.05 |
| hsa-miR-135a | 27 | 2.70 | 1.52 | 20 | 3.77 | 0.54 | 1.07 |
| hsa-miR-374 | 100 | 4.71 | 0.74 | 100 | 5.82 | 0.57 | 1.11 |
| mmu-miR-215 | 18 | 2.61 | 1.94 | 60 | 3.74 | 0.73 | 1.13 |
| rno-miR-333 | 9 | 2.70 | 0.82 | 40 | 3.85 | 1.25 | 1.15 |
| rno-miR-349 | 0 | 1.62 | 0.50 | 0 | 2.78 | 0.45 | 1.16 |
| hsa-miR-101 | 100 | 5.69 | 0.55 | 100 | 6.88 | 0.06 | 1.19 |
| mmu-miR-429 | 0 | 2.47 | 0.49 | 20 | 3.67 | 0.27 | 1.20 |
| mmu-miR-7b | 21 | 3.21 | 0.94 | 100 | 4.44 | 0.27 | 1.23 |
| hsa-miR-301 | 24 | 2.76 | 0.83 | 40 | 4.02 | 0.22 | 1.25 |
| hsa-miR-377 | 64 | 3.99 | 0.95 | 100 | 5.33 | 0.39 | 1.34 |
| mmu-miR-337 | 0 | 1.87 | 0.55 | 40 | 3.21 | 0.96 | 1.34 |
| hsa-miR-376a | 100 | 5.23 | 0.94 | 100 | 6.63 | 0.31 | 1.40 |
| hsa-miR-148b | 100 | 5.71 | 0.38 | 100 | 7.11 | 0.60 | 1.40 |
| hsa-miR-335 | 100 | 6.77 | 0.90 | 100 | 8.19 | 0.35 | 1.42 |
| mmu-miR-297 | 0 | 1.79 | 0.49 | 40 | 3.34 | 0.92 | 1.55 |
| hsa-miR-153 | 39 | 3.49 | 1.17 | 100 | 5.26 | 0.25 | 1.76 |
| hsa-miR-29c | 100 | 8.37 | 0.51 | 100 | 10.25 | 0.26 | 1.87 |
| hsa-miR-194 | 100 | 6.75 | 2.13 | 100 | 8.63 | 0.21 | 1.88 |
| hsa-miR-96 | 94 | 4.80 | 0.97 | 100 | 6.69 | 0.38 | 1.89 |
| hsa-miR-182 | 91 | 5.39 | 1.29 | 100 | 7.39 | 0.37 | 2.00 |
| hsa-miR-215 | 42 | 4.09 | 2.37 | 100 | 6.34 | 0.50 | 2.25 |
| hsa-miR-429 | 70 | 4.88 | 1.88 | 100 | 7.13 | 0.09 | 2.25 |
| hsa-miR-494 | 100 | 6.78 | 0.54 | 100 | 9.07 | 0.97 | 2.28 |
| hsa-miR-7 | 94 | 5.07 | 1.66 | 100 | 7.68 | 0.56 | 2.61 |
| hsa-miR-192 | 97 | 6.47 | 2.25 | 100 | 9.13 | 0.25 | 2.66 |
| mmu-miR-192 | 97 | 6.32 | 2.25 | 100 | 9.00 | 0.28 | 2.67 |
| hsa-miR-130b | 100 | 5.56 | 0.66 | 100 | 8.27 | 0.25 | 2.71 |
| hsa-miR-338 | 58 | 4.09 | 1.31 | 100 | 6.82 | 0.19 | 2.73 |
| hsa-miR-200b | 82 | 6.47 | 2.59 | 100 | 9.49 | 0.14 | 3.02 |
| hsa-miR-200c | 94 | 7.37 | 2.65 | 100 | 10.59 | 0.24 | 3.22 |
| hsa-miR-148a | 100 | 7.34 | 1.13 | 100 | 10.57 | 0.36 | 3.23 |
| hsa-miR-200a | 67 | 5.31 | 2.78 | 100 | 8.57 | 0.17 | 3.26 |
| hsa-miR-141 | 67 | 5.52 | 2.29 | 100 | 9.49 | 0.20 | 3.98 |
| hsa-miR-375 | 58 | 4.63 | 2.20 | 100 | 9.21 | 0.39 | 4.59 |
| hsa-miR-216 | 3 | 2.79 | 0.69 | 100 | 9.09 | 0.28 | 6.30 |
| mmu-miR-217 | 6 | 2.37 | 0.89 | 100 | 9.70 | 0.21 | 7.32 |
| hsa-miR-217 | 12 | 2.38 | 1.07 | 100 | 9.86 | 0.22 | 7.48 |

| | | | | | | | |
|---|---|---|---|---|---|---|---|
| threshold value = 3.47 for Ref; threshold value = 3.70 for N; %*, percentage of miRNAs above threshold value | | | | | | | |

### EXAMPLE 4:

### IDENTIFICATION OF MIRNAS DIFFERENTIALLY EXPRESSED IN PDAC

To uncover miRNAs potentially relevant to pancreatic carcinogenesis, the inventors normalized miRNA array data from eight PDAC samples together with the normal pancreas set (Table 3). A direct comparison of mea miRNA expression levels showed that miRNA expression is profoundly affected in PDAC (Table 3, FIG. 3). Interestingly, most of the miRNAs down regulated in PDAC are miRNAs strongly enriched in pancreas relatively to the 33 human tissues reference set (Tables 3 and 5). Among these miRNAs, the highly expressed, pancreas-enriched miR-216 and -217 were down-regulated more than 200-fold in PDAC samples, to levels barely detectable on the array.

Further data analysis revealed 84 miRNAs with a significant differential expression between normal pancreas and PDAC (Table 6, Flag (N vs Ca)=1). Among these miRNAs, 41 were down-regulated and 32 were up-regulated by at least 2-fold in PDAC (|Δh|(N-Ca)>0.6). Together with miR-216 and -217, a total of 11 miRNAs were strongly down-regulated by more than 5-fold in PDAC (Δh(N-Ca)>1.6; hsa-miR-29c, -30a-3p, -96, -130b, -141, -148a, -148b, -216, -217, -375, and -494), and 11 others were strongly enriched in PDAC samples (Δh(N-Ca)<-1.6; hsa-miR-31, -143, - 145, -146a, -150, -155, -196a, -196b, -210, -222, and -223).

**Table 6. miRNAs Differentially Expressed Among Normal Pancreas (N), Chronic Pancreatitis (Ch), and PDAC (Ca) Tissue Samples**

| | **Mean** | **Mean** | **Mean** | **p-value** | **Δh** | **p-value** | **Flag*** | **Δh** | **p-value** | **Flag*** | **Δh** | **p-value** | **Flag*** |
|---|---|---|---|---|---|---|---|---|---|---|---|---|---|
| **miR Name** | **Ch** | **N** | **Ca** | **ANOVA** | **Ch-N** | **Ch vs N** | **Ch vs N** | **Ch-Ca** | **Ch vs Ca** | **Ch vs Ca** | **N-Ca** | **N vs Ca** | **N vs Ca** |
| hsa-let-7a | 8.90 | 9.50 | 8.90 | 1.97E-03 | -0.60 | 1.58E-03 | 1 | 0.00 | 9.92E-01 | 0 | 0.60 | 2.51E-03 | 1 |
| hsa-let-7b | 8.85 | 9.22 | 8.48 | 2.84E-04 | -0.37 | 3.53E-02 | 1 | 0.36 | 1.77E-02 | 1 | 0.73 | 2.21E-04 | 1 |
| hsa-let-7c | 8.81 | 9.33 | 8.55 | 2.03E-04 | -0.52 | 1.81E-03 | 1 | 0.27 | 9.06E-02 | 0 | 0.78 | 3.35E-04 | 1 |
| hsa-let-7d | 7.92 | 8.83 | 8.14 | 3.95E-04 | -0.91 | 9.38E-05 | 1 | -0.22 | 2.21E-01 | 0 | 0.69 | 4.02E-03 | 1 |
| hsa-let-7e | 6.27 | 7.14 | 6.70 | 1.15E-03 | -0.87 | 1.57E-03 | 1 | -0.43 | 7.80E-03 | 1 | 0.44 | 5.59E-02 | 0 |
| hsa-let-7f | 7.58 | 8.66 | 7.89 | 6.23E-04 | -1.08 | 135E-04 | 1 | -0.30 | 1.85E-01 | 0 | 0.78 | 6.06E-03 | 1 |
| hsa-let-7g | 7.09 | 7.68 | 7.26 | 3.13E-03 | -0.59 | 2.02E-04 | 1 | -0.17 | 2.47E-01 | 0 | 0.42 | 2.03E-02 | 1 |
| hsa-let-7i | 7.19 | 6.47 | 7.44 | 4.78E-05 | 0.72 | 1.60E-03 | 1 | -0.25 | 1.34E-01 | 0 | -0.97 | 4.08E-05 | 1 |
| hsa-miR-100 | 6.52 | 5.32 | 6.29 | 2.31E-04 | 1.19 | 2.96E-05 | 1 | 0.23 | 3.39E-01 | 0 | -0.97 | 2.23E-03 | 1 |
| hsa-miR-101 | 4.42 | 5.03 | 4.47 | 1.27E-02 | -0.61 | 1.62E-03 | 1 | -0.04 | 8.36E-01 | 0 | 0.57 | 1.74E-02 | 1 |
| hsa-miR-103 | 6.10 | 6.01 | 6.67 | 8.84E-05 | 0.08 | 4.53E-01 | 0 | -0.58 | 6.81E-04 | 1 | -0.66 | 6.54E-04 | 1 |
| hsa-miR-106a | 6.18 | 6.46 | 6.70 | 6.89E-04 | -0.28 | 4.24E-02 | 0 | -0.52 | 6.70E-04 | 1 | -0.24 | 4.19E-02 | 0 |
| hsa-miR-106b | 5.11 | 5.14 | 5.60 | 9.30E-04 | -0.04 | 6.88E-01 | 0 | -0.50 | 2.55E-03 | 1 | -0.46 | 6.75E-03 | 1 |
| hsa-miR-107 | 6.18 | 5.98 | 6.71 | 1.47E-05 | 0.20 | 7.46E-02 | 0 | -0.53 | 4.44E-04 | 1 | -0.73 | 9.89E-05 | 1 |
| hsa-miR-10a | 5.77 | 5.09 | 6.19 | 1.73E-05 | 0.69 | 1.72E-05 | 1 | -0.41 | 3.24E-02 | 0 | -1.10 | 9.72E-05 | 1 |
| hsa-miR-125a | 6.07 | 5.48 | 6.00 | 1.59E-02 | 0.59 | 3.05E-03 | 1 | 0.07 | 7.06E-01 | 0 | -0.51 | 238E-02 | 1 |
| hsa-miR-125b | 7.44 | 6.12 | 6.88 | 1.56E-05 | 1.32 | 4.88E-07 | 1 | 0.56 | 1.37E-02 | 1 | -0.76 | 3.07E-03 | 1 |
| hsa-miR-130a | 5.98 | 6.20 | 5.58 | 1.75E-04 | -0.23 | 3.63E-02 | 1 | 0.40 | 5.49E-03 | 1 | 0.62 | 4.94E-04 | 1 |
| hsa-miR-130b | 5.01 | 6.42 | 3.83 | 8.26E-08 | -1.41 | 2.02E-03 | 1 | 1.19 | 8.11E-04 | 1 | 2.60 | 1.18E-09 | 1 |
| hsa-miR-134 | 2.84 | 3.21 | 2.52 | 9.05E-03 | -0.36 | 1.41E-01 | 0 | 0.33 | 9.25E-02 | 0 | 0.69 | 3.19E-03 | 1 |
| hsa-miR-140 | 3.75 | 3.44 | 4.07 | 8.46E-03 | 0.32 | 9.77E-02 | 0 | -0.32 | 1.13E-01 | 0 | -0.63 | 3.01E-03 | 1 |
| hsa-miR-141 | 6.72 | 7.65 | 5.95 | 1.82E-05 | -0.93 | 4.62E-03 | 1 | 0.77 | 133E-02 | 1 | 1.70 | 1.20E-05 | 1 |
| hsa-miR-143 | 7.59 | 6.18 | 7.91 | 3.86E-06 | 1.41 | 4.01E-06 | 1 | -0.32 | 2.16E-01 | 0 | -1.73 | 3.17E-05 | 1 |
| hsa-miR-145 | 7.89 | 6.47 | 8.10 | 1.60E-04 | 1.42 | 4.88E-05 | 1 | -0.21 | 5.34E-01 | 0 | -1.62 | 4.84E-04 | 1 |
| hsa-miR-146a | 4.96 | 4.03 | 5.89 | 6.66E-05 | 0.93 | 1.98E-02 | 1 | -0.93 | 1.19E-02 | 1 | -1.86 | 3.34E-05 | 1 |
| hsa-miR-148a | 7.21 | 8.72 | 5.23 | 9.28E-09 | -1.52 | 3.00E-03 | 1 | 1.98 | 2.83E-05 | 1 | 3.49 | 2.32E-09 | 1 |
| hsa-miR-148b | 3.14 | 5.27 | 2.91 | 1.53E-06 | -2.13 | 1.80E-04 | 1 | 0.24 | 3.90E-01 | 0 | 2.37 | 6.17E-06 | 1 |
| hsa-miR-150 | 3.83 | 2.11 | 4.35 | 5.86E-04 | 1.72 | 2.15E-02 | 1 | -0.52 | 2.82E-01 | 0 | -2.25 | 5.58E-06 | 1 |
| hsa-miR-154 | 3.10 | 3.47 | 2.28 | 3.24E-04 | -0.37 | 2.27E-01 | 0 | 0.82 | 6.63E-03 | 1 | 1.19 | 3.93E-05 | 1 |
| hsa-miR-155 | 4.32 | 3.47 | 5.55 | 3.19E-04 | 0.85 | 1.04E-01 | 0 | -1.24 | 234E-03 | 1 | -2.08 | 6.49E-04 | 1 |
| hsa-miR-15b | 5.00 | 5.33 | 5.72 | 2.08E-03 | -0.33 | 939E-02 | 0 | -0.72 | 1.38E-03 | 1 | -0.39 | 5.20E-02 | 0 |
| hsa-miR-17-5p | 6.12 | 6.31 | 6.62 | 4.11E-03 | -0.19 | 2.06E-01 | 0 | -0.50 | 2.95E-03 | 1 | -0.31 | 3.75E-02 | 0 |
| hsa-miR-18 | 3.32 | 2.83 | 4.24 | 6.85E-05 | 0.49. | 3.04E-02 | 1 | -0.92 | 3.74E-03 | 1 | -1.41 | 1.74E-04 | 1 |
| hsa-miR-181b | 4.51 | 4.52 | 5.01 | 1.47E-02 | -0.01 | 9.49E-01 | 0 | -0.50 | 2.13E-02 | 1 | -0.49 | 1.68E-02 | 1 |
| hsa-miR-182 | 4.26 | 5.55 | 4.54 | 4.58E-04 | -1.29 | 9.47E-04 | 1 | -0.28 | 2.99E-01 | 0 | 1.01 | 8.44E-04 | 1 |
| hsa-miR-186 | 3.19 | 3.87 | 3.40 | 3.49E-03 | -0.68 | 4.90E-03 | 1 | -0.21 | 2.01E-01 | 0 | 0.47 | 7.99E-03 | 1 |
| hsa-miR-196a | 1.89 | 1.63 | 3.77 | 9.61E-07 | 0.26 | 4.15E-01 | 0 | -1.88 | 2.25E-05 | 1 | -2.14 | 8.31E-06 | 1 |
| hsa-miR-196b | 1.63 | 1.20 | 3.28 | 8.92E-06 | 0.44 | 2.14E-01 | 0 | -1.64 | 2.22E-04 | 1 | -2.08 | 2.86E-05 | 1 |
| hsa-miR-199a | 6.58 | 5.51 | 6.30 | 2.36E-05 | 1.08 | 1.63E-04 | 1 | 0.28 | 7.96E-02 | 0 | -0.79 | 2.63E-04 | 1 |
| hsa-miR-199a-AS | 7.41 | 6.59 | 7.01 | 9.52E-06 | 0.82 | 8.95E-05 | 1 | 0.39 | 4.14E-04 | 1 | -0.42 | 4.69E-03 | 1 |
| hsa-miR-19a | 4.12 | 4.90 | 4.25 | 1.90E-03 | -0.78 | 9.51E-04 | 1 | -0.13 | 4.70E-01 | 0 | 0.65 | 737E-03 | 1 |
| hsa-miR-19b | 6.22 | 6.66 | 6.16 | 8.53E-03 | -0.44 | 9.80E-03 | 1 | 0.06 | 6.78E-01 | 0 | 0.50 | 7.49E-03 | 1 |
| hsa-miR-200a | 5.69 | 6.73 | 5.48 | 4.11E-04 | -1.04 | 1.67E-03 | 1 | 0.21 | 4.47E-01 | 0 | 1.25 | 2.62E-04 | 1 |
| hsa-miR-200b | 6.50 | 7.65 | 6.68 | 5.63E-04 | -1.15 | 7.01E-04 | 1 | -0.17 | 5.10E-01 | 0 | 0.98 | 6.23E-04 | 1 |
| hsa-miR-200c | 7.67 | 8.74 | 7.23 | 2.20E-06 | -1.07 | 4.06E-04 | 1 | 0.44 | 4.79E-02 | 0 | 1.51 | 1.23E-06 | 1 |
| hsa-miR-203 | 2.85 | 3.69 | 5.15 | 5.07E-07 | -0.84 | 1.13E-02 | 1 | -2.30 | 9.16E-07 | 1 | -1.46 | 5.28E-04 | 1 |
| hsa-miR-21 | 9.26 | 8.86 | 9.75 | 6.29E-03 | 0.39 | 2.12E-01 | 0 | -0.49 | 2.41E-02 | 1 | -0.89 | 439E-03 | 1 |
| hsa-miR-210 | 4.53 | 3.97 | 6.56 | 1.07E-07 | 0.56 | 9.02E-03 | 1 | -2.03 | 1.87E-05 | 1 | -2.59 | 5.51E-06 | 1 |
| hsa-miR-214 | 6.14 | 4.81 | 6.10 | 1.21E-04 | 1.32 | 3.61E-04 | 1 | 0.04 | 8.67E-01 | 0 | -1.28 | 335E-04 | 1 |
| hsa-miR-216 | 5.97 | 7.25 | 2.01 | 1.86E-10 | -1.27 | 1.11E-02 | 1 | 3.97 | 2.97E-07 | 1 | 5.24 | 1.40E-09 | 1 |
| hsa-miR-217 | 6.77 | 8.02 | 2.42 | 2.77E-10 | -1.25 | 9.44E-03 | 1 | 4.35 | 3.68E-07 | 1 | 5.60 | 8.83E-09 | 1 |
| hsa-miR-221 | 5.49 | 5.18 | 6.45 | 1.69E-06 | 0.31 | 4.08E-02 | 0 | -0.96 | 9.55E-05 | 1 | -1.27 | 2.95E-05 | 1 |
| hsa-miR-222 | 4.72 | 4.10 | 5.94 | 8.14E-07 | 0.62 | 1.38E-02 | 1 | -1.22 | 6.91E-05 | 1 | -1.84 | 1.29E-05 | 1 |
| hsa-miR-223 | 5.70 | 4.64 | 6.81 | 8.24E-04 | 1.06 | 3.62E-02 | 1 | -1.11 | 3.14E-02 | 0 | -2.17 | 7.94E-04 | 1 |
| hsa-miR-224 | 2.66 | 2.81 | 3.82 | 1.47E-06 | -0.15 | 4.33E-01 | 0 | -1.16 | 2.91E-06 | 1 | -1.01 | 9.27E-05 | 1 |
| hsa-miR-23a | 7.15 | 7.07 | 7.62 | 6.46E-04 | 0.09 | 4.23E-01 | 0 | -0.47 | 4.25E-03 | 1 | -0.56 | 1.83E-03 | 1 |
| hsa-miR-24 | 7.65 | 7.31 | 8.02 | 1.20E-04 | 0.34 | 1.14E-02 | 1 | -0.37 | 1.07E-02 | 1 | -0.72 | 2.72E-04 | 1 |
| hsa-miR-25 | 5.19 | 5.58 | 5.64 | 3.16E-04 | -0.40 | 2.83E-03 | 1 | -0.46 | 5.50E-04 | 1 | -0.06 | 5.28E-01 | 0 |
| hsa-miR-26a | 8.76 | 9.15 | 8.75 | 1.48E-02 | -0.39 | 5.67E-03 | 1 | 0.02 | 9.06E-01 | 0 | 0.41 | 1.75E-02 | 1 |
| hsa-miR-26b | 6.66 | 7.62 | 6.93 | 1.96E-04 | -0.96 | 1.80E-04 | 1 | -0.28 | 1.57E-01 | 0 | 0.68 | 8.90E-04 | 1 |
| hsa-miR-27a | 6.90 | 7.03 | 7.34 | 3.70E-03 | -0.12 | 3.33E-01 | 0 | -0.44 | 5.02E-03 | 1 | -0.31 | 1.35E-02 | 1 |
| hsa-miR-27b | 7.14 | 7.62 | 7.09 | 3.00E-03 | -0.48 | 4.54E-03 | 1 | 0.06 | 6.74E-01 | 0 | 0.54 | 3.46E-03 | 1 |
| hsa-miR-28 | 4.65 | 4.99 | 5.05 | 1.08E-02 | -0.34 | 1.55E-02 | 1 | -0.40 | 6.31E-03 | 1 | -0.06 | 6.73E-01 | 0 |
| hsa-miR-29a | 7.29 | 7.71 | 7.58 | 1.37E-03 | -0.42 | 9.41E-04 | 1 | -0.29 | 8.00E-03 | 1 | 0.13 | 1.98E-01 | 0 |
| hsa-miR-29b | 6.09 | 6.73 | 6.43 | 1.03E-02 | -0.64 | 2.59E-03 | 1 | -0.33 | 8.98E-02 | 0 | 0.31 | 9.90E-02 | 0 |
| hsa-miR-29c | 7.02 | 8.40 | 6.45 | 7.11E-07 | -1.38 | 2.86E-04 | 1 | 0.58 | 2.51E-02 | 1 | 1.96 | 1.03E-06 | 1 |
| hsa-miR-30a-3p | 3.55 | 4.27 | 2.62 | 3.98E-08 | -0.72 | 4.32E-03 | 1 | 0.93 | 9.37E-05 | 1 | 1.65 | 8.25E-09 | 1 |
| hsa-miR-30a-5p | 7.22 | 7.77 | 6.64 | 1.15E-06 | -0.55 | 4.54E-03 | 1 | 0.58 | 1.17E-03 | 1 | 1.13 | 1.16E-06 | 1 |
| hsa-miR-30b | 6.27 | 7.00 | 5.69 | 2.84E-07 | -0.73 | 1.42E-03 | 1 | 0.58 | 1.06E-03 | 1 | 1.31 | 3.87E-07 | 1 |
| hsa-miR-30c | 6.20 | 6.93 | 5.58 | 2.29E-07 | -0.73 | 1.56E-03 | 1 | 0.62 | 7.64E-04 | 1 | 1.35 | 2.95E-07 | 1 |
| hsa-miR-30d | 6.65 | 7.24 | 6.25 | 4.37E-06 | -0.59 | 4.66E-04 | 1 | 0.40 | 1.01E-02 | 1 | 0.99 | 1.61E-05 | 1 |
| hsa-miR-30e-3p | 2.77 | 3.77 | 2.26 | 1.07E-05 | -1.01 | 3.30E-03 | 1 | 0.51 | 3.77E-02 | 0 | 1.51 | 1.74E-06 | 1 |
| hsa-miR-30e-5p | 6.57 | 7.22 | 6.22 | 2.68E-07 | -0.65 | 5.92E-04 | 1 | 0.36 | 6.79E-03 | 1 | 1.00 | 9.08E-08 | 1 |
| hsa-miR-31 | 5.65 | 4.08 | 6.66 | 8.86E-03 | 1.57 | 1.51E-03 | 1 | -1.01 | 2.21E-01 | 0 | -2.58 | 9.27E-03 | 1 |
| hsa-miR-331 | 2.63 | 2.25 | 3.46 | 1.48E-04 | 0.38 | 1.29E-01 | 0 | -0.83 | 2.06E-03 | 1 | -1.22 | 3.35E-04 | 1 |
| hsa-miR-335 | 5.13 | 6.35 | 4.92 | 3.98E-04 | -1.21 | 2.37E-03 | 1 | 0.21 | 4.89E-01 | 0 | 1.42 | 2.48E-04 | 1 |
| hsa-miR-365 | 2.43 | 3.61 | 2.20 | 7.58E-06 | -1.18 | 8.46E-04 | 1 | 0.23 | 1.33E-01 | 0 | 1.41 | 4.70E-05 | 1 |
| hsa-miR-3 68 | 5.16 | 5.71 | 4.38 | 1.16E-06 | -0.55 | 7.65E-03 | 1 | 0.78 | 5.11E-04 | 1 | 1.33 | 2.60E-06 | 1 |
| hsa-miR-374 | 2.71 | 3.99 | 3.11 | 1.34E-03 | -1.28 | 1.96E-03 | 1 | -0.40 | 1.03E-01 | 0 | 0.88 | 1.20E-02 | 1 |
| hsa-miR-375 | 6.51 | 7.37 | 4.68 | 4.82E-05 | -0.86 | 4.56E-02 | 0 | 1.83 | 2.56E-03 | 1 | 2.69 | 1.15E-04 | 1 |
| hsa-miR-376a | 3.97 | 4.79 | 3.70 | 1.42E-04 | -0.82 | 2.67E-03 | 1 | 0.27 | 2.03E-01 | 0 | 1.09 | 8.06E-05 | 1 |
| hsa-miR-377 | 3.21 | 3.50 | 2.70 | 3.28E-03 | -0.29 | 1.83E-01 | 0 | 0.51 | 2.95E-02 | 1 | 0.81 | 2.03E-03 | 1 |
| hsa-miR-379 | 4.16 | 4.11 | 3.50 | 5.83E-04 | 0.05 | 6.63E-01 | 0 | 0.66 | 1.19E-03 | 1 | 0.61 | 6.13E-03 | 1 |
| hsa-miR-429 | 4.14 | 5.29 | 4.68 | 1.94E-03 | -1.16 | 6.86E-04 | 1 | -0.54 | 7.15E-02 | 0 | 0.62 | 2.23E-02 | 1 |
| hsa-miR-93 | 5.07 | 4.99 | 5.90 | 3.64E-05 | 0.08 | 6.36E-01 | 0 | -0.83 | 2.61E-04 | 1 | -0.90 | 2.34E-04 | 1 |
| hsa-miR-95 | 2.73 | 4.05 | 3.22 | 1.91E-03 | -1.32 | 1.71E-04 | 1 | -0.49 | 1.32E-01 | 0 | 0.83 | 2.08E-02 | 1 |
| hsa-miR-96 | 2.99 | 4.85 | 3.09 | 2.27E-06 | -1.86 | 1.90E-04 | 1 | -0.10 | 6.74E-01 | 0 | 1.76 | 1.36E-06 | 1 |
| hsa-miR-98 | 4.16 | 5.22 | 4.56 | 3.32E-03 | -1.06 | 9.57E-04 | 1 | -0.40 | 9.13E-02 | 0 | 0.66 | 4.25E-02 | 0 |
| hsa-miR-99a | 6.71 | 5.54 | 6.23 | 2.73E-03 | 1.18 | 1.21E-04 | 1 | 0.49 | 1.01E-01 | 0 | -0.69 | 4.14E-02 | 0 |
| hsa-miR-99b | 4.68 | 4.29 | 4.91 | 1.23E-02 | 0.39 | 5.10E-02 | 0 | -0.24 | 1.74E-01 | 0 | -0.62 | 1.14E-02 | 1 |
| hsa-miR-452 | 2.52 | 2.73 | 3.44 | 1.61E-05 | -0.21 | 2.11E-01 | 0 | -0.91 | 8.82E-05 | 1 | -0.70 | 1.69E-04 | 1 |
| hsa-miR-494 | 6.96 | 7.22 | 5.14 | 5.26E-03 | -0.26 | 7.20E-01 | 0 | 1.82 | 9.59E-03 | 1 | 2.09 | 3.98E-03 | 1 |
| hsa-miR-497 | 5.29 | 4.17 | 4.83 | 7.94E-05 | 1.12 | 1.31E-04 | 1 | 0.46 | 2.37E-02 | 1 | -0.66 | 2.60E-03 | 1 |
| ambi-miR-7105 | 2.37 | 2.68 | 3.03 | 2.02E-05 | -0.30 | 2.75E-02 | 1 | -0.66 | 1.53E-05 | 1 | -0.35 | 5.31E-03 | 1 |

| | | | | | | | | | | | | | |
|---|---|---|---|---|---|---|---|---|---|---|---|---|---|
| *** Significant differential expression is indicated by a Flag = 1** | | | | | | | | | | | | | |

### EXAMPLE 5:

### COMPARISON OF NORMAL VS PDAC VS CHRONIC PANCREATITIS TISSUE SAMPLES

Because some of the miRNA expression changes identified above may in fact be related to non-neoplastic processes, the inventors added to our analysis miRNA expression data from a control set consisting of six chronic pancreatitis tissue samples. Hierarchical clustering analysis showed that miRNA expression profiles from chronic pancreatitis samples are in-between the normal and PDAC profiles and are more similar overall to expression profiles from normal pancreas than to expression profiles from PDAC. Pair comparison of Pearson correlation values, calculated using mean normalized data for all the miRNAs, confirmed that global miRNA expression levels in chronic pancreatitis tissues are intermediate amid those in the normal and PDAC tissues (Table 7). Consistent with this observation, 45 out of the 52 miRNAs differentially expressed between PDAC and chronic tissue samples (Table 6, Flag (Ch vs Ca)=1) were also deregulated in PDAC respective to normal tissues (FIG. 4A, top). All of these miRNAs had expression levels in chronic samples that were in-between those of the normal and PDAC expression levels.

**Table 7. Paired Pearson Correlation Values.**

| | Normal | Chronic | Cancer | Cell Line |
|---|---|---|---|---|
| Normal | 1 | 0.96 | 0.93 | 0.78 |
| Chronic | 0.96 | 1 | 0.95 | 0.79 |
| Cancer | 0.93 | 0.95 | 1 | 0.84 |
| Cell Line | 0.78 | 0.79 | 0.84 | 1 |

Ninety-four (94) miRNAs were found differentially expressed at a significant level between any 2 of the 3 tissue types (Table 6). Among the 68 miRNA differentially expressed in chronic samples versus normal samples (Table 6, Flag (Ch vs N)=1), 61 were also deregulated in PDAC versus normal pancreas (FIG. 4A, bottom). These miRNAs whose expression is affected in both PDAC and chronic diseased tissues are more likely to reflect the desmoplastic reaction of the tumor rather than changes specific to PDAC. Nonetheless, Principal Component Analysis (PCA) using the 94 miRNAs identified above showed a perfect segregation of the PDAC samples away from the normal pancreas and chronic disease groups showing that miRNA expression classifies pancreatic tissues (FIG. 4B).

### EXAMPLE 6:

### MIRNA BIOMARKERS FOR PANCREATIC DISEASES

To identify the best miRNA markers for PDAC, the inventors selected those most differentially expressed among the three tissue types using stringent parameters: |Δh|>1.6 (5-fold) and p-value<0.0001 between at least 2 sample types (FIG. 5). Using this subset of 20 miRNAs, a clear discrimination between the tissue types could be achieved. For example, expression of miR-29c, -96, -143, -145, -148b and -150 were mis-regulated in both chronic and cancer samples while miR-196a, - 196b, -203, -210, -222, -216, -217 and -375 were mis-regulated only in PDAC samples. These data indicate that expression levels of a few miRNAs can be used to classify normal, chronic pancreatitis, and PDAC tissues and discriminate between neoplastic and non-neoplastic processes in PDAC.

### EXAMPLE 7:

### MIRNA EXPRESSION IN PANCREATIC CARCINOMA CELL LINES

Currently, pancreatic carcinoma cell lines represent the best available cell model systems for in vitro analyses of miRNA function. Therefore, the same array profiling strategy was deployed to characterize miRNA expression in six pancreatic primary ductal adenocarcinoma cell lines, IMIMPC2, PT45, PL45, SKPC1, PancTuI, and PaCa44. To compare miRNA expression profiles in cell lines and primary tissues, the inventors normalized miRNA array data from the cell lines together with the 19 tissue set (Table 4).

Hierarchical clustering analysis on the global miRNA population as well as clustering and principal component analyses (FIG. 6) on the differentially expressed miRNAs showed a clear segregation of the cell line samples away from the primary tissues. This divergence resulted mainly from the lack of detectable expression of ∼60 miRNAs in cancer cell line samples relative to tissue samples, including seven miRNAs from our list of top 20 differentially expressed miRNAs in PDAC (miR-143, -145, -150, -216, -217, -223 and -375; FIG. 5). Array data indicate that only 140 miRNAs were detected in the cancer cell lines, and each of them was also expressed in pancreatic tissues (see % of samples with miRNA detected above threshold value in Table 4).

Overall, the mea miRNA expression levels in cancer cell lines correlated better with PDAC than with normal or chronic tissue (Pearson correlation values of 0.84, 0.78 and 0.79 respectively; Table 7). Furthermore, 12 out the top 20 miRNAs differentially expressed in PDAC had expression levels in the cell lines that were very similar to those in PDAC. Thus, miRNA expression profiles in these cancer cell lines more closely resemble those from PDAC primary tumors than those from normal pancreatic tissues.

### EXAMPLE 8:

### IDENTIFICATION OF MIRNA BIOMARKERS OVER-EXPRESSED IN PDAC AND CANCER CELL LINES

Consistent with the differences described in Example 7, 87 miRNAs were identified as differentially expressed between PDAC and the cancer cell lines, and 108 were differentially expressed between normal pancreas and the cancer cell lines (Table 8A, 8B, and 8C, Flag (Ca vs CL)=1 or Flag (N vs CL) =1). Strikingly, one miRNA, miR-205, was highly over-expressed by more than 600-fold in cell lines versus normal tissue and was also up-regulated in 5 out of 8 PDAC tissues relative to chronic or normal tissues (Table 3). This observation prompted us to look for other miRNAs highly expressed in cell lines and up-regulated in PDAC that could have escaped our initial stringent screening because of a high p-value or low Δh (Example 6). By this approach, the inventors identified five additional miRNAs: miR-18a, -31, -93, -221, and -224 (FIG. 7). These six miRNAs (FIG. 7) are over-expressed in neoplastic ductal cells both in vivo and ex vivo, and therefore, represent additional potential biomarkers for PDAC.

**Table 8A. miRNAs Differentially Expressed Between Normal Pancreas (N), Chronic Pancreatitis (Ch), PDAC (Ca), and Pancreatic Cancer Cell lines (CL)**

| | **Mean Ch** | **Mean N** | **Mean Ca** | **Mean CL** | **p-value ANOVA** |
|---|---|---|---|---|---|
| **miRNA** | | | | | |
| hsa-let-7b | 8.78 | 9.06 | 8.53 | 7.96 | 8.56E-04 |
| hsa-let-7c | 8.74 | 9.18 | 8.60 | 8.08 | 1.01E-03 |
| hsa-let-7d | 7.85 | 8.68 | 8.19 | 8.01 | 3.71E-03 |
| hsa-let-7e | 6.19 | 6.98 | 6.75 | 6.46 | 8.94E-03 |
| hsa-let-7f | 7.51 | 8.51 | 7.94 | 7.36 | 8.57E-04 |
| hsa-let-7g | 7.01 | 7.52 | 7.31 | 6.45 | 8.72E-06 |
| hsa-let-7i | 7.12 | 6.31 | 7.49 | 7.96 | 1.21E-03 |
| hsa-miR-1 | 2.84 | 2.19 | 3.23 | 0.78 | 5.65E-03 |
| hsa-miR-100 | 6.44 | 5.16 | 6.34 | 7.11 | 1.35E-05 |
| hsa-miR-101 | 4.33 | 4.87 | 4.49 | 2.48 | 3.47E-10 |
| hsa-miR-103 | 6.02 | 5.86 | 6.72 | 6.80 | 3.86E-05 |
| hsa-miR-106a | 6.10 | 6.30 | 6.74 | 7.91 | 1.34E-08 |
| hsa-miR-106b | 5.02 | 4.98 | 5.65 | 6.02 | 3.88E-08 |
| hsa-miR-107 | 6.10 | 5.82 | 6.76 | 6.83 | 2.28E-05 |
| hsa-miR-10a | 5.70 | 4.92 | 6.23 | 5.87 | 5.54E-04 |
| hsa-miR-10b | 4.96 | 4.54 | 5.24 | 3.97 | 2.20E-04 |
| hsa-miR-125b | 7.37 | 5.96 | 6.93 | 6.06 | 4.23E-05 |
| hsa-miR-126 | 7.54 | 7.12 | 7.41 | 3.77 | 2.53E-11 |
| hsa-miR-126-AS | 3.27 | 3.91 | 3.55 | 0.90 | 2.08E-06 |
| hsa-miR-128a | 2.23 | 2.16 | 2.77 | 3.50 | 1.53E-06 |
| hsa-miR-130b | 4.93 | 6.27 | 3.84 | 4.98 | 4.01E-08 |
| hsa-miR-132 | 3.92 | 3.06 | 4.09 | 2.21 | 1.06E-05 |
| hsa-miR-140 | 3.63 | 3.24 | 4.08 | 1.77 | 1.57E-09 |
| hsa-miR-141 | 6.65 | 7.50 | 6.00 | 6.34 | 1.06E-05 |
| hsa-miR-142-3p | 2.76 | 2.71 | 3.68 | 0.87 | 3.52E-07 |
| hsa-miR-143 | 7.52 | 6.02 | 7.96 | 0.49 | 0.00E+00 |
| hsa-miR-145 | 7.82 | 6.32 | 8.15 | 0.65 | 0.00E+00 |
| hsa-miR-146a | 4.87 | 3.85 | 5.93 | 3.13 | 1.24E-05 |
| hsa-miR-148a | 7.14 | 8.57 | 5.27 | 2.55 | 1.91E-10 |
| hsa-miR-148b | 2.97 | 5.11 | 2.82 | 2.88 | 1.82E-06 |
| hsa-miR-150 | 3.68 | 1.73 | 4.38 | 1.19 | 1.49E-06 |
| hsa-miR-151 | 3.86 | 4.02 | 4.00 | 4.62 | 3.84E-05 |
| hsa-miR-152 | 5.05 | 4.75 | 5.15 | 2.90 | 2.40E-08 |
| hsa-miR-153 | 2.47 | 3.23 | 2.73 | 1.37 | 6.95E-04 |
| hsa-miR-154 | 2.92 | 3.27 | 2.07 | 0.63 | 2.49E-07 |
| hsa-miR-155 | 4.22 | 3.25 | 5.59 | 4.97 | 2.75E-03 |
| hsa-miR-15a | 6.19 | 6.14 | 6.47 | 5.60 | 1.48E-04 |
| hsa-miR-15b | 4.92 | 5.17 | 5.77 | 7.63 | 6.94E-11 |
| hsa-miR-17-3p | 2.13 | 2.31 | 2.43 | 3.33 | 1.60E-04 |
| hsa-miR-17-5p | 6.04 | 6.15 | 6.66 | 7.86 | 4.51E-08 |
| hsa-miR-18a | 3.17 | 2.58 | 4.26 | 5.14 | 3.68E-07 |
| hsa-miR-181a | 5.38 | 5.00 | 5.76 | 6.23 | 4.65E-04 |
| hsa-miR-181b | 4.42 | 4.35 | 5.05 | 5.99 | 1.54E-05 |
| hsa-miR-182 | 4.16 | 5.39 | 4.57 | 5.08 | 5.77E-04 |
| hsa-miR-183 | 1.94 | 2.14 | 1.57 | 3.23 | 3.96E-04 |
| hsa-miR-186 | 3.04 | 3.69 | 3.39 | 2.62 | 1.02E-03 |
| hsa-miR-192 | 6.07 | 7.13 | 6.63 | 2.23 | 5.47E-11 |
| hsa-miR-193a | 2.60 | 2.20 | 2.33 | 3.09 | 1.52E-03 |
| hsa-miR-194 | 5.95 | 6.63 | 7.13 | 2.27 | 9.00E-11 |
| hsa-miR-195 | 6.72 | 6.12 | 6.49 | 3.49 | 1.05E-12 |
| hsa-miR-196a | 1.41 | 1.13 | 3.77 | 2.85 | 1.00E-05 |
| hsa-miR-196b | 1.04 | 0.57 | 3.24 | 2.35 | 3.79E-04 |
| hsa-miR-197 | 2.07 | 1.72 | 2.11 | 3.74 | 2.46E-05 |
| hsa-miR-199a | 6.51 | 5.35 | 6.35 | 0.56 | 0.00E+00 |
| hsa-miR-199a-AS | 7.33 | 6.43 | 7.06 | 0.71 | 0.00E+00 |
| hsa-miR-199b | 4.79 | 4.36 | 4.58 | 0.73 | 7.77E-16 |
| hsa-miR-20a | 5.52 | 5.64 | 5.99 | 6.93 | 4.27E-06 |
| hsa-miR-200a | 5.61 | 6.57 | 5.52 | 5.63 | 1.38E-03 |
| hsa-miR-200b | 6.43 | 7.50 | 6.72 | 7.10 | 1.26E-03 |
| hsa-miR-200c | 7.60 | 8.59 | 7.28 | 8.46 | 3.51E-08 |
| hsa-miR-203 | 2.66 | 3.50 | 5.19 | 2.68 | 2.06E-04 |
| hsa-miR-205 | 1.44 | 0.90 | 3.12 | 7.35 | 3.47E-06 |
| hsa-miR-21 | 9.18 | 8.71 | 9.80 | 9.77 | 5.27E-04 |
| hsa-miR-210 | 4.43 | 3.79 | 6.61 | 4.71 | 2.38E-08 |
| hsa-miR-214 | 6.06 | 4.65 | 6.14 | 1.91 | 2.52E-13 |
| hsa-miR-215 | 2.54 | 4.33 | 3.91 | 0.96 | 3.13E-05 |
| hsa-miR-216 | 5.90 | 7.09 | 1.64 | 1.05 | 4.56E-13 |
| hsa-miR-217 | 6.70 | 7.86 | 2.19 | 1.27 | 3.81E-13 |
| hsa-miR-218 | 4.06 | 3.54 | 4.10 | 2.24 | 4.34E-07 |
| hsa-miR-22 | 7.07 | 6.79 | 7.25 | 5.59 | 2.00E-07 |
| hsa-miR-221 | 5.41 | 5.02 | 6.50 | 7.90 | 1.55E-11 |
| hsa-miR-222 | 4.63 | 3.92 | 5.99 | 7.22 | 3.43E-11 |
| hsa-miR-223 | 5.62 | 4.47 | 6.86 | 1.36 | 1.33E-10 |
| hsa-miR-224 | 2.44 | 2.56 | 3.83 | 4.20 | 1.51E-03 |
| hsa-miR-23a | 7.08 | 6.91 | 7.67 | 7.95 | 1.62E-06 |
| hsa-miR-23b | 7.21 | 7.16 | 7.62 | 7.74 | 2.71E-03 |
| hsa-miR-24 | 7.58 | 7.15 | 8.07 | 7.26 | 6.80E-06 |
| hsa-miR-25 | 5.10 | 5.42 | 5.69 | 5.66 | 2.18E-04 |
| hsa-miR-26a | 8.69 | 9.00 | 8.80 | 7.40 | 2.29E-08 |
| hsa-miR-26b | 6.58 | 7.46 | 6.98 | 5.28 | 1.94E-08 |
| hsa-miR-27a | 6.83 | 6.87 | 7.39 | 7.30 | 5.46E-04 |
| hsa-miR-27b | 7.07 | 7.47 | 7.13 | 6.74 | 2.55E-02 |
| hsa-miR-28 | 4.56 | 4.82 | 5.08 | 4.95 | 9.11E-03 |
| hsa-miR-29c | 6.95 | 8.25 | 6.49 | 4.60 | 1.48E-10 |
| hsa-miR-301 | 1.77 | 1.95 | 2.64 | 3.27 | 1.91E-06 |
| hsa-miR-30a-3p | 3.42 | 4.10 | 2.51 | 3.87 | 1.50E-04 |
| hsa-miR-30a-5p | 7.15 | 7.61 | 6.69 | 6.92 | 1.07E-03 |
| hsa-miR-30b | 6.20 | 6.84 | 5.74 | 5.37 | 2.31E-07 |
| hsa-miR-30c | 6.13 | 6.77 | 5.63 | 6.30 | 1.71E-04 |
| hsa-miR-30d | 6.58 | 7.08 | 6.29 | 6.41 | 6.28E-04 |
| hsa-miR-30e-3p | 2.54 | 3.58 | 2.06 | 3.04 | 1.91E-04 |
| hsa-miR-30e-5p | 6.50 | 7.06 | 6.26 | 5.88 | 9.81E-05 |
| hsa-miR-320 | 5.80 | 5.70 | 6.00 | 6.49 | 2.23E-03 |
| hsa-miR-324-3p | 2.47 | 1.44 | 2.58 | 3.04 | 3.63E-06 |
| hsa-miR-331 | 2.40 | 1.91 | 3.45 | 3.87 | 1.48E-06 |
| hsa-miR-335 | 5.05 | 6.19 | 4.96 | 3.37 | 3.25E-04 |
| hsa-miR-338 | 4.01 | 4.82 | 3.97 | 0.96 | 8.80E-07 |
| hsa-miR-339 | 2.39 | 2.43 | 2.33 | 3.47 | 1.37E-05 |
| hsa-miR-342 | 5.88 | 6.07 | 6.15 | 5.24 | 2.21E-04 |
| hsa-miR-34a | 5.56 | 5.29 | 5.87 | 3.31 | 1.16E-06 |
| hsa-miR-34b | 3.06 | 3.48 | 3.70 | 1.95 | 3.79E-06 |
| hsa-miR-361 | 4.39 | 4.45 | 4.76 | 5.22 | 1.48E-04 |
| hsa-miR-365 | 2.16 | 3.41 | 1.97 | 2.78 | 6.50E-06 |
| hsa-miR-368 | 5.08 | 5.56 | 4.41 | 0.71 | 0.00E+00 |
| hsa-miR-374 | 2.48 | 3.80 | 3.06 | 1.58 | 4.81E-06 |
| hsa-miR-375 | 6.43 | 7.21 | 4.70 | 0.85 | 1.41E-11 |
| hsa-miR-376a | 3.86 | 4.63 | 3.70 | 0.82 | 6.59E-13 |
| hsa-miR-377 | 3.05 | 3.30 | 2.59 | 0.85 | 9.82E-07 |
| hsa-miR-379 | 4.06 | 3.93 | 3.49 | 2.02 | 6.22E-09 |
| hsa-miR-381 | 2.31 | 2.08 | 1.39 | 0.96 | 1.97E-07 |
| hsa-miR-382 | 2.96 | 3.07 | 3.04 | 1.00 | 7.09E-08 |
| hsa-miR-422a | 1.89 | 2.60 | 2.77 | 3.54 | 3.60E-03 |
| hsa-miR-422b | 3.41 | 3.43 | 4.13 | 4.26 | 1.47E-02 |
| hsa-miR-423 | 3.51 | 3.05 | 3.79 | 5.07 | 2.76E-07 |
| hsa-miR-424 | 3.07 | 3.66 | 3.91 | 1.56 | 4.57E-05 |
| hsa-miR-429 | 4.03 | 5.13 | 4.71 | 4.42 | 2.57E-03 |
| hsa-miR-92 | 4.80 | 4.82 | 4.92 | 6.35 | 1.72E-08 |
| hsa-miR-93 | 4.99 | 4.83 | 5.94 | 6.78 | 1.26E-09 |
| hsa-miR-95 | 2.51 | 3.87 | 3.17 | 1.39 | 2.22E-06 |
| hsa-miR-96 | 2.79 | 4.69 | 3.04 | 3.10 | 9.15E-06 |
| hsa-miR-98 | 4.05 | 5.05 | 4.58 | 4.22 | 1.33E-02 |
| hsa-miR-99a | 6.64 | 5.38 | 6.27 | 5.97 | 1.29E-02 |
| hsa-miR-99b | 4.59 | 4.12 | 4.95 | 5.22 | 5.07E-03 |
| ambi-miR-7029 | 5.26 | 5.58 | 5.30 | 1.00 | 7.87E-09 |
| hsa-miR-193b | 3.17 | 3.00 | 3.11 | 5.08 | 2.48E-06 |
| ambi-miR-7058 | 3.97 | 4.03 | 4.13 | 4.85 | 3.23E-04 |
| hsa-miR-452 | 2.27 | 2.48 | 3.42 | 2.41. | 9.61E-04 |
| hsa-miR-432 | 3.36 | 3.09 | 3.11 | 2.21 | 3.11E-05 |
| hsa-miR-494 | 6.89 | 7.07 | 5.17 | 4.50 | 2.72E-04 |
| hsa-miR-497 | 5.20 | 3.99 | 4.86 | 1.94 | 3.17E-12 |
| ambi-miR-7105 | 2.10 | 2.42 | 2.99 | 2.78 | 2.38E-04 |

**Table 8B. miRNAs Differentially Expressed Between Normal Pancreas (N), Chronic Pancreatitis (Ch), PDAC (Ca), and Pancreatic Cancer Cell lines (CL)**

| | Ch vs N | | | Ch vs Ca | | | Ch vs CL | | |
|---|---|---|---|---|---|---|---|---|---|
| miRNA | Δh | p-value | Flag | Δh | p-value | Flag | Δh | p-value | Flag |
| hsa-let-7b | -0.29 | 1.41E-01 | 0 | 0.24 | 1.32E-01 | 0 | 0.81 | 1.50E-02 | 1 |
| hsa-let-7c | -0.44 | 1.64E-02 | 1 | 0.14 | 4.00E-01 | 0 | 0.66 | 2.62E-02 | 1 |
| hsa-let-7d | -0.83 | 5.50E-04 | 1 | -0.35 | 9.89E-02 | 0 | -0.16 | 3.63E-01 | 0 |
| hsa-let-7e | -0.79 | 4.08E-03 | 1 | -0.55 | 4.46E-03 | 1 | -0.27 | 2.04E-01 | 0 |
| hsa-let-7f | -1.00 | 2.23E-04 | 1 | -0.43 | 8.60E-02 | 0 | 0.15 | 5.20E-01 | 0 |
| hsa-let-7g | -0.51 | 1.48E-03 | 1 | -0.30 | 7.94E-02 | 0 | 0.57 | 1.94E-03 | 1 |
| hsa-let-7i | 0.80 | 1.97E-03 | 1 | -0.38 | 4.30E-02 | 0 | -0.84 | 1.01E-01 | 0 |
| hsa-miR-1 | 0.65 | 2.56E-01 | 0 | -0.39 | 6.41E-01 | 0 | 2.06 | 2.40E-03 | 1 |
| hsa-miR-100 | 1.28 | 6.57E-05 | 1 | 0.10 | 6.78E-01 | 0 | -0.67 | 3.71E-02 | 1 |
| hsa-miR-101 | -0.54 | 1.90E-03 | 1 | -0.16 | 4.62E-01 | 0 | 1.84 | 8.66E-07 | 1 |
| hsa-miR-103 | 0.16 | 2.75E-01 | 0 | -0.70 | 3.00E-04 | 1 | -0.78 | 4.04E-03 | 1 |
| hsa-miR-106a | -0.20 | 1.21E-01 | 0 | -0.64 | 7.38E-05 | 1 | -1.81 | 2.39E-05 | 1 |
| hsa-miR-106b | 0.04 | 6.93E-01 | 0 | -0.62 | 5.74E-04 | 1 | -1.00 | 1.77E-06 | 1 |
| hsa-miR-107 | 0.28 | 6.53E-02 | 0 | -0.66 | 1.89E-04 | 1 | -0.73 | 7.38E-03 | 1 |
| hsa-miR-10a | 0.77 | 4.48E-05 | 1 | -0.54 | 1.26E-02 | 1 | -0.17 | 5.96E-01 | 0 |
| hsa-miR-10b | 0.42 | 7.48E-02 | 0 | -0.29 | 2.02E-01 | 0 | 0.98 | 1.11E-02 | 1 |
| hsa-miR-125b | 1.41 | 2.37E-06 | 1 | 0.44 | 5.93E-02 | 0 | 1.31 | 1.46E-03 | 1 |
| hsa-miR-126 | 0.43 | 1.33E-02 | 1 | 0.14 | 3.66E-01 | 0 | 3.77 | 3.35E-06 | 1 |
| hsa-miR-126-AS | -0.64 | 1.62E-01 | 0 | -0.28 | 5.55E-01 | 0 | 2.37 | 4.29E-04 | 1 |
| hsa-miR-128a | 0.08 | 7.15E-01 | 0 | -0.53 | 8.44E-04 | 1 | -1.27 | 2.59E-05 | 1 |
| hsa-miR-130b | -1.34 | 2.00E-03 | 1 | 1.09 | 9.55E-04 | 1 | -0.05 | 8.74E-01 | 0 |
| hsa-miR-132 | 0.86 | 7.20E-02 | 0 | -0.17 | 5.05E-01 | 0 | 1.71 | 1.73E-04 | 1 |
| hsa-miR-140 | 0.40 | 8.93E-02 | 0 | -0.45 | 5.63E-02 | 0 | 1.86 | 1.20E-05 | 1 |
| hsa-miR-141 | -0.85 | 4.45E-03 | 1 | 0.65 | 2.27E-02 | 0 | 0.31 | 2.03E-01 | 0 |
| hsa-miR-142-3p | 0.05 | 9.27E-01 | 0 | -0.92 | 1.24E-02 | 1 | 1.89 | 8.70E-05 | 1 |
| hsa-miR-143 | 1.50 | 6.47E-06 | 1 | -0.44 | 1.17E-01 | 0 | 7.02 | 1.97E-13 | 1 |
| hsa-miR-145 | 1.50 | 6.33E-05 | 1 | -0.33 | 3.52E-01 | 0 | 7.17 | 2.20E-11 | 1 |
| hsa-miR-146a | 1.03 | 1.80E-02 | 1 | -1.06 | 5.94E-03 | 1 | 1.75 | 1.51E-02 | 1 |
| hsa-miR-148a | -1.43 | 3.09E-03 | 1 | 1.86 | 3.58E-05 | 1 | 4.59 | 2.84E-05 | 1 |
| hsa-miR-148b | -2.14 | 2.19E-04 | 1 | 0.15 | 6.41E-01 | 0 | 0.09 | 7.82E-01 | 0 |
| hsa-miR-150 | 1.95 | 2.07E-02 | 1 | -0.70 | 1.89E-01 | 0 | 2.49 | 2.35E-03 | 1 |
| hsa-miR-151 | -0.16 | 2.99E-01 | 0 | -0.14 | 2.25E-01 | 0 | -0.76 | 6.36E-04 | 1 |
| hsa-miR-152 | 0.30 | 1.80E-01 | 0 | -0.10 | 6.08E-01 | 0 | 2.16 | 7.33E-05 | 1 |
| hsa-miR-153 | -0.75 | 5.97E-02 | 0 | -0.25 | 5.55E-01 | 0 | 1.10 | 1.50E-02 | 1 |
| hsa-miR-154 | -0.35 | 3.23E-01 | 0 | 0.85 | 2.22E-02 | 0 | 2.29 | 1.01E-04 | 1 |
| hsa-miR-155 | 0.96 | 9.15E-02 | 0 | -1.38 | 1.24E-03 | 1 | -0.76 | 2.75E-01 | 0 |
| hsa-miR-15a | 0.05 | 6.09E-01 | 0 | -0.28 | 7.62E-02 | 0 | 0.59 | 8.98E-03 | 1 |
| hsa-miR-15b | -0.25 | 2.67E-01 | 0 | -0.85 | 9.62E-04 | 1 | -2.71 | 2.05E-07 | 1 |
| hsa-miR-17-3p | -0.18 | 3.80E-01 | 0 | -0.30 | 1.57E-01 | 0 | -1.20 | 1.45E-03 | 1 |
| hsa-miR-17-5p | -0.11 | 4.37E-01 | 0 | -0.63 | 3.90E-04 | 1 | -1.82 | 3.81E-05 | 1 |
| hsa-miR-18a | 0.59 | 3.25E-02 | 0 | -1.09 | 1.50E-03 | 1 | -1.97 | 2.84E-04 | 1 |
| hsa-miR-181a | 0.38 | 1.19E-01 | 0 | -0.39 | 1.41E-01 | 0 | -0.86 | 9.46E-03 | 1 |
| hsa-miR-181b | 0.06 | 7.66E-01 | 0 | -0.63 | 7.94E-03 | 1 | -1.57 | 9.14E-04 | 1 |
| hsa-miR-182 | -1.23 | 1.25E-03 | 1 | -0.41 | 1.33E-01 | 0 | -0.92 | 7.06E-03 | 1 |
| hsa-miR-183 | -0.20 | 4.93E-01 | 0 | 0.37 | 3.61E-01 | 0 | -1.29 | 1.11E-03 | 1 |
| hsa-miR-186 | -0.65 | 1.10E-02 | 1 | -0.35 | 8.00E-02 | 0 | 0.42 | 1.62E-01 | 0 |
| hsa-miR-192 | -1.06 | 1.99E-04 | 1 | -0.56 | 2.53E-01 | 0 | 3.85 | 2.56E-10 | 1 |
| hsa-miR-193a | 0.40 | 3.78E-02 | 0 | 0.27 | 1.69E-01 | 0 | -0.49 | 4.00E-02 | 0 |
| hsa-miR-194 | -0.68 | 1.54E-03 | 1 | -1.18 | 2.92E-02 | 0 | 3.68 | 5.59E-10 | 1 |
| hsa-miR-195 | 0.60 | 1.21E-02 | 1 | 0.24 | 1.78E-01 | 0 | 3.24 | 1.92E-08 | 1 |
| hsa-miR-196a | 0.28 | 5.14E-01 | 0 | -2.36 | 2.01E-05 | 1 | -1.43 | 2.89E-02 | 1 |
| hsa-miR-196b | 0.47 | 3.27E-01 | 0 | -2.20 | 1.45E-04 | 1 | -1.31 | 1.14E-01 | 0 |
| hsa-miR-197 | 0.35 | 8.86E-02 | 0 | -0.04 | 8.75E-01 | 0 | -1.67 | 2.02E-03 | 1 |
| hsa-miR-199a | 1.16 | 3.52E-04 | 1 | 0.16 | 3.37E-01 | 0 | 5.95 | 7.62E-10 | 1 |
| hsa-miR-199a-AS | 0.90 | 1.34E-04 | 1 | 0.27 | 1.07E-02 | 1 | 6.62 | 1.00E-10 | 1 |
| hsa-miR-199b | 0.43 | 4.60E-02 | 0 | 0.21 | 2.72E-01 | 0 | 4.06 | 6.93E-09 | 1 |
| hsa-miR-20a | -0.12 | 4.09E-01 | 0 | -0.47 | 3.29E-03 | 1 | -1.41 | 3.99E-04 | 1 |
| hsa-miR-200a | -0.96 | 1.26E-03 | 1 | 0.09 | 7.33E-01 | 0 | -0.02 | 9.46E-01 | 0 |
| hsa-miR-200b | -1.07 | 6.18E-04 | 1 | -0.30 | 2.57E-01 | 0 | -0.67 | 2.06E-02 | 1 |
| hsa-miR-200c | -0.99 | 4.17E-04 | 1 | 0.32 | 9.97E-02 | 0 | -0.86 | 4.32E-04 | 1 |
| hsa-miR-203 | -0.84 | 1.82E-02 | 1 | -2.54 | 6.14E-07 | 1 | -0.03 | 9.74E-01 | 0 |
| hsa-miR-205 | 0.53 | 2.66E-01 | 0 | -1.69 | 1.15E-01 | 0 | -5.91 | 2.04E-06 | 1 |
| hsa-miR-21 | 0.48 | 1.49E-01 | 0 | -0.62 | 1.03E-02 | 1 | -0.59 | 2.17E-02 | 1 |
| hsa-miR-210 | 0.65 | 7.35E-03 | 1 | -2.17 | 9.55E-06 | 1 | -0.28 | 3.79E-01 | 0 |
| hsa-miR-214 | 1.41 | 5.77E-04 | 1 | -0.08 | 7.67E-01 | 0 | 4.15 | 1.56E-08 | 1 |
| hsa-miR-215 | -1.79 | 2.60E-04 | 1 | -1.37 | 5.99E-02 | 0 | 1.58 | 6.37E-04 | 1 |
| hsa-miR-216 | -1.19 | 1.35E-02 | 1 | 4.25 | 7.01E-07 | 1 | 4.85 | 3.16E-07 | 1 |
| hsa-miR-217 | -1.17 | 1.18E-02 | 1 | 4.51 | 1.71E-06 | 1 | 5.42 | 1.96E-08 | 1 |
| hsa-miR-218 | 0.52 | 2.03E-02 | 1 | -0.04 | 8.61E-01 | 0 | 1.81 | 3.05E-05 | 1 |
| hsa-miR-22 | 0.28 | 5.62E-02 | 0 | -0.18 | 1.87E-01 | 0 | 1.48 | 3.17E-04 | 1 |
| hsa-miR-221 | 0.39 | 3.59E-02 | 0 | -1.09 | 5.77E-05 | 1 | -2.48 | 2.61E-07 | 1 |
| hsa-miR-222 | 0.71 | 1.53E-02 | 1 | -1.36 | 5.16E-05 | 1 | -2.58 | 1.34E-07 | 1 |
| hsa-miR-223 | 1.15 | 3.12E-02 | 0 | -1.24 | 2.21E-02 | 0 | 4.27 | 6.85E-07 | 1 |
| hsa-miR-224 | -0.12 | 5.49E-01 | 0 | -1.39 | 3.30E-07 | 1 | -1.76 | 2.14E-02 | 1 |
| hsa-miR-23a | 0.17 | 2.54E-01 | 0 | -0.59 | 2.24E-03 | 1 | -0.87 | 8.03E-05 | 1 |
| hsa-miR-23b | 0.05 | 7.24E-01 | 0 | -0.41 | 3.04E-02 | 0 | -0.53 | 1.29E-03 | 1 |
| hsa-miR-24 | 0.42 | 9.32E-03 | 1 | -0.50 | 3.00E-03 | 1 | 0.31 | 5.64E-02 | 0 |
| hsa-miR-25 | -0.32 | 2.01E-02 | 1 | -0.58 | 1.11E-04 | 1 | -0.56 | 2.52E-03 | 1 |
| hsa-miR-26a | -0.31 | 4.14E-02 | 0 | -0.11 | 4.83E-01 | 0 | 1.29 | 1.87E-05 | 1 |
| hsa-miR-26b | -0.88 | 4.21E-04 | 1 | -0.40 | 6.53E-02 | 0 | 1.30 | 4.78E-04 | 1 |
| hsa-miR-27a | -0.04 | 7.85E-01 | 0 | -0.56 | 2.68E-03 | 1 | -0.47 | 1.12E-02 | 1 |
| hsa-miR-27b | -0.40 | 1.64E-02 | 1 | -0.06 | 6.72E-01 | 0 | 0.33 | 1.99E-01 | 0 |
| hsa-miR-28 | -0.26 | 8.53E-02 | 0 | -0.52 | 2.47E-03 | 1 | -0.39 | 1.09E-02 | 1 |
| hsa-miR-29c | -1.30 | 2.24E-04 | 1 | 0.45 | 7.08E-02 | 0 | 2.35 | 1.68E-05 | 1 |
| hsa-miR-301 | -0.17 | 3.45E-01 | 0 | -0.86 | 2.11E-04 | 1 | -1.50 | 1.82E-04 | 1 |
| hsa-miR-30a-3p | -0.68 | 4.81E-03 | 1 | 0.91 | 2.27E-04 | 1 | -0.45 | 3.32E-01 | 0 |
| hsa-miR-30a-5p | -0.47 | 6.63E-03 | 1 | 0.46 | 5.87E-03 | 1 | 0.23 | 3.88E-01 | 0 |
| hsa-miR-30b | -0.65 | 2.61E-03 | 1 | 0.46 | 7.43E-03 | 1 | 0.83 | 1.81E-03 | 1 |
| hsa-miR-30c | -0.65 | 1.75E-03 | 1 | 0.50 | 3.89E-03 | 1 | -0.18 | 5.25E-01 | 0 |
| hsa-miR-30d | -0.51 | 1.04E-03 | 1 | 0.28 | 6.70E-02 | 0 | 0.16 | 3.67E-01 | 0 |
| hsa-miR-30e-3p | -1.05 | 4.39E-03 | 1 | 0.48 | 9.68E-02 | 0 | -0.50 | 1.93E-01 | 0 |
| hsa-miR-30e-5p | -0.57 | 9.20E-04 | 1 | 0.24 | 5.77E-02 | 0 | 0.62 | 4.20E-02 | 0 |
| hsa-miR-320 | 0.10 | 4.74E-01 | 0 | -0.20 | 2.52E-01 | 0 | -0.69 | 5.77E-03 | 1 |
| hsa-miR-324-3p | 1.04 | 7.28E-04 | 1 | -0.10 | 4.47E-01 | 0 | -0.57 | 2.71E-02 | 1 |
| hsa-miR-331 | 0.49 | 1.38E-01 | 0 | -1.04 | 1.41E-03 | 1 | -1.47 | 1.61E-04 | 1 |
| hsa-miR-335 | -1.14 | 3.22E-03 | 1 | 0.09 | 7.68E-01 | 0 | 1.68 | 3.19E-02 | 1 |
| hsa-miR-338 | -0.81 | 1.03E-03 | 1 | 0.04 | 9.09E-01 | 0 | 3.05 | 3.99E-04 | 1 |
| hsa-miR-339 | -0.03 | 8.52E-01 | 0 | 0.06 | 6.76E-01 | 0 | -1.08 | 1.37E-03 | 1 |
| hsa-miR-342 | -0.19 | 1.06E-01 | 0 | -0.27 | 9.69E-03 | 1 | 0.65 | 2.69E-02 | 1 |
| hsa-miR-34a | 0.27 | 3.14E-01 | 0 | -0.31 | 1.46E-01 | 0 | 2.25 | 5.36E-04 | 1 |
| hsa-miR-34b | -0.43 | 2.47E-01 | 0 | -0.64 | 2.11E-03 | 1 | 1.10 | 3.01E-04 | 1 |
| hsa-miR-361 | -0.06 | 6.74E-01 | 0 | -0.37 | 4.08E-02 | 0 | -0.83 | 7.48E-05 | 1 |
| hsa-miR-365 | -1.26 | 9.64E-04 | 1 | 0.18 | 3.90E-01 | 0 | -0.63 | 3.65E-03 | 1 |
| hsa-miR-368 | -0.48 | 2.87E-02 | 0 | 0.67 | 2.41E-03 | 1 | 4.37 | 4.17E-10 | 1 |
| hsa-miR-374 | -1.32 | 2.21E-03 | 1 | -0.57 | 6.22E-02 | 0 | 0.90 | 6.73E-03 | 1 |
| hsa-miR-375 | -0.78 | 5.79E-02 | 0 | 1.73 | 4.49E-03 | 1 | 5.58 | 4.23E-08 | 1 |
| hsa-miR-376a | -0.77 | 8.25E-03 | 1 | 0.16 | 4.96E-01 | 0 | 3.04 | 1.68E-07 | 1 |
| hsa-miR-377 | -0.25 | 3.33E-01 | 0 | 0.46 | 8.57E-02 | 0 | 2.20 | 2.28E-04 | 1 |
| hsa-miR-379 | 0.13 | 3.93E-01 | 0 | 0.57 | 5.30E-03 | 1 | 2.04 | 2.56E-06 | 1 |
| hsa-miR-381 | 0.23 | 3.56E-01 | 0 | 0.92 | 4.15E-05 | 1 | 1.35 | 1.03E-05 | 1 |
| hsa-miR-382 | -0.11 | 7.49E-01 | 0 | -0.07 | 7.57E-01 | 0 | 1.96 | 5.44E-05 | 1 |
| hsa-miR-422a | -0.71 | 1.05E-01 | 0 | -0.88 | 4.70E-02 | 0 | -1.65 | 4.83E-03 | 1 |
| hsa-miR-422b | -0.02 | 9.53E-01 | 0 | -0.72 | 6.64E-03 | 1 | -0.85 | 2.94E-02 | 1 |
| hsa-miR-423 | 0.47 | 1.23E-01 | 0 | -0.28 | 2.76E-01 | 0 | -1.55 | 1.13E-05 | 1 |
| hsa-miR-424 | -0.59 | 1.93E-01 | 0 | -0.85 | 6.16E-03 | 1 | 1.51 | 1.58E-02 | 1 |
| hsa-miR-429 | -1.10 | 8.23E-04 | 1 | -0.68 | 3.37E-02 | 0 | -0.39 | 1.37E-01 | 0 |
| hsa-miR-92 | -0.02 | 8.95E-01 | 0 | -0.12 | 3.21E-01 | 0 | -1.55 | 3.42E-05 | 1 |
| hsa-miR-93 | 0.16 | 4.03E-01 | 0 | -0.95 | 1.11E-04 | 1 | -1.80 | 2.50E-06 | 1 |
| hsa-miR-95 | -1.36 | 4.92E-04 | 1 | -0.66 | 9.37E-02 | 0 | 1.12 | 1.69E-03 | 1 |
| hsa-miR-96 | -1.90 | 2.51E-04 | 1 | -0.25 | 3.60E-01 | 0 | -0.31 | 3.91E-01 | 0 |
| hsa-miR-98 | -1.00 | 1.86E-03 | 1 | -0.53 | 4.71E-02 | 0 | -0.16 | 5.25E-01 | 0 |
| hsa-miR-99a | 1.26 | 2.09E-04 | 1 | 0.37 | 2.34E-01 | 0 | 0.67 | 8.83E-02 | 0 |
| hsa-miR-99b | 0.47 | 4.28E-02 | 0 | -0.36 | 7.55E-02 | 0 | -0.63 | 6.85E-02 | 0 |
| ambi-miR-7029 | -0.31 | 6.21E-01 | 0 | -0.04 | 9.43E-01 | 0 | 4.26 | 8.68E-06 | 1 |
| hsa-miR-193b | 0.17 | 4.09E-01 | 0 | 0.06 | 8.57E-01 | 0 | -1.91 | 3.32E-05 | 1 |
| ambi-miR-7058 | -0.06 | 7.77E-01 | 0 | -0.17 | 4.02E-01 | 0 | -0.89 | 1.98E-03 | 1 |
| hsa-miR-452 | -0.21 | 3.49E-01 | 0 | -1.15 | 5.38E-05 | 1 | -0.13 | 7.43E-01 | 0 |
| hsa-miR-432 | 0.27 | 1.65E-01 | 0 | 0.25 | 1.67E-01 | 0 | 1.16 | 2.25E-04 | 1 |
| hsa-miR-494 | -0.18 | 7.96E-01 | 0 | 1.71 | 1.18E-02 | 1 | 2.39 | 1.71E-03 | 1 |
| hsa-miR-497 | 1.21 | 1.68E-04 | 1 | 0.34 | 9.57E-02 | 0 | 3.27 | 1.89E-07 | 1 |
| ambi-miR-7105 | -0.32 | 8.49E-02 | 0 | -0.89 | 1.91E-05 | 1 | -0.68 | 1.37E-02 | 1 |

| | | | | | | | | | |
|---|---|---|---|---|---|---|---|---|---|
| Significant differential expression is indicated by a Flag = 1 | | | | | | | | | |

**Table 8C. miRNAs Differentially Expressed Between Normal Pancreas (N), Chronic Pancreatitis (Ch), PDAC (Ca), and Pancreatic Cancer Cell lines (CL)**

| | N vs Ca | | | N vs CL | | | Ca vs CL | | |
|---|---|---|---|---|---|---|---|---|---|
| miRNA | Δh | p-value | Flag | Δh | p-value | Flag | Δh | p-value | Flag |
| hsa-let-7b | 0.53 | 6.62E-03 | 1 | 1.10 | 5.35E-03 | 1 | 0.57 | 3.54E-02 | 0 |
| hsa-let-7c | 0.58 | 7.45E-03 | 1 | 1.10 | 3.20E-03 | 1 | 0.52 | 5.63E-02 | 0 |
| hsa-let-7d | 0.48 | 4.50E-02 | 0 | 0.67 | 5.25E-03 | 1 | 0.19 | 3.80E-01 | 0 |
| hsa-let-7e | 0.23 | 3.08E-01 | 0 | 0.52 | 8.49E-02 | 0 | 0.28 | 1.93E-01 | 0 |
| hsa-let-7f | 0.57 | 4.31E-02 | 0 | 1.15 | 1.43E-03 | 1 | 0.58 | 5.37E-02 | 0 |
| hsa-let-7g | 0.21 | 2.50E-01 | 0 | 1.08 | 7.91E-05 | 1 | 0.86 | 3.81E-04 | 1 |
| hsa-let-7i | -1.18 | 8.05E-06 | 1 | -1.65 | 9.36E-03 | 1 | -0.47 | 2.60E-01 | 0 |
| hsa-miR-1 | -1.04 | 2.09E-01 | 0 | 1.41 | 8.60E-05 | 1 | 2.45 | 5.23E-03 | 1 |
| hsa-miR-100 | -1.18 | 9.86E-04 | 1 | -1.95 | 1.11E-04 | 1 | -0.77 | 2.69E-02 | 1 |
| hsa-miR-101 | 0.38 | 1.13E-01 | 0 | 2.39 | 9.82E-08 | 1 | 2.01 | 1.36E-06 | 1 |
| hsa-miR-103 | -0.86 | 1.56E-04 | 1 | -0.95 | 2.85E-03 | 1 | -0.08 | 6.80E-01 | 0 |
| hsa-miR-106a | -0.44 | 1.37E-03 | 1 | -1.61 | 1.69E-04 | 1 | -1.17 | 1.34E-04 | 1 |
| hsa-miR-106b | -0.67 | 5.10E-04 | 1 | -1.04 | 1.11E-06 | 1 | -0.37 | 1.24E-02 | 1 |
| hsa-miR-107 | -0.94 | 2.95E-05 | 1 | -1.02 | 2.40E-03 | 1 | -0.08 | 7.09E-01 | 0 |
| hsa-miR-10a | -1.31 | 3.92E-05 | 1 | -0.95 | 2.24E-02 | 1 | 0.37 | 2.62E-01 | 0 |
| hsa-miR-10b | -0.70 | 2.30E-03 | 1 | 0.57 | 9.28E-02 | 0 | 1.27 | 5.34E-04 | 1 |
| hsa-miR-125b | -0.97 | 8.80E-04 | 1 | -0.10 | 7.62E-01 | 0 | 0.87 | 1.48E-02 | 1 |
| hsa-miR-126 | -0.29 | 5.67E-02 | 0 | 3.34 | 3.35E-05 | 1 | 3.63 | 2.76E-07 | 1 |
| hsa-miR-126-AS | 0.36 | 3.72E-01 | 0 | 3.01 | 1.23E-05 | 1 | 2.65 | 3.72E-05 | 1 |
| hsa-miR-128a | -0.61 | 1.00E-02 | 1 | -1.35 | 5.30E-04 | 1 | -0.74 | 8.50E-04 | 1 |
| hsa-miR-130b | 2.43 | 1.13E-09 | 1 | 1.29 | 3.62E-04 | 1 | -1.14 | 6.70E-05 | 1 |
| hsa-miR-132 | -1.03 | 9.27E-03 | 1 | 0.85 | 5.45E-02 | 0 | 1.88 | 1.39E-06 | 1 |
| hsa-miR-140 | -0.85 | 1.05E-03 | 1 | 1.46 | 4.84E-05 | 1 | 2.31 | 1.26E-07 | 1 |
| hsa-miR-141 | 1.50 | 2.56E-05 | 1 | 1.16 | 3.50E-05 | 1 | -0.34 | 1.33E-01 | 0 |
| hsa-miR-142-3p | -0.97 | 2.74E-02 | 0 | 1.85 | 9.03E-04 | 1 | 2.81 | 1.20E-08 | 1 |
| hsa-miR-143 | -1.94 | 2.65E-05 | 1 | 5.53 | 1.52E-11 | 1 | 7.46 | 1.36E-12 | 1 |
| hsa-miR-145 | -1.83 | 3.22E-04 | 1 | 5.67 | 7.09E-10 | 1 | 7.50 | 3.98E-11 | 1 |
| hsa-miR-146a | -2.09 | 1.35E-05 | 1 | 0.72 | 2.71E-01 | 0 | 2.81 | 1.14E-04 | 1 |
| hsa-miR-148a | 3.30 | 4.26E-09 | 1 | 6.02 | 5.64E-06 | 1 | 2.73 | 1.49E-04 | 1 |
| hsa-miR-148b | 2.29 | 2.74E-05 | 1 | 2.23 | 8.93E-05 | 1 | -0.06 | 8.42E-01 | 0 |
| hsa-miR-150 | -2.65 | 6.12E-06 | 1 | 0.54 | 2.52E-01 | 0 | 3.19 | 7.12E-08 | 1 |
| hsa-miR-151 | 0.02 | 8.75E-01 | 0 | -0.60 | 3.14E-03 | 1 | -0.61 | 1.91E-04 | 1 |
| hsa-miR-152 | -0.40 | 2.81E-02 | 0 | 1.85 | 4.01E-04 | 1 | 2.25 | 3.04E-06 | 1 |
| hsa-miR-153 | 0.50 | 1.98E-01 | 0 | 1.86 | 4.81E-05 | 1 | 1.36 | 3.49E-03 | 1 |
| hsa-miR-154 | 1.20 | 6.30E-04 | 1 | 2.64 | 6.56E-06 | 1 | 1.44 | 3.34E-04 | 1 |
| hsa-miR-155 | -2.34 | 3.56E-04 | 1 | -1.72 | 5.94E-02 | 0 | 0.62 | 3.01E-01 | 0 |
| hsa-miR-15a | -0.33 | 3.81E-02 | 0 | 0.53 | 1.65E-02 | 1 | 0.86 | 5.46E-04 | 1 |
| hsa-miR-15b | -0.60 | 1.32E-02 | 1 | -2.46 | 1.59E-06 | 1 | -1.86 | 8.21E-07 | 1 |
| hsa-miR-17-3p | -0.11 | 5.23E-01 | 0 | -1.02 | 3.80E-03 | 1 | -0.90 | 2.51E-03 | 1 |
| hsa-miR-17-5p | -0.52 | 2.51E-03 | 1 | -1.71 | 1.88E-04 | 1 | -1.20 | 2.27E-04 | 1 |
| hsa-miR-18a | -1.68 | 4.02E-05 | 1 | -2.56 | 5.93E-05 | 1 | -0.88 | 2.40E-02 | 1 |
| hsa-miR-181a | -0.76 | 3.68E-03 | 1 | -1.23 | 2.89E-04 | 1 | -0.47 | 7.69E-02 | 0 |
| hsa-miR-181b | -0.69 | 2.68E-03 | 1 | -1.64 | 1.05E-03 | 1 | -0.94 | 6.92E-03 | 1 |
| hsa-miR-182 | 0.82 | 4.46E-03 | 1 | 0.31 | 2.27E-01 | 0 | -0.51 | 5.09E-02 | 0 |
| hsa-miR-183 | 0.57 | 1.66E-01 | 0 | -1.09 | 1.01E-03 | 1 | -1.66 | 6.66E-04 | 1 |
| hsa-miR-186 | 0.30 | 1.01E-01 | 0 | 1.07 | 3.57E-03 | 1 | 0.77 | 6.10E-03 | 1 |
| hsa-miR-192 | 0.51 | 3.35E-01 | 0 | 4.91 | 1.30E-11 | 1 | 4.40 | 4.92E-07 | 1 |
| hsa-miR-193a | -0.13 | 5.21E-01 | 0 | -0.89 | 3.35E-03 | 1 | -0.76 | 4.66E-03 | 1 |
| hsa-miR-194 | -0.51 | 3.49E-01 | 0 | 4.36 | 1.84E-10 | 1 | 4.86 | 2.86E-07 | 1 |
| hsa-miR-195 | -0.36 | 1.24E-01 | 0 | 2.64 | 3.37E-06 | 1 | 3.00 | 9.57E-09 | 1 |
| hsa-miR-196a | -2.64 | 3.99E-06 | 1 | -1.72 | 1.39E-02 | 1 | 0.92 | 6.51E-02 | 0 |
| hsa-miR-196b | -2.67 | 1.01E-05 | 1 | -1.78 | 4.65E-02 | 0 | 0.89 | 1.84E-01 | 0 |
| hsa-miR-197 | -0.39 | 1.42E-01 | 0 | -2.02 | 9.22E-04 | 1 | -1.63 | 1.20E-03 | 1 |
| hsa-miR-199a | -1.00 | 7.20E-05 | 1 | 4.79 | 3.95E-08 | 1 | 5.79 | 5.24E-12 | 1 |
| hsa-miR-199a-AS | -0.63 | 4.67E-04 | 1 | 5.72 | 8.48E-09 | 1 | 6.35 | 1.10E-12 | 1 |
| hsa-miR-199b | -0.22 | 1.56E-01 | 0 | 3.63 | 1.81E-08 | 1 | 3.85 | 9.95E-11 | 1 |
| hsa-miR-20a | -0.36 | 1.60E-02 | 1 | -1.29 | 1.53E-03 | 1 | -0.94 | 1.82E-03 | 1 |
| hsa-miR-200a | 1.05 | 9.04E-04 | 1 | 0.95 | 1.35E-03 | 1 | -0.11 | 6.83E-01 | 0 |
| hsa-miR-200b | 0.77 | 3.94E-03 | 1 | 0.40 | 5.26E-02 | 0 | -0.37 | 1.36E-01 | 0 |
| hsa-miR-200c | 1.31 | 3.88E-06 | 1 | 0.12 | 2.97E-01 | 0 | -1.19 | 2.66E-06 | 1 |
| hsa-miR-203 | -1.69 | 2.33E-04 | 1 | 0.82 | 3.61E-01 | 0 | 2.51 | 2.79E-03 | 1 |
| hsa-miR-205 | -2.22 | 7.67E-02 | 0 | -6.44 | 1.42E-OS | 1 | -4.23 | 2.34E-03 | 1 |
| hsa-miR-21 | -1.09 | 1.60E-03 | 1 | -1.07 | 5.06E-03 | 1 | 0.03 | 8.91E-01 | 0 |
| hsa-miR-210 | -2.82 | 2.13E-06 | 1 | -0.93 | 1.63E-02 | 1 | 1.90 | 1.80E-04 | 1 |
| hsa-miR-214 | -1.49 | 1.67E-04 | 1 | 2.73 | 1.64E-06 | 1 | 4.23 | 9.20E-10 | 1 |
| hsa-miR-215 | 0.42 | 5.77E-01 | 0 | 3.37 | 4.18E-06 | 1 | 2.95 | 8.70E-04 | 1 |
| hsa-miR-216 | 5.45 | 2.52E-08 | 1 | 6.04 | 2.98E-09 | 1 | 0.60 | 1.57E-01 | 0 |
| hsa-miR-217 | 5.68 | 1.66E-07 | 1 | 6.59 | 1.50E-12 | 1 | 0.91 | 6.31E-02 | 0 |
| hsa-miR-218 | -0.56 | 4.59E-02 | 0 | 1.29 | 1.39E-03 | 1 | 1.85 | 2.29E-05 | 1 |
| hsa-miR-22 | -0.46 | 2.33E-03 | 1 | 1.20 | 2.59E-03 | 1 | 1.66 | 1.53E-05 | 1 |
| hsa-miR-221 | -1.48 | 1.18E-05 | 1 | -2.88 | 4.44E-07 | 1 | -1.40 | 3.25E-05 | 1 |
| hsa-miR-222 | -2.06 | 8.61E-06 | 1 | -3.29 | 3.63E-07 | 1 | -1.23 | 1.81 E-04 | 1 |
| hsa-miR-223 | -2.39 | 4.27E-04 | 1 | 3.12 | 1.32E-05 | 1 | 5.50 | 1.99E-08 | 1 |
| hsa-miR-224 | -1.27 | 2.18E-05 | 1 | -1.64 | 4.94E-02 | 0 | -0.37 | 5.21E-01 | 0 |
| hsa-miR-23a | -0.76 | 5.15E-04 | 1 | -1.04 | 2.49E-05 | 1 | -0.28 | 8.92E-02 | 0 |
| hsa-miR-23b | -0.46 | 3.14E-02 | 0 | -0.58 | 2.32E-03 | 1 | -0.12 | 4.71E-01 | 0 |
| hsa-miR-24 | -0.92 | 7.49E-05 | 1 | -0.11 | 5.14E-01 | 0 | 0.81 | 2.16E-04 | 1 |
| hsa-miR-25 | -0.26 | 2.80E-02 | 0 | -0.24 | 1.31E-01 | 0 | 0.02 | 8.60E-01 | 0 |
| hsa-miR-26a | 0.20 | 2.61E-01 | 0 | 1.60 | 2.31E-05 | 1 | 1.39 | 8.09E-06 | 1 |
| hsa-miR-26b | 0.48 | 1.85E-02 | 1 | 2.18 | 7.78E-06 | 1 | 1.70 | 1.22E-05 | 1 |
| hsa-miR-27a | -0.52 | 2.02E-03 | 1 | -0.43 | 5.72E-03 | 1 | 0.09 | 5.26E-01 | 0 |
| hsa-miR-27b | 0.33 | 7.68E-02 | 0 | 0.72 | 2.45E-02 | 1 | 0.39 | 1.19E-01 | 0 |
| hsa-miR-28 | -0.26 | 1.43E-01 | 0 | -0.12 | 4.52E-01 | 0 | 0.14 | 3.81E-01 | 0 |
| hsa-miR-29c | 1.75 | 5.23E-06 | 1 | 3.65 | 6.72E-07 | 1 | 1.89 | 1.85E-05 | 1 |
| hsa-miR-301 | -0.69 | 1.02E-03 | 1 | -1.32 | 7.49E-04 | 1 | -0.64 | 1.55E-02 | 1 |
| hsa-miR-30a-3p | 1.59 | 1.12E-07 | 1 | 0.23 | 6.25E-01 | 0 | -1.36 | 3.12E-03 | 1 |
| hsa-miR-30a-5p | 0.93 | 2.08E-05 | 1 | 0.70 | 2.74E-02 | 1 | -0.23 | 3.26E-01 | 0 |
| hsa-miR-30b | 1.11 | 4.45E-06 | 1 | 1.48 | 3.32E-05 | 1 | 0.37 | 4.73E-02 | 0 |
| hsa-miR-30c | 1.15 | 3.24E-06 | 1 | 0.47 | 1.32E-01 | 0 | -0.68 | 1.33E-02 | 1 |
| hsa-miR-30d | 0.79 | 2.84E-04 | 1 | 0.67 | 6.02E-03 | 1 | -0.12 | 5.33E-01 | 0 |
| hsa-miR-30e-3p | 1.53 | 1.57E-05 | 1 | 0.55 | 1.14E-01 | 0 | -0.98 | 5.37E-03 | 1 |
| hsa-miR-30e-5p | 0.80 | 3.20E-06 | 1 | 1.18 | 2.06E-03 | 1 | 0.38 | 1.14E-01 | 0 |
| hsa-miR-320 | -0.30 | 1.13E-01 | 0 | -0.79 | 4.19E-03 | 1 | -0.49 | 3.52E-02 | 0 |
| hsa-miR-324-3p | -1.14 | 1.09E-04 | 1 | -1.60 | 3.30E-04 | 1 | -0.46 | 4.05E-02 | 0 |
| hsa-miR-331 | -1.53 | 2.41 E-04 | 1 | -1.96 | 8.19E-05 | 1 | -0.43 | 1.09E-01 | 0 |
| hsa-miR-335 | 1.23 | 9.74E-04 | 1 | 2.82 | 3.36E-03 | 1 | 1.59 | 1.91E-02 | 1 |
| hsa-miR-338 | 0.85 | 5.46E-02 | 0 | 3.86 | 1.78E-04 | 1 | 3.01 | 3.14E-04 | 1 |
| hsa-miR-339 | 0.10 | 4.92E-01 | 0 | -1.04 | 2.63E-03 | 1 | -1.14 | 9.66E-05 | 1 |
| hsa-miR-342 | -0.08 | 4.44E-01 | 0 | 0.84 | 1.44E-02 | 1 | 0.92 | 1.16E-03 | 1 |
| hsa-miR-34a | -0.58 | 3.89E-02 | 0 | 1.98 | 3.82E-03 | 1 | 2.56 | 3.43E-05 | 1 |
| hsa-miR-34b | -0.21 | 4.92E-01 | 0 | 1.53 | 2.12E-03 | 1 | 1.74 | 5.33E-07 | 1 |
| hsa-miR-361 | -0.31 | 1.15E-01 | 0 | -0.77 | 5.53E-04 | 1 | -0.46 | 1.70E-02 | 1 |
| hsa-miR-365 | 1.44 | 1.53E-04 | 1 | 0.63 | 2.26E-02 | 1 | -0.81 | 7.95E-04 | 1 |
| hsa-miR-368 | 1.15 | 2.52E-05 | 1 | 4.85 | 3.48E-10 | 1 | 3.70 | z-11 | 1 |
| hsa-miR-374 | 0.75 | 4.31E-02 | 0 | 2.23 | 7.29E-05 | 1 | 1.48 | 2.34E-04 | 1 |
| hsa-miR-375 | 2.51 | 3.16E-04 | 1 | 6.36 | 5.62E-09 | 1 | 3.85 | 3.08E-06 | 1 |
| hsa-miR-376a | 0.93 | 9.59E-04 | 1 | 3.81 | 2.09E-08 | 1 | 2.88 | 1.39E-08 | 1 |
| hsa-miR-377 | 0.72 | 1.38E-02 | 1 | 2.45 | 2.09E-04 | 1 | 1.73 | 3.69E-04 | 1 |
| hsa-miR-379 | 0.44 | 4.32E-02 | 0 | 1.91 | 2.83E-05 | 1 | 1.47 | 3.26E-05 | 1 |
| hsa-miR-381 | 0.69 | 1.72E-03 | 1 | 1.12 | 2.40E-04 | 1 | 0.43 | 1.25E-03 | 1 |
| hsa-miR-3 82 | 0.03 | 8.80E-01 | 0 | 2.07 | 4.91E-05 | 1 | 2.03 | 2.68E-07 | 1 |
| hsa-miR-422a | -0.17 | 5.78E-01 | 0 | -0.94 | 2.23E-02 | 1 | -0.77 | 5.96E-02 | 0 |
| hsa-miR-422b | -0.71 | 2.49E-02 | 0 | -0.83 | 6.51E-02 | 0 | -0.13 | 6.95E-01 | 0 |
| hsa-miR-423 | -0.75 | 1.56E-02 | 1 | -2.02 | 3.00E-06 | 1 | -1.27 | 3.02E-05 | 1 |
| hsa-miR-424 | -0.26 | 4.13E-01 | 0 | 2.10 | 6.06E-03 | 1 | 2.35 | 8.92E-05 | 1 |
| hsa-miR-429 | 0.42 | 1.14E-01 | 0 | 0.71 | 1.00E-03 | 1 | 0.29 | 2.78E-01 | 0 |
| hsa-miR-92 | -0.10 | 4.21E-01 | 0 | -1.53 | 1.21E-04 | 1 | -1.42 | 8.10E-06 | 1 |
| hsa-miR-93 | -1.11 | 4.56E-05 | 1 | -1.96 | 2.80E-06 | 1 | -0.84 | 4.32E-04 | 1 |
| hsa-miR-95 | 0.71 | 7.99E-02 | 0 | 2.48 | 1.94E-06 | 1 | 1.77 | 2.73E-04 | 1 |
| hsa-miR-96 | 1.64 | 6.25E-06 | 1 | 1.59 | 4.07E-04 | 1 | -0.06 | 8.20E-01 | 0 |
| hsa-miR-98 | 0.47 | 1.51E-01 | 0 | 0.84 | 3.07E-02 | 1 | 0.37 | 2.44E-01 | 0 |
| hsa-miR-99a | -0.90 | 1.70E-02 | 1 | -0.60 | 1.58E-01 | 0 | 0.30 | 4.49E-01 | 0 |
| hsa-miR-99b | -0.83 | 4.04E-03 | 1 | -1.10 | 1.40E-02 | 1 | -0.27 | 3.90E-01 | 0 |
| ambi-miR-7029 | 0.27 | 5.70E-01 | 0 | 4.57 | 1.29E-07 | 1 | 4.30 | 1.02E-07 | 1 |
| hsa-miR-193b | -0.11 | 7.67E-01 | 0 | -2.08 | 1.63E-05 | 1 | -1.97 | 1.50E-04 | 1 |
| ambi-miR-7058 | -0.10 | 5.18E-01 | 0 | -0.82 | 7.83E-04 | 1 | -0.72 | 7.04E-04 | 1 |
| hsa-miR-452 | -0.94 | 7.38E-06 | 1 | 0.07 | 8.60E-01 | 0 | 1.02 | 7.99E-03 | 1 |
| hsa-miR-432 | -0.01 | 9.38E-01 | 0 | 0.89 | 2.28E-03 | 1 | 0.90 | 5.65E-04 | 1 |
| hsa-miR-494 | 1.89 | 5.80E-03 | 1 | 2.57 | 6.42E-04 | 1 | 0.68 | 1.60E-01 | 0 |
| hsa-miR-497 | -0.87 | 4.23E-04 | 1 | 2.06 | 2.01E-05 | 1 | 2.93 | 2.13E-08 | 1 |
| ambi-miR-7105 | -0.57 | 9.24E-04 | 1 | -0.36 | 1.68E-01 | 0 | 0.21 | 2.83E-01 | 0 |

| | | | | | | | | | |
|---|---|---|---|---|---|---|---|---|---|
| Significant differential expression is indicated by a Flag = 1 | | | | | | | | | |

### EXAMPLE 9:

### 26 MIRNA BIOMARKERS FOR PDAC AND OTHER PANCREATIC DISEASES

Microarray profiling of pancreatic cell lines and normal and diseased primary tissues allowed us to identify 131 mis-regulated (differentially expressed) miRNAs among the samples (Table 8). The inventors further identified a subset of 94 miRNAs differentially expressed between normal, chronic, and PDAC samples (Table 6), and 84 miRNAs differentially expressed between normal and PDAC samples (Table 6, Flag (N vs Ca)=1). The inventors identified 20 miRNAs with |Δh|>1.6 (5-fold) and p-value<0.0001 between at least 2 out of 3 primary tissue types (FIG. 5), and six miRNAs over-expressed in neoplastic ductal cells both *in vivo* and *ex vivo* (FIG. 7). These 26 miRNAs whose expression is significantly affected in PDAC represent novel biomarkers and therapeutic targets for PDAC and other pancreatic diseases. Their identity, associated array data, and chromosomal location are summarized in Table 9.

**Table 9 Top 26 miRNAs differentially expressed in PDAC**

| **miRNA ID** | **Chr.** | **Mean (N)** | **Mean (Ch)** | **Mean (Ca)** | **Mean (CL)** | **p-value** | **Δh (Ca-N)** | **Δ fold** | **# targets** |
|---|---|---|---|---|---|---|---|---|---|
| miR-205 | 1 q32.2 | 0.9 | 1.44 | 3.12 | 7.35 | 3.47E-06 | 2.22 | 9 | 19 |
| miR-29c | 1 q32.2 | 8.25 | 6.95 | 6.49 | 4.6 | 1.48E-10 | -1.75 | 6 | 45 |
| miR-216 | 2 p16.1 | 7.09 | 5.9 | 1.64 | 1.05 | 4.56E-13 | -5.45 | 233 | 6 |
| miR-217 | 2 p16.1 | 7.86 | 6.7 | 2.19 | 1.27 | 3.81E-13 | -5.68 | 293 | 19 |
| miR-375 | 2 q35 | 7.21 | 6.43 | 4.7 | 0.85 | 1.41E-11 | -2.51 | 12 | 5 |
| miR-143 | 5 q32 | 6.02 | 7.52 | 7.96 | 0.49 | < 1E-11 | 1.94 | 7 | 7 |
| miR-145 | 5 q32 | 6.32 | 7.82 | 8.15 | 0.65 | < 1E-11 | 1.83 | 6 | 15 |
| miR-146a | 5 q33.3 | 3.85 | 4.87 | 5.93 | 3.13 | 1.24E-05 | 2.09 | 8 | 4 |
| miR-148a | 7 p15.2 | 8.57 | 7.14 | 5.27 | 2.55 | 1.91E-10 | -3.3 | 27 | 28 |
| miR-196b | 7 p15.2 | 0.57 | 1.04 | 3.24 | 2.35 | 3.79E-04 | 2.67 | 14 | 7 |
| miR-93 | 7 q22.1 | 4.83 | 4.99 | 5.94 | 6.78 | 1.26E-09 | 1.11 | 3 | 26 |
| miR-96 | 7 q32.2 | 4.69 | 2.79 | 3.04 | 3.1 | 9.15E-06 | -1.64 | 5 | 33 |
| miR-31 | 9 p21.3 | 3.9 | 5.57 | 6.69 | 6.06 | 1.52E-01 | 2.79 | 16 | 10 |
| miR-210 | 11 p15.5 | 3.79 | 4.43 | 6.61 | 4.71 | 2.38E-08 | 2.82 | 17 | 0 |
| miR-148b | 12 q13.13 | 5.11 | 2.97 | 2.82 | 2.88 | 1.82E-06 | -2.29 | 10 | 28 |
| miR-196a | 12 q13.13 | 1.13 | 1.41 | 3.77 | 2.85 | 1.00E-05 | 2.64 | 14 | 6 |
| miR-141 | 12 p13.31 | 7.5 | 6.65 | 6 | 6.34 | 1.06E-05 | -1.5 | 4.5 | 29 |
| miR-18a | 13 q31.3 | 2.58 | 3.17 | 4.26 | 5.14 | 3.68E-07 | 1.68 | 5 | 4 |
| miR-203 | 14 q32.33 | 3.5 | 2.66 | 5.19 | 2.68 | 2.06E-04 | 1.69 | 5 | 14 |
| miR-150 | 19 p13.33 | 1.73 | 3.68 | 4.38 | 1.19 | 1.49E-06 | 2.65 | 14 | 6 |
| miR-155 | 21 q21.3 | 3.25 | 4.22 | 5.59 | 4.97 | 2.75E-03 | 2.34 | 10 | 8 |
| miR-130b | 22 q11.21 | 6.27 | 4.93 | 3.84 | 4.98 | 4.01E-08 | -2.43 | 11 | 41 |
| miR-221 | X p11.3 | 5.02 | 5.41 | 6.5 | 7.9 | 1.55E-11 | 1.48 | 4 | 13 |
| miR-222 | X p11.3 | 3.92 | 4.63 | 5.99 | 7.22 | 3.43E-11 | 2.06 | 8 | 12 |
| miR-223 | X q12 | 4.47 | 5.62 | 6.86 | 1.36 | 1.33E-10 | 2.39 | 11 | 10 |
| miR-224 | X q28 | 2.56 | 2.44 | 3.83 | 4.2 | 1.51E-03 | 1.27 | 3.5 | 7 |

### EXAMPLE 10:

### MICROARRAY DATA VALIDATION BY QRT-PCR

To verify our array data, the inventors performed real-time PCR for 5 miRNAs with very distinct expression patterns within the 3 tissue types (miR-143, - 155, -196a, -217, and -223) and one miRNA with no significant variation (miR-16). qRT-PCR reactions were performed using SuperTaq™ Polymerase (Ambion) and the mirVana™ qRT-PCR miRNA Detection Kit and Primer Sets (Ambion) following the manufacturer's instructions. qRT-PCR were performed with 5 to 50 ng of total RNA input on an ABI7500 thermocycler (Applied Biosystems; Foster City, CA, USA). Data analysis was performed using 7500 Fast System SDS Software.

The inventors analyzed the 19 total RNA samples previously profiled as well as an independent set of tissues consisting of 2 normal pancreas, 2 PDAC, and one chronic pancreatitis samples showing different extent of RNA degradation (N6, N7, Ca9, Ca10, and Ch7; FIG. 1; see Table 2 for pathology report). The relative variations of miRNA expression levels were similar for the normalized array and qRT-PCR data (FIG. 8). Moreover, all of the 24 samples had the expected miRNA expression patterns characteristic of normal, cancer and chronic tissues, thus validating our array data and further illustrating the stability of mature miRNA molecules.

### EXAMPLE 11:

### TISSUE CLASSIFICATION BY QRT-PCR

Analyses of global miRNA expression profiles, differentially expressed miRNAs (FIG. 4B and 6), and the 26 miRNA markers (FIG. 5, 7, and 8) indicated that miRNA expression can distinguish and classify normal and diseased pancreatic tissues. To take this classification one step further, the minimal set of miRNAs that could discriminate between neoplastic and non-neoplastic tissues were identified by applying a quantitative RT-PCR analysis of mature miRNAs. The inventors found that the difference between raw Ct values of two miRNAs (miR-196 and -217) provided a simple index to identify diseased tissues independently of the total RNA sample input (FIG. 9A). The same analysis performed on the 24 independent tissue samples showed a perfect segregation between normal, PDAC, and chronic pancreatitis samples with a p-value of 1.77E-13 (FIG. 9B). This segregation was confirmed using a different real-time PCR assay, the TaqMan® MicroRNA Assay (Applied Biosystems; Foster City, CA, USA). qRT-PCR reactions were performed with 10 ng RNA input using the 7900HT Fast Real-Time PCR System (Applied Biosystems) on a subset of 20 frozen pancreatic tissue samples (6 N, 6 Ch and 8 Ca, Example 1). RT reactions were carried out using random primers, while for PCR reactions gene-specific priming was used. Initial data analysis was done using the 7900HT Sequence Detection System Software v2.3. The inventors observed segregation between normal, cancer, and chronic tissues that was nearly identical to the previously performed assay, with a p-value of 8.18E-10 (FIG. 10).

### EXAMPLE 12

### MRNA EXPRESSION SIGNATURES IN CLASSIFICATION OF DISEASED PANCREATIC TISSUE

Several reports have described the potential diagnostic significance of carcinoembryonic antigen-related cell adhesion molecule 6 (CEACAM6), survivin (BIRC5), mucin 4 (MUC4) and urokinase plasminogen activator receptor (UPAR) in clinical samples obtained from patients with pancreatic disease (Balague *et al.,* 1995; Bhanot *et al.,* 2006; Chen *et al.,* 2007; Duxbury *et al.,* 2004; Hollingsworth *et al.,* 1994; Lopes *et al.,* 2007). All four genes are up-regulated in pancreatic cancer and a majority of pancreatic cancer cell lines, not expressed (MUC4) or elevated (survivin, UPAR, CEACAM) in chronic pancreatitis, and not expressed or barely expressed in normal pancreatic tissue (Jhala *et al.,* 2006; Andrianifahanana *et al.,* 2001; Friess *et al.,* 1997; Shimzu *et al.,* 1990).

The inventors interrogated the expression levels of CEACAM6, BIRC5, MUC4 and UPAR in a subset of frozen tissue samples from Example 1 (6 N, 6 Ch and 8 Ca) using real time RT-PCR. qRT-PCR reactions were carried out using 5 ng total RNA input and the ABI TaqMan® Gene Expression Assay system (Applied Biosystems). RT reactions used random primers, and PCR reactions used gene-specific priming. Initial data analysis was done using the 7900HT Sequence Detection System Software v2.3. The comparison of mean mRNA expression levels for CEACAM6, BIRC5, MUC4 or UPAR between the experimental groups demonstrated a general up-regulation of these genes in PDAC (data not shown). However, an analysis of expression levels in individual samples revealed no clear segregation between normal pancreas, chronic pancreatitis and PDAC sample sets, as reflected by high p-values of 7.64E-03, 1.11E-02 and 2.11E-03, respectively. The expression profiles in chronic pancreatitis samples were either intermediate between the normal and PDAC profiles or closer to normal (FIG 11A).

### EXAMPLE 13

### COMBINATIONS OF MIRNA AND MRNA EXPRESSION SIGNATURES IMPROVE CLASSIFICATION OF DISEASED PANCREATIC TISSUE

The inventors sought to determine if the individual diagnostic performance of CEACAM6, BIRC5, MUC4 and UPAR could be improved by combining them together or with miRNAs. The inventors found that combined expression signatures of two or more mRNA genes did not offer any advantage in segregating between normal pancreas, chronic pancreatitis, and pancreatic cancer samples (p >4.35 x 10⁻⁰⁷, FIG. 11B), when compared with the miRNA index of miR-196a and miR-217 (p =8.18 x 10⁻¹⁰, FIG. 10). However, combinations of miRNA and mRNA expression signatures increased the separation between normal tissue and chronic pancreatitis samples and enabled the differentiation of pancreatic cancer samples from normal, non-malignant tissue samples. The best shown combination, 196a-217+CEACAM, had p-value of 5.24 x10⁻¹⁰.

### EXAMPLE 14

### DETECTION OF MIRNA AND MRNA BIOMARKERS FOR DIAGNOSIS OF PANCREATIC ADENOCARCINOMA IN PANCREATIC FINE NEEDLE ASPIRATES (FNAs)

The inventors interrogated expression signatures of a subset of the top 20 differentially expressed miRNAs (Example 6). Because pancreatic tissues contain high levels of ribonucleases, FNAs were collected within 30 min post surgery in RNARetain™ (tissue collection and storage solution), kept at 4°C for up to two days, and shipped on dry ice. The targets interrogated by qRT-PCR included: miR-130b, - 148a, -155, -196a, -217 and -375, as well as two mRNAs previously described in the literature, CEACAM6 and BIRC5. qRT-PCR reactions were carried out as described in Example 12 using 10 ng (for miRNA quantification) and 5 ng (for mRNA quantification) total RNA input. The miRNA expression patterns in 10 PDAC FNAs (Ca FNA) were consistent with the reference frozen pancreatic ductal adenocarcinoma samples (FIG. 12). The expression levels of CEACAM6 and BIRC5 mRNAs were also consistent with PDAC, although they overlapped with chronic pancreatitis expression levels and normal pancreas specimens, as in the case of BIRC5. In the three non-PDAC FNA samples (FNA; FNA-8-solid circle, FNA-12-solid triangle and FNA-13-star), the expression patterns varied depending on the interrogated marker. Further analysis by combining two or more miRNA and/or mRNA markers, confirmed that the difference between raw Ct values of miR-196 and miR-217 improved segregation between pancreatic cancer FNA samples and normal or chronic pancreatitis specimens (FIG. 13). In addition, these miRNAs alone, or in combination with mRNA markers, enabled classification of FNA-8 (suspect for PDAC, solid circle) as a PDAC specimen. This result demonstrates that miRNAs could aid pathological evaluation of suspicious cases and become a valuable asset in definitive diagnosis of pancreatic adenocarcinoma. Additionally, the combination of miRNA and mRNA expression levels enabled an increased separation between the pancreatic cancer FNAs and frozen normal pancreas as well as chronic pancreatitis specimen.

### EXAMPLE 15:

### TARGET PREDICTION FOR MIRNAS POTENTIALLY INVOLVED IN PANCREATIC CARCINOGENESIS

To gain further insights into the biological pathways potentially regulated by miRNA during pancreatic carcinogenesis, the inventors performed a comprehensive comparison between the published genes known to be deregulated in pancreatic carcinoma and the predicted target genes for the 26 miRNA biomarkers described above. A representative number of published data sets reporting differentially expressed genes in PDAC was used to build a PDAC candidate gene expression database. In parallel, a search was performed on the predicted targets for the 26 miRNA biomarkers identified in our study using the publicly available PicTar web interface (http://pictar.bio.nyu.edu). Following comparison of both datasets, a subset of genes which were found in both datasets was generated. Lastly, miRNAs up-regulated in PDAC were linked to the common set of predicted target genes which were known to be down-regulated in PDAC. The opposite selection criteria was applied for the miRNAs down-regulated in PDAC.

Relying on the current view that miRNAs are negative regulators of gene expression, and therefore, anticipating an inverse correlation between miRNA and predicted target gene expression, the inventors identified 246 unique genes deregulated in PDAC (Table 10). Importantly, about 37% of these genes were predicted to be targeted by multiple miRNAs. 47 genes are predicted to be targeted by 2 miRNAs, 21 genes by 3 miRNAs, 13 genes by 4 miRNAs, and 7 genes by up to 5 different miRNAs. These data indicate that miRNA-driven pathophysiological mechanisms may be directly involved in pancreatic diseases development and as importantly, offer new targets for diagnostic uses and therapeutic interventions.

**Table 10 Twenty-six miRNA biomarkers and their predicted target genes differentially expressed in PDAC**

| | **miRNA in** | | | | | |
|---|---|---|---|---|---|---|
| **miRNA** | **PDAC** | **Target Gene** | **Description of Predicted Target Gene** | **Ensembl Gene** | **UniGene** | **Literature Source** |
| hsa-miR-145 hsa-miR-18a hsa-miR-205 | up | ACTB | Actin, beta | ENSG00000075624 | Hs.520640 | Crnogorac-Jurcevic et al., 2001 |
| hsa-miR-141 | down | AK3 | Adenylate kinase 3-like 1 | ENSG00000162433 | Hs.10862 | Nakamura et al., 2004, Logsdon et al., 2003 |
| hsa-miR-203 hsa-miR-205 | up | AMOT | Angiomotin | ENSG00000126016 | Hs.528051 | Nakamura et al., 2004 |
| hsa-miR-145 | up | ANGPT2 | Angiopoietin 2 | ENSG00000091879 | Hs.553484 | Sato et al., 2003 |
| hsa-miR-217 | down | ANLN | Anillin, actin binding protein (scraps homolog, Drosophila) | ENSG00000011426 | Hs.62180 | lacobuzio-Donahue et al., 2003; Nakamura et al., 2004 |
| hsa-miR-29c | down | RND3 | Rho family GTPase 3 | ENSG00000115963 | Hs.6838 | Gress et al., 1996 |
| hsa-miR-203 hsa-miR-93 | up | ARHGAP12 | Rho GTPase activating protein 12 | ENSG00000165322 | Hs.499264 | Buchholz et al., 2005 |
| hsa-miR-93 | up | ARHGEF11 | Rho guanine nucleotide exchange factor (GEF) 11 | ENSG00000132694 | Hs.516954 | Han et al., 2002 |
| hsa-miR-130b hsa-miR-148a hsa-miR-148b hsa-miR-375 hsa-miR-96 | down | ARHGEF12 | Rho guanine nucleotide exchange factor (GEF) 12 | ENSG00000196914 | Hs.24598 | Gress et al., 1996 |
| hsa-miR-141 | down | ARPC5 | Actin related protein 2/3 complex, subunit 5, 16kDa | ENSG00000162704 | Hs.518609 | Iacobuzio-Donahue et al., 2003; Grützmann et al., 2003 |
| hsa-miR-29c | down | ASPH | Aspartate beta-hydroxylase | ENSG00000198363 | Hs.332422 | lacobuzio-Donahue et al., 2003, Gress et al., 1996 |
| hsa-miR-155 | up | ASTN2 | Astrotactin 2 | ENSG00000148219 | Hs.209217 | Buchholz et al., 2005 |
| hsa-miR-145 | up | ATXN2 | Ataxin 2 | ENSG00000204842 | Hs.76253 | Buchholz et al., 2005 |
| hsa-miR-205 | up | AXIN2 | Axin 2 (conductin, axil) | ENSG00000168646 | Hs.156527 | Buchholz et al., 2005 |
| hsa-miR-145 | up | BAZ2A | Bromodomain adjacent to zinc finger domain, 2A | ENSG00000076108 | Hs.314263 | Nakamura et al., 2004 |
| hsa-miR-217 | down | BCL2 | B-cell CLL/lymphoma 2 | ENSG00000171791 | Hs.150749 | Friess et al., 1998 |
| hsa-miR-96 | | | | | | |
| hsa-miR-150 | up | BET1 | BET1 homolog (S. cerevisiae) | ENSG00000105829 | Hs.489132 | Buchholz et al., 2005 |
| hsa-miR-148b | down | BLCAP | Bladder cancer associated protein | ENSG00000166619 | Hs.472651 | Gress et al., 1996 |
| hsa-miR-145 | up | C14orfl40 | Chromosome 14 open reading frame 140 | ENSG00000119703 | Hs.48642 | Nakamura et al., 2004 |
| hsa-miR-150 hsa-miR-205 | up | C1orf16 | Chromosome 1 open reading frame 16 | ENSG00000116698 | Hs.270775 | Crnogorac-Jurcevic et al., 2003 |
| hsa-miR-130b | down | C1QDC1 | C1q domain containing 1 | ENSG00000110888 | Hs.234355 | Iacobuzio-Donahue et al., 2002 |
| hsa-miR-150 | up | C20orf102 | Chromosome 20 open reading frame 102 | ENSG00000132821 | Hs.517029 | Nakamura et al., 2004 |
| hsa-miR-93 | up | C20orf161 | Chromosome 20 open reading frame 161 | ENSG00000124104 | Hs.472854 | Buchholz et al., 2005 |
| hsa-miR-205 | up | C21orf63 | Chromosome 21 open reading frame 63 | ENSG00000166979 | Hs.208358 | Nakamura et al., 2004 |
| hsa-miR-93 | up | C2orf17 | Chromosome 2 open reading frame 17 | ENSG00000144567 | Hs.516707 | Buchholz et al., 2005 |
| hsa-miR-130b hsa-miR-148a hsa-miR-148b | down | C6orf62 | Chromosome 6 open reading frame 62 | ENSG00000112308 | Hs.519930 | Gress et al., 1996 |
| hsa-miR-31 | up | C7orf23 | Chromosome 7 open reading frame 23 | ENSG00000135185 | Hs.196129 | Buchholz et al., 2005 |
| hsa-miR-155 | up | C8orf4 | Chromosome 8 open reading frame 4 | ENSG00000176907 | Hs.283683 | Nakamura et al., 2004 |
| hsa-miR-203 | up | C9orf58 | Chromosome 9 open reading frame 58 | ENSG00000126878 | Hs.4944 | Buchholz et al., 2005 |
| hsa-miR-150 | up | CACNA1G | Calcium channel, voltage-dependent, alpha 1G subunit | ENSG00000006283 | Hs.194746 | Sato et al., 2003 |
| hsa-miR-31 | up | CADPS | Ca2+-dependent secretion activator | ENSG00000163618 | Hs.127013 | Nakamura et al., 2004 |
| hsa-miR-205 | up | CANX | Calnexin | ENSG00000127022 | Hs.529890 | Tan et al., 2003 |
| hsa-miR-96 | down | CAV1 | Caveolin 1, caveolae protein, 22kDa | ENSG00000105974 | Hs.74034 | Iacobuzio-Donahue et al., 2003; Crnogorac-Jurcevic et al., 2001 |
| hsa-miR-29c | down | CAV2 | Caveolin 2 | ENSG00000105971 | Hs.212332 | Iacobuzio-Donahue et al., 2003; lacobuzio-Donahue et al., 2003; Iacobuzio-Donahue et al., 2002; Sato et al., 2003 |
| hsa-miR-130b | down | CBFB | Core-binding factor, beta subunit | ENSG00000067955 | Hs.460988 | Buchholz et al., 2005 |
| hsa-miR-203 | up | CCNC | Cyclin C | ENSG00000112237 | Hs.430646 | Crnogorac-Jurcevic et al., 2002 |
| hsa-miR-224 | up | CD28 | CD28 antigen (Tp44) | ENSG00000178562 | Hs.1987 | Nakamura et al., 2004 |
| hsa-miR-31 | up | CD28 | CD28 antigen (Tp44) | ENSG00000178562 | Hs.1987 | Nakamura et al., 2004 |
| hsa-miR-148a hsa-miR-148b | down | CDC25B | Cell division cycle 25B | ENSG00000101224 | Hs.153752 | Grützmann et al., 2003 |
| hsa-miR-130b hsa-miR-148a hsa-miR-148b | down | CDC2L6 | Cell division cycle 2-like 6 (CDK8-like) | ENSG00000155111 | Hs.193251 | Gress et al, 1996 |
| hsa-miR-96 | down | CDC37 | CDC37 cell division cycle 37 homolog (S. cerevisiae) | ENSG00000105401 | Hs.160958 | Buchholz et al., 2005 |
| hsa-miR-93 hsa-miR-196a hsa-miR-196b hsa-miR-221 hsa-miR-222 | up | CDKN1A | Cyclin-dependent kinase inhibitor 1A (p21, Cip1) | ENSG00000124762 | Hs.370771 | Sato et al., 2003 |
| hsa-miR-221 hsa-miR-222 | up | CDKN1C | Cyclin-dependent kinase inhibitor 1C (p57, Kip2) | ENSG00000129757 | Hs.106070 | Sato et al., 2003 |
| hsa-miR-216 | down | CEBPG | CCAAT/enhancer binding protein (C/EBP), gamma | ENSG00000153879 | Hs.429666 | Gress et al., 1996 |
| hsa-miR-96 | down | CELSR1 | Cadherin, EGF LAG seven-pass G-type receptor | ENSG00000075275 | Hs.252387 | lacobuzio-Donahue et al., 2003 |
| hsa-miR-96 | down | CFL1 | Cofilin 1 (non-muscle) | ENSG00000172757 | Hs.170622 | Nakamura et al., 2004 |
| hsa-miR-130b hsa-miR-141 hsa-miR-148a hsa-miR-148b hsa-miR-217 | down | CHD9 | Chromodomain helicase DNA binding protein 9 | ENSG00000177200 | Hs.59159 | Buchholz et al., 2005 |
| hsa-miR-224 | up | CHGB | Chromogranin B (secretogranin 1) | ENSG00000089199 | Hs.516874 | Crnogorac-Jurcevic et al., 2003 |
| hsa-miR-145 | up | CHKB | Choline kinase beta | ENSG00000100288 | Hs.439777 | Buchholz et al., 2005 |
| hsa-miR-29c | down | CLDN1 | Claudin 1 | ENSG00000163347 | Hs.439060 | Iacobuzio-Donahue et al., 2002 |
| hsa-miR-375 | down | CLECSF2 | C-type lectin domain family 2, | ENSG00000110852 | Hs.85201 | Friess et al., 2003 |
| | | | member B | | | |
| hsa-miR-143 | up | CNNM3 | Cyclin M3 | ENSG00000168763 | Hs.150895 | Nakamura et al., 2004 |
| hsa-miR-29c | down | COL11A1 | Collagen, type XI, alpha 1 | ENSG00000060718 | Hs.523446 | Iacobuzio-Donahue et al., 2002 |
| hsa-miR-29c | down | COL1A1 | Collagen, type I, alpha 1 | ENSG00000108821 | Hs.172928 | Iacobuzio-Donahue et al., 2002; Nakamura et al., 2004; Gress et al., 1997; Crnogorac-Jurcevic et al., 2003; Yoshida et al., 2003 |
| hsa-miR-29c | down | COL1A2 | Collagen, type I, alpha 2 | ENSG00000164692 | Hs.489142 | Friess et al., 2003; Crnogorac-Jurcevic et al., 2002; Tan et al., 2003; Iacobuzio-Donahue et al., 2002; Nakamura et al., 2004 |
| hsa-miR-130b hsa-miR-148a hsa-miR-148b hsa-miR-29c | down | COL2A1 | Collagen, type II, alpha 1 | ENSG00000139219 | Hs.408182 | Friess et al., 2003 |
| hsa-miR-29c | down | COL3A1 | Collagen, type III, alpha 1 | ENSG00000168542 | Hs.443625 | Buchholz et al., 2005; Gress et al., 1997; Nakamura et al., 2004; Crnogorac-Jurcevic et al., 2001; Friess et al., 2003 |
| hsa-miR-29c | down | COL4A2 | Collagen, type IV, alpha 2 | ENSG00000134871 | Hs.508716 | Friess et al., 2003 |
| hsa-miR-29c | down | COL5A2 | Collagen, type V, alpha 2 | ENSG00000204262 | Hs.445827 | Gress et al., 1997 |
| hsa-miR-29c | down | COL6A3 | Collagen, type VI, alpha 3 | ENSG00000163359 | Hs.233240 | Gress et al., 1997 |
| hsa-miR-205 | up | CROP | Cisplatin resistance-associated overexpressed protein | ENSG00000108848 | Hs.130293 | Grützmann et al., 2003 |
| hsa-miR-96 | down | CRR9 | Cisplatin resistance related protein CRR9p | ENSG00000049656 | Hs.444673 | Gress et al., 1996 |
| hsa-miR-141 | down | CSNK1A1 | Casein kinase 1, alpha 1 | ENSG00000113712 | Hs.529862 | Nakamura et al., 2004 |
| hsa-miR-375 | down | CTGF | Connective tissue growth factor | ENSG00000118523 | Hs.410037 | Iacobuzio-Donahue et al., 2002 |
| hsa-miR-141 | down | CTNNA1 | Catenin (cadherin-associated protein), alpha 1, 102kDa | ENSG00000044115 | Hs.445981 | Li, 2003 |
| hsa-miR-141 hsa-miR-217 | down | CTNNB1 | Catenin (cadherin-associated protein), beta 1, 88kDa | ENSG00000168036 | Hs.476018 | Iacobuzio-Donahue et al., 2002; Gress et al., 1996; Li, 2003 |
| hsa-miR-29c hsa-miR-96 | down | CTNND1 | Catenin (cadherin-associated protein), delta 1 | ENSG00000198561 | Hs.166011 | Gress et al., 1996 |
| hsa-miR-31 | up | CXCL12 | Chemokine (C-X-C motif) ligand 12 (stromal cell-derived factor 1) | ENSG00000107562 | Hs.522891 | Grützmann et al., 2003 |
| hsa-miR-146a | up | CXYorf2 | Chromosome X and Y open reading frame 2 | ENSG00000169098 | Hs.521856 | Buchholz et al., 2005 |
| hsa-miR-223 | up | CYB5 | Cytochrome b-5 | ENSG00000166347 | Hs.465413 | Nakamura et al., 2004 |
| hsa-miR-145 | up | DAG1 | Dystroglycan 1 (dystrophin-associated glycoprotein 1) | ENSG00000173402 | Hs.76111 | Han et al., 2002 |
| hsa-miR-223 | up | DAG1 | Dystroglycan 1 (dystrophin-associated glycoprotein 1) | ENSG00000173402 | Hs.76111 | Han et al., 2002 |
| hsa-miR-141 | down | DEK | DEK oncogene (DNA binding) | ENSG00000124795 | Hs.484813 | Gress et al., 1996 |
| hsa-miR-29c | down | DGKD | Diacylglycerol kinase, delta 130kDa | ENSG00000077044 | Hs.471675 | Grützmann et al., 2003 |
| hsa-miR-224 | up | DKFZp434K2435 | Hypothetical protein DKFZp434K2435 | ENSG00000139173 | Hs.444668 | Nakamura et al., 2004 |
| hsa-miR-150 | up | DKFZP586A0522 | DKFZP586A0522 protein | ENSG00000185432 | Hs.288771 | Nakamura et al., 2004 |
| hsa-miR-217 | down | DKK1 | Dickkopfhomolog 1 (Xenopus laevis) | ENSG00000107984 | Hs.40499 | lacobuzio-Donahue et al., 2003 |
| hsa-miR-205 | up | DLG2 | Discs, large homolog 2, chapsyn-110 (Drosophila) | ENSG00000150672 | Hs.503453 | Friess et al., 2003 |
| hsa-miR-217 | down | DNAJA1 | DnaJ (Hsp40) homolog, subfamily A, member 1 | ENSG00000086061 | Hs.445203 | Yoshida et al., 2003 |
| hsa-miR-29c | down | DNM3 | Dynamin 3 | ENSG00000197959 | Hs.567444 | Nakamura et al., 2004 |
| hsa-miR-130b | down | DPYSL2 | Dihydropyrimidinase-like 2 | ENSG00000092964 | Hs.173381 | Gress et al., 1996 |
| hsa-miR-148a hsa-miR-148b | down | DUST1 | Dual specificity phosphatase 1 | ENSG00000120129 | Hs.171695 | Yoshida et al., 2003 |
| hsa-miR-223 | up | EFNA1 | Ephrin-A1 | ENSG00000169242 | Hs.516664 | Crnogorac-Jurcevic et al., 2001 |
| hsa-miR-203 | up | EGR1 | Early growth response 1 | ENSG00000120738 | Hs.326035 | Nakamura et al., 2004 |
| hsa-miR-130b hsa-miR-148a hsa-miR-148b | down | EIF2C4 | Eukaryotic translation initiation factor 2C, 4 | ENSG00000134698 | Hs.471492 | Nakamura et al., 2004 |
| hsa-miR-217 | down | EIF4A2 | Eukaryotic translation initiation | ENSG00000156976 | Hs.478553 | Gress et al., 1996 |
| | | | factor 4A, isoform 2 | | | |
| hsa-miR-141 | down | EIF4E | Eukaryotic translation initiation factor 4E | ENSG00000151247 | Hs.249718 | Gress et al., 1996 |
| hsa-miR-141 hsa-miR-29c | down | ELF2 | E74-like factor 2 (ets domain transcription factor) | ENSG00000109381 | Hs.480763 | Gress et al., 1996 |
| hsa-miR-93 | up | EPAS1 | Endothelial PAS domain protein 1 | ENSG00000116016 | Hs.468410 | Friess et al., 2003 |
| hsa-miR-375 | down | EPB41L2 | Erythrocyte membrane protein band 4.1-like 2 | ENSG00000079819 | Hs.486470 | Nakamura et al., 2004 |
| hsa-miR-141 | down | EPHA2 | EPH receptor A2 | ENSG00000142627 | Hs.171596 | Iacobuzio-Donahue et al., 2002 |
| hsa-miR-130b | down | EREG | Epiregulin | ENSG00000124882 | Hs.115263 | lacobuzio-Donahue et al., 2003 |
| hsa-miR-145 | up | EYA3 | Eyes absent homolog 3 (Drosophila) | ENSG00000158161 | Hs.185774 | Buchholz et al., 2005 |
| hsa-miR-223 | up | FGFR2 | Fibroblast growth factor receptor 2 | ENSG00000066468 | Hs.533683 | Nakamura et al., 2004 |
| hsa-miR-93 | up | FLT1 | Fms-related tyrosine kinase 1 | ENSG00000102755 | Hs.507621 | Crnogorac-Jurcevic et al., 2001 |
| hsa-miR-217 | down | FN1 | Fibronectin 1 | ENSG00000115414 | Hs.203717 | Buchholz et al., 2005, Tan et al., 2003; Friess et al., 2003; Nakamura et al., 2004; Gress et al., 1997; Crnogorac-Jurcevic et al., 2003 |
| hsa-miR-221 hsa-miR-222 | up | FOS | V-fos FBJ murine osteosarcoma viral oncogene homolog | ENSG00000170345 | Hs.25647 | Grützmann et al., 2003; Han et al., 2002 |
| hsa-miR-29c | down | FSTL1 | Follistatin-like 1 | ENSG00000163430 | Hs.269512 | Tan et al., 2003 |
| hsa-miR-205 | up | FXYD2 | FXYD domain containing ion transport regulator 2 | ENSG00000137731 | Hs.413137 | Buchholz et al., 2005, Nakamura et al., 2004, Crnogorac-Jurcevic et al., 2003 |
| hsa-miR-96 | down | FYN | FYN oncogene related to SRC, FGR, YES | ENSG00000010810 | Hs.390567 | Yoshida et al., 2003; Nakamura et al., 2004 |
| hsa-miR-141 hsa-miR-96 | down | GALNT2 | UDP-N-acetyl-alpha-D-galactosamine:polypeptide N-acetylgalactosaminyltransferase 2 | ENSG00000143641 | Hs.567272 | Nakamura et al., 2004 |
| hsa-miR-155 | up | GNAS | GNAS complex locus | ENSG00000087460 | Hs.125898 | Buchholz et al., 2005 |
| hsa-miR-143 | up | HABP2 | Hyaluronan binding protein 2 | ENSG00000148702 | Hs.422542 | Nakamura et al., 2004; |
| hsa-miR-224 | | | | | | Grützmann et al., 2003; Friess et al., 2003 |
| hsa-miR-93 | up | HABP4 | Hyaluronan binding protein 4 | ENSG00000130956 | Hs.494567 | Buchholz et al., 2005 |
| hsa-miR-141 | down | HAPIP | Kalirin, RhoGEF kinase | ENSG00000160145 | Hs.8004 | Iacobuzio-Donahue et al., 2003 |
| hsa-miR-130b hsa-miR-148a hsa-miR-148b hsa-miR-29c hsa-miR-96 | down | HAS3 | Hyaluronan synthase 3 | ENSG00000103044 | Hs.85962 | Iacobuzio-Donahue et al., 2003 |
| hsa-miR-130b | down | HECA | Headcase homolog (Drosophila) | ENSG00000112406 | Hs. 197644 | Nakamura et al., 2004 |
| hsa-miR-205 | up | HMGB1 | High-mobility group box 1 | ENSG00000189403 | Hs.434102 | Buchholz et al., 2005 |
| hsa-miR-196a hsa-miR-196b hsa-miR-203 | up | HOXA1 | Homeo box A1 | ENSG00000105991 | Hs.67397 | Sato et al., 2003 |
| hsa-miR-29c | down | HOXA10 | Homeo box A10 | ENSG00000153807 | Hs.110637 | lacobuzio-Donahue et al., 2003 |
| hsa-miR-141 | down | HOXA11 | Homeo box A11 | ENSG00000005073 | Hs.249171 | Buchholz et al., 2005 |
| hsa-miR-141 | down | HOXB5 | Homeo box B5 | ENSG00000120075 | Hs.149548 | Iacobuzio-Donahue et al., 2003 |
| hsa-miR-205 | up | HS3ST1 | Heparan sulfate (glucosamine) 3-O-sulfotransferase 1 | ENSG00000002587 | Hs.507348 | Nakamura et al., 2004 |
| hsa-miR-130b hsa-miR-148a hsa-miR-148b hsa-miR-217 hsa-miR-29c | down | HSHIN1 | OTU domain containing 4 | ENSG00000164164 | Hs.270851 | Gress et al., 1996 |
| hsa-miR-223 | up | CDH12 | Cadherin 12, type 2 (N-cadherin 2) | ENSG00000154162 | Hs.113684 | Crnogorac-Jurcevic et al., 2003 |
| hsa-miR-93 | up | IL17E | Interleukin 17E | ENSG00000166090 | Hs.302036 | Buchholz et al.,2005 |
| hsa-miR-224 | up | ILF3 | Interleukin enhancer binding factor 3, 90kDa | ENSG00000129351 | Hs.465885 | Nakamura et al., 2004 |
| hsa-miR-29c | down | IMPDH1 | IMP (inosine monophosphate) dehydirogenase 1 | ENSG00000156802 | Hs.558347 | Nakamura et al., 2004 |
| hsa-miR-130b hsa-miR-148a hsa-miR-148b | down | INHBB | Inhibin, beta B (activin AB beta polypeptide) | ENSG00000163083 | Hs.1735 | Sato et al., 2003; Iacobuzio-Donahue et al., 2003 |
| hsa-miR-155 | up | INPP5D | Inositol polyphosphate-5-phosphatase, 145kDa | ENSG00000168918 | Hs.262886 | Nakamura et al., 2004 |
| hsa-miR-130b hsa-miR-148a hsa-miR-148b hsa-miR-29c | down | ITGA11 | Integrin, alpha 11 | ENSG00000137809 | Hs.436416 | Gress et al., 1997 |
| hsa-miR-217 | down | ITM1 | Integral membrane protein 1 | ENSG00000134910 | Hs.504237 | Nakamura et al., 2004 |
| hsa-miR-130b hsa-miR-96 | down | KAB | KARP-1-binding protein | ENSG00000143702 | Hs.533635 | Iacobuzio-Donahue et al., 2002 |
| hsa-miR-143 hsa-miR-203 hsa-miR-93 | up | KIAA0063 | Josephin domain containing 1 | ENSG00000100221 | Hs.3094 | Nakamura et al., 2004 |
| hsa-miR-155 | up | KIAA0276 | DCN1, defective in cullin neddylation 1, domain containing 4 (S. cerevisiae) | ENSG00000109184 | Hs.221407 | Buchholz et al.,2005 |
| hsa-miR-216 hsa-miR-29c | down | KIAA1199 | KIAA1199 | ENSG00000103888 | Hs.459088 | lacobuzio-Donahue et al., 2003 |
| hsa-miR-146a | up | KLF7 | Kruppel-like factor 7 (ubiquitous) | ENSG00000118263 | Hs.471221 | Nakamura et al., 2004 |
| hsa-miR-93 | up | KLF9 | Kruppel-like factor 9 | ENSG00000119138 | Hs.150557 | Nakamura et al., 2004 |
| hsa-miR-29c | down | LAST1 | LIM and SH3 protein 1 | ENSG00000002834 | Hs.334851 | Gress et al., 1996 |
| hsa-miR-130b hsa-miR-148a hsa-miR-148b | down | LBR | Lamin B receptor | ENSG00000143815 | Hs.435166 | Gress et al., 1996 |
| hsa-miR-96 | down | LCP1 | Lymphocyte cytosolic protein 1 (L-plastin) | ENSG00000136167 | Hs.381099 | Nakamura et al., 2004 |
| hsa-miR-217 | down | LGALS3 | Lectin, galactoside-binding, soluble, (galectin 3) | ENSG00000131981 | Hs.531081 | Iacobuzio-Donahue et al., 2003 |
| hsa-miR-130b hsa-miR-148a hsa-miR-148b | down | MAF1 | MAF1 homolog (S. cerevisiae) | ENSG00000179632 | Hs.19673 | Gress et al., 1996 |
| hsa-miR-217 | down | MAPK1 | Mitogen-activated protein kinase 1 | ENSG00000100030 | Hs.431850 | Gress et al., 1996 |
| hsa-miR-203 hsa-miR-205 hsa-miR-93 | up | MAPK9 | Mitogen-activated protein kinase 9 | ENSG00000050748 | Hs.484371 | Crnogorac-Jurcevic et al., 2001 |
| hsa-miR-29c | down | MARK3 | MAP/microtubule affinity-regulating kinase 3 | ENSG00000075413 | Hs.35828 | Friess et al., 2003 |
| hsa-miR-93 | up | MASTL | Microtubule associated | ENSG00000120539 | Hs.276905 | Buchholz et al.,2005 |
| | | | serine/threonine kinase-like | | | |
| hsa-miR-93 | up | MCL1 | Myeloid cell leukemia sequence 1 (BCL2-related) | ENSG00000143384 | Hs.532826 | Gratzmann et al., 2003 |
| hsa-miR-203 hsa-miR-223 hsa-miR-93 | up | MEF2C | MADS box transcription enhancer factor 2, polypeptide C (myocyte enhancer factor 2C) | ENSG00000081189 | Hs.444409 | Nakamura et al., 2004 |
| hsa-miR-18a | up | MEF2D | MADS box transcription enhancer factor 2, polypeptide D (myocyte enhancer factor 2D) | ENSG00000116604 | Hs.314327 | Nakamura et al., 2004 |
| hsa-miR-196b | up | MEIS2 | Meis 1, myeloid ecotropic viral integration site 1 homolog 2 (mouse) | ENSG00000134138 | Hs.510989 | Nakamura et al., 2004 |
| hsa-miR-29c | down | MFAP2 | Microfibrillar-associated protein 2 | ENSG00000117122 | Hs.389137 | lacobuzio-Donahue et al., 2003 |
| hsa-miR-196a hsa-miR-196b | up | MGC61598 | Similar to ankyrin-repeat protein Nrarp | ENSG00000198435 | Hs.535075 | Nakamura et al., 2004 |
| hsa-miR-141 | down | MMP11 | Matrix metallopeptidase 11 (stromelysin 3) | ENSG00000099953 | Hs.143751 | Nakamura et al., 2004; lacobuzio-Donahue et al., 2003; Bramhall et al., 1997; Crnogorac-Jurcevic et al., 2003 |
| hsa-miR-29c | down | MMP2 | Matrix metallopeptidase 2 (gelatinase A, 72kDa gelatinase, 72kDa type IV collagenase) | ENSG00000087245 | Hs.513617 | Bramhall et al., 1997; Ellenrieder et al, 2000; Gress et al., 1997 |
| hsa-miR-143 hsa-miR-145 | up | MSI2 | Musashi homolog 2 (Drosophila) | ENSG00000153944 | Hs.134470 | Nakamura et al., 2004 |
| hsa-miR-29c | down | MYBL2 | V-myb myeloblastosis viral oncogene homolog (avian)-like 2 | ENSG00000101057 | Hs.179718 | Nakamura et al., 2004 |
| hsa-miR-130b | down | N4BP1 | Nedd4 binding protein 1 | ENSG00000102921 | Hs.511839 | Gress et al., 1997 |
| hsa-miR-96 | down | N4BP1 | Nedd4 binding protein 1 | ENSG00000102921 | Hs.511839 | Gress et al., 1997 |
| hsa-miR-29c | down | NAV2 | Neuron navigator 2 | ENSG00000166833 | Hs.502116 | Nakamura et al., 2004 |
| hsa-miR-29c | down | NCOA3 | Nuclear receptor coactivator 3 | ENSG00000124151 | Hs.382168 | Nakamura et al., 2004 |
| hsa-miR-145 hsa-miR-93 | up | NEDD4L | Neural precursor cell expressed, developmentally down-regulated 4-like | ENSG00000049759 | Hs.185677 | Nakamura et al., 2004 |
| hsa-miR-223 hsa-miR-93 | up | NFIB | Nuclear factor I/B | ENSG00000147862 | Hs.370359 | Nakamura et al., 2004 |
| hsa-miR-130b hsa-miR-148a hsa-miR-148b hsa-miR-217 | down | NHS | Nance-Horan syndrome (congenital cataracts and dental anomalies) | ENSG00000188158 | Hs.201623 | Nakamura et al., 2004 |
| hsa-miR-205 | up | NPR2 | Natriuretic peptide receptor B/guanylate cyclase B (atrionatriuretic peptide receptor B) | ENSG00000159899 | Hs.78518 | Nakamura et al., 2004 |
| hsa-miR-150 hsa-miR-155 | up | NR2F2 | Nuclear receptor subfamily 2, group F, member 2 | ENSG00000185551 | Hs.347991 | Nakamura et al., 2004 |
| hsa-miR-130b hsa-miR-141 | down | NR3C2 | Nuclear receptor subfamily 3, group C, member 2 | ENSG00000151623 | Hs.163924 | Gress et al., 1996 |
| hsa-miR-224 | up | NR4A1 | Nuclear receptor subfamily 4, group A, member 1 | ENSG00000123358 | Hs.524430 | Nakamura et al., 2004 |
| hsa-miR-93 | up | NR4A2 | Nuclear receptor subfamily 4, group A, member 2 | ENSG00000153234 | Hs.165258 | Nakamura et al., 2004 |
| hsa-miR-31 | up | NR5A2 | Nuclear receptor subfamily 5, group A, member 2 | ENSG00000116833 | Hs.33446 | Crnogorac-Jurcevic et al., 2003 |
| hsa-miR-143 hsa-miR-146a hsa-miR-31 | up | NUMB | Numb homolog (Drosophila) | ENSG00000133961 | Hs.509909 | Buchholz et al.,2005 |
| hsa-miR-205 hsa-miR-223 | up | NUTF2 | Nuclear transport factor 2 | ENSG00000102898 | Hs.356630 | Buchholz et al.,2005 |
| hsa-miR-96 | down | OSBPL8 | Oxysterol binding protein-like 8 | ENSG00000091039 | Hs.430849 | Iacobuzio-Donahue et al., 2002 |
| hsa-miR-29c | down | OXTR | Oxytocin receptor | ENSG00000180914 | Hs.2820 | Sato et al., 2003 |
| hsa-miR-130b hsa-miR-141 hsa-miR-96 | down | PAFAH1B1 | Platelet-activating factor acetylhydrolase, isoform Ib, alpha subunit 45kDa | ENSG00000007168 | Hs.77318 | Gress et al., 1996 |
| hsa-miR-221 hsa-miR-222 hsa-miR-93 | up | PCDHA6 | Protocadherin alpha subfamily C, 1 | ENSG00000081842 | Hs.199343 | Buchholz et al.,2005 |
| hsa-miR-221 hsa-miR-222 hsa-miR-31 | up | PCGF3 | Polycomb group ring finger 3 | ENSG00000185619 | Hs.144309 | Nakamura et al., 2004 |
| hsa-miR-217 | down | PCNA | Proliferating cell nuclear antigen | ENSG00000132646 | Hs.147433 | Han et al., 2002 |
| hsa-miR-145 | up | PDCD4 | Programmed cell death 4 (neoplastic transformation | ENSG00000150593 | Hs.232543 | Tan et al., 2003 |
| | | | inhibitor) | | | |
| hsa-miR-29c | down | PDGFRB | Platelet-derived growth factor receptor, beta polypeptide | ENSG00000113721 | Hs.509067 | Gress et al., 1997 |
| hsa-miR-205 | up | PHB | Prohibitin | ENSG00000167085 | Hs.514303 | Nakamura et al., 2004 |
| hsa-miR-143 hsa-miR-18a hsa-miR-203 hsa-miR-223 | up | PHF20L1 | PHD finger protein 20-like 1 | ENSG00000197967 | Hs.304362 | Buchholz et al., 2005 |
| hsa-miR-203 | up | PLD2 | Phospholipase D2 | ENSG00000129219 | Hs.104519 | Nakamura et al., 2004 |
| hsa-miR-31 | up | PLEK2 | Pleckstrin 2 | ENSG00000100558 | Hs.170473 | Sato et al., 2003 |
| hsa-miR-96 | down | PLOD2 | Procollagen-lysine, 2-oxoglutarate 5-dioxygenase 2 | ENSG00000152952 | Hs.477866 | Buchholz et al., 2005 |
| hsa-miR-93 | up | PPARA | Peroxisome proliferative activated receptor, alpha | ENSG00000186951 | Hs.275711 | Nakamura et al., 2004 |
| hsa-miR-29c | down | PPIC | Peptidylprolyl isomerase C (cyclophilin C) | ENSG00000168938 | Hs.110364 | Nakamura et al., 2004 |
| hsa-miR-216 | down | PPM1B | Protein phosphatase 1B ((formerly 2C), magnesium-dependent, beta isoform | ENSG00000138032 | Hs.416769 | Nakamura et al., 2004 |
| hsa-miR-141 | down | PPP2R2A | Protein phosphatase 2 (formerly 2A), regulatory subtmit B (PR 52), alpha isoform | ENSG00000104762 | Hs.146339 | Yoshida et al., 2003 |
| hsa-miR-141 | down | PPT2 | Palmitoyl-protein thioesterase 2 | ENSG00000168452 | Hs.332138 | Buchholz et al., 2005 |
| hsa-miR-29c | down | PRO014 | PRO0149 protein | ENSG00000182831 | Hs.221497 | Gress et al., 1996 |
| hsa-miR-130b hsa-miR-141 hsa-miR-29c hsa-miR-96 | down | PTP4A1 | Protein tyrosine phosphatase type IVA, member 1 | ENSG00000112245 | Hs.227777 | Han et al., 2002 |
| hsa-miR-130b hsa-miR-148a hsa-miR-148b hsa-miR-96 | down | RAB34 | RAB34, member RAS oncogene family | ENSG00000109113 | Hs.301853 | Buchholz et al., 2005; lacobuzio-Donahue et al., 2003 |
| hsa-miR-96 | down | RAC1 | Ras-related C3 botulinum toxin substrate 1 (rho family, small GTP binding protein Rac 1) | ENSG00000136238 | Hs.413812 | Crnogorac-Jurcevic et al., 2001 |
| hsa-miR-141 hsa-miR-29c | down | RARB | Retinoic acid receptor, beta | ENSG00000077092 | Hs.436538 | Crnogorac-Jurcevic et al., 2001 |
| hsa-miR-141 | down | RBMS1 | RNA binding motif, single stranded interacting protein 1 | ENSG00000153250 | Hs.470412 | Nakamura et al., 2004 |
| hsa-miR-223 | up | RBPSUH | Recombining binding protein suppressor of hairless (Drosophila) | ENSG00000168214 | Hs.479396 | Han et al., 2002 |
| hsa-miR-196a hsa-miR-196b | up | RGL2 | Ral guanine nucleotide dissociation stimulator-like 2 | ENSG00000056687 | Hs.509622 | Nakamura et al., 2004 |
| hsa-miR-130b hsa-miR-148a hsa-miR-148b hsa-miR-29c | down | RNPEPL1 | Arginyl aminopeptidase (aminopeptidase B)-like 1 | ENSG00000142327 | Hs.5345 | Gress et al., 1996 |
| hsa-miR-141 hsa-miR-375 | down | RUNX1 | Runt-related transcription factor 1 (acute myeloid leukemia 1; amll oncogene) | ENSG00000159216 | Hs.149261 | Iacobuzio-Donahue et al., 2002; lacobuzio-Donahue et al., 2003 |
| hsa-miR-221 hsa-miR-222 | up | SAFB | Scaffold attachment factor B | ENSG00000160633 | Hs.23978 | Nakamura et al., 2004 |
| hsa-miR-141 | down | SCD | Stearoyl-CoA desaturase (delta-9-desaturase) | ENSG00000099194 | Hs.558396 | Nakamura et al., 2004 |
| hsa-miR-205 | up | SCMH1 | Sex comb on midleg homolog 1 (Drosophila) | ENSG00000010803 | Hs.87464 | Nakamura et al., 2004 |
| hsa-miR-130b hsa-miR-148a hsa-miR-148b hsa-miR-217 | down | SDFR1 | Stromal cell derived factor receptor 1 | ENSG00000156642 | Hs.187866 | Gress et al., 1996 |
| hsa-miR-205 | up | SEL1L | Sel-1 suppressor of lin-12-like (C. elegans) | ENSG00000071537 | Hs.181300 | Crnogorac-Jurcevic et al., 2003 |
| hsa-miR-31 | up | SEMA3F | Sema domain, immimoglobulin domain (Ig), short basic domain, secreted, semaphorin 3F | ENSG00000001617 | Hs.32981 | Nakamura et al., 2004 |
| hsa-miR-205 hsa-miR-93 | up | SENP1 | SUMO1/sentrin specific peptidase 1 | ENSG00000079387 | Hs.371957 | Nakamura et al., 2004 |
| hsa-miR-96 | down | SEPT 11 | Septin 11 | ENSG00000138758 | Hs.128199 | Iacobuzio-Donahue et al., 2002 |
| hsa-miR-141 hsa-miR-216 | down | SEPT7 | Septin 7 | ENSG00000122545 | Hs.191346 | Nakamura et al., 2004 |
| hsa-miR-221 hsa-miR-222 | up | SFRP2 | Secreted frizzled-related protein 2 | ENSG00000145423 | Hs.481022 | Crnogorac-Jurcevic et al., 2001 |
| hsa-miR-96 | down | SH3BGRL3 | SH3 domain binding glutamic acid-rich protein like 3 | ENSG00000142669 | Hs.109051 | Buchholz et al., 2005 |
| hsa-miR-130b | down | SIAHBP1 | Fuse-binding protein-interacting repressor | ENSG00000179950 | Hs.521924 | Gress et al., 1996 |
| hsa-miR-29c | down | SLC16A1 | AKR7 family pseudogene | ENSG00000155380 | Hs.75231 | Iacobuzio-Donahue et al., 2003 |
| hsa-miR-141 | down | SLC20A1 | Solute carrier family 20 (phosphate transporter), member 1 | ENSG00000144136 | Hs.187946 | Nakamura et al., 2004 |
| hsa-miR-145 | up | SLC25A25 | Solute carrier family 25 (mitochondrial carrier; phosphate carrier), member 25 | ENSG00000148339 | Hs.5476 | Nakamura et al., 2004 |
| hsa-miR-130b hsa-miR-148a hsa-miR-148b | down | SLC2A1 | Solute carrier family 2 (facilitated glucose transporter), member 1 | ENSG00000117394 | Hs.473721 | Logsdon et al., 2003; Crnogorac-Jurcevic et al., 2003; Nakamura et al., 2004; Iacobuzio-Donahue et al., 2003 |
| hsa-miR-96 | down | SLC35A1 | Solute carrier family 35 (CMP-sialic acid transporter), member A1 | ENSG00000055291 | Hs.423163 | Friess et al., 2003 |
| hsa-miR-93 | up | SLC40A1 | Solute carrier family 40 (iron-regulated transporter), member 1 | ENSG00000138449 | Hs.529285 | Nakamura et al., 2004 |
| hsa-miR-145 hsa-miR-203 hsa-miR-221 hsa-miR-222 hsa-miR-224 | up | SLC4A4 | Solute carrier family 4, sodium bicarbonate cotransporter, member 4 | ENSG00000080493 | Hs.5462 | Buchholz et al.,2005; lacobuzio-Donahue et al., 2003; Crnogorac-Jurcevic et al., 2003; Nakamura et al., 2004 |
| hsa-miR-96 | down | SLCO3A1 | Solute carrier organic anion transporter family, member 3A1 | ENSG00000176463 | Hs.311187 | lacobuzio-Donahue et al., 2003 |
| hsa-miR-216 | down | SMAD7 | SMAD, mothers against DPP homolog 7 (Drosophila) | ENSG00000101665 | Hs.465087 | Kleeff et al., 1999 |
| hsa-miR-96 | down | SMAD7 | SMAD, mothers against DPP homolog 7 (Drosophila) | ENSG00000101665 | Hs.465087 | Kleeff et al., 1999 |
| hsa-miR-221 hsa-miR-222 | up | SMARCA1 | SWI/SNF related, matrix associated, actin dependent regulator of chromatin, subfamily a, member 1 | ENSG00000102038 | Hs. 152292 | Sato et al., 2003 |
| hsa-miR-148a hsa-miR-148b | down | SMARCD1 | SWI/SNF related, matrix associated, actin dependent regulator of chromatin, subfamily d, member 1 | ENSG00000066117 | Hs.79335 | Buchholz et al., 2005 |
| hsa-miR-130b | down | SMC4L1 | SMC4 structural maintenance of | ENSG00000113810 | Hs.58992 | Nakamura et al., 2004; |
| hsa-miR-141 hsa-miR-216 | | | chromosomes 4-like 1 (yeast) | | | Iacobuzio-Donahue et al., 2003 |
| hsa-miR-130b hsa-miR-141 hsa-miR-148a hsa-miR-148b hsa-miR-96 | down | SOX5 | SRY (sex determining region Y)-box 5 | ENSG00000134532 | Hs.434948 | Tan et al., 2003 |
| hsa-miR-155 hsa-miR-221 | up | SOX6 | SRY (sex determining region Y)-box 6 | ENSG00000110693 | Hs.368226 | Nakamura et al., 2004 |
| hsa-miR-93 | up | SP8 | Sp8 transcription factor | ENSG00000164651 | Hs. 195922 | Nakamura et al., 2004 |
| hsa-miR-29c | down | SPARC | Secreted protein, acidic, cysteine-rich (osteonectin) | ENSG00000113140 | Hs.111779 | Tan et al., 2003; Nakamura et al., 2004; Friess et al., 2003 |
| hsa-miR-93 | up | STAT3 | Signal transducer and activator of transcription 3 (acute-phase response factor) | ENSG00000168610 | Hs.463059 | Han et al., 2002 |
| hsa-miR-217 | down | STRBP | Spermatid perinuclear RNA binding protein | ENSG00000165209 | Hs.287659 | Iacobuzio-Donahue et al., 2003 |
| hsa-miR-130b hsa-miR-148a hsa-miR-148b | down | SULF1 | Sulfatase 1 | ENSG00000137573 | Hs.409602 | Buchholz et al., 2005; Iacobuzio-Donahue et al., 2002 |
| hsa-miR-29c | down | SYT7 | Synaptotagmin VII | ENSG00000011347 | Hs.502730 | Buchholz et al., 2005 |
| hsa-miR-217 hsa-miR-96 | down | TACC1 | Transforming, acidic coiled-coil containing protein 1 | ENSG00000147526 | Hs.279245 | Gress et al.,1996 |
| hsa-miR-196a hsa-miR-196b | up | TCF7 | Transcription factor 7 (T-cell specific, HMG-box) | ENSG00000081059 | Hs.519580 | Buchholz et al., 2005 |
| hsa-miR-96 | down | TEGT | Testis enhanced gene transcript (BAX inhibitor 1) | ENSG00000139644 | Hs.35052 | Nakamura et al., 2004 |
| hsa-miR-130b | down | TIMP2 | TIMP metallopeptidase inhibitor 2 | ENSG00000035862 | Hs.104839 | Gress et al., 1996; Bramhall et al., 1997 |
| hsa-miR-221 hsa-miR-222 | up | TIMP3 | TIMP metallopeptidase inhibitor 3 (Sorsby fundus dystrophy, pseudoinflammatory) | ENSG00000197047 | Hs.297324 | Sato et al., 2003 |
| hsa-miR-93 | up | TTPARP | TCDD-inducible poly(ADP-ribose) polymerase | ENSG00000163659 | Hs.12813 | Nakamura et al., 2004 |
| hsa-miR-29c hsa-miR-96 | down | TM4SF14 | Tetraspanin 14 | ENSG00000108219 | Hs.310453 | Nakamura et al., 2004 |
| hsa-miR-130b | down | TMEPAI | Transmembrane, prostate androgen | ENSG00000124225 | Hs.517155 | Iacobuzio-Donahue et al., |
| hsa-miR-96 | | | induced RNA | | | 2002 |
| hsa-miR-29c | down | TMPRSS3 | Transmembrane protease, serine 3 | ENSG00000160183 | Hs.208600 | lacobuzio-Donahue et al., 2003 |
| hsa-miR-130b hsa-miR-148a hsa-miR-148b hsa-miR-96 | down | TMSB10 | Thymosin, beta 10 | ENSG00000034510 | Hs.446574 | Tan et al., 2003; Nakamura et al., 2004 |
| hsa-miR-13 Ob hsa-miR-148a hsa-miR-148b hsa-miR-217 | down | TMSB4X | Thymosin, beta 4, X-linked | ENSG00000188364 | Hs.522584 | Tan et al., 2003 |
| hsa-miR-145 | up | STEAP4 | STEAP family member 4 | ENSG00000127954 | Hs.521008 | Nakamura et al., 2004 |
| hsa-miR-155 hsa-miR-93 | up | TP53INP1 | Tumor protein p53 inducible nuclear protein 1 | ENSG00000164938 | Hs.492261 | Nakamura et al., 2004 |
| hsa-miR-29c | down | TRIB2 | Tribbles homolog 2 (Drosophila) | ENSG00000071575 | Hs.467751 | Nakamura et al., 2004 |
| hsa-miR-130b hsa-miR-96 | down | TTYH3 | Tweety homolog 3 (Drosophila) | ENSG00000136295 | Hs.440899 | Buchholz et al.,2005 |
| hsa-miR-130b | down | UBE2D2 | Ubiquitin-conjugating enzyme E2D 2 (UBC4/5 homolog, yeast) | ENSG00000131508 | Hs.108332 | Nakamura et al., 2004 |
| hsa-miR-221 hsa-miR-222 | up | UNC84B | Unc-84 homolog B (C. elegans) | ENSG00000100242 | Hs.517622 | Nakamura et al., 2004 |
| hsa-miR-29c | down | USP37 | Ubiquitin specific peptidase 37 | ENSG00000135913 | Hs.166068 | Gress et al., 1996 |
| hsa-miR-143 hsa-miR-221 hsa-miR-222 hsa-miR-31 | up | VAPB | VAMP (vesicle-associated membrane protein)-associated protein B and C | ENSG00000124164 | Hs.182625 | Buchholz et al.,2005 |
| hsa-miR-130b hsa-miR-141 hsa-miR-148a hsa-miR-148b hsa-miR-29c | down | WHSC1 | Wolf-Hirschhorn syndrome candidate 1 | ENSG00000109685 | Hs.113876 | Nakamura et al., 2004 |
| hsa-miR-130b hsa-miR-148a hsa-miR-148b | down | WNT10B | Wingless-type MMTV integration site family, member 10B | ENSG00000169884 | Hs.91985 | Buchholz et al.,2005 |
| hsa-miR-18a hsa-miR-203 | up | YPEL5 | Yippee-like 5 (Drosophila) | ENSG00000119801 | Hs.515890 | Grützmann et al., 2003 |
| hsa-miR-130b hsa-miR-148a | down | ZCCHC2 | Zinc finger, CCHC domain containing 2 | ENSG00000141664 | Hs.114191 | Nakamura et al., 2004 |
| hsa-miR-148b hsa-miR-217 | | | | | | |
| hsa-miR-96 | down | ZIC2 | Zic family member 2 (odd-paired homolog, Drosophila) | ENSG00000043355 | Hs.369063 | lacobuzio-Donahue et al., 2003 |
| hsa-miR-145 hsa-miR-205 hsa-miR-196a hsa-miR-196b | up | ZNF462 | Zinc finger protein 462 | ENSG00000148143 | Hs.370379 | Buchholz et al.,2005 |

### EXAMPLE 16

### DIAGNOSTIC METHODS

A patient may present for evaluation with symptoms that include one or more of jaundice, weight loss, bruising, abdominal pain, vomiting, diarrhea, and/or nausea. Peripheral blood is drawn in order to evaluate the patient's plasma for the presence of CA 19-9, a cancer tumor marker that exhibits 50-75% sensitivity and 83% specificity for pancreatic cancer (Freelove and Walling, 2006). At the same time total RNA, including the miRNA fraction, are purified from a sample of the patient's plasma. The methods of the invention are used to determine if levels of miRNAs listed in Table 6, Table 9 and FIG. 9A and 9B are altered in a manner that suggests pancreatic ductal adenocarcinoma or chronic pancreatitis. Typically, under these circumstances the invention will be used to diagnose a case of chronic pancreatitis.

### EXAMPLE 17

### DIAGNOSTIC METHODS

A patient may have symptoms suggesting pancreatic cancer or chronic pancreatitis (see Example 13) and may be found to have elevated levels of the serum tumor marker CA19-9 and is scheduled for an endoscopic ultrasonography-guided fine needle aspiration (Freelove and Walling, 2006). During the procedure, fine needle aspirates containing pancreatic cells are collected. Alternatively, pancreatic juice may be aspirated during the procedure. Pancreatic juice may contain sloughed pancreatic cells and contents of lysed pancreatic cells. Total RNA, including the miRNA fraction, is purified from the contents of the fine needle aspirate, fresh, frozen or fixed, or from the pancreatic juice sample. The methods of the invention are used to determine if levels of miRNAs listed in Table 6, Table 9, and FIG. 9A and 9B are altered in a manner that suggests pancreatic ductal adenocarcinoma or chronic pancreatitis. Typically, under these circumstances the invention will be used to confirm a suspected diagnosis of pancreatic cancer.

### EXAMPLE 18

### DIAGNOSTIC METHODS

An asymptomatic patient may be found to have a pancreatic mass on CT scan imaging. Chest x-ray and colonoscopy are normal. The patient has a family history of pancreatic cancer and may have experienced some recent weight loss. Peripheral blood is drawn in order to evaluate the patient's plasma for the presence of tumor marker antigens. A sample of the plasma also may be processed to purify miRNAs. The methods of the invention then may be used to determine if levels of plasma-isolated miRNAs are altered in a manner that suggests pancreatic ductal adenocarcinoma or chronic pancreatitis (Table 6, Table 9, FIG. 9A and 9B). Under these circumstances the invention can be used to provide a diagnosis of pancreatic cancer.

### REFERENCES

The following references provide exemplary procedural or other details supplementary to those set forth herein.
U.S. Patent 6,723,509
U.S. Patent 4,337,063
U.S. Patent 4,404,289
U.S. Patent 4,405,711
U.S. Patent 4,659,774
U.S. Patent 4,682,195
U.S. Patent 4,683,202
U.S. Patent 4,704,362
U.S. Patent 4,816,571
U.S. Patent 4,959,463
U.S. Patent 5,141,813
U.S. Patent 5,143,854
U.S. Patent 5,202,231
U.S. Patent 5,214,136
U.S. Patent 5,221,619
U.S. Patent 5,223,618
U.S. Patent 5,242,974
U.S. Patent 5,264,566
U.S. Patent 5,268,486
U.S. Patent 5,288,644
U.S. Patent 5,324,633
U.S. Patent 5,378,825
U.S. Patent 5,384,261
U.S. Patent 5,405,783
U.S. Patent 5,412,087
U.S. Patent 5,424,186
U.S. Patent 5,428,148
U.S. Patent 5,429,807
U.S. Patent 5,432,049
U.S. Patent 5,436,327
U.S. Patent 5,445,934
U.S. Patent 5,446,137
U.S. Patent 5,466,786
U.S. Patent 5,468,613
U.S. Patent 5,470,710
U.S. Patent 5,470,967
U.S. Patent 5,472,672
U.S. Patent 5,480,980
U.S. Patent 5,492,806
U.S. Patent 5,503,980
U.S. Patent 5,510,270
U.S. Patent 5,525,464
U.S. Patent 5,527,681
U.S. Patent 5,529,756
U.S. Patent 5,532,128
U.S. Patent 5,545,531
U.S. Patent 5,547,839
U.S. Patent 5,554,501
U.S. Patent 5,554,744
U.S. Patent 5,556,752
U.S. Patent 5,561,071
U.S. Patent 5,571,639
U.S. Patent 5,574,146
U.S. Patent 5,580,726
U.S. Patent 5,580,732
U.S. Patent 5,583,013
U.S. Patent 5,593,839
U.S. Patent 5,599,672
U.S. Patent 5,599,695
U.S. Patent 5,602,240
U.S. Patent 5,602,244
U.S. Patent 5,610,287
U.S. Patent 5,610,289
U.S. Patent 5,614,617
U.S. Patent 5,623,070
U.S. Patent 5,624,711
U.S. Patent 5,631,134
U.S. Patent 5,637,683
U.S. Patent 5,639,603
U.S. Patent 5,645,897
U.S. Patent 5,652,099
U.S. Patent 5,654,413
U.S. Patent 5,658,734
U.S. Patent 5,661,028
U.S. Patent 5,665,547
U.S. Patent 5,667,972
U.S. Patent 5,670,663
U.S. Patent 5,672,697
U.S. Patent 5,681,947
U.S. Patent 5,695,940
U.S. Patent 5,700,637
U.S. Patent 5,700,922
U.S. Patent 5,705,629
U.S. Patent 5,708,154
U.S. Patent 5,714,606
U.S. Patent 5,728,525
U.S. Patent 5,744,305
U.S. Patent 5,763,167
U.S. Patent 5,777,092
U.S. Patent 5,792,847
U.S. Patent 5,800,992
U.S. Patent 5,807,522
U.S. Patent 5,830,645
U.S. Patent 5,837,196
U.S. Patent 5,847,219
U.S. Patent 5,858,988
U.S. Patent 5,859,221
U.S. Patent 5,871,928
U.S. Patent 5,872,232
U.S. Patent 5,876,932
U.S. Patent 5,886,165
U.S. Patent 5,919,626
U.S. Patent 6,004,755
U.S. Patent 6,087,102
U.S. Patent 6,251,666
U.S. Patent 6,368,799
U.S. Patent 6,383,749
U.S. Patent 6,617,112
U.S. Patent 6,638,717
U.S. Patent 6,720,138
U.S. Patent Application serial number 09/545,207
U.S. Patent Application serial number 10/667,126
U.S. Patent Application serial number 11/141,707
U.S. Patent Application serial number 11/273,640
U.S. Patent Prov. Appn. serial number 60/575,743
U.S. Patent Prov. Appn. serial number 60/649,584
U.S. Patent Prov. Appn. serial number 60/826,173
Ambros, Cell, 107(7):823-826, 2001.
Andrianifahanana et al., Clin. Cancer Res., 7(12): 4033-4040, 2001.
Balague et al., Gastroenterology, 109(3):953-964, 1995.
Beaucage, and Lyer, Tetrahedron, 48:2223-2311, 1992.
Bhanot et al., Am. J Surg. Pathol., 30(6): 754-759, 2006.
Brennecke et al., Cell, 113:25-36, 2003.
Calin et al., Proc. Natl. Acad. Sci. USA, 99:15524-15529, 2002.
Carrington et al. Science, 301(5631):336-338, 2003.
Chen et al., Int. J. Cancer, 120(7):1511-1517, 2007.
Cummins et al., In: IRT: Nucleosides and nucleosides, La Jolla CA, 72, 1996.
Denli et al., Trends Biochem. Sci., 28:196, 2003.
Didenko, Biotechniques, 31(5):1106-16, 1118, 1120-1, 2001.
Duxbury et al., Br. J. Cancer, 91(7)1384-1390, 2004.
Emptage et al., : Neuron, 2001 Jan;29(1):197-208, 2001.
EP 266,032
EP 373 203
EP 785 280
EP 799 897
Esquela-Kerscher and Slack, Nat Rev Cancer, 6(4):259-269, 2006.
Fodor et al., Science, 251:767-777, 1991.
Freelove and Walling, Am. Fam. Physician, 73(3):485-492, 2006.
Friess et al., Gastroenterology, 113(3):904-913, 1997.
Froehler et al., Nucleic Acids Res., 14(13):5399-5407, 1986.
Gillam et al., J Biol. Chem., 253:2532, 1978.
Gillam et al., Nucleic Acids Res., 6:2973, 1979.
Griffey et al., JMass Spectrom, 32(3):305-13, 1997.
Griffiths-Jones et al., Nucleic Acids Res., 34 (Database Issue):D140-D144, 2006.
He et al., Proc. Natl. Acad. Sci. USA, 102(52):19075-19080, 2005.
Hollingsworth et al.., Int. J. Cancer, 57(2):198-203, 1994.
Huber et al., Bioinformatics, 18:Suppl 1:596-104, 2002.
Itakura and Riggs, Science, 209:1401-1405, 1980.
Itakura et al., J. Biol. Chem., 250:4592, 1975.
Jemal et al., CA CancerJClin., 56(2):106-130, 2006.
Jhala et al., Am. J. Clin. Pathol. 126(4):572-579, 2006.
Khorana, Science, 203, 614, 1979.
Kleeff et al., Oncogene, (39):5363-5372, 1999.
Klostermeier and Millar, Biopolymers, 61(3):159-79, 2001-2002.
Kornberg and Baker, In: DNA Replication, 2d Ed., Freeman, San Francisco, 1992.
Lagos-Quintana et al., Science, 294(5543):853-858, 2001.
Lau et al., Science, 294(5543):858-862, 2001.
Lee and Ambros, Science, 294(5543):862-864, 2001.
Lee et al., EMBO J. 21:4663-70, 2002.
Lopes et al., Int. J. Cancer, 120(11):2344-2352, 2007
Lu et al., Nature, 435(7043):834-838, 2005.
Monti et al., Virchows Arch., 445(3):236-247, 2004.
Olsen et al., Dev. Biol., 216:671, 1999.
Sambrook et al., In: DNA microaarays: a molecular cloning manual, Cold Spring Harbor Laboratory Press, Cold Spring Harbor, NY, 2003.
Sambrook et al., In: Molecular cloning: a laboratory manual, 2nd Ed., Cold Spring Harbor Laboratory Press, Cold Spring Harbor, NY, 1989.
Sambrook et al., In: Molecular cloning: a laboratory manual, 3rd Ed., Cold Spring Harbor Laboratory Press, Cold Spring Harbor, NY, 2001.
Seggerson et al., Dev. Biol., 243:215, 2002.
Shimzu et al., Arch. Pathol. Lab. Med., 114(2):195-200, 1990.
U.K. Patent 8 803 000
U.K. Patent 1,529,202
WO 0168255
WO 03020898
WO 03022421
WO 03023058
WO 03029485
WO 03040410
WO 03053586
WO 03066906
WO 03067217
WO 03076928
WO 03087297
WO 03091426
WO 03093810
WO 03100448A1
WO 04020085
WO 04027093
WO 09923256
WO 09936760
WO 93/17126
WO 95/11995
WO 95/21265
WO 95/21944
WO 95/21944
WO 95/35505
WO 96/31622
WO 97/10365
WO 97/27317
WO 9743450
WO 99/35505
WO0138580
WO03100012
Xu et al., Curr. Biol., 13:790-795, 2003.

## Claims

1. A method for diagnosing a pancreatitis or pancreatic ductal adenocarcinoma (PDAC) in a patient comprising measuring an expression profile of a miRNA in a pancreatic sample obtained from the patient, wherein a decrease in the expression profile in the sample from the patient as compared to a reference expression profile of a normal sample is indicative of a pancreatitis or pancreatic ductal adenocarcinoma (PDAC); wherein the miRNA is hsa-miR-130b.

2. The method of claim 1 further comprising measuring an expression profile of one or more miRNAs in a sample or from the patient, wherein a difference in the expression profile in the sample from the patient and a reference expression profile is indicative of pancreatitis or pancreatic ductal adenocarcinoma (PDAC); wherein the miRNA is: hsa-let-7a, hsa-let-7b, hsa-let-7c, hsa-let-7d, hsa-let-7e, hsa-let-7f, hsa-let-7g, hsa-let-7i, hsa-miR-100, hsa-miR-101, hsa-miR-103, hsa-miR-106a, hsa-miR-106b, hsa-miR-107, hsa-iR-10a, hsa-miR-125a, hsa-miR-125b, hsa-miR-130a, hsa-miR-134, hsa-miR-140, hsa-miR-141, hsa-miR-143, hsa-miR-145, hsa-miR-146a, hsa-miR-148a, hsa-miR-148b, hsa-miR-150, hsa-miR-154, hsa-miR-155, hsa-miR-15b, hsa-miR-17-5p, hsa-miR-18a, hsa-miR-181b, hsa-miR-182, hsa-miR-186, hsa-miR-196a, hsa-miR-196b, hsa-miR-199a, hsa-miR-199a-AS, hsa-miR-19a, hsa-miR-19b, hsa-miR-200a, hsa-miR-200b, hsa-miR-200c, hsa-miR-203, hsa-miR-21, hsa-miR-210, hsa-miR-214, hsa-miR-216, hsa-miR-217, hsa-miR-221, hsa-miR-222, hsa-miR-223, hsa-miR-224, hsa-miR-23a, hsa-miR-24, hsa-miR-25, hsa-miR-26a, hsa-miR-26b, hsa-miR-27a, hsa-miR-27b, hsa-miR-28, hsa-miR-29a, hsa-miR-29b, hsa-miR-29c, hsa-miR-30a-3p, hsa-miR-30a-5p, hsa-miR-30b, hsa-miR-30c, hsa-miR-30d, hsa-miR-30e-3p, hsa-miR-30e-5p, hsa-miR-31, hsa-miR-331, hsa-miR-335, hsa-miR-365, hsa-miR-368, hsa-miR-374, hsa-miR-375, hsa-miR-376a, hsa-miR-377, hsa-miR-379, hsa-miR-429, hsa-miR-93, hsa-miR-95, hsa-miR-96, hsa-miR-98, hsa-miR-99a, hsa-miR-99b, hsa-miR-452, hsa-miR-494, hsa-miR-497, miR-205, or ambi-miR-7105, preferably where the miRNA is one or more of miR-205, miR-29c, miR-216, miR-217, miR-375, miR-143, miR-145, miR-146a, miR-148a, miR-196b, miR-93, miR-96, miR-31, miR-210, miR-148b, miR-196a, miR-141, miR-18a, miR-203, miR-150, miR-155, miR-130b, miR-221, miR-222, miR-223, or miR-224, in particular preferred wherein the miRNA is miR-196a, miR-217, or both miR-196a and miR-217.

3. The method of claim 1 or 2, **characterized in that** the pathological condition is pancreatitis and
• differential expression of one or more miRNA is indicative of pancreatitis, wherein the miRNA is hsa-miR-130b, preferably further one or more of hsa-let-7a, hsa-let-7b, hsa-let-7c, hsa-let-7d, hsa-let-7e, hsa-let-7f, hsa-let-7g, hsa-let-7i, hsa-miR-100, hsa-miR-101, hsa-miR-10a, hsa-miR-125a, hsa-miR-125b, hsa-miR-130a, hsa-miR-141, hsa-miR-143, hsa-miR-145, hsa-miR-146a, hsa-miR-148a, hsa-miR-148b, hsa-miR-150, hsa-miR-18a, hsa-miR-182, hsa-miR-186, hsa-miR-199a hsa-miR-199a-AS, hsa-miR-19a, hsa-miR-19b, hsa-miR-200a, hsa-miR-200b, hsa-miR-200c, hsa-miR-203, hsa-miR-210, hsa-miR-214, hsa-miR-216, hsa-miR-217, hsa-miR-222, hsa-miR-223, hsa-miR-24, hsa-miR-25, hsa-miR-26a, hsa-miR-26b, hsa-miR-27b, hsa-miR-28, hsa-miR-29a, hsa-miR-29b, hsa-miR-29c, hsa-miR-30a-3p, hsa-miR-30a-5p, hsa-miR-30b, hsa-miR-30c, hsa-miR-30d, hsa-miR-30e-3p, hsa-miR-30e-5p, hsa-miR-31, hsa-miR-335, hsa-miR-365, hsa-miR-368, hsa-miR-374, hsa-miR-376a, hsa-miR-429, hsa-miR-95, hsa-miR-96, hsa-miR-98, hsa-miR-99a, hsa-miR-497, or ambi-miR-7105, or
• wherein a decrease in expression of one or more miRNA in a patient sample is indicative of pancreatitis, wherein the miRNA is hsa-miR-130b, preferably further one or more of hsa-let-7a, hsa-let-7b, hsa-let-7c, hsa-let-7d, hsa-let-7e, hsa-let-7f, hsa-let-7g, hsa-miR-101, hsa-miR-130a, hsa-miR-141, hsa-miR-148a, hsa-miR-148b, hsa-miR-182, hsa-miR-186, hsa-miR-19a, hsa-miR-19b, hsa-miR-200a, hsa-miR-200b, hsa-miR-200c, hsa-miR-203, hsa-miR-216, hsa-miR-217, hsa-miR-25, hsa-miR-26a, hsa-miR-26b, hsa-miR-27b, hsa-miR-28, hsa-miR-29a, hsa-miR-29b, hsa-miR-29c, hsa-miR-30a-3p, hsa-miR-30a-5p, hsa-miR-30b, hsa-miR-30c, hsa-miR-30d, hsa-miR-30e-3p, hsa-miR-30e-5p, hsa-miR-335, hsa-miR-365, hsa-miR-368, hsa-miR-374, hsa-miR-376a, hsa-miR-429, hsa-miR-95, hsa-miR-96, hsa-miR-98, or ambi-miR-7105.

4. The method of claim 1 or 2, **characterized in that** the pathological condition is pancreatic ductal adenocarcinoma (PDAC) and
• differential expression of one or more miRNA is indicative of PDAC, wherein the miRNA is hsa-miR-130b, preferably further one or more of hsa-let-7a, hsa-let-7b, hsa-let-7c, hsa-let-7d, hsa-let-7f, hsa-let-7g, hsa-let-7i, hsa-miR-100, hsa-miR-101, hsa-miR-103, hsa-miR-106b, hsa-miR-107, hsa-iR-10a, hsa-miR-125a, hsa-miR-125b, hsa-miR-130a, hsa-miR-134, hsa-miR-140, hsa-miR-141, hsa-miR-143, hsa-miR-145, hsa-miR-146a, hsa-miR-148a, hsa-miR-148b, hsa-miR-150, hsa-miR-154, hsa-miR-155, hsa-miR-18a, hsa-miR-181b, hsa-miR-182, hsa-miR-186, hsa-miR-196a, hsa-miR-196b, hsa-miR-199a, hsa-miR-199a-AS, hsa-miR-19a, hsa-miR-19b, hsa-miR-200a, hsa-miR-200b, hsa-miR-200c, hsa-miR-203, hsa-miR-21, hsa-miR-210, hsa-miR-214, hsa-miR-216, hsa-miR-217, hsa-miR-221, hsa-miR-222, hsa-miR-223, hsa-miR-224, hsa-miR-23a, hsa-miR-24, hsa-miR-26a, hsa-miR-26b, hsa-miR-27a, hsa-miR-27b, hsa-miR-29c, hsa-miR-30a-3p, hsa-miR-30a-5p, hsa-miR-30b, hsa-miR-30c, hsa-miR-30d, hsa-miR-30e-3p, hsa-miR-30e-5p, hsa-miR-31, hsa-miR-331, hsa-miR-335, hsa-miR-365, hsa-miR-368, hsa-miR-374, hsa-miR-375, hsa-miR-376a, hsa-miR-377, hsa-miR-379, hsa-miR-429, hsa-miR-93, hsa-miR-95, hsa-miR-96, hsa-miR-99b, hsa-miR-452, hsa-miR-494, hsa-miR-497, miR-205, or ambi-miR-7105, or
• wherein a decrease in expression of one or more miRNA is indicative of PDAC, wherein the miRNA is hsa-miR-130b, preferably further one or more of hsa-let-7a, hsa-let-7b, hsa-let-7c, hsa-let-7d, hsa-let-7e, hsa-let-7f, hsa-let-7g, hsa-miR-101, hsa-miR-130a, hsa-miR-134, hsa-miR-141, hsa-miR-148a, hsa-miR-148b, hsa-miR-154, hsa-miR-182, hsa-miR-186, hsa-miR-19a, hsa-miR-19b, hsa-miR-200a, hsa-miR-200b, hsa-miR-200c, hsa-miR-216, hsa-miR-217, hsa-miR-26a, hsa-miR-26b, hsa-miR-27b, hsa-miR-29c, hsa-miR-30a-3p, hsa-miR-30a-5p, hsa-miR-30b, hsa-miR-30c, hsa-miR-30d, hsa-miR-30e-3p, hsa-miR-30e-5p, hsa-miR-335, hsa-miR-365, hsa-miR-368, hsa-miR-374, hsa-miR-375, hsa-miR-376a, hsa-miR-377, hsa-miR-379, hsa-miR-429, hsa-miR-95, hsa-miR-96, or hsa-miR-494.

5. The method of claim 1 or 2, **characterized in that** pancreatitis is distinguished from PDAC by differential expression of one or more hsa-let-7b, hsa-let-7e, hsa-miR-103, hsa-miR-106a, hsa-miR-106b, hsa-miR-107, hsa-miR-125b, hsa-miR-130a, hsa-miR-130b, hsa-miR-141, hsa-miR-146a, hsa-miR-148a, hsa-miR-154, hsa-miR-155, hsa-miR-15b, hsa-miR-17-5p, hsa-miR-18a, hsa-miR-181b, hsa-miR-196a, hsa-miR-196b, hsa-miR-199a-AS, hsa-miR-203, hsa-miR-21, hsa-miR-210, hsa-miR-216, hsa-miR-217, hsa-miR-221, hsa-miR-222, hsa-miR-224, hsa-miR-23a, hsa-miR-24, hsa-miR-25, hsa-miR-27a, hsa-miR-28, hsa-miR-29a, hsa-miR-29c, hsa-miR-30a-3p, hsa-miR-30a-5p, hsa-miR-30b, hsa-miR-30c, hsa-miR-30d, hsa-miR-30e-5p, hsa-miR-331, hsa-miR-368, hsa-miR-375, hsa-miR-377, hsa-miR-379, hsa-miR-93, hsa-miR-452, hsa-miR-494, hsa-miR-497, or ambi-miR-7105.

6. The method of any one of claims 1 to 5, further comprising measuring an expression profile of one or more mRNA whose expression level is correlated with pancreatitis or PDAC, preferably wherein the mRNA measured is selected from the group comprising carcinoembryonic antigen-related cell adhesion molecule 6 mRNA, survivin mRNA, mucin 4 mRNA, and urokinase plasminogen activator receptor mRNA, and optionally wherein measuring an expression profile of one or more mRNA is by quantitative reverse transcription PCR (qRT-PCR).

7. The method of any one of claims 1 to 6, **characterized in that** the sample is a tissue, a blood, a serum, a plasma, or a pancreatic juice sample, preferably wherein the sample from the patient and the normal sample are pancreatic samples, and wherein the sample is optionally fresh, frozen, fixed, or embedded.

8. The method of any one of claims 1 to 7, further comprising labeling miRNA from the sample, preferably further comprising hybridizing the labeled miRNA to one or more miRNA probes, in particular wherein the miRNA probes are coupled to a support, preferably wherein the support is glass, plastic, metal, or latex, and in particular preferred wherein the support is planar, or wherein the support is a bead.

9. The method of any one of claims 1 to 8, **characterized in that** expression of the miRNA is determined by an amplification assay or a hybridization assay, preferably wherein the amplification assay is a quantitative amplification assay, in particular preferred quantitative RT-PCR, or wherein preferably the hybridization assay is an array hybridization assay or a solution hybridization assay.

10. The method of any one of claims 1 to 9, **characterized in that** diagnosing comprises screening for pancreatitis or pancreatic ductal adenocarcinoma (PDAC), assessing prognosis of pancreatitis or pancreatic ductal adenocarcinoma (PDAC), staging a pancreatitis or pancreatic ductal adenocarcinoma (PDAC), or assessing response of pancreatitis or pancreatic ductal adenocarcinoma (PDAC) to therapy.

11. A miRNA molecule or inhibitor for use in the treatment of pancreatic ductal adenocarcinoma in a subject comprising administering to the subject an effective amount of one or more synthetic miRNA molecules or inhibitors having a nucleic acid segment having the sequence of hsa-miR-130b, preferably further one or more of hsa-let-7a, hsa-let-7b, hsa-let-7c, hsa-let-7d, hsa-let-7f, hsa-let-7g, hsa-let-7i, hsa-miR-100, hsa-miR-101, hsa-miR-103, hsa-miR-106b, hsa-miR-107, hsa-iR-10a, hsa-miR-125a, hsa-miR-125b, hsa-miR-130a, hsa-miR-134, hsa-miR-140, hsa-miR-141, hsa-miR-143, hsa-miR-145, hsa-miR-146a, hsa-miR-148a, hsa-miR-148b, hsa-miR-150, hsa-miR-154, hsa-miR-155, hsa-miR-18a, hsa-miR-181b, hsa-miR-182, hsa-miR-186, hsa-miR-196a, hsa-miR-196b, hsa-miR-199a, hsa-miR-199a-AS, hsa-miR-19a, hsa-miR-19b, hsa-miR-200a, hsa-miR-200b, hsa-miR-200c, hsa-miR-203, hsa-miR-21, hsa-miR-210, hsa-miR-214, hsa-miR-216, hsa-miR-217, hsa-miR-221, hsa-miR-222, hsa-miR-223, hsa-miR-224, hsa-miR-23a, hsa-miR-24, hsa-miR-26a, hsa-miR-26b, hsa-miR-27a, hsa-miR-27b, hsa-miR-29c, hsa-miR-30a-3p, hsa-miR-30a-5p, hsa-miR-30b, hsa-miR-30c, hsa-miR-30d, hsa-miR-30e-3p, hsa-miR-30e-5p, hsa-miR-31, hsa-miR-331, hsa-miR-335, hsa-miR-365, hsa-miR-368, hsa-miR-374, hsa-miR-375, hsa-miR-376a, hsa-miR-377, hsa-miR-379, hsa-miR-429, hsa-miR-93, hsa-miR-95, hsa-miR-96, hsa-miR-99b, hsa-miR-452, hsa-miR-494, hsa-miR-497, miR-205, or ambi-miR-7105.

12. A miRNA molecule or inhibitor for use according to claim 11; wherein the further
(a) one or more miRNA inhibitors has the nucleic acid sequence of hsa-let-7i, hsa-miR-100, hsa-miR-103, hsa-miR-106b, hsa-miR-107, hsa-miR-10a, hsa-miR-125a, hsa-miR-125b, hsa-miR-140, hsa-miR-143, hsa-miR-145, hsa-miR-146a, hsa-miR-150, hsa-miR-155, hsa-miR-18a, hsa-miR-181b, hsa-miR-196a, hsa-miR-196b, hsa-miR-199a, hsa-miR-199a-AS, hsa-miR-203, hsa-miR-21, hsa-miR-210, hsa-miR-214, hsa-miR-221, hsa-miR-222, hsa-miR-223, hsa-miR-224, hsa-miR-23a, hsa-miR-24, hsa-miR-27a, hsa-miR-31, hsa-miR-331, hsa-miR-93, hsa-miR-99b, hsa-miR-452, hsa-miR-497, or ambi-miR-7105; and/or
(b) one or more miRNAs has the nucleic acid sequence of hsa-let-7a, hsa-let-7b, hsa-let-7c, hsa-let-7d, hsa-let-7e, hsa-let-7f, hsa-let-7g, hsa-miR-101, hsa-miR-130a, hsa-miR-130b, hsa-miR-134, hsa-miR-141, hsa-miR-148a, hsa-miR-148b, hsa-miR-154, hsa-miR-182, hsa-miR-186, hsa-miR-19a, hsa-miR-19b, hsa-miR-200a, hsa-miR-200b, hsa-miR-200c, hsa-miR-216, hsa-miR-217, hsa-miR-26a, hsa-miR-26b, hsa-miR-27b, hsa-miR-29c, hsa-miR-30a-3p, hsa-miR-30a-5p, hsa-miR-30b, hsa-miR-30c, hsa-miR-30d, hsa-miR-30e-3p, hsa-miR-30e-5p, hsa-miR-335, hsa-miR-365, hsa-miR-368, hsa-miR-374, hsa-miR-375, hsa-miR-376a, hsa-miR-377, hsa-miR-379, hsa-miR-429, hsa-miR-95, hsa-miR-96, or hsa-miR-494.

13. A miRNA molecule or inhibitor for use in the treatment of chronic pancreatitis in a subject comprising administering to the subject an effective amount of one or more synthetic miRNA molecules or miRNA inhibitors comprising the sequence of miR-130b, preferably further one or more of hsa-let-7a, hsa-let-7b, hsa-let-7c, hsa-let-7d, hsa-let-7e, hsa-let-7f, hsa-let-7g, hsa-let-7i, hsa-miR-100, hsa-miR-101, hsa-miR-10a, hsa-miR-125a, hsa-miR-125b, hsa-miR-130a, hsa-miR-141, hsa-miR-143, hsa-miR-145, hsa-miR-146a, hsa-miR-148a, hsa-miR-148b, hsa-miR-150, hsa-miR-18a, hsa-miR-182, hsa-miR-186, hsa-miR-199a hsa-miR-199a-AS, hsa-miR-19a, hsa-miR-19b, hsa-miR-200a, hsa-miR-200b, hsa-miR-200c, hsa-miR-203, hsa-miR-210, hsa-miR-214, hsa-miR-216, hsa-miR-217, hsa-miR-222, hsa-miR-223, hsa-miR-24, hsa-miR-25, hsa-miR-26a, hsa-miR-26b, hsa-miR-27b, hsa-miR-28, hsa-miR-29a, hsa-miR-29b, hsa-miR-29c, hsa-miR-30a-3p, hsa-miR-30a-5p, hsa-miR-30b, hsa-miR-30c, hsa-miR-30d, hsa-miR-30e-3p, hsa-miR-30e-5p, hsa-miR-31, hsa-miR-335, hsa-miR-365, hsa-miR-368, hsa-miR-374, hsa-miR-376a, hsa-miR-429, hsa-miR-95, hsa-miR-96, hsa-miR-98, hsa-miR-99a, hsa-miR-497, or ambi-miR-7105.

14. The miRNA molecule or inhibitor for use according to claim 12; wherein the further
(a) one or more miRNA inhibitors has the nucleic acid sequence of hsa-let-7i, hsa-miR-100, hsa-iR-10a, hsa-miR-125a, hsa-miR-125b, hsa-miR-143, hsa-miR-145, hsa-miR-146a, hsa-miR-150, hsa-miR-18a, hsa-miR-199a, hsa-miR-199a-AS, hsa-miR-210, hsa-miR-214, hsa-miR-222, hsa-miR-223, hsa-miR-24, hsa-miR-31, hsa-miR-99a, or hsa-miR-497; and/or
(b) one or more miRNAs has the nucleic acid sequence of hsa-let-7a, hsa-let-7b, hsa-let-7c, hsa-let-7d, hsa-let-7e, hsa-let-7f, hsa-let-7g, hsa-miR-101, hsa-miR-130a, hsa-miR-130b, hsa-miR-141, hsa-miR-148a, hsa-miR-148b, hsa-miR-182, hsa-miR-186, hsa-miR-19a, hsa-miR-19b, hsa-miR-200a, hsa-miR-200b, hsa-miR-200c, hsa-miR-203, hsa-miR-216, hsa-miR-217, hsa-miR-25, hsa-miR-26a, hsa-miR-26b, hsa-miR-27b, hsa-miR-28, hsa-miR-29a, hsa-miR-29b, hsa-miR-29c, hsa-miR-30a-3p, hsa-miR-30a-5p, hsa-miR-30b, hsa-miR-30c, hsa-miR-30d, hsa-miR-30e-3p, hsa-miR-30e-5p, hsa-miR-335, hsa-miR-365, hsa-miR-368, hsa-miR-374, hsa-miR-376a, hsa-miR-429, hsa-miR-95, hsa-miR-96, hsa-miR-98, or ambi-miR-7105.

15. Use of a kit in the analysis of a pathological sample, by assessing a miRNA profile for the sample in a method according to any one of claims 1-10, the kit comprising, in suitable container means, two or more miRNA probes, wherein the miRNA probes detect has-miR-130b, and further one or more of hsa-let-7a, hsa-let-7b, hsa-let-7c, hsa-let-7d, hsa-let-7f, hsa-let-7g, hsa-let-7i, hsa-miR-100, hsa-miR-101, hsa-miR-103, hsa-miR-106b, hsa-miR-107, hsa-iR-10a, hsa-miR-125a, hsa-miR-125b, hsa-miR-130a, hsa-miR-130b, hsa-miR-134, hsa-miR-140, hsa-miR-141, hsa-miR-143, hsa-miR-145, hsa-miR-146a, hsa-miR-148a, hsa-miR-148b, hsa-miR-150, hsa-miR-154, hsa-miR-155, hsa-miR-18a, hsa-miR-181b, hsa-miR-182, hsa-miR-186, hsa-miR-196a, hsa-miR-196b, hsa-miR-199a, hsa-miR-199a-AS, hsa-miR-19a, hsa-miR-19b, hsa-miR-200a, hsa-miR-200b, hsa-miR-200c, hsa-miR-203, hsa-miR-21, hsa-miR-210, hsa-miR-214, hsa-miR-216, hsa-miR-217, hsa-miR-221, hsa-miR-222, hsa-miR-223, hsa-miR-224, hsa-miR-23a, hsa-miR-24, hsa-miR-26a, hsa-miR-26b, hsa-miR-27a, hsa-miR-27b, hsa-miR-29c, hsa-miR-30a-3p, hsa-miR-30a-5p, hsa-miR-30c, hsa-miR-30d, hsa-miR-30e-3p, hsa-miR-30e-5p, hsa-miR-31, hsa-miR-331, hsa-miR-335, hsa-miR-365, hsa-miR-368, hsa-miR-374, hsa-miR-375, hsa-miR-376a, hsa-miR-377, hsa-miR-379, hsa-miR-429, hsa-miR-93, hsa-miR-95, hsa-miR-96, hsa-miR-99b, hsa-miR-452, hsa-miR-494, hsa-miR-497, miR-205, or ambi-miR-7105.

## Patentansprüche

1. Verfahren zum Diagnostizieren von Pankreatitis oder pankreatischem duktalem Adenokarzinom (PDAC) bei einem Patienten, umfassend das Messen eines Expressionsprofils einer miRNA in einer von dem Patienten erhaltenen Pankreasprobe, wobei ein Rückgang im Expressionsprofil in der Probe von dem Patienten verglichen zu einem Referenz-Expressionsprofil einer normalen Probe indikativ für eine Pankreatitis oder ein pankreatisches duktales Adenokarzinom (PDAC) ist; wobei die miRNA hsa-miR-130b ist.

2. Verfahren nach Anspruch 1, außerdem umfassend das Messen eines Expressionsprofils einer oder mehrerer miRNAs in einer Probe oder von einem Patienten, wobei ein Unterschied in dem Expressionsprofil in der Probe von dem Patienten und einem Referenz-Expressionsprofil indikativ für Pankreatitis oder pankreatisches duktales Adenokarzinom (PDAC) ist; wobei die miRNA ist: hsa-let-7a, hsa-let-7b, hsa-let-7c, hsa-let-7d, hsa-let-7e, hsa-let-7f, hsa-let-7g, hsa-let-7i, hsa-miR-100, hsa-miR-101, hsa-miR-103, hsa-miR-106a, hsa-miR-106b, hsa-miR-107, hsa-miR-10a, hsa-miR-125a, hsa-miR-125b, hsa-miR-130a, hsa-miR-134, hsa-miR-140, hsa-miR-141, hsa-miR-143, hsa-miR-145, hsa-miR-146a, hsa-miR-148a, hsa-miR-148b, hsa-miR-150, hsa-miR-154, hsa-miR-155, hsa-miR-15b, hsa-miR-17-5p, hsa-miR-18a, hsa-miR-181b, hsa-miR-182, hsa-miR-186, hsa-miR-196a, hsa-miR-196b, hsa-miR-199a, hsa-miR-199a-AS, hsa-miR-19a, hsa-miR-19b, hsa-miR-200a, hsa-miR-200b, hsa-miR-200c, hsa-miR-203, hsa-miR-21, hsa-miR-210, hsa-miR-214, hsa-miR-216, hsa-miR-217, hsa-miR-221, hsa-miR-222, hsa-miR-223, hsa-miR-224, hsa-miR-23a, hsa-miR-24, hsa-miR-25, hsa-miR-26a, hsa-miR-26b, hsa-miR-27a, hsa-miR-27b, hsa-miR-28, hsa-miR-29a, hsa-miR-29b, hsa-miR-29c, hsa-miR-30a-3p, hsa-miR-30a-5p, hsa-miR-30b, hsa-miR-30c, hsa-miR-30d, hsa-miR-30e-3p, hsa-miR-30e-5p, hsa-miR-31, hsa-miR-331, hsa-miR-335, hsa-miR-365, hsa-miR-368, hsa-miR-374, hsa-miR-375, hsa-miR-376a, hsa-miR-377, hsa-miR-379, hsa-miR-429, hsa-miR-93, hsa-miR-95, hsa-miR-96, hsa-miR-98, hsa-miR-99a, hsa-miR-99b, hsa-miR-452, hsa-miR-494, hsa-miR-497, miR-205 oder ambi-miR-7105, vorzugsweise wobei die miRNA eines oder mehreres ist von miR-205, miR-29c, miR-216, miR-217, miR-375, miR-143, miR-145, miR-146a, miR-148a, miR-196b, miR-93, miR-96, miR-31, miR-210, miR-148b, miR-196a, miR-141, miR-18a, miR-203, miR-150, miR-155, miR-130b, miR-221, miR-222, miR-223 oder miR-224, besonders bevorzugt wobei die miRNA miR-196a, miR-217 oder sowohl miR-196a als auch miR-217 ist.

3. Verfahren nach Anspruch 1 oder 2, **dadurch gekennzeichnet, dass** die pathologische Bedingung Pankreatitis ist und
• die differenzielle Expression einer oder mehrerer miRNAs indikativ für Pankreatitis ist, wobei die miRNA hsa-miR-130b ist, vorzugsweise außerdem eines oder mehrere von hsa-let-7a, hsa-let-7b, hsa-let-7c, hsa-let-7d, hsa-let-7e, hsa-let-7f, hsa-let-7g, hsa-let-7i, hsa-miR-100, hsa-miR-101, hsa-miR-10a, hsa-miR-125a, hsa-miR-125b, hsa-miR-130a, hsa-miR-141, hsa-miR-143, hsa-miR-145, hsa-miR-146a, hsa-miR-148a, hsa-miR-148b, hsa-miR-150, hsa-miR-18a, hsa-miR-182, hsa-miR-186, hsa-miR-199a, hsa-miR-199a-AS, hsa-miR-19a, hsa-miR-19b, hsa-miR-200a, hsa-miR-200b, hsa-miR-200c, hsa-miR-203, hsa-miR-210, hsa-miR-214, hsa-miR-216, hsa-miR-217, hsa-miR-222, hsa-miR-223, hsa-miR-24, hsa-miR-25, hsa-miR-26a, hsa-miR-26b, hsa-miR-27b, hsa-miR-28, hsa-miR-29a, hsa-miR-29b, hsa-miR-29c, hsa-miR-30a-3p, hsa-miR-30a-5p, hsa-miR-30b, hsa-miR-30c, hsa-miR-30d, hsa-miR-30e-3p, hsa-miR-30e-5p, hsa-miR-31, hsa-miR-335, hsa-miR-365, hsa-miR-368, hsa-miR-374, hsa-miR-376a, hsa-miR-429, hsa-miR-95, hsa-miR-96, hsa-miR-98, hsa-miR-99a, hsa-miR-497 oder ambi-miR-7105, oder
• wobei ein Rückgang in der Expression einer oder mehrerer miRNAs in einer Patientenprobe indikativ für Pankreatitis ist, wobei die miRNA hsa-miR-130b ist, vorzugsweise außerdem eines oder mehrere von hsa-let-7a, hsa-let-7b, hsa-let-7c, hsa-let-7d, hsa-let-7e, hsa-let-7f, hsa-let-7g, hsa-miR-101, hsa-miR-130a, hsa-miR-141, hsa-miR-148a, hsa-miR-148b, hsa-miR-182, hsa-miR-186, hsa-miR-19a, hsa-miR-19b, hsa-miR-200a, hsa-miR-200b, hsa-miR-200c, hsa-miR-203, hsa-miR-216, hsa-miR-217, hsa-miR-25, hsa-miR-26a, hsa-miR-26b, hsa-miR-27b, hsa-miR-28, hsa-miR-29a, hsa-miR-29b, hsa-miR-29c, hsa-miR-30a-3p, hsa-miR-30a-5p, hsa-miR-30b, hsa-miR-30c, hsa-miR-30d, hsa-miR-30e-3p, hsa-miR-30e-5p, hsa-miR-335, hsa-miR-365, hsa-miR-368, hsa-miR-374, hsa-miR-376a, hsa-miR-429, hsa-miR-95, hsa-miR-96, hsa-miR-98 oder ambi-miR-7105.

4. Verfahren nach Anspruch 1 oder 2, **dadurch gekennzeichnet, dass** der pathologische Zustand pankreatisches duktales Adenokarzinom (PDAC) ist und
• die differenzielle Expression einer oder mehrerer miRNAs indikativ für PDAC ist, wobei die miRNA hsa-miR-130b ist, vorzugsweise außerdem eines oder mehrere von hsa-let-7a, hsa-let-7b, hsa-let-7c, hsa-let-7d, hsa-let-7f, hsa-let-7g, hsa-let-7i, hsa-miR-100, hsa-miR-101, hsa-miR-103, hsa-miR-106b, hsa-miR-107, hsa-miR-10a, hsa-miR-125a, hsa-miR-125b, hsa-miR-130a, hsa-miR-134, hsa-miR-140, hsa-miR-141, hsa-miR-143, hsa-miR-145, hsa-miR-146a, hsa-miR-148a, hsa-miR-148b, hsa-miR-150, hsa-miR-154, hsa-miR-155, hsa-miR-18a, hsa-miR-181b, hsa-miR-182, hsa-miR-186, hsa-miR-196a, hsa-miR-196b, hsa-miR-199a, hsa-miR-199a-AS, hsa-miR-19a, hsa-miR-19b, hsa-miR-200a, hsa-miR-200b, hsa-miR-200c, hsa-miR-203, hsa-miR-21, hsa-miR-210, hsa-miR-214, hsa-miR-216, hsa-miR-217, hsa-miR-221, hsa-miR-222, hsa-miR-223, hsa-miR-224, hsa-miR-23a, hsa-miR-24, hsa-miR-26a, hsa-miR-26b, hsa-miR-27a, hsa-miR-27b, hsa-miR-29c, hsa-miR-30a-3p, hsa-miR-30a-5p, hsa-miR-30b, hsa-miR-30c, hsa-miR-30d, hsa-miR-30e-3p, hsa-miR-30e-5p, hsa-miR-31, hsa-miR-331, hsa-miR-335, hsa-miR-365, hsa-miR-368, hsa-miR-374, hsa-miR-375, hsa-miR-376a, hsa-miR-377, hsa-miR-379, hsa-miR-429, hsa-miR-93, hsa-miR-95, hsa-miR-96, hsa-miR-99b, hsa-miR-452, hsa-miR-494, hsa-miR-497, miR-205 oder ambi-miR-7105, oder
• wobei ein Rückgang in der Expression einer oder mehrerer miRNAs indikativ für PDAC ist, wobei die miRNA hsa-miR-130b ist, vorzugsweise außerdem eines oder mehrere von hsa-let-7a, hsa-let-7b, hsa-let-7c, hsa-let-7d, hsa-let-7e, hsa-let-7f, hsa-let-7g, hsa-miR-101, hsa-miR-130a, hsa-miR-134, hsa-miR-141, hsa-miR-148a, hsa-miR-148b, hsa-miR-154, hsa-miR-182, hsa-miR-186, hsa-miR-19a, hsa-miR-19b, hsa-miR-200a, hsa-miR-200b, hsa-miR-200c, hsa-miR-216, hsa-miR-217, hsa-miR-26a, hsa-miR-26b, hsa-miR-27b, hsa-miR-29c, hsa-miR-30a-3p, hsa-miR-30a-5p, hsa-miR-30b, hsa-miR-30c, hsa-miR-30d, hsa-miR-30e-3p, hsa-miR-30e-5p, hsa-miR-335, hsa-miR-365, hsa-miR-368, hsa-miR-374, hsa-miR-375, hsa-miR-376a, hsa-miR-377, hsa-miR-379, hsa-miR-429, hsa-miR-95, hsa-miR-96 oder hsa-miR-494.

5. Verfahren nach Anspruch 1 oder 2, **dadurch gekennzeichnet, dass** Pankreatitis von PDAC unterschieden wird durch die differenzielle Expression eines oder mehrerer von hsa-let-7b, hsa-let-7e, hsa-miR-103, hsa-miR-106a, hsa-miR-106b, hsa-miR-107, hsa-miR-125b, hsa-miR-130a, hsa-miR-130b, hsa-miR-141, hsa-miR-146a, hsa-miR-148a, hsa-miR-154, hsa-miR-155, hsa-miR-15b, hsa-miR-17-5p, hsa-miR-18a, hsa-miR-181b, hsa-miR-196a, hsa-miR-196b, hsa-miR-199a-AS, hsa-miR-203, hsa-miR-21, hsa-miR-210, hsa-miR-216, hsa-miR-217, hsa-miR-221, hsa-miR-222, hsa-miR-224, hsa-miR-23a, hsa-miR-24, hsa-miR-25, hsa-miR-27a, hsa-miR-28, hsa-miR-29a, hsa-miR-29c, hsa-miR-30a-3p, hsa-miR-30a-5p, hsa-miR-30b, hsa-miR-30c, hsa-miR-30d, hsa-miR-30e-5p, hsa-miR-331, hsa-miR-368, hsa-miR-375, hsa-miR-377, hsa-miR-379, hsa-miR-93, hsa-miR-452, hsa-miR-494, hsa-miR-497 oder ambi-miR-7105.

6. Verfahren nach einem der Ansprüche 1 bis 5, außerdem umfassend das Messen eines Expressionsprofils von ein oder mehreren mRNAs, deren Expressionshöhe mit Pankreatitis oder PDAC korreliert ist, vorzugsweise wobei die gemessene mRNA ausgewählt ist aus der Gruppe, umfassend karzinoembryonales Antigen-verwandtes Zelladhäsionsmolekül 6-mRNA, Survivin-mRNA, Mucin-4-mRNA und Urokinase-Plasminogen-Aktivator-Rezeptor-mRNA, und optional wobei das Messen eines Expressionsprofils der ein oder mehreren mRNAs durch quantitative Reverse Transkriptase-PCR (qRT-PCR) erfolgt.

7. Verfahren nach einem der Ansprüche 1 bis 6, **dadurch gekennzeichnet, dass** die Probe eine Gewebe-, eine Blut-, eine Serum-, eine Plasma- oder eine Bauchspeicheldrüsensaftprobe ist, vorzugsweise wobei die Probe von dem Patienten ist und die normale Probe Pankreasproben sind, und wobei die Probe optional frisch, gefroren, fixiert oder eingebettet ist.

8. Verfahren nach einem der Ansprüche 1 bis 7, außerdem umfassend das Markieren der miRNA von der Probe, vorzugsweise außerdem umfassend das Hybridisieren der markierten miRNA an ein oder mehrere miRNA-Sonden, insbesondere wobei die miRNA-Sonden an einen Träger gekoppelt sind, vorzugsweise wobei der Träger Glas, Kunststoff, Metall oder Latex ist, und besonders bevorzugt wobei der Träger planar ist oder wobei der Träger ein Kügelchen ist.

9. Verfahren nach einem der Ansprüche 1 bis 8, **dadurch gekennzeichnet, dass** die Expression der miRNA durch einen Amplifikationsassay oder einen Hybridisationsassay bestimmt wird, vorzugsweise wobei der Amplifikationsassay ein quantitativer Amplifikationsassay, besonders bevorzugt quantitative RT-PCR, ist oder wobei vorzugsweise der Hybridisationsassay ein Array-Hybridisationsassay oder ein Lösungs-Hybridisationsassay ist.

10. Verfahren nach einem der Ansprüche 1 bis 9, **dadurch gekennzeichnet, dass** das Diagnostizieren das Screening auf Pankreatitis oder pankreatisches duktales Adenokarzinom (PDAC), Beurteilen der Prognose der Pankreatitis oder des pankreatischen duktalen Adenokarzinoms (PDAC), Stadienbestimmen einer Pankreatitis oder eines pankreatischen duktalen Adenokarzinoms (PDAC) oder Auswerten des Ansprechens der Pankreatitis oder des pankreatischen duktalen Adenokarzinoms (PDAC) auf Therapie umfasst.

11. miRNA-Molekül oder -Inhibitor zur Verwendung in der Behandlung von pankreatischem duktalem Adenokarzinom (PDAC) bei einem Individuum, umfassend das Verabreichen an das Individuum einer wirksamen Menge eines oder mehrerer synthetischer miRNA-Moleküle oder -Inhibitoren mit einem Nukleinsäure-Segment mit der Sequenz von hsa-miR-130b, vorzugsweise außerdem einer oder mehrerer von hsa-let-7a, hsa-let-7b, hsa-let-7c, hsa-let-7d, hsa-let-7f, hsa-let-7g, hsa-let-7i, hsa-miR-100, hsa-miR-101, hsa-miR-103, hsa-miR-106b, hsa-miR-107, hsa-miR-10a, hsa-miR-125a, hsa-miR-125b, hsa-miR-130a, hsa-miR-134, hsa-miR-140, hsa-miR-141, hsa-miR-143, hsa-miR-145, hsa-miR-146a, hsa-miR-148a, hsa-miR-148b, hsa-miR-150, hsa-miR-154, hsa-miR-155, hsa-miR-18a, hsa-miR-181b, hsa-miR-182, hsa-miR-186, hsa-miR-196a, hsa-miR-196b, hsa-miR-199a, hsa-miR-199a-AS, hsa-miR-19a, hsa-miR-19b, hsa-miR-200a, hsa-miR-200b, hsa-miR-200c, hsa-miR-203, hsa-miR-21, hsa-miR-210, hsa-miR-214, hsa-miR-216, hsa-miR-217, hsa-miR-221, hsa-miR-222, hsa-miR-223, hsa-miR-224, hsa-miR-23a, hsa-miR-24, hsa-miR-26a, hsa-miR-26b, hsa-miR-27a, hsa-miR-27b, hsa-miR-29c, hsa-miR-30a-3p, hsa-miR-30a-5p, hsa-miR-30b, hsa-miR-30c, hsa-miR-30d, hsa-miR-30e-3p, hsa-miR-30e-5p, hsa-miR-31, hsa-miR-331, hsa-miR-335, hsa-miR-365, hsa-miR-368, hsa-miR-374, hsa-miR-375, hsa-miR-376a, hsa-miR-377, hsa-miR-379, hsa-miR-429, hsa-miR-93, hsa-miR-95, hsa-miR-96, hsa-miR-99b, hsa-miR-452, hsa-miR-494, hsa-miR-497, miR-205 oder ambi-miR-7105.

12. miRNA-Molekül oder -Inhibitor zur Verwendung gemäß Anspruch 11, worin die weiteren
(a) ein oder mehreren miRNA-Inhibitoren die Nukleinsäuresequenz von hsa-let-7i, hsa-miR-100, hsa-miR-103, hsa-miR-106b, hsa-miR-107, hsa-miR-10a, hsa-miR-125a, hsa-miR-125b, hsa-miR-140, hsa-miR-143, hsa-miR-145, hsa-miR-146a, hsa-miR-150, hsa-miR-155, hsa-miR-18a, hsa-miR-181b, hsa-miR-196a, hsa-miR-196b, hsa-miR-199a, hsa-miR-199a-AS, hsa-miR-203, hsa-miR-21, hsa-miR-210, hsa-miR-214, hsa-miR-221, hsa-miR-222, hsa-miR-223, hsa-miR-224, hsa-miR-23a, hsa-miR-24, hsa-miR-27a, hsa-miR-31, hsa-miR-331, hsa-miR-93, hsa-miR-99b, hsa-miR-452, hsa-miR-497 oder ambi-miR-7105 aufweisen; und/oder
(b) ein oder mehreren miRNAs die Nukleinsäuresequenz von hsa-let-7a, hsa-let-7b, hsa-let-7c, hsa-let-7d, hsa-let-7e, hsa-let-7f, hsa-let-7g, hsa-miR-101, hsa-miR-130a, hsa-miR-130b, hsa-miR-134, hsa-miR-141, hsa-miR-148a, hsa-miR-148b, hsa-miR-154, hsa-miR-182, hsa-miR-186, hsa-miR-19a, hsa-miR-19b, hsa-miR-200a, hsa-miR-200b, hsa-miR-200c, hsa-miR-216, hsa-miR-217, hsa-miR-26a, hsa-miR-26b, hsa-miR-27b, hsa-miR-29c, hsa-miR-30a-3p, hsa-miR-30a-5p, hsa-miR-30b, hsa-miR-30c, hsa-miR-30d, hsa-miR-30e-3p, hsa-miR-30e-5p, hsa-miR-335, hsa-miR-365, hsa-miR-368, hsa-miR-374, hsa-miR-375, hsa-miR-376a, hsa-miR-377, hsa-miR-379, hsa-miR-429, hsa-miR-95, hsa-miR-96 oder hsa-miR-494 aufweisen.

13. miRNA-Molekül oder -Inhibitor zur Verwendung in der Behandlung von chronischer Pankreatitis bei einem Individuum, umfassend das Verabreichen an das Individuum einer wirksamen Menge eines oder mehrerer synthetischer miRNA-Moleküle oder miRNA-Inhibitoren, umfassend die Sequenz von miR-130b, vorzugsweise außerdem eines oder mehrere von hsa-let-7a, hsa-let-7b, hsa-let-7c, hsa-let-7d, hsa-let-7e, hsa-let-7f, hsa-let-7g, hsa-let-7i, hsa-miR-100, hsa-miR-101, hsa-miR-10a, hsa-miR-125a, hsa-miR-125b, hsa-miR-130a, hsa-miR-141, hsa-miR-143, hsa-miR-145, hsa-miR-146a, hsa-miR-148a, hsa-miR-148b, hsa-miR-150, hsa-miR-18a, hsa-miR-182, hsa-miR-186, hsa-miR-199a, hsa-miR-199a-AS, hsa-miR-19a, hsa-miR-19b, hsa-miR-200a, hsa-miR-200b, hsa-miR-200c, hsa-miR-203, hsa-miR-210, hsa-miR-214, hsa-miR-216, hsa-miR-217, hsa-miR-222, hsa-miR-223, hsa-miR-24, hsa-miR-25, hsa-miR-26a, hsa-miR-26b, hsa-miR-27b, hsa-miR-28, hsa-miR-29a, hsa-miR-29b, hsa-miR-29c, hsa-miR-30a-3p, hsa-miR-30a-5p, hsa-miR-30b, hsa-miR-30c, hsa-miR-30d, hsa-miR-30e-3p, hsa-miR-30e-5p, hsa-miR-31, hsa-miR-335, hsa-miR-365, hsa-miR-368, hsa-miR-374, hsa-miR-376a, hsa-miR-429, hsa-miR-95, hsa-miR-96, hsa-miR-98, hsa-miR-99a, hsa-miR-497 oder ambi-miR-7105.

14. miRNA-Molekül oder -Inhibitor zur Verwendung gemäß Anspruch 12, wobei die weiteren
(a) ein oder mehreren miRNA-Inhibitoren die Nukleinsäuresequenz von hsa-let-7i, hsa-miR-100, hsa-miR-10a, hsa-miR-125a, hsa-miR-125b, hsa-miR-143, hsa-miR-145, hsa-miR-146a, hsa-miR-150, hsa-miR-18a, hsa-miR-199a, hsa-miR-199a-AS, hsa-miR-210, hsa-miR-214, hsa-miR-222, hsa-miR-223, hsa-miR-24, hsa-miR-31, hsa-miR-99a oder hsa-miR-497 aufweisen; und/oder
(b) ein oder mehreren miRNAs die Nukleinsäuresequenz von hsa-let-7a, hsa-let-7b, hsa-let-7c, hsa-let-7d, hsa-let-7e, hsa-let-7f, hsa-let-7g, hsa-miR-101, hsa-miR-130a, hsa-miR-130b, hsa-miR-141, hsa-miR-148a, hsa-miR-148b, hsa-miR-182, hsa-miR-186, hsa-miR-19a, hsa-miR-19b, hsa-miR-200a, hsa-miR-200b, hsa-miR-200c, hsa-miR-203, hsa-miR-216, hsa-miR-217, hsa-miR-25, hsa-miR-26a, hsa-miR-26b, hsa-miR-27b, hsa-miR-28, hsa-miR-29a, hsa-miR-29b, hsa-miR-29c, hsa-miR-30a-3p, hsa-miR-30a-5p, hsa-miR-30b, hsa-miR-30c, hsa-miR-30d, hsa-miR-30e-3p, hsa-miR-30e-5p, hsa-miR-335, hsa-miR-365, hsa-miR-368, hsa-miR-374, hsa-miR-376a, hsa-miR-429, hsa-miR-95, hsa-miR-96, hsa-miR-98 oder ambi-miR-7105 aufweisen.

15. Verwendung eines Kits in der Analyse einer pathologischen Probe, durch Auswerten eines miRNA-Profils für die Probe bei einem Verfahren gemäß einem der Ansprüche 1-10, wobei der Kit, in einem geeigneten Behältnis, zwei oder mehrere miRNA-Sonden umfasst, wobei die miRNA-Sonden hsa-miR-130b detektieren, und außerdem eines oder mehrere von hsa-let-7a, hsa-let-7b, hsa-let-7c, hsa-let-7d, hsa-let-7f, hsa-let-7g, hsa-let-7i, hsa-miR-100, hsa-miR-101, hsa-miR-103, hsa-miR-106b, hsa-miR-107, hsa-miR-10a, hsa-miR-125a, hsa-miR-125b, hsa-miR-130a, hsa-miR-130b, hsa-miR-134, hsa-miR-140, hsa-miR-141, hsa-miR-143, hsa-miR-145, hsa-miR-146a, hsa-miR-148a, hsa-miR-148b, hsa-miR-150, hsa-miR-154, hsa-miR-155, hsa-miR-18a, hsa-miR-181b, hsa-miR-182, hsa-miR-186, hsa-miR-196a, hsa-miR-196b, hsa-miR-199a, hsa-miR-199a-AS, hsa-miR-19a, hsa-miR-19b, hsa-miR-200a, hsa-miR-200b, hsa-miR-200c, hsa-miR-203, hsa-miR-21, hsa-miR-210, hsa-miR-214, hsa-miR-216, hsa-miR-217, hsa-miR-221, hsa-miR-222, hsa-miR-223, hsa-miR-224, hsa-miR-23a, hsa-miR-24, hsa-miR-26a, hsa-miR-26b, hsa-miR-27a, hsa-miR-27b, hsa-miR-29c, hsa-miR-30a-3p, hsa-miR-30a-5p, hsa-miR-30c, hsa-miR-30d, hsa-miR-30e-3p, hsa-miR-30e-5p, hsa-miR-31, hsa-miR-331, hsa-miR-335, hsa-miR-365, hsa-miR-368, hsa-miR-374, hsa-miR-375, hsa-miR-376a, hsa-miR-377, hsa-miR-379, hsa-miR-429, hsa-miR-93, hsa-miR-95, hsa-miR-96, hsa-miR-99b, hsa-miR-452, hsa-miR-494, hsa-miR-497, miR-205 oder ambi-miR-7105.

## Revendications

1. Procédé de diagnostic d'une pancréatite ou d'un adénocarcinome canalaire pancréatique (ACCP) chez un patient comprenant la mesure d'un profil d'expression d'un miARN dans un échantillon pancréatique obtenu du patient, dans lequel une diminution dans le profil d'expression dans l'échantillon provenant du patient comparativement à un profil d'expression de référence d'un échantillon normal indique une pancréatite ou un adénocarcinome canalaire pancréatique (ACCP) ; dans lequel le miARN est hsa-miR-130b.

2. Procédé selon la revendication 1 comprenant en outre la mesure d'un profil d'expression d'un ou de plusieurs miARN dans un échantillon provenant du patient, dans lequel une différence dans le profil d'expression dans l'échantillon provenant du patient et un profil d'expression de référence indique une pancréatite ou un adénocarcinome canalaire pancréatique (ACCP) ; dans lequel le miARN est : hsa-let-7a, hsa-let-7b, hsa-let-7c, hsa-let-7d, hsa-let-7e, hsa-let-7f, hsa-let-7g, hsa-let-7i, hsa-miR-100, hsa-miR-101, hsa-miR-103, hsa-miR-106a, hsa-miR-106b, hsa-miR-107, hsa-iR-10a, hsa-miR-125a, hsa-miR-125b, hsa-miR-130a, hsa-miR-134, hsa-miR-140, hsa-miR-141, hsa-miR-143, hsa-miR-145, hsa-miR-146a, hsa-miR-148a, hsa-miR-148b, hsa-miR-150, hsa-miR-154, hsa-miR-155, hsa-miR-15b, hsa-miR-17-5p, hsa-miR-18a, hsa-miR-181b, hsa-miR-182, hsa-miR-186, hsa-miR-196a, hsa-miR-196b, hsa-miR-199a, hsa-miR-199a-AS, hsa-miR-19a, hsa-miR-19b, hsa-miR-200a, hsa-miR-200b, hsa-miR-200c, hsa-miR-203, hsa-miR-21, hsa-miR-210, hsa-miR-214, hsa-miR-216, hsa-miR-217, hsa-miR-221, hsa-miR-222, hsa-miR-223, hsa-miR-224, hsa-miR-23a, hsa-miR-24, hsa-miR-25, hsa-miR-26a, hsa-miR-26b, hsa-miR-27a, hsa-miR-27b, hsa-miR-28, hsa-miR-29a, hsa-miR-29b, hsa-miR-29c, hsa-miR-30a-3p, hsa-miR-30a-5p, hsa-miR-30b, hsa-miR-30c, hsa-miR-30d, hsa-miR-30e-3p, hsa-miR-30e-5p, hsa-miR-31, hsa-miR-331, hsa-miR-335, hsa-miR-365, hsa-miR-368, hsa-miR-374, hsa-miR-375, hsa-miR-376a, hsa-miR-377, hsa-miR-379, hsa-miR-429, hsa-miR-93, hsa-miR-95, hsa-miR-96, hsa-miR-98, hsa-miR-99a, hsa-miR-99b, hsa-miR-452, hsa-miR-494, hsa-miR-497, miR-205, ou ambi-miR-7105, de préférence où le miARN est un ou plusieurs de miR-205, miR-29c, miR-216, miR-217, miR-375, miR-143, miR-145, miR-146a, miR-148a, miR-196b, miR-93, miR-96, miR-31, miR-210, miR-148b, miR-196a, miR-141, miR-18a, miR-203, miR-150, miR-155, miR-130b, miR-221, miR-222, miR-223, ou miR-224, en particulier préféré où le miARN est miR-196a, miR-217, ou à la fois miR-196a et miR-217.

3. Procédé selon la revendication 1 ou 2, **caractérisé en ce que** l'affection pathologique est la pancréatite et
• l'expression différentielle d'un ou de plusieurs miARN indique une pancréatite, le miARN étant miR-130b, de préférence en outre un ou plusieurs de hsa-let-7a, hsa-let-7b, hsa-let-7c, hsa-let-7d, hsa-let-7e, hsa-let-7f, hsa-let-7g, hsa-let-7i, hsa-miR-100, hsa-miR-101, hsa-miR-10a, hsa-miR-125a, hsa-miR-125b, hsa-miR-130a, hsa-miR-141, hsa-miR-143, hsa-miR-145, hsa-miR-146a, hsa-miR-148a, hsa-miR-148b, hsa-miR-150, hsa-miR-18a, hsa-miR-182, hsa-miR-186, hsa-miR-199a hsa-miR-199a-AS, hsa-miR-19a, hsa-miR-19b, hsa-miR-200a, hsa-miR-200b, hsa-miR-200c, hsa-miR-203, hsa-miR-210, hsa-miR-214, hsa-miR-216, hsa-miR-217, hsa-miR-222, hsa-miR-223, hsa-miR-24, hsa-miR-25, hsa-miR-26a, hsa-miR-26b, hsa-miR-27b, hsa-miR-28, hsa-miR-29a, hsa-miR-29b, hsa-miR-29c, hsa-miR-30a-3p, hsa-miR-30a-5p, hsa-miR-30b, hsa-miR-30c, hsa-miR-30d, hsa-miR-30e-3p, hsa-miR-30e-5p, hsa-miR-31, hsa-miR-335, hsa-miR-365, hsa-miR-368, hsa-miR-374, hsa-miR-376a, hsa-miR-429, hsa-miR-95, hsa-miR-96, hsa-miR-98, hsa-miR-99a, hsa-miR-497, ou ambi-miR-7105, ou
• dans lequel une diminution de l'expression d'un ou de plusieurs miARN dans un échantillon de patient indique une pancréatite, le miARN étant miR-130b, de préférence en outre un ou plusieurs de hsa-let-7a, hsa-let-7b, hsa-let-7c, hsa-let-7d, hsa-let-7e, hsa-let-7f, hsa-let-7g, hsa-miR-101, hsa-miR-130a, hsa-miR-141, hsa-miR-148a, hsa-miR-148b, hsa-miR-182, hsa-miR-186, hsa-miR-19a, hsa-miR-19b, hsa-miR-200a, hsa-miR-200b, hsa-miR-200c, hsa-miR-203, hsa-miR-216, hsa-miR-217, hsa-miR-25, hsa-miR-26a, hsa-miR-26b, hsa-miR-27b, hsa-miR-28, hsa-miR-29a, hsa-miR-29b, hsa-miR-29c, hsa-miR-30a-3p, hsa-miR-30a-5p, hsa-miR-30b, hsa-miR-30c, hsa-miR-30d, hsa-miR-30e-3p, hsa-miR-30e-5p, hsa-miR-335, hsa-miR-365, hsa-miR-368, hsa-miR-374, hsa-miR-376a, hsa-miR-429, hsa-miR-95, hsa-miR-96, hsa-miR-98, ou ambi-miR-7105.

4. Procédé selon la revendication 1 ou 2, **caractérisé en ce que** l'affection pathologique est l'adénocarcinome canalaire pancréatique (ACCP) et
• l'expression différentielle d'un ou de plusieurs miARN indique un ACCP, le miARN étant miR-130b, de préférence en outre un ou plusieurs de hsa-let-7a, hsa-let-7b, hsa-let-7c, hsa-let-7d, hsa-let-7f, hsa-let-7g, hsa-let-7i, hsa-miR-100, hsa-miR-101, hsa-miR-103, hsa-miR-106b, hsa-miR-107, hsa-iR-10a, hsa-miR-125a, hsa-miR-125b, hsa-miR-130a, hsa-miR-134, hsa-miR-140, hsa-miR-141, hsa-miR-143, hsa-miR-145, hsa-miR-146a, hsa-miR-148a, hsa-miR-148b, hsa-miR-150, hsa-miR-154, hsa-miR-155, hsa-miR-18a, hsa-miR-181b, hsa-miR-182, hsa-miR-186, hsa-miR-196a, hsa-miR-196b, hsa-miR-199a, hsa-miR-199a-AS, hsa-miR-19a, hsa-miR-19b, hsa-miR-200a, hsa-miR-200b, hsa-miR-200c, hsa-miR-203, hsa-miR-21, hsa-miR-210, hsa-miR-214, hsa-miR-216, hsa-miR-217, hsa-miR-221, hsa-miR-222, hsa-miR-223, hsa-miR-224, hsa-miR-23a, hsa-miR-24, hsa-miR-26a, hsa-miR-26b, hsa-miR-27a, hsa-miR-27b, hsa-miR-29c, hsa-miR-30a-3p, hsa-miR-30a-5p, hsa-miR-30b, hsa-miR-30c, hsa-miR-30d, hsa-miR-30e-3p, hsa-miR-30e-5p, hsa-miR-31, hsa-miR-331, hsa-miR-335, hsa-miR-365, hsa-miR-368, hsa-miR-374, hsa-miR-375, hsa-miR-376a, hsa-miR-377, hsa-miR-379, hsa-miR-429, hsa-miR-93, hsa-miR-95, hsa-miR-96, hsa-miR-99b, hsa-miR-452, hsa-miR-494, hsa-miR-497, miR-205, ou ambi-miR-7105, ou
• dans lequel une diminution de l'expression d'un ou de plusieurs miARN indique un ACCP, le miARN étant miR-130b, de préférence en outre un ou plusieurs de hsa-let-7a, hsa-let-7b, hsa-let-7c, hsa-let-7d, hsa-let-7e, hsa-let-7f, hsa-let-7g, hsa-miR-101, hsa-miR-130a, hsa-miR-134, hsa-miR-141, hsa-miR-148a, hsa-miR-148b, hsa-miR-154, hsa-miR-182, hsa-miR-186, hsa-miR-19a, hsa-miR-19b, hsa-miR-200a, hsa-miR-200b, hsa-miR-200c, hsa-miR-216, hsa-miR-217, hsa-miR-26a, hsa-miR-26b, hsa-miR-27b, hsa-miR-29c, hsa-miR-30a-3p, hsa-miR-30a-5p, hsa-miR-30b, hsa-miR-30c, hsa-miR-30d, hsa-miR-30e-3p, hsa-miR-30e-5p, hsa-miR-335, hsa-miR-365, hsa-miR-368, hsa-miR-374, hsa-miR-375, hsa-miR-376a, hsa-miR-377, hsa-miR-379, hsa-miR-429, hsa-miR-95, hsa-miR-96, ou hsa-miR-494.

5. Procédé selon la revendication 1 ou 2, **caractérisé en ce que** la pancréatite se distingue de l'ACCP par l'expression différentielle d'un ou de plusieurs de hsa-let-7b, hsa-let-7e, hsa-miR-103, hsa-miR-106a, hsa-miR-106b, hsa-miR-107, hsa-miR-125b, hsa-miR-130a, hsa-miR-130b, hsa-miR-141, hsa-miR-146a, hsa-miR-148a, hsa-miR-154, hsa-miR-155, hsa-miR-15b, hsa-miR-17-5p, hsa-miR-18a, hsa-miR-181b, hsa-miR-196a, hsa-miR-196b, hsa-miR-199a-AS, hsa-miR-203, hsa-miR-21, hsa-miR-210, hsa-miR-216, hsa-miR-217, hsa-miR-221, hsa-miR-222, hsa-miR-224, hsa-miR-23a, hsa-miR-24, hsa-miR-25, hsa-miR-27a, hsa-miR-28, hsa-miR-29a, hsa-miR-29c, hsa-miR-30a-3p, hsa-miR-30a-5p, hsa-miR-30b, hsa-miR-30c, hsa-miR-30d, hsa-miR-30e-5p, hsa-miR-331, hsa-miR-368, hsa-miR-375, hsa-miR-377, hsa-miR-379, hsa-miR-93, hsa-miR-452, hsa-miR-494, hsa-miR-497, ou ambi-miR-7105.

6. Procédé selon l'une quelconque des revendications 1 à 5, comprenant en outre la mesure d'un profil d'expression d'un ou de plusieurs ARNm dont le taux d'expression est en corrélation avec une pancréatite ou un ACCP, de préférence dans lequel l'ARNm mesuré est choisi dans le groupe comprenant l'ARNm de la molécule d'adhérence cellulaire 6 associée à l'antigène carcinoembryonnaire, l'ARNm de la survivine, l'ARNm de la mucine 4, et l'ARNm du récepteur de l'activateur du plasminogène de type urokinase, et éventuellement dans lequel la mesure d'un profil d'expression d'un ou de plusieurs ARNm est réalisée par transcription inverse-PCR quantitative (qRT-PCR).

7. Procédé selon l'une quelconque des revendications 1 à 6, **caractérisé en ce que** l'échantillon est un tissu, un sang, un plasma, ou un échantillon de suc pancréatique, de préférence dans lequel l'échantillon provenant du patient et l'échantillon normal sont des échantillons pancréatiques, et dans lequel l'échantillon est éventuellement frais, congelé, fixé, ou incorporé.

8. Procédé selon l'une quelconque des revendications 1 à 7, comprenant en outre le marquage de miARN provenant de l'échantillon, comprenant en outre de préférence l'hybridation du miARN marqué à une ou plusieurs sondes de miARN, en particulier dans lequel les sondes de miARN sont couplées à un support, de préférence dans lequel le support est du verre, du plastique, du métal, ou du latex, et en particulier préféré dans lequel le support est planaire, ou dans lequel le support est une bille.

9. Procédé selon l'une quelconque des revendications 1 à 8, **caractérisé en ce que** l'expression du miARN est déterminée par un test d'amplification ou un test d'hybridation, de préférence dans lequel le test d'amplification est un test d'amplification quantitative, en particulier préféré une RT-PCR quantitative, ou dans lequel de préférence le test d'hybridation est un test d'hybridation sur puce ou un test d'hybridation en solution.

10. Procédé selon l'une quelconque des revendications 1 à 9, **caractérisé en ce que** le diagnostic comprend le criblage pour la pancréatite ou l'adénocarcinome canalaire pancréatique (ACCP), l'estimation du pronostic d'une pancréatite ou d'un adénocarcinome canalaire pancréatique (ACCP), la détermination du stade d'une pancréatite ou d'un adénocarcinome canalaire pancréatique (ACCP), ou l'estimation de la réponse de la pancréatite ou de l'adénocarcinome canalaire pancréatique (ACCP) au traitement.

11. Molécule ou inhibiteur de miARN pour une utilisation dans le traitement de l'adénocarcinome canalaire pancréatique chez un sujet comprenant l'administration au sujet d'une quantité efficace d'une ou de plusieurs molécules ou d'un ou de plusieurs inhibiteurs de miARN de synthèse comportant un segment d'acide nucléique ayant la séquence de miR-130b, de préférence en outre un ou plusieurs de hsa-let-7a, hsa-let-7b, hsa-let-7c, hsa-let-7d, hsa-let-7f, hsa-let-7g, hsa-let-7i, hsa-miR-100, hsa-miR-101, hsa-miR-103, hsa-miR-106b, hsa-miR-107, hsa-iR-10a, hsa-miR-125a, hsa-miR-125b, hsa-miR-130a, hsa-miR-134, hsa-miR-140, hsa-miR-141, hsa-miR-143, hsa-miR-145, hsa-miR-146a, hsa-miR-148a, hsa-miR-148b, hsa-miR-150, hsa-miR-154, hsa-miR-155, hsa-miR-18a, hsa-miR-181b, hsa-miR-182, hsa-miR-186, hsa-miR-196a, hsa-miR-196b, hsa-miR-199a, hsa-miR-199a-AS, hsa-miR-19a, hsa-miR-19b, hsa-miR-200a, hsa-miR-200b, hsa-miR-200c, hsa-miR-203, hsa-miR-21, hsa-miR-210, hsa-miR-214, hsa-miR-216, hsa-miR-217, hsa-miR-221, hsa-miR-222, hsa-miR-223, hsa-miR-224, hsa-miR-23a, hsa-miR-24, hsa-miR-26a, hsa-miR-26b, hsa-miR-27a, hsa-miR-27b, hsa-miR-29c, hsa-miR-30a-3p, hsa-miR-30a-5p, hsa-miR-30b, hsa-miR-30c, hsa-miR-30d, hsa-miR-30e-3p, hsa-miR-30e-5p, hsa-miR-31, hsa-miR-331, hsa-miR-335, hsa-miR-365, hsa-miR-368, hsa-miR-374, hsa-miR-375, hsa-miR-376a, hsa-miR-377, hsa-miR-379, hsa-miR-429, hsa-miR-93, hsa-miR-95, hsa-miR-96, hsa-miR-99b, hsa-miR-452, hsa-miR-494, hsa-miR-497, miR-205, ou ambi-miR-7105.

12. Molécule ou inhibiteur de miARN pour une utilisation selon la revendication 11, où les autres
(a) un ou plusieurs inhibiteurs de miARN ont la séquence d'acide nucléique de hsa-let-7i, hsa-miR-100, hsa-miR-103, hsa-miR-106b, hsa-miR-107, hsa-miR-10a, hsa-miR-125a, hsa-miR-125b, hsa-miR-140, hsa-miR-143, hsa-miR-145, hsa-miR-146a, hsa-miR-150, hsa-miR-155, hsa-miR-18a, hsa-miR-181b, hsa-miR-196a, hsa-miR-196b, hsa-miR-199a, hsa-miR-199a-AS, hsa-miR-203, hsa-miR-21, hsa-miR-210, hsa-miR-214, hsa-miR-221, hsa-miR-222, hsa-miR-223, hsa-miR-224, hsa-miR-23a, hsa-miR-24, hsa-miR-27a, hsa-miR-31, hsa-miR-331, hsa-miR-93, hsa-miR-99b, hsa-miR-452, hsa-miR-497, ou ambi-miR-7105 ; et/ou
(b) un ou plusieurs miARN ont la séquence d'acide nucléique de hsa-let-7a, hsa-let-7b, hsa-let-7c, hsa-let-7d, hsa-let-7e, hsa-let-7f, hsa-let-7g, hsa-miR-101, hsa-miR-130a, hsa-miR-130b, hsa-miR-134, hsa-miR-141, hsa-miR-148a, hsa-miR-148b, hsa-miR-154, hsa-miR-182, hsa-miR-186, hsa-miR-19a, hsa-miR-19b, hsa-miR-200a, hsa-miR-200b, hsa-miR-200c, hsa-miR-216, hsa-miR-217, hsa-miR-26a, hsa-miR-26b, hsa-miR-27b, hsa-miR-29c, hsa-miR-30a-3p, hsa-miR-30a-5p, hsa-miR-30b, hsa-miR-30c, hsa-miR-30d, hsa-miR-30e-3p, hsa-miR-30e-5p, hsa-miR-335, hsa-miR-365, hsa-miR-368, hsa-miR-374, hsa-miR-375, hsa-miR-376a, hsa-miR-377, hsa-miR-379, hsa-miR-429, hsa-miR-95, hsa-miR-96, ou hsa-miR-494.

13. Molécule ou inhibiteur de miARN pour une utilisation dans le traitement de la pancréatite chronique chez un sujet comprenant l'administration au sujet d'une quantité efficace d'une ou de plusieurs molécules de miARN ou d'un ou de plusieurs inhibiteurs de miARN de synthèse comprenant la séquence de miR-130b, de préférence en outre un ou plusieurs de hsa-let-7a, hsa-let-7b, hsa-let-7c, hsa-let-7d, hsa-let-7e, hsa-let-7f, hsa-let-7g, hsa-let-7i, hsa-miR-100, hsa-miR-101, hsa-miR-10a, hsa-miR-125a, hsa-miR-125b, hsa-miR-130a, hsa-miR-141, hsa-miR-143, hsa-miR-145, hsa-miR-146a, hsa-miR-148a, hsa-miR-148b, hsa-miR-150, hsa-miR-18a, hsa-miR-182, hsa-miR-186, hsa-miR-199a hsa-miR-199a-AS, hsa-miR-19a, hsa-miR-19b, hsa-miR-200a, hsa-miR-200b, hsa-miR-200c, hsa-miR-203, hsa-miR-210, hsa-miR-214, hsa-miR-216, hsa-miR-217, hsa-miR-222, hsa-miR-223, hsa-miR-24, hsa-miR-25, hsa-miR-26a, hsa-miR-26b, hsa-miR-27b, hsa-miR-28, hsa-miR-29a, hsa-miR-29b, hsa-miR-29c, hsa-miR-30a-3p, hsa-miR-30a-5p, hsa-miR-30b, hsa-miR-30c, hsa-miR-30d, hsa-miR-30e-3p, hsa-miR-30e-5p, hsa-miR-31, hsa-miR-335, hsa-miR-365, hsa-miR-368, hsa-miR-374, hsa-miR-376a, hsa-miR-429, hsa-miR-95, hsa-miR-96, hsa-miR-98, hsa-miR-99a, hsa-miR-497, ou ambi-miR-7105.

14. Molécule ou inhibiteur de miARN pour une utilisation selon la revendication 12, où les autres
(a) un ou plusieurs inhibiteurs de miARN ont la séquence d'acide nucléique de hsa-let-7i, hsa-miR-100, hsa-iR-10a, hsa-miR-125a, hsa-miR-125b, hsa-miR-143, hsa-miR-145, hsa-miR-146a, hsa-miR-150, hsa-miR-18a, hsa-miR-199a, hsa-miR-199a-AS, hsa-miR-210, hsa-miR-214, hsa-miR-222, hsa-miR-223, hsa-miR-24, hsa-miR-31, hsa-miR-99a, ou hsa-miR-497 ; et/ou
(b) un ou plusieurs miARN ont la séquence d'acide nucléique de hsa-let-7a, hsa-let-7b, hsa-let-7c, hsa-let-7d, hsa-let-7e, hsa-let-7f, hsa-let-7g, hsa-miR-101, hsa-miR-130a, hsa-miR-130b, hsa-miR-141, hsa-miR-148a, hsa-miR-148b, hsa-miR-182, hsa-miR-186, hsa-miR-19a, hsa-miR-19b, hsa-miR-200a, hsa-miR-200b, hsa-miR-200c, hsa-miR-203, hsa-miR-216, hsa-miR-217, hsa-miR-25, hsa-miR-26a, hsa-miR-26b, hsa-miR-27b, hsa-miR-28, hsa-miR-29a, hsa-miR-29b, hsa-miR-29c, hsa-miR-30a-3p, hsa-miR-30a-5p, hsa-miR-30b, hsa-miR-30c, hsa-miR-30d, hsa-miR-30e-3p, hsa-miR-30e-5p, hsa-miR-335, hsa-miR-365, hsa-miR-368, hsa-miR-374, hsa-miR-376a, hsa-miR-429, hsa-miR-95, hsa-miR-96, hsa-miR-98, ou ambi-miR-7105.

15. Utilisation d'un kit dans l'analyse d'un échantillon pathologique, par estimation d'un profil de miARN pour l'échantillon dans un procédé selon l'une quelconque des revendications 1 à 10, le kit comprenant, dans un récipient approprié, deux sondes de miARN ou plus, dans lequel les sondes de miARN détectent miR-130b, et en outre un ou plusieurs de hsa-let-7a, hsa-let-7b, hsa-let-7c, hsa-let-7d, hsa-let-7e, hsa-let-7f, hsa-let-7g, hsa-let-7i, hsa-miR-100, hsa-miR-101, hsa-miR-103, hsa-miR-106b, hsa-miR-107, hsa-iR-10a, hsa-miR-125a, hsa-miR-125b, hsa-miR-130a, hsa-miR-130b, hsa-miR-134, hsa-miR-140, hsa-miR-141, hsa-miR-143, hsa-miR-145, hsa-miR-146a, hsa-miR-148a, hsa-miR-148b, hsa-miR-150, hsa-miR-154, hsa-miR-155, hsa-miR-18a, hsa-miR-181b, hsa-miR-182, hsa-miR-186, hsa-miR-196a, hsa-miR-196b, hsa-miR-199a, hsa-miR-199a-AS, hsa-miR-19a, hsa-miR-19b, hsa-miR-200a, hsa-miR-200b, hsa-miR-200c, hsa-miR-203, hsa-miR-21, hsa-miR-210, hsa-miR-214, hsa-miR-216, hsa-miR-217, hsa-miR-221, hsa-miR-222, hsa-miR-223, hsa-miR-224, hsa-miR-23a, hsa-miR-24, hsa-miR-26a, hsa-miR-26b, hsa-miR-27a, hsa-miR-27b, hsa-miR-29c, hsa-miR-30a-3p, hsa-miR-30a-5p, hsa-miR-30c, hsa-miR-30d, hsa-miR-30e-3p, hsa-miR-30e-5p, hsa-miR-31, hsa-miR-331, hsa-miR-335, hsa-miR-365, hsa-miR-368, hsa-miR-374, hsa-miR-375, hsa-miR-376a, hsa-miR-377, hsa-miR-379, hsa-miR-429, hsa-miR-93, hsa-miR-95, hsa-miR-96, hsa-miR-99b, hsa-miR-452, hsa-miR-494, hsa-miR-497, miR-205, ou ambi-miR-7105.


### SEQUENCE LISTING

<110> ASURAGEN, INC.
<120> MICRORNAS DIFFERENTIALLY EXPRESSED IN PANCREATIC DISEASES AND USES THEREOF
<130> R 61479
<140> UNKNOWN
   <141> 2007-09-19
<150> EP 07842816.6
   <151> 2007-09-19
<150> 60/826,173
   <151> 2006-09-19
<160> 350
<170> PatentIn version 3.3
<210> 1
   <211> 22
   <212> RNA
   <213> Homo sapiens
<400> 1
   ugagguagua gguuguauag uu 22
<210> 2
   <211> 22
   <212> RNA
   <213> Homo sapiens
<400> 2
   ugagguagua gguugugugg uu 22
<210> 3
   <211> 22
   <212> RNA
   <213> Homo sapiens
<400> 3
   ugagguagua gguuguaugg uu 22
<210> 4
   <211> 21
   <212> RNA
   <213> Homo sapiens
<400> 4
   agagguagua gguugcauag u 21
<210> 5
   <211> 21
   <212> RNA
   <213> Homo sapiens
<400> 5
   ugagguagga gguuguauag u 21
<210> 6
   <211> 22
   <212> RNA
   <213> Homo sapiens
<400> 6
   ugagguagua gauuguauag uu 22
<210> 7
   <211> 21
   <212> RNA
   <213> Homo sapiens
<400> 7
   ugagguagua guuuguacag u 21
<210> 8
   <211> 21
   <212> RNA
   <213> Homo sapiens
<400> 8
   ugagguagua guuugugcug u 21
<210> 9
   <211> 21
   <212> RNA
   <213> Homo sapiens
<400> 9
   uggaauguaa agaaguaugu a 21
<210> 10
   <211> 22
   <212> RNA
   <213> Homo sapiens
<400> 10
   aacccguaga uccgaacuug ug 22
<210> 11
   <211> 22
   <212> RNA
   <213> Homo sapiens
<400> 11
   uacaguacug ugauaacuga ag 22
<210> 12
   <211> 23
   <212> RNA
   <213> Homo sapiens
<400> 12
   agcagcauug uacagggcua uga 23
<210> 13
   <211> 20
   <212> RNA
   <213> Homo sapiens
<400> 13
   ucaaaugcuc agacuccugu 20
<210> 14
   <211> 24
   <212> RNA
   <213> Homo sapiens
<400> 14
   aaaagugcuu acagugcagg uagc 24
<210> 15
   <211> 21
   <212> RNA
   <213> Homo sapiens
<400> 15
   uaaagugcug acagugcaga u 21
<210> 16
   <211> 23
   <212> RNA
   <213> Homo sapiens
<400> 16
   agcagcauug uacagggcua uca 23
<210> 17
   <211> 23
   <212> RNA
   <213> Homo sapiens
<400> 17
   uacccuguag auccgaauuu gug 23
<210> 18
   <211> 22
   <212> RNA
   <213> Homo sapiens
<400> 18
   uacccuguag aaccgaauuu gu 22
<210> 19
   <211> 23
   <212> RNA
   <213> Homo sapiens
<400> 19
   uggaguguga caaugguguu ugu 23
<210> 20
   <211> 22
   <212> RNA
   <213> Homo sapiens
<400> 20
   uuaaggcacg cggugaaugc ca 22
<210> 21
   <211> 23
   <212> RNA
   <213> Homo sapiens
<400> 21
   ucccugagac ccuuuaaccu gug 23
<210> 22
   <211> 22
   <212> RNA
   <213> Homo sapiens
<400> 22
   ucccugagac ccuaacuugu ga 22
<210> 23
   <211> 21
   <212> RNA
   <213> Homo sapiens
<400> 23
   ucguaccgug aguaauaaug c 21
<210> 24
   <211> 21
   <212> RNA
   <213> Homo sapiens
<400> 24
   cauuauuacu uuugguacgc g 21
<210> 25
   <211> 22
   <212> RNA
   <213> Homo sapiens
<400> 25
   ucggauccgu cugagcuugg cu 22
<210> 26
   <211> 22
   <212> RNA
   <213> Homo sapiens
<400> 26
   ucacagugaa ccggucucuu uu 22
<210> 27
   <211> 21
   <212> RNA
   <213> Homo sapiens
<400> 27
   cuuuuugcgg ucugggcuug c 21
<210> 28
   <211> 22
   <212> RNA
   <213> Homo sapiens
<400> 28
   cagugcaaug uuaaaagggc au 22
<210> 29
   <211> 22
   <212> RNA
   <213> Homo sapiens
<400> 29
   cagugcaaug augaaagggc au 22
<210> 30
   <211> 22
   <212> RNA
   <213> Homo sapiens
<400> 30
   uaacagucua cagccauggu cg 22
<210> 31
   <211> 22
   <212> RNA
   <213> Homo sapiens
<400> 31
   uugguccccu ucaaccagcu gu 22
<210> 32
   <211> 21
   <212> RNA
   <213> Homo sapiens
<400> 32
   ugugacuggu ugaccagagg g 21
<210> 33
   <211> 23
   <212> RNA
   <213> Homo sapiens
<400> 33
   uauggcuuuu uauuccuaug uga 23
<210> 34
   <211> 22
   <212> RNA
   <213> Homo sapiens
<400> 34
   uauggcuuuu cauuccuaug ug 22
<210> 35
   <211> 23
   <212> RNA
   <213> Homo sapiens
<400> 35
   acuccauuug uuuugaugau gga 23
<210> 36
   <211> 22
   <212> RNA
   <213> Homo sapiens
<400> 36
   uauugcuuaa gaauacgcgu ag 22
<210> 37
   <211> 17
   <212> RNA
   <213> Homo sapiens
<400> 37
   agcugguguu gugaauc 17
<210> 38
   <211> 18
   <212> RNA
   <213> Homo sapiens
<400> 38
   ucuacagugc acgugucu 18
<210> 39
   <211> 21
   <212> RNA
   <213> Homo sapiens
<400> 39
   agugguuuua cccuauggua g 21
<210> 40
   <211> 22
   <212> RNA
   <213> Homo sapiens
<400> 40
   uaacacuguc ugguaaagau gg 22
<210> 41
   <211> 23
   <212> RNA
   <213> Homo sapiens
<400> 41
   uguaguguuu ccuacuuuau gga 23
<210> 42
   <211> 20
   <212> RNA
   <213> Homo sapiens
<400> 42
   cauaaaguag aaagcacuac 20
<210> 43
   <211> 22
   <212> RNA
   <213> Homo sapiens
<400> 43
   ugagaugaag cacuguagcu ca 22
<210> 44
   <211> 22
   <212> RNA
   <213> Homo sapiens
<400> 44
   uacaguauag augauguacu ag 22
<210> 45
   <211> 24
   <212> RNA
   <213> Homo sapiens
<400> 45
   guccaguuuu cccaggaauc ccuu 24
<210> 46
   <211> 22
   <212> RNA
   <213> Homo sapiens
<400> 46
   ugagaacuga auuccauggg uu 22
<210> 47
   <211> 20
   <212> RNA
   <213> Homo sapiens
<400> 47
   guguguggaa augcuucugc 20
<210> 48
   <211> 22
   <212> RNA
   <213> Homo sapiens
<400> 48
   ucagugcacu acagaacuuu gu 22
<210> 49
   <211> 22
   <212> RNA
   <213> Homo sapiens
<400> 49
   ucagugcauc acagaacuuu gu 22
<210> 50
   <211> 22
   <212> RNA
   <213> Homo sapiens
<400> 50
   ucuggcuccg ugucuucacu cc 22
<210> 51
   <211> 22
   <212> RNA
   <213> Homo sapiens
<400> 51
   ucucccaacc cuuguaccag ug 22
<210> 52
   <211> 22
   <212> RNA
   <213> Homo sapiens
<400> 52
   acuagacuga agcuccuuga gg 22
<210> 53
   <211> 22
   <212> RNA
   <213> Homo sapiens
<400> 53
   ucagugcaug acagaacuug gg 22
<210> 54
   <211> 20
   <212> RNA
   <213> Homo sapiens
<400> 54
   uugcauaguc acaaaaguga 20
<210> 55
   <211> 22
   <212> RNA
   <213> Homo sapiens
<400> 55
   uagguuaucc guguugccuu cg 22
<210> 56
   <211> 22
   <212> RNA
   <213> Homo sapiens
<400> 56
   uuaaugcuaa ucgugauagg gg 22
<210> 57
   <211> 22
   <212> RNA
   <213> Homo sapiens
<400> 57
   uagcagcaca uaaugguuug ug 22
<210> 58
   <211> 22
   <212> RNA
   <213> Homo sapiens
<400> 58
   uagcagcaca ucaugguuua ca 22
<210> 59
   <211> 22
   <212> RNA
   <213> Homo sapiens
<400> 59
   uagcagcacg uaaauauugg cg 22
<210> 60
   <211> 20
   <212> RNA
   <213> Homo sapiens
<400> 60
   acugcaguga aggcacuugu 20
<210> 61
   <211> 24
   <212> RNA
   <213> Homo sapiens
<400> 61
   caaagugcuu acagugcagg uagu 24
<210> 62
   <211> 22
   <212> RNA
   <213> Homo sapiens
<400> 62
   uaaggugcau cuagugcaga ua 22
<210> 63
   <211> 23
   <212> RNA
   <213> Homo sapiens
<400> 63
   aacauucaac gcugucggug agu 23
<210> 64
   <211> 22
   <212> RNA
   <213> Homo sapiens
<400> 64
   aacauucauu gcugucggug gg 22
<210> 65
   <211> 22
   <212> RNA
   <213> Homo sapiens
<400> 65
   aacauucaac cugucgguga gu 22
<210> 66
   <211> 22
   <212> RNA
   <213> Homo sapiens
<400> 66
   uuuggcaaug guagaacuca ca 22
<210> 67
   <211> 21
   <212> RNA
   <213> Homo sapiens
<400> 67
   ugguucuaga cuugccaacu a 21
<210> 68
   <211> 23
   <212> RNA
   <213> Homo sapiens
<400> 68
   uauggcacug guagaauuca cug 23
<210> 69
   <211> 22
   <212> RNA
   <213> Homo sapiens
<400> 69
   uggacggaga acugauaagg gu 22
<210> 70
   <211> 18
   <212> RNA
   <213> Homo sapiens
<400> 70
   uggagagaaa ggcaguuc 18
<210> 71
   <211> 23
   <212> RNA
   <213> Homo sapiens
<400> 71
   caaagaauuc uccuuuuggg cuu 23
<210> 72
   <211> 21
   <212> RNA
   <213> Homo sapiens
<400> 72
   ucgugucuug uguugcagcc g 21
<210> 73
   <211> 22
   <212> RNA
   <213> Homo sapiens
<400> 73
   caucccuugc augguggagg gu 22
<210> 74
   <211> 23
   <212> RNA
   <213> Homo sapiens
<400> 74
   gugccuacug agcugauauc agu 23
<210> 75
   <211> 22
   <212> RNA
   <213> Homo sapiens
<400> 75
   ugauauguuu gauauauuag gu 22
<210> 76
   <211> 22
   <212> RNA
   <213> Homo sapiens
<400> 76
   caacggaauc ccaaaagcag cu 22
<210> 77
   <211> 21
   <212> RNA
   <213> Homo sapiens
<400> 77
   cugaccuaug aauugacagc c 21
<210> 78
   <211> 21
   <212> RNA
   <213> Homo sapiens
<400> 78
   aacuggccua caaaguccca g 21
<210> 79
   <211> 22
   <212> RNA
   <213> Homo sapiens
<400> 79
   uguaacagca acuccaugug ga 22
<210> 80
   <211> 21
   <212> RNA
   <213> Homo sapiens
<400> 80
   uagcagcaca gaaauauugg c 21
<210> 81
   <211> 21
   <212> RNA
   <213> Homo sapiens
<400> 81
   uagguaguuu cauguuguug g 21
<210> 82
   <211> 21
   <212> RNA
   <213> Homo sapiens
<400> 82
   uagguaguuu ccuguuguug g 21
<210> 83
   <211> 22
   <212> RNA
   <213> Homo sapiens
<400> 83
   uucaccaccu ucuccaccca gc 22
<210> 84
   <211> 19
   <212> RNA
   <213> Homo sapiens
<400> 84
   gguccagagg ggagauagg 19
<210> 85
   <211> 23
   <212> RNA
   <213> Homo sapiens
<400> 85
   cccaguguuc agacuaccug uuc 23
<210> 86
   <211> 22
   <212> RNA
   <213> Homo sapiens
<400> 86
   uacaguaguc ugcacauugg uu 22
<210> 87
   <211> 23
   <212> RNA
   <213> Homo sapiens
<400> 87
   cccaguguuu agacuaucug uuc 23
<210> 88
   <211> 23
   <212> RNA
   <213> Homo sapiens
<400> 88
   ugugcaaauc uaugcaaaac uga 23
<210> 89
   <211> 23
   <212> RNA
   <213> Homo sapiens
<400> 89
   ugugcaaauc caugcaaaac uga 23
<210> 90
   <211> 23
   <212> RNA
   <213> Homo sapiens
<400> 90
   uaaagugcuu auagugcagg uag 23
<210> 91
   <211> 22
   <212> RNA
   <213> Homo sapiens
<400> 91
   uaacacuguc ugguaacgau gu 22
<210> 92
   <211> 23
   <212> RNA
   <213> Homo sapiens
<400> 92
   uaauacugcc ugguaaugau gac 23
<210> 93
   <211> 22
   <212> RNA
   <213> Homo sapiens
<400> 93
   uaauacugcc ggguaaugau gg 22
<210> 94
   <211> 22
   <212> RNA
   <213> Homo sapiens
<400> 94
   gugaaauguu uaggaccacu ag 22
<210> 95
   <211> 22
   <212> RNA
   <213> Homo sapiens
<400> 95
   uucccuuugu cauccuaugc cu 22
<210> 96
   <211> 22
   <212> RNA
   <213> Homo sapiens
<400> 96
   uccuucauuc caccggaguc ug 22
<210> 97
   <211> 22
   <212> RNA
   <213> Homo sapiens
<400> 97
   uggaauguaa ggaagugugu gg 22
<210> 98
   <211> 22
   <212> RNA
   <213> Homo sapiens
<400> 98
   auaagacgag caaaaagcuu gu 22
<210> 99
   <211> 22
   <212> RNA
   <213> Homo sapiens
<400> 99
   uagcuuauca gacugauguu ga 22
<210> 100
   <211> 22
   <212> RNA
   <213> Homo sapiens
<400> 100
   cugugcgugu gacagcggcu ga 22
<210> 101
   <211> 22
   <212> RNA
   <213> Homo sapiens
<400> 101
   uucccuuugu cauccuucgc cu 22
<210> 102
   <211> 21
   <212> RNA
   <213> Homo sapiens
<400> 102
   uaacagucuc cagucacggc c 21
<210> 103
   <211> 22
   <212> RNA
   <213> Homo sapiens
<400> 103
   accaucgacc guugauugua cc 22
<210> 104
   <211> 21
   <212> RNA
   <213> Homo sapiens
<400> 104
   acagcaggca cagacaggca g 21
<210> 105
   <211> 21
   <212> RNA
   <213> Homo sapiens
<400> 105
   augaccuaug aauugacaga c 21
<210> 106
   <211> 21
   <212> RNA
   <213> Homo sapiens
<400> 106
   uaaucucagc uggcaacugu g 21
<210> 107
   <211> 24
   <212> RNA
   <213> Homo sapiens
<400> 107
   uacugcauca ggaacugauu ggau 24
<210> 108
   <211> 21
   <212> RNA
   <213> Homo sapiens
<400> 108
   uugugcuuga ucuaaccaug u 21
<210> 109
   <211> 21
   <212> RNA
   <213> Homo sapiens
<400> 109
   ugauugucca aacgcaauuc u 21
<210> 110
   <211> 22
   <212> RNA
   <213> Homo sapiens
<400> 110
   aagcugccag uugaagaacu gu 22
<210> 111
   <211> 21
   <212> RNA
   <213> Homo sapiens
<400> 111
   ccacaccgua ucugacacuu u 21
<210> 112
   <211> 23
   <212> RNA
   <213> Homo sapiens
<400> 112
   agcuacauug ucugcugggu uuc 23
<210> 113
   <211> 24
   <212> RNA
   <213> Homo sapiens
<400> 113
   agcuacaucu ggcuacuggg ucuc 24
<210> 114
   <211> 21
   <212> RNA
   <213> Homo sapiens
<400> 114
   ugucaguuug ucaaauaccc c 21
<210> 115
   <211> 23
   <212> RNA
   <213> Homo sapiens
<400> 115
   caagucacua gugguuccgu uua 23
<210> 116
   <211> 21
   <212> RNA
   <213> Homo sapiens
<400> 116
   aucacauugc cagggauuuc c 21
<210> 117
   <211> 21
   <212> RNA
   <213> Homo sapiens
<400> 117
   aucacauugc cagggauuac c 21
<210> 118
   <211> 22
   <212> RNA
   <213> Homo sapiens
<400> 118
   uggcucaguu cagcaggaac ag 22
<210> 119
   <211> 22
   <212> RNA
   <213> Homo sapiens
<400> 119
   cauugcacuu gucucggucu ga 22
<210> 120
   <211> 21
   <212> RNA
   <213> Homo sapiens
<400> 120
   uucaaguaau ccaggauagg c 21
<210> 121
   <211> 22
   <212> RNA
   <213> Homo sapiens
<400> 121
   uucaaguaau ucaggauagg uu 22
<210> 122
   <211> 21
   <212> RNA
   <213> Homo sapiens
<400> 122
   uucacagugg cuaaguuccg c 21
<210> 123
   <211> 21
   <212> RNA
   <213> Homo sapiens
<400> 123
   uucacagugg cuaaguucug c 21
<210> 124
   <211> 22
   <212> RNA
   <213> Homo sapiens
<400> 124
   aaggagcuca cagucuauug ag 22
<210> 125
   <211> 21
   <212> RNA
   <213> Homo sapiens
<400> 125
   agggcccccc cucaauccug u 21
<210> 126
   <211> 22
   <212> RNA
   <213> Homo sapiens
<400> 126
   ugguuuaccg ucccacauac au 22
<210> 127
   <211> 21
   <212> RNA
   <213> Homo sapiens
<400> 127
   uagcaccauc ugaaaucggu u 21
<210> 128
   <211> 23
   <212> RNA
   <213> Homo sapiens
<400> 128
   uagcaccauu ugaaaucagu guu 23
<210> 129
   <211> 20
   <212> RNA
   <213> Homo sapiens
<400> 129
   uagcaccauu ugaaaucggu 20
<210> 130
   <211> 23
   <212> RNA
   <213> Homo sapiens
<400> 130
   cagugcaaua guauugucaa agc 23
<210> 131
   <211> 23
   <212> RNA
   <213> Homo sapiens
<400> 131
   uaagugcuuc cauguuuugg uga 23
<210> 132
   <211> 23
   <212> RNA
   <213> Homo sapiens
<400> 132
   uaagugcuuc cauguuuuag uag 23
<210> 133
   <211> 23
   <212> RNA
   <213> Homo sapiens
<400> 133
   acuuuaacau ggaagugcuu ucu 23
<210> 134
   <211> 23
   <212> RNA
   <213> Homo sapiens
<400> 134
   uaagugcuuc cauguuucag ugg 23
<210> 135
   <211> 22
   <212> RNA
   <213> Homo sapiens
<400> 135
   uuuaacaugg ggguaccugc ug 22
<210> 136
   <211> 23
   <212> RNA
   <213> Homo sapiens
<400> 136
   uaagugcuuc cauguuugag ugu 23
<210> 137
   <211> 22
   <212> RNA
   <213> Homo sapiens
<400> 137
   cuuucagucg gauguuugca gc 22
<210> 138
   <211> 22
   <212> RNA
   <213> Homo sapiens
<400> 138
   uguaaacauc cucgacugga ag 22
<210> 139
   <211> 22
   <212> RNA
   <213> Homo sapiens
<400> 139
   uguaaacauc cuacacucag cu 22
<210> 140
   <211> 23
   <212> RNA
   <213> Homo sapiens
<400> 140
   uguaaacauc cuacacucuc agc 23
<210> 141
   <211> 22
   <212> RNA
   <213> Homo sapiens
<400> 141
   uguaaacauc cccgacugga ag 22
<210> 142
   <211> 22
   <212> RNA
   <213> Homo sapiens
<400> 142
   cuuucagucg gauguuuaca gc 22
<210> 143
   <211> 20
   <212> RNA
   <213> Homo sapiens
<400> 143
   uguaaacauc cuugacugga 20
<210> 144
   <211> 21
   <212> RNA
   <213> Homo sapiens
<400> 144
   ggcaagaugc uggcauagcu g 21
<210> 145
   <211> 21
   <212> RNA
   <213> Homo sapiens
<400> 145
   uauugcacau uacuaaguug c 21
<210> 146
   <211> 23
   <212> RNA
   <213> Homo sapiens
<400> 146
   aaaagcuggg uugagagggc gaa 23
<210> 147
   <211> 22
   <212> RNA
   <213> Homo sapiens
<400> 147
   gcacauuaca cggucgaccu cu 22
<210> 148
   <211> 22
   <212> RNA
   <213> Homo sapiens
<400> 148
   ccacugcccc aggugcugcu gg 22
<210> 149
   <211> 23
   <212> RNA
   <213> Homo sapiens
<400> 149
   cgcauccccu agggcauugg ugu 23
<210> 150
   <211> 23
   <212> RNA
   <213> Homo sapiens
<400> 150
   ccuaguaggu guccaguaag ugu 23
<210> 151
   <211> 20
   <212> RNA
   <213> Homo sapiens
<400> 151
   ccucugggcc cuuccuccag 20
<210> 152
   <211> 22
   <212> RNA
   <213> Homo sapiens
<400> 152
   cuggcccucu cugcccuucc gu 22
<210> 153
   <211> 19
   <212> RNA
   <213> Homo sapiens
<400> 153
   gugcauugua guugcauug 19
<210> 154
   <211> 23
   <212> RNA
   <213> Homo sapiens
<400> 154
   gcaaagcaca cggccugcag aga 23
<210> 155
   <211> 21
   <212> RNA
   <213> Homo sapiens
<400> 155
   gccccugggc cuauccuaga a 21
<210> 156
   <211> 23
   <212> RNA
   <213> Homo sapiens
<400> 156
   ucaagagcaa uaacgaaaaa ugu 23
<210> 157
   <211> 23
   <212> RNA
   <213> Homo sapiens
<400> 157
   uccagcuccu auaugaugcc uuu 23
<210> 158
   <211> 23
   <212> RNA
   <213> Homo sapiens
<400> 158
   uccagcauca gugauuuugu uga 23
<210> 159
   <211> 21
   <212> RNA
   <213> Homo sapiens
<400> 159
   ucccuguccu ccaggagcuc a 21
<210> 160
   <211> 23
   <212> RNA
   <213> Homo sapiens
<400> 160
   uccgucucag uuacuuuaua gcc 23
<210> 161
   <211> 24
   <212> RNA
   <213> Homo sapiens
<400> 161
   ucucacacag aaaucgcacc cguc 24
<210> 162
   <211> 21
   <212> RNA
   <213> Homo sapiens
<400> 162
   ugcugacucc uaguccaggg c 21
<210> 163
   <211> 23
   <212> RNA
   <213> Homo sapiens
<400> 163
   ugucugcccg caugccugcc ucu 23
<210> 164
   <211> 23
   <212> RNA
   <213> Homo sapiens
<400> 164
   uggcaguguc uuagcugguu guu 23
<210> 165
   <211> 23
   <212> RNA
   <213> Homo sapiens
<400> 165
   uaggcagugu cauuagcuga uug 23
<210> 166
   <211> 23
   <212> RNA
   <213> Homo sapiens
<400> 166
   aggcagugua guuagcugau ugc 23
<210> 167
   <211> 22
   <212> RNA
   <213> Homo sapiens
<400> 167
   uuaucagaau cuccaggggu ac 22
<210> 168
   <211> 22
   <212> RNA
   <213> Homo sapiens
<400> 168
   uaaugccccu aaaaauccuu au 22
<210> 169
   <211> 22
   <212> RNA
   <213> Homo sapiens
<400> 169
   aauugcacuu uagcaauggu ga 22
<210> 170
   <211> 22
   <212> RNA
   <213> Homo sapiens
<400> 170
   acauagagga aauuccacgu uu 22
<210> 171
   <211> 21
   <212> RNA
   <213> Homo sapiens
<400> 171
   aauaauacau gguugaucuu u 21
<210> 172
   <211> 21
   <212> RNA
   <213> Homo sapiens
<400> 172
   gccugcuggg guggaaccug g 21
<210> 173
   <211> 21
   <212> RNA
   <213> Homo sapiens
<400> 173
   gugccgccau cuuuugagug u 21
<210> 174
   <211> 23
   <212> RNA
   <213> Homo sapiens
<400> 174
   aaagugcugc gacauuugag cgu 23
<210> 175
   <211> 23
   <212> RNA
   <213> Homo sapiens
<400> 175
   gaagugcuuc gauuuugggg ugu 23
<210> 176
   <211> 22
   <212> RNA
   <213> Homo sapiens
<400> 176
   acucaaaaug ggggcgcuuu cc 22
<210> 177
   <211> 22
   <212> RNA
   <213> Homo sapiens
<400> 177
   uuauaauaca accugauaag ug 22
<210> 178
   <211> 22
   <212> RNA
   <213> Homo sapiens
<400> 178
   uuuguucguu cggcucgcgu ga 22
<210> 179
   <211> 21
   <212> RNA
   <213> Homo sapiens
<400> 179
   aucauagagg aaaauccacg u 21
<210> 180
   <211> 22
   <212> RNA
   <213> Homo sapiens
<400> 180
   aucacacaaa ggcaacuuuu gu 22
<210> 181
   <211> 22
   <212> RNA
   <213> Homo sapiens
<400> 181
   cuccugacuc cagguccugu gu 22
<210> 182
   <211> 19
   <212> RNA
   <213> Homo sapiens
<400> 182
   ugguagacua uggaacgua 19
<210> 183
   <211> 22
   <212> RNA
   <213> Homo sapiens
<400> 183
   uauguaauau gguccacauc uu 22
<210> 184
   <211> 22
   <212> RNA
   <213> Homo sapiens
<400> 184
   ugguugacca uagaacaugc gc 22
<210> 185
   <211> 22
   <212> RNA
   <213> Homo sapiens
<400> 185
   uauacaaggg caagcucucu gu 22
<210> 186
   <211> 22
   <212> RNA
   <213> Homo sapiens
<400> 186
   gaaguuguuc gugguggauu cg 22
<210> 187
   <211> 22
   <212> RNA
   <213> Homo sapiens
<400> 187
   agaucagaag gugauugugg cu 22
<210> 188
   <211> 20
   <212> RNA
   <213> Homo sapiens
<400> 188
   auuccuagaa auuguucaua 20
<210> 189
   <211> 22
   <212> RNA
   <213> Homo sapiens
<400> 189
   cuggacuuag ggucagaagg cc 22
<210> 190
   <211> 22
   <212> RNA
   <213> Homo sapiens
<400> 190
   cuggacuugg agucagaagg cc 22
<210> 191
   <211> 22
   <212> RNA
   <213> Homo sapiens
<400> 191
   agcucggucu gaggccccuc ag 22
<210> 192
   <211> 22
   <212> RNA
   <213> Homo sapiens
<400> 192
   cagcagcaau ucauguuuug aa 22
<210> 193
   <211> 21
   <212> RNA
   <213> Homo sapiens
<400> 193
   aucgggaaug ucguguccgc c 21
<210> 194
   <211> 22
   <212> RNA
   <213> Homo sapiens
<400> 194
   uaauacuguc ugguaaaacc gu 22
<210> 195
   <211> 22
   <212> RNA
   <213> Homo sapiens
<400> 195
   uugcauaugu aggauguccc au 22
<210> 196
   <211> 22
   <212> RNA
   <213> Homo sapiens
<400> 196
   uggcagugua uuguuagcug gu 22
<210> 197
   <211> 22
   <212> RNA
   <213> Homo sapiens
<400> 197
   uuuuugcgau guguuccuaa ua 22
<210> 198
   <211> 22
   <212> RNA
   <213> Homo sapiens
<400> 198
   uggaagacua gugauuuugu ug 22
<210> 199
   <211> 23
   <212> RNA
   <213> Homo sapiens
<400> 199
   ucuuugguua ucuagcugua uga 23
<210> 200
   <211> 21
   <212> RNA
   <213> Homo sapiens
<400> 200
   uaaagcuaga uaaccgaaag u 21
<210> 201
   <211> 21
   <212> RNA
   <213> Homo sapiens
<400> 201
   uauugcacuu gucccggccu g 21
<210> 202
   <211> 22
   <212> RNA
   <213> Homo sapiens
<400> 202
   aaagugcugu ucgugcaggu ag 22
<210> 203
   <211> 22
   <212> RNA
   <213> Homo sapiens
<400> 203
   uucaacgggu auuuauugag ca 22
<210> 204
   <211> 22
   <212> RNA
   <213> Homo sapiens
<400> 204
   uuuggcacua gcacauuuuu gc 22
<210> 205
   <211> 22
   <212> RNA
   <213> Homo sapiens
<400> 205
   ugagguagua aguuguauug uu 22
<210> 206
   <211> 22
   <212> RNA
   <213> Homo sapiens
<400> 206
   aacccguaga uccgaucuug ug 22
<210> 207
   <211> 22
   <212> RNA
   <213> Homo sapiens
<400> 207
   cacccguaga accgaccuug cg 22
<210> 208
   <211> 22
   <212> RNA
   <213> Mus musculus
<400> 208
   cuauacgacc ugcugccuuu cu 22
<210> 209
   <211> 22
   <212> RNA
   <213> Mus musculus
<400> 209
   uacaguacug ugauagcuga ag 22
<210> 210
   <211> 22
   <212> RNA
   <213> Mus musculus
<400> 210
   caaagugcua acagugcagg ua 22
<210> 211
   <211> 22
   <212> RNA
   <213> Mus musculus
<400> 211
   aagcccuuac cccaaaaagc au 22
<210> 212
   <211> 22
   <212> RNA
   <213> Mus musculus
<400> 212
   uaccacaggg uagaaccacg ga 22
<210> 213
   <211> 21
   <212> RNA
   <213> Mus musculus
<400> 213
   cuagacugag gcuccuugag g 21
<210> 214
   <211> 22
   <212> RNA
   <213> Mus musculus
<400> 214
   uuaaugcuaa uugugauagg gg 22
<210> 215
   <211> 21
   <212> RNA
   <213> Mus musculus
<400> 215
   acugcaguga gggcacuugu a 21
<210> 216
   <211> 18
   <212> RNA
   <213> Mus musculus
<400> 216
   cugaccuaug aauugaca 18
<210> 217
   <211> 23
   <212> RNA
   <213> Mus musculus
<400> 217
   cccaguguuu agacuaccug uuc 23
<210> 218
   <211> 21
   <212> RNA
   <213> Mus musculus
<400> 218
   uacucaguaa ggcauuguuc u 21
<210> 219
   <211> 22
   <212> RNA
   <213> Mus musculus
<400> 219
   agagguauag cgcaugggaa ga 22
<210> 220
   <211> 23
   <212> RNA
   <213> Mus musculus
<400> 220
   gcuucuccug gcucuccucc cuc 23
<210> 221
   <211> 22
   <212> RNA
   <213> Mus musculus
<400> 221
   uucccuuugu cauccuuugc cu 22
<210> 222
   <211> 21
   <212> RNA
   <213> Mus musculus
<400> 222
   augaccuaug auuugacaga c 21
<210> 223
   <211> 24
   <212> RNA
   <213> Mus musculus
<400> 223
   uacugcauca ggaacugacu ggau 24
<210> 224
   <211> 23
   <212> RNA
   <213> Mus musculus
<400> 224
   cucaaacuau gggggcacuu uuu 23
<210> 225
   <211> 23
   <212> RNA
   <213> Mus musculus
<400> 225
   aaagugcuuc cacuuugugu gcc 23
<210> 226
   <211> 22
   <212> RNA
   <213> Mus musculus
<400> 226
   caucaaagug gaggcccucu cu 22
<210> 227
   <211> 23
   <212> RNA
   <213> Mus musculus
<400> 227
   aagugccgcc agguuuugag ugu 23
<210> 228
   <211> 22
   <212> RNA
   <213> Mus musculus
<400> 228
   acucaaacug ggggcucuuu ug 22
<210> 229
   <211> 22
   <212> RNA
   <213> Mus musculus
<400> 229
   agugccgcag aguuuguagu gu 22
<210> 230
   <211> 22
   <212> RNA
   <213> Mus musculus
<400> 230
   aaagugcuuc ccuuuugugu gu 22
<210> 231
   <211> 23
   <212> RNA
   <213> Mus musculus
<400> 231
   aaagugcuac uacuuuugag ucu 23
<210> 232
   <211> 21
   <212> RNA
   <213> Mus musculus
<400> 232
   auguaugugu gcaugugcau g 21
<210> 233
   <211> 22
   <212> RNA
   <213> Mus musculus
<400> 233
   ggcagaggag ggcuguucuu cc 22
<210> 234
   <211> 22
   <212> RNA
   <213> Mus musculus
<400> 234
   uaugcaaggg caagcucucu uc 22
<210> 235
   <211> 20
   <212> RNA
   <213> Mus musculus
<400> 235
   aaacaugaag cgcugcaaca 20
<210> 236
   <211> 22
   <212> RNA
   <213> Mus musculus
<400> 236
   cagcagcaau ucauguuuug ga 22
<210> 237
   <211> 23
   <212> RNA
   <213> Mus musculus
<400> 237
   ccuaguaggu gcucaguaag ugu 23
<210> 238
   <211> 22
   <212> RNA
   <213> Mus musculus
<400> 238
   aacacaccca gcuaaccuuu uu 22
<210> 239
   <211> 23
   <212> RNA
   <213> Mus musculus
<400> 239
   gcaaagcaca gggccugcag aga 23
<210> 240
   <211> 23
   <212> RNA
   <213> Mus musculus
<400> 240
   uucagcuccu auaugaugcc uuu 23
<210> 241
   <211> 21
   <212> RNA
   <213> Mus musculus
<400> 241
   ucgaucgguc ggucggucag u 21
<210> 242
   <211> 23
   <212> RNA
   <213> Mus musculus
<400> 242
   ugaucuagcc aaagccugac ugu 23
<210> 243
   <211> 21
   <212> RNA
   <213> Mus musculus
<400> 243
   ugcugacccc uaguccagug c 21
<210> 244
   <211> 23
   <212> RNA
   <213> Mus musculus
<400> 244
   ugucugcccg agugccugcc ucu 23
<210> 245
   <211> 23
   <212> RNA
   <213> Mus musculus
<400> 245
   uaggcagugu aauuagcuga uug 23
<210> 246
   <211> 23
   <212> RNA
   <213> Mus musculus
<400> 246
   uucacaaagc ccauacacuu uca 23
<210> 247
   <211> 24
   <212> RNA
   <213> Mus musculus
<400> 247
   ucccugagga gcccuuugag ccug 24
<210> 248
   <211> 21
   <212> RNA
   <213> Mus musculus
<400> 248
   aucguagagg aaaauccacg u 21
<210> 249
   <211> 22
   <212> RNA
   <213> Mus musculus
<400> 249
   aucauagagg aacauccacu uu 22
<210> 250
   <211> 22
   <212> RNA
   <213> Mus musculus
<400> 250
   uauguaguau gguccacauc uu 22
<210> 251
   <211> 22
   <212> RNA
   <213> Mus musculus
<400> 251
   agaucagaag gugacugugg cu 22
<210> 252
   <211> 20
   <212> RNA
   <213> Mus musculus
<400> 252
   auuccuagaa auuguucaca 20
<210> 253
   <211> 23
   <212> RNA
   <213> Mus musculus
<400> 253
   gaauguugcu cggugaaccc cuu
<210> 254
   <211> 21
   <212> RNA
   <213> Homo sapiens
<400> 254
   aauauaacac agauggccug u 21
<210> 255
   <211> 23
   <212> RNA
   <213> Mus musculus
<400> 255
   aacacggucc acuaacccuc agu 23
<210> 256
   <211> 23
   <212> RNA
   <213> Homo sapiens
<400> 256
   acuucaccug guccacuagc cgu 23
<210> 257
   <211> 22
   <212> RNA
   <213> Mus musculus
<400> 257
   uaauacuguc ugguaaugcc gu 22
<210> 258
   <211> 21
   <212> RNA
   <213> Mus musculus
<400> 258
   uggaagacuu gugauuuugu u 21
<210> 259
   <211> 22
   <212> RNA
   <213> Rattus norvegicus
<400> 259
   ucgaggagcu cacagucuag ua 22
<210> 260
   <211> 21
   <212> RNA
   <213> Rattus norvegicus
<400> 260
   acugcauuac gagcacuuac a 21
<210> 261
   <211> 23
   <212> RNA
   <213> Rattus norvegicus
<400> 261
   auguaugugu gcauguaugc aug 23
<210> 262
   <211> 19
   <212> RNA
   <213> Rattus norvegicus
<400> 262
   ccuugagggg caugagggu 19
<210> 263
   <211> 20
   <212> RNA
   <213> Rattus norvegicus
<400> 263
   guggugugcu aguuacuuuu 20
<210> 264
   <211> 21
   <212> RNA
   <213> Rattus norvegicus
<400> 264
   ucacccuucc auaucuaguc u 21
<210> 265
   <211> 20
   <212> RNA
   <213> Rattus norvegicus
<400> 265
   ucucccuccg ugugcccaga 20
<210> 266
   <211> 23
   <212> RNA
   <213> Rattus norvegicus
<400> 266
   ugaucuagcc aaagccugac cgu 23
<210> 267
   <211> 23
   <212> RNA
   <213> Rattus norvegicus
<400> 267
   ugucugccug agugccugcc ucu 23
<210> 268
   <211> 19
   <212> RNA
   <213> Rattus norvegicus
<400> 268
   ugucccucug ggucgccca 19
<210> 269
   <211> 22
   <212> RNA
   <213> Rattus norvegicus
<400> 269
   cagcccugcu gucuuaaccu cu 22
<210> 270
   <211> 21
   <212> RNA
   <213> Rattus norvegicus
<400> 270
   agaguaguag guugcauagu a 21
<210> 271
   <211> 21
   <212> RNA
   <213> Rattus norvegicus
<400> 271
   ggccucauua aauguuuguu g 21
<210> 272
   <211> 22
   <212> RNA
   <213> Rattus norvegicus
<400> 272
   caacaaauca cagucugcca ua 22
<210> 273
   <211> 23
   <212> RNA
   <213> Homo sapiens
<400> 273
   aaaaugguuc ccuuuagagu guu 23
<210> 274
   <211> 23
   <212> RNA
   <213> Homo sapiens
<400> 274
   aaagugcauc cuuuuagagg uuu 23
<210> 275
   <211> 23
   <212> RNA
   <213> Homo sapiens
<400> 275
   aaagugcuuc cuuuuagagg guu 23
<210> 276
   <211> 22
   <212> RNA
   <213> Homo sapiens
<400> 276
   aaagugccuc cuuuuagagu gu 22
<210> 277
   <211> 23
   <212> RNA
   <213> Homo sapiens
<400> 277
   caaagugccu cccuuuagag ugu 23
<210> 278
   <211> 21
   <212> RNA
   <213> Homo sapiens
<400> 278
   aaagugcuuc cuuuuagagg g 21
<210> 279
   <211> 22
   <212> RNA
   <213> Homo sapiens
<400> 279
   aaagugcauc uuuuuagagg au 22
<210> 280
   <211> 21
   <212> RNA
   <213> Homo sapiens
<400> 280
   aaagugcuuc cuuuuagagg c 21
<210> 281
   <211> 21
   <212> RNA
   <213> Homo sapiens
<400> 281
   aaagugcuuc cuuuuugagg g 21
<210> 282
   <211> 22
   <212> RNA
   <213> Homo sapiens
<400> 282
   aaagugcuuc ccuuuggacu gu 22
<210> 283
   <211> 23
   <212> RNA
   <213> Homo sapiens
<400> 283
   aaagugcuuc ucuuuggugg guu 23
<210> 284
   <211> 22
   <212> RNA
   <213> Homo sapiens
<400> 284
   acaaagugcu ucccuuuaga gu 22
<210> 285
   <211> 23
   <212> RNA
   <213> Homo sapiens
<400> 285
   aucgugcauc ccuuuagagu guu 23
<210> 286
   <211> 22
   <212> RNA
   <213> Homo sapiens
<400> 286
   aaagcgcuuc ccuucagagu gu 22
<210> 287
   <211> 22
   <212> RNA
   <213> Homo sapiens
<400> 287
   aacgcacuuc ccuuuagagu gu 22
<210> 288
   <211> 21
   <212> RNA
   <213> Homo sapiens
<400> 288
   aacgcgcuuc ccuauagagg g 21
<210> 289
   <211> 21
   <212> RNA
   <213> Homo sapiens
<400> 289
   aaagcgcuuc ucuuuagagg a 21
<210> 290
   <211> 22
   <212> RNA
   <213> Homo sapiens
<400> 290
   caaagcgcuu cucuuuagag ug 22
<210> 291
   <211> 22
   <212> RNA
   <213> Homo sapiens
<400> 291
   caaagcgcuc cccuuuagag gu 22
<210> 292
   <211> 21
   <212> RNA
   <213> Homo sapiens
<400> 292
   caaagcgcuu cccuuuggag c 21
<210> 293
   <211> 21
   <212> RNA
   <213> Homo sapiens
<400> 293
   gaaggcgcuu cccuuuagag c 21
<210> 294
   <211> 21
   <212> RNA
   <213> Homo sapiens
<400> 294
   gaaggcgcuu cccuuuggag u 21
<210> 295
   <211> 21
   <212> RNA
   <213> Homo sapiens
<400> 295
   aaagcgcuuc ccuuugcugg a 21
<210> 296
   <211> 21
   <212> RNA
   <213> Homo sapiens
<400> 296
   gagugccuuc uuuuggagcg u 21
<210> 297
   <211> 18
   <212> RNA
   <213> Homo sapiens
<400> 297
   ugcuuccuuu cagagggu 18
<210> 298
   <211> 22
   <212> RNA
   <213> Homo sapiens
<400> 298
   aagugcuguc auagcugagg uc 22
<210> 299
   <211> 23
   <212> RNA
   <213> Homo sapiens
<400> 299
   ggaaaccguu accauuacug agu 23
<210> 300
   <211> 23
   <212> RNA
   <213> Homo sapiens
<400> 300
   aguggggaac ccuuccauga gga 23
<210> 301
   <211> 21
   <212> RNA
   <213> Homo sapiens
<400> 301
   uaaggcaccc uucugaguag a 21
<210> 302
   <211> 20
   <212> RNA
   <213> Homo sapiens
<400> 302
   auugacacuu cugugaguag 20
<210> 303
   <211> 23
   <212> RNA
   <213> Homo sapiens
<400> 303
   ugauugguac gucugugggu aga 23
<210> 304
   <211> 23
   <212> RNA
   <213> Homo sapiens
<400> 304
   ugauuguagc cuuuuggagu aga 23
<210> 305
   <211> 21
   <212> RNA
   <213> Homo sapiens
<400> 305
   uuuugcaccu uuuggaguga a 21
<210> 306
   <211> 24
   <212> RNA
   <213> Homo sapiens
<400> 306
   aacuggcccu caaagucccg cuuu 24
<210> 307
   <211> 23
   <212> RNA
   <213> Homo sapiens
<400> 307
   gccgagacua gagucacauc cug 23
<210> 308
   <211> 21
   <212> RNA
   <213> Homo sapiens
<400> 308
   cccagauaau ggcacucuca a 21
<210> 309
   <211> 23
   <212> RNA
   <213> Homo sapiens
<400> 309
   uacucaggag aguggcaauc aca 23
<210> 310
   <211> 22
   <212> RNA
   <213> Homo sapiens
<400> 310
   ccucuagaug gaagcacugu cu 22
<210> 311
   <211> 23
   <212> RNA
   <213> Homo sapiens
<400> 311
   cucuagaggg aagcacuuuc ucu 23
<210> 312
   <211> 23
   <212> RNA
   <213> Homo sapiens
<400> 312
   ucucuggagg gaagcacuuu cug 23
<210> 313
   <211> 24
   <212> RNA
   <213> Homo sapiens
<400> 313
   cucuagaggg aagcgcuuuc uguu 24
<210> 314
   <211> 24
   <212> RNA
   <213> Homo sapiens
<400> 314
   cucuugaggg aagcacuuuc uguu 24
<210> 315
   <211> 21
   <212> RNA
   <213> Homo sapiens
<400> 315
   cuccagaggg aaguacuuuc u 21
<210> 316
   <211> 21
   <212> RNA
   <213> Homo sapiens
<400> 316
   cuccagaggg augcacuuuc u 21
<210> 317
   <211> 22
   <212> RNA
   <213> Homo sapiens
<400> 317
   cuacaaaggg aagcacuuuc uc 22
<210> 318
   <211> 23
   <212> RNA
   <213> Homo sapiens
<400> 318
   ucuacaaagg gaagcccuuu cug 23
<210> 319
   <211> 21
   <212> RNA
   <213> Homo sapiens
<400> 319
   cugcaaaggg aagcccuuuc u 21
<210> 320
   <211> 24
   <212> RNA
   <213> Homo sapiens
<400> 320
   uucuccaaaa gaaagcacuu ucug 24
<210> 321
   <211> 22
   <212> RNA
   <213> Homo sapiens
<400> 321
   uucuccaaaa gggagcacuu uc 22
<210> 322
   <211> 23
   <212> RNA
   <213> Homo sapiens
<400> 322
   uuucaagcca gggggcguuu uuc 23
<210> 323
   <211> 22
   <212> RNA
   <213> Homo sapiens
<400> 323
   uucacaggga ggugucauuu au 22
<210> 324
   <211> 23
   <212> RNA
   <213> Homo sapiens
<400> 324
   ugaggggcag agagcgagac uuu 23
<210> 325
   <211> 22
   <212> RNA
   <213> Homo sapiens
<400> 325
   uguuugcaga ggaaacugag ac 22
<210> 326
   <211> 22
   <212> RNA
   <213> Homo sapiens
<400> 326
   uuguacaugg uaggcuuuca uu 22
<210> 327
   <211> 23
   <212> RNA
   <213> Homo sapiens
<400> 327
   ucuuggagua ggucauuggg ugg 23
<210> 328
   <211> 23
   <212> RNA
   <213> Homo sapiens
<400> 328
   aaacaaacau ggugcacuuc uuu 23
<210> 329
   <211> 24
   <212> RNA
   <213> Homo sapiens
<400> 329
   ugaaacauac acgggaaacc ucuu 24
<210> 330
   <211> 17
   <212> RNA
   <213> Homo sapiens
<400> 330
   auuacauggc caaucuc 17
<210> 331
   <211> 23
   <212> RNA
   <213> Homo sapiens
<400> 331
   aggaccugcg ggacaagauu cuu 23
<210> 332
   <211> 22
   <212> RNA
   <213> Homo sapiens
<400> 332
   caaccuggag gacuccaugc ug 22
<210> 333
   <211> 21
   <212> RNA
   <213> Homo sapiens
<400> 333
   cagcagcaca cugugguuug u 21
<210> 334
   <211> 24
   <212> RNA
   <213> Homo sapiens
<400> 334
   aauccuugga accuaggugu gagu 24
<210> 335
   <211> 22
   <212> RNA
   <213> Homo sapiens
<400> 335
   aauccuuugu cccuggguga ga 22
<210> 336
   <211> 21
   <212> RNA
   <213> Homo sapiens
<400> 336
   auccuugcua ucugggugcu 21
<210> 337
   <211> 22
   <212> RNA
   <213> Homo sapiens
<400> 337
   uuuccuaugc auauacuucu uu 22
<210> 338
   <211> 21
   <212> RNA
   <213> Homo sapiens
<400> 338
   gcaguccaug ggcauauaca c 21
<210> 339
   <211> 23
   <212> RNA
   <213> Homo sapiens
<400> 339
   cacucagccu ugagggcacu uuc 23
<210> 340
   <211> 21
   <212> RNA
   <213> Homo sapiens
<400> 340
   agacccuggu cugcacucua u 21
<210> 341
   <211> 21
   <212> RNA
   <213> Homo sapiens
<400> 341
   gugucuuuug cucugcaguc a 21
<210> 342
   <211> 21
   <212> RNA
   <213> Homo sapiens
<400> 342
   ucagucucau cugcaaagaa g 21
<210> 343
   <211> 23
   <212> RNA
   <213> Homo sapiens
<400> 343
   uagcagcggg aacaguucug cag 23
<210> 344
   <211> 22
   <212> RNA
   <213> Homo sapiens
<400> 344
   agaggcuggc cgugaugaau uc 22
<210> 345
   <211> 23
   <212> RNA
   <213> Homo sapiens
<400> 345
   uuaagacuug cagugauguu uaa 23
<210> 346
   <211> 22
   <212> RNA
   <213> Homo sapiens
<400> 346
   gucaacacuu gcugguuucc uc 22
<210> 347
   <211> 23
   <212> RNA
   <213> Homo sapiens
<400> 347
   agugacauca cauauacggc age 23
<210> 348
   <211> 21
   <212> RNA
   <213> Homo sapiens
<400> 348
   cuggauggcu ccuccauguc u 21
<210> 349
   <211> 22
   <212> RNA
   <213> Homo sapiens
<400> 349
   augcaccugg gcaaggauuc ug 22
<210> 350
   <211> 23
   <212> RNA
   <213> Homo sapiens
<400> 350
   ucccccaggu gugauucuga uuu 23